# FASCICULE DE BREVET EUROPEEN

(11) **EP 1 112 251 B1**
(45) Date de publication et mention de la délivrance du brevet: **07.05.2003**
(21) Numéro de dépôt: 99941728.0
(22) Date de dépôt: 09.09.1999
(51) Int. Cl.: C07D 205/06, C07D 401/06, C07D 409/06, C07D 409/14, C07D 417/06, A61K 31/397

(54) **DERIVES D'AZETIDINE, LEUR PREPARATION ET LES MEDICAMENTS LES CONTENANT**
AZETIDINDERIVATE, DEREN HERSTELLUNG UND SIE ENTHALTENDE ARZNEIMITTEL
AZETIDINE DERIVATIVES, PREPARATION AND MEDICINES CONTAINING THEM

(30) Priorité: 11.09.1998 FR 9811342; 12.02.1999 US 119929 P
(43) Date de publication de la demande: 04.07.2001
(73) Titulaire: Aventis Pharma S.A., 92160 Antony (FR)
(72) Inventeur: MIGNANI, Serge, F-92290 Chatenay-Malabry (FR); HITTINGER, Augustin, F-91430 Igny (FR); ACHARD, Daniel, F-94320 Thiais (FR); BOUCHARD, Hervé, F-94320 Thiais (FR); BOUQUEREL, Jean, F-93700 Drancy (FR); CAPET, Marc, F-91170 Viry-Chatillon (FR); GRISONI, Serge, F-94600 Choisy le Roi (FR); MALLERON, Jean-Luc, F-91460 Marcoussis (FR)
(86) Numéro de dépôt international: FR9902147
(87) Numéro de publication internationale: WO00015609

(56) Documents cités:
- FR-A- 2 388 793
- GB-A- 2 055 818
- US-A- 4 242 261

## Description

La présente invention concerne des dérivés d'azétidine de formule : 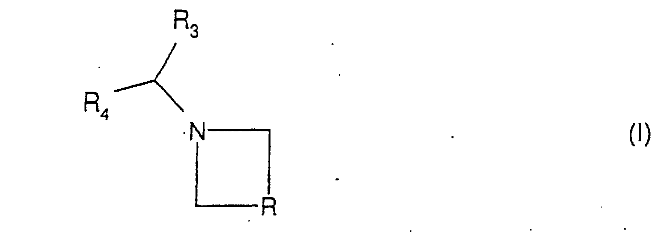 leurs isomères optiques, leurs sels, leur préparation et les médicaments les contenant.

Des dérivés d'azétidine sont decrits dans les documents suivants: US 4 242 261, GB 2 055 818 et FR 2 388 793

Dans la formule (I),
R représente une chaîne 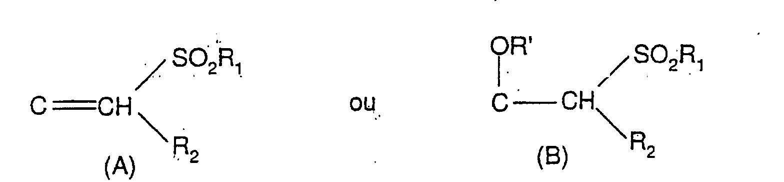 R₁ représente un radical méthyle ou éthyle,
R₂ représente soit un aromatique choisi parmi phényle, naphtyle ou indényle, ces aromatiques étant non substitués ou substitués par un ou plusieurs halogène, alkyle, alcoxy, -CO-alk, hydroxy, -COOR₅, formyle, trifluorométhyle, trifluorométhylsulfanyle, trifluorométhoxy, nitro, -NR₆R₇, -CO-NH-NR₆R₇, -N(alk)COOR₈, cyano, -CONHR₉, -CO-NR₁₆R₁₇, alkylsulfanyle, hydroxyalkyle, -O-alk-NR₁₂R₁₃ ou alkylthioalkyle soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, indolinyle, indolyle, isochromannyle, isoquinolyle, pyridyle, quinolyle, 1,2,3,4-tétrahydroisoquinolyle, 1,2,3,4-tétrahydroquinolyle, thiazolyle, thiényle, ces hétéroaromatiques pouvant être non substitués ou substitués par un halogène, alkyle, alcoxy, -COOR₅, trifluorométhyle, trifluorométhylsulfanyle, trifluorométhoxy, nitro, -NR₆R₇, -CO-NH-NR₆R₇, cyano, -CONHR₉, alkylsulfanyle, hydroxyalkyle ou alkylthioalkyle,
R₃ et R₄, identiques ou différents, représentent soit un aromatique choisi parmi phényle, naphtyle ou indényle, ces aromatiques étant non substitués ou substitués par un ou plusieurs halogène, alkyle, alcoxy, formyle, hydroxy, trifluorométhyle, trifluorométhoxy, -CO-alk, cyano, -COOR₅, -CONR₁₀R₁₁, -CO-NH-NR₆R₇, alkylsulfanyle, hydroxyalkyle, -alk-NR₆R₇ ou alkylthioalkyle; soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, furyle, isochromannyle, isoquinolyle, pyrrolyle, quinolyle, 1,2,3,4-tétrahydroisoquinolyle, thiazolyle, thiényle, ces hétéroaromatiques pouvant être non substitués ou substitués par un halogène, alkyle, alcoxy, hydroxy, trifluorométhyle, trifluorométhoxy, cyano, -COOR₅, -CO-NH-NR₆R₇, -CONR₁₀R₁₁, -alk-NR₆R₇, alkylsulfanyle, hydroxyalkyle ou alkylthioalkyle,
R₅ est un radical alkyle ou phényle éventuellement substitué par un ou plusieurs atomes d'halogène,
R₆ et R₇, identiques ou différents représentent un atome d'hydrogène ou un radical alkyle, -COOalk, cycloalkyle, alkylcycloalkyle, -alk-O-alk, hydroxyalkyle ou bien R₆ et R₇ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, -COalk, -COOalk, -CO-NHalk, -CS-NHalk, -CO-alk-NR₁₄R₁₅, oxo, hydroxyalkyle, -alk-O-alk, -CO-NH₂,
R₈ représente un radical alkyle,
R₉ représente un atome d'hydrogène ou un radical alkyle ou alkyle substitué par dialkylamino, phényle, cycloalkyle (éventuellement substitué par -COOalk) ou un hétérocycle mono ou bicyclique saturé ou insaturé ayant 3 à 10 chaînons, contenant éventuellement un ou plusieurs hétéroatomes choisis parmi oxygène, soufre et azote et étant éventuellement substitué par un ou plusieurs radicaux alkyle,
R₁₀ et R₁₁, identiques ou différents, représentent un atome d'hydrogène ou un radical alkyle ou bien R₁₀ et R₁₁ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un radical alkyle,
R₁₂ et R₁₃, identiques ou différents, représentent un atome d'hydrogène ou un radical alkyle, cycloalkyle ou bien R₁₂ et R₁₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un radical alkyle, -COalk, -COOalk, -CO-NHalk, -CS-NHalk, -CO-alk-NR₁₄R₁₅ ou un hétérocycle mono ou bicyclique saturé ayant 3 à 10 chaînons et contenant un hétéroatome choisi parmi oxygène, soufre et azote,
R₁₄ et R₁₅, identiques ou différents, représentent un atome d'hydrogène ou un radical alkyle ou -COOalk,
R₁₆ et R₁₇ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote,
R' représente un atome d'hydrogène ou un radical -CO-alk,
alk représente un radical alkyle ou alkylène.

Dans les définitions précédentes et celles qui suivent, sauf mention contraire, les radicaux et portions alkyle et alkylène et les radicaux et portions alcoxy sont en chaîne droite ou ramifiée et contiennent 1 à 6 atomes de carbone.

Parmi les radicaux alkyle on peut citer les radicaux méthyle, éthyle, n-propyle, isopropyle, n-butyle, sec-butyle, iso-butyle, tert-butyle, pentyle, hexyle. Parmi les radicaux alcoxy on peut citer les radicaux méthoxy, éthoxy, n-propoxy, iso-propoxy, n-butoxy, iso-butoxy, sec-butoxy, tert-butoxy, pentyloxy.

Le terme halogène comprend chlore, fluor, brome et iode.

Lorsque R₂ et/ou R₃ et/ou R₄ représentent indépendamment un phényle substitué celui-ci est de préférence mono, di ou trisubstitué.

Lorsque R₆ et R₇ forment ensemble avec l'atome d'azote auquel ils sont rattachés un hétérocycle mono ou bicyclique, saturé ou insaturé ayant 3 à 10 chaînons celui-ci est de préférence un cycle azétidinyle, pyrrolidinyle, pipérazinyle, pipéridyle, morpholinyle, imidazolyle, thiomorpholinyle ou furyle, ces cycles étant éventuellement substitués par un radical alkyle, hydroxyalkyle, -alk-O-alk, -CONH₂, -COalk, -COOalk, oxo, -CSNHalk, -CONHalk ou -CO-alk-NR₁₄R₁₅ et, en particulier, par un radical méthyle, éthyle, propyle, isobutyl, acétyle, N,N-diméthylamino-méthylcarbonyle, méthyloxycarbonyle, méthylcarbamoyle, méthylthiocarbamoyle, N-méthylaminométhylcarbonyle, N-méthyl N-tertbutoxycarbonylaminométhylcarbonyle, oxo, -CSNHCH₃, -CONHCH₃.

Lorsque R₁₀ et R₁₁ forment ensemble avec l'atome d'azote auquel ils sont rattachés un hétérocycle mono ou bicyclique saturé et ayant 3 à 10 chaînons, celui-ci est de préférence un cycle azétidinyle, pyrrolidinyle, pipérazinyle, pipéridyle, morpholinyle ou thiomorpholinyle, ces cycles étant éventuellement substitués par un alkyle.

Lorsque R₁₂ et R₁₃ forment ensemble avec l'atome d'azote auquel ils sont rattachés un hétérocycle mono ou bicyclique saturé et ayant 3 à 10 chaînons, celui-ci est de préférence un cycle azétidinyle, pyrrolidinyle, pipérazinyle, pipéridyle, morpholinyle ou thiomorpholinyle, ces cycles étant éventuellement substitués par un radical alkyle, -COalk, -COOalk, -CO-NHalk, -CS-NHalk, -CO-alk-NR₁₄R₁₅ ou un hétérocycle mono ou bicyclique saturé ayant 3 à 10 chaînons et contenant un hétéroatome choisi parmi oxygène, soufre et azote, et, en particulier, par un radical thiomorpholinyle.

Lorsque R₁₆ et R₁₇ forment ensemble avec l'atome d'azote auquel ils sont rattachés un hétérocycle mono ou bicyclique saturé et ayant 3 à 10 chaînons, celui-ci est de préférence un cycle pipéridyle.

Lorsque R₉ représente un radical alkyle substitué par un hétérocycle mono ou bicyclique saturé ou insaturé ayant 3 à 10 chaînons, contenant éventuellement un ou plusieurs hétéroatomes choisis parmi oxygène, soufre et azote, ce dernier est, de préférence, un cycle pyrrolidinyle, tétrahydrofuryle, morpholinyle, pyrrolyle, ces cycles étant éventuellement substitués par un ou plusieurs radicaux alkyle.

Les composés de formule (I) peuvent se présenter sous forme d'énantiomères et de diastéréoisomères. Ces isomères optiques et leurs mélanges font partie de l'invention.

Les composés de formule (I) pour lesquels R représente une chaîne de formule (A) peuvent être préparés par déshydratation d'un composé de formule (la) correspondant : 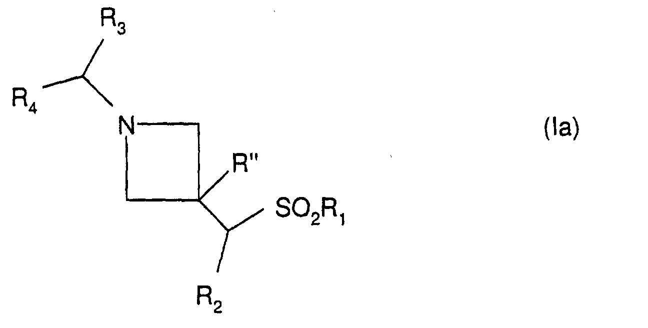 dans laquelle R₁, R₂, R₃ et R₄ ont les mêmes significations que dans la formule (I) et R" représente un radical hydroxy, méthanesulfonyloxy ou acétyloxy.

Cette déshydratation s'effectue par toute méthode connue de l'homme de l'art permettant de déshydrater un alcool ou un de ses dérivés pour obtenir l'alcène correspondant. De préférence, on utilise les dérivés pour lesquels R" est un radical méthanesulfonyloxy ou acétyloxy obtenus à partir du dérivé correspondant pour lequel R" est un radical hydroxy par action de chlorure de méthane sulfonyle ou de chlorure d'acétyle, au sein d'un solvant inerte tel que la pyridine, le tétrahydrofuranne, le dioxanne, un solvant chloré (dichlorométhane, chloroforme par exemple), à une température comprise entre 5°C et 20°C puis on traite avec une base telle qu'un hydroxyde de métal alcalin (soude par exemple), un carbonate de métal alcalin (carbonate de sodium ou de potassium par exemple), une amine telle qu'une trialkylamine (triéthylamine par exemple), la 4-diméthylaminopyridine, le diaza-1,8-bicyclo[5.4.0]undécène-7, à une température comprise entre 0°C et la température d'ébullition du milieu réactionnel. Le méthanesulfonyloxy et l'acétyloxy peuvent être isolés ou non.

Les composés de formule (I) pour lesquels R représente une chaîne (B) dans laquelle R' est un atome d'hydrogène peuvent être préparés par action du dérivé R₁SO₂CH₂R₂ (II) pour lequel R₁ et R₂ ont les mêmes significations que dans la formule (I) sur une azétidinone de formule : 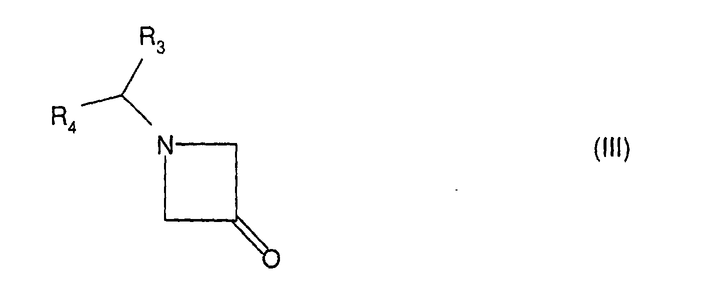 dans laquelle R₃ et R₄ ont les mêmes significations que dans la formule (I).

On opère généralement au sein d'un solvant inerte tel qu'un éther (tétrahydrofuranne par exemple), en présence d'une base forte telle que le diisopropylamidure de lithium, le tert-butylate de potassium ou le n-butyllithium, à une température comprise entre -70°C et -15°C.

Les dérivés de formule (II) peuvent être obtenus par application ou adaptation des méthodes décrites dans les exemples. Notamment on opère selon les schémas réactionnels suivants : 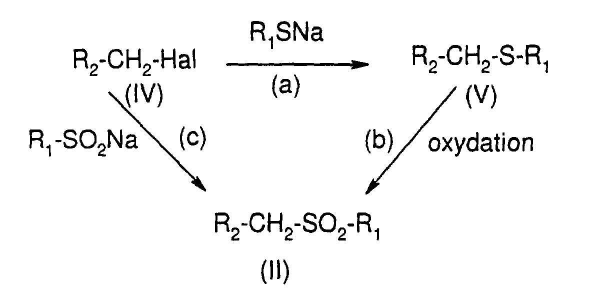 dans ces formules Hal représente un atome d'halogène et, de préférence, chlore, brome ou iode, R₁ et R₂ ont les mêmes significations que dans la formule (I).

La réaction (a) s'effectue généralement au sein d'un solvant inerte tel que le diméthylformamide ou un alcool aliphatique 1-4C, à une température comprise entre 20 et 30°C.

La réaction (b) s'effectue par toutes méthodes connues permettant d'oxyder un dérivé soufré sans toucher au reste de la molécule comme celles décrites par M. HUDLICKY, Oxidations in Organic Chemistry, ACS Monograph, 186, 252-263 (1990). Par exemple, on opère par action d'un peroxyacide organique ou un sel d'un tel peroxyacide (acides peroxycarboxyliques ou peroxysulfoniques, notamment l'acide peroxybenzoïque, l'acide 3-chloroperoxybenzoïque, l'acide 4-nitroperoxybenzoïque, l'acide peroxyacétique, l'acide trifluoroperoxyacétique, l'acide peroxyformique, l'acide monoperoxyphtalique) ou les peracides minéraux ou un sel d'un tel acide (par exemple l'acide periodique ou persulfurique), au sein d'un solvant inerte tel qu'un solvant chloré (chloroforme, dichlorométhane par exemple), à une température comprise entre 0 et 25°C. On peut utiliser également le peroxyde d'hydrogène ou un periodate (periodate de sodium par exemple), au sein d'un solvant inerte tel qu'un alcool aliphatique 1-4C (méthanol, éthanol par exemple), l'eau ou un mélange de ces solvants, à une température comprise entre 0 et 20°C. Il est également possible d'opérer au moyen de tertiobutylhydroperoxyde en présence de tétraisopropylate de titane au sein d'un alcool aliphatique 1-4C (méthanol, éthanol par exemple) ou un mélange eau-alcool, à une température voisine de 25°C ou au moyen d'oxone^{R} (peroxymonosulfate de potassium), au sein d'un alcool aliphatique 1-4C (méthanol, éthanol par exemple), en présence d'eau, d'acide acétique ou d'acide sulfurique, à une température voisine de 20°C.

La réaction (c) s'effectue de préférence au sein d'un solvant inerte tel qu'un alcool aliphatique 1-4C (méthanol, éthanol par exemple), à une température comprise entre 20°C et la température d'ébullition du milieu réactionnel.

Les dérivés de formule (IV) sont commercialisés ou peuvent être obtenus par application ou adaptation des méthodes décrites dans les exemples. En particulier, on halogène le dérivé méthylé ou l'alcool correspondant, au moyen d'un agent d'halogénation tel que l'acide bromhydrique, au sein de l'acide acétique, à une température voisine de 20°C ou le N-bromo ou N-chlorosuccinimide en présence de peroxyde de benzoyle, au sein d'un solvant inerte tel que le tétrachlorométhane, à la température d'ébullition du milieu réactionnel. Les dérivés méthylés ou les alcools correspondants sont commercialisés ou peuvent être obtenus selon les méthodes décrites par BRINE G. A. et coll., J. Heterocyl. Chem, 26, 677 (1989) et NAGARATHNAM D., Synthesis, 8, 743 (1992) et dans les exemples.

Les azétidinones de formule (III) peuvent être obtenues par application ou adaptation des méthodes décrites par KATRITZKY A.R et coll., J. Heterocycl. Chem., 271 (1994), ou DAVE P.R., J. Org. Chem., 61, 5453 (1996) et dans les exemples. On opère généralement selon le schéma réactionnel suivant : 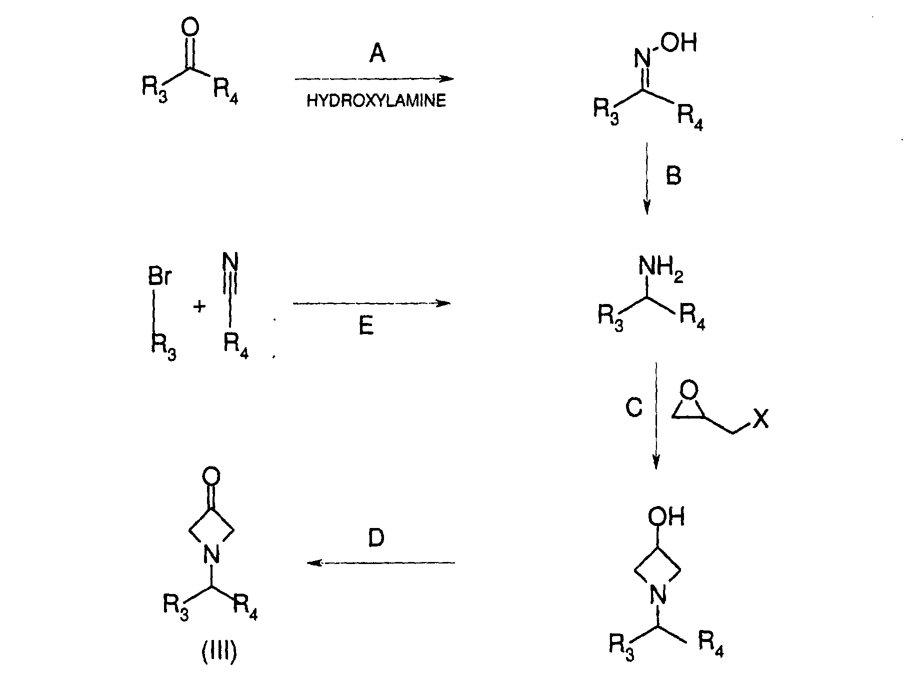 dans ces formules R₃ et R₄ ont les mêmes significations que dans la formule (I) et X représente un atome de chlore ou de brome.

Dans l'étape A, on opère de préférence au sein d'un solvant inerte tel qu'un alcool aliphatique 1-4C (éthanol, méthanol par exemple), éventuellement en présence d'un hydroxyde de métal alcalin, à la température d'ébullition du milieu réactionnel.

Dans l'étape B, la réduction s'effectue généralement, au moyen d'hydrure de lithium et d'aluminium, au sein du tétrahydrofuranne à la température d'ébullition du milieu réactionnel.

Dans l'étape C, on opère de préférence au sein d'un solvant inerte tel qu'un alcool aliphatique 1-4C (éthanol, méthanol par exemple), en présence d'hydrogénocarbonate de sodium, à une température comprise entre 20°C et la température d'ébullition du milieu réactionnel.

Dans l'étape D on oxyde de préférence au sein de DMSO, au moyen du complexe trioxyde de soufre-pyridine, à une température voisine de 20°C ou au moyen de diméthylsulfoxyde, en présence de chlorure d'oxalyle et de triéthylamine, à une température comprise entre -70 et -50°C.

Dans l'étape E, on opère selon la méthode décrite par GRISAR M. et coll. dans J. Med. Chem., 885 (1973). On forme le magnésien du dérivé bromé puis on fait réagir le nitrile, au sein d'un éther tel que l'éther éthylique, à une température comprise entre 0°C et la température d'ébullition du milieu réactionnel. Après hydrolyse avec un alcool, l'imine intermédiaire est réduite *in situ* par du borohydrure de sodium à une température comprise entre 0°C et la température d'ébullition du milieu réactionnel.

Les dérivés R₃-CO-R₄ sont commercialisés ou peuvent être obtenus par application ou adaptation des méthodes décrites par KUNDER N.G. et coll. J. Chem. Soc. Perkin Trans 1, 2815 (1997); MORENO-MARRAS M., Eur. J. Med. Chem., 23 (5) 477 (1988); SKINNER et coll., J. Med. Chem., 14 (6) 546 (1971); HURN N.K., Tet. Lett., 36 (52) 9453 (1995); MEDICI A. et coll., Tet. Lett., 24 (28) 2901 (1983); RIECKE R.D. et coll., J. Org. Chem., 62 (20) 6921 (1997); KNABE J. et coll., Arch. Pharm., 306 (9) 648 (1973); CONSONNI R. et coll., J. Chem. Soc. Perkin Trans 1, 1809 (1996); FR-96-2481 et JP-94-261393.

Les dérivés R₃Br sont commercialisés ou peuvent être obtenus par application ou adaptation des méthodes décrites par BRANDSMA L. et coll., Synth. Comm., 20 (11) 1697 et 3153 (1990); LEMAIRE M. et coll., Synth. Comm., 24 (1) 95 (1994); GODA H. et coll., Synthesis, 9 849 (1992); BAEUERLE P. et coll., J. Chem. Soc. Perkin Trans 2, 489 (1993).

Les dérivés R₄CN sont commercialisés ou peuvent être obtenus par application ou adaptation des méthodes décrites par BOUYSSOU P. et coll., J. Het. Chem., 29 (4) 895 (1992); SUZUKI N. et coll., J. Chem. Soc. Chem. Comm., 1523 (1984); MARBURG S. et coll., J. Het. Chem., 17 1333 (1980); PERCEC V. et coll., J. Org. Chem., 60 (21) 6895 (1995).

Les composés de formule (I) pour lesquels R représente une chaîne (B) dans laquelle R' est un atome d'hydrogène peuvent également être préparés par action d'un dérivé R₃CH(Br)R₄ (VI) pour lequel R₃ et R₄ ont les mêmes significations que dans la formule (I) sur un dérivé de formule : 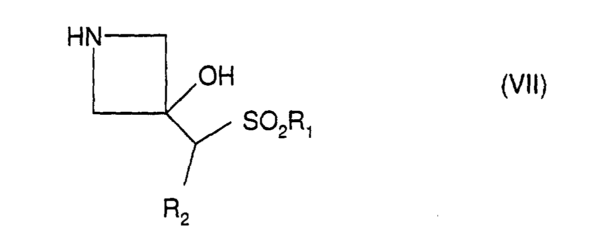 dans laquelle R₁ et R₂ ont les mêmes significations que dans la formule (I).

Cette réaction s'effectue généralement en présence d'une base telle qu'un carbonate de métal alcalin (carbonate de potassium par exemple), au sein d'un solvant inerte tel que l'acétonitrile, à la température d'ébullition du milieu réactionnel.

Les dérivés de formule (VI) sont commercialisés ou peuvent être obtenus par application ou adaptation de la méthode décrite par BACHMANN W.E., J. Am. Chem. Soc., 2135 (1933). Généralement, on brome l'alcool correspondant R₃CHOHR₄ au moyen d'acide bromhydrique, au sein de l'acide acétique, à une température comprise entre 0°C et la température d'ébullition du milieu réactionnel.

Les alcools correspondants R₃CHOHR₄ sont commercialisés ou peuvent être obtenus par application ou adaptation des méthodes décrites par PLASZ A.C. et coll., J. Chem. Soc. Chem. Comm., 527 (1972).

Les dérivés de formule (VII) peuvent être obtenus par hydrolyse d'un dérivé de formule : 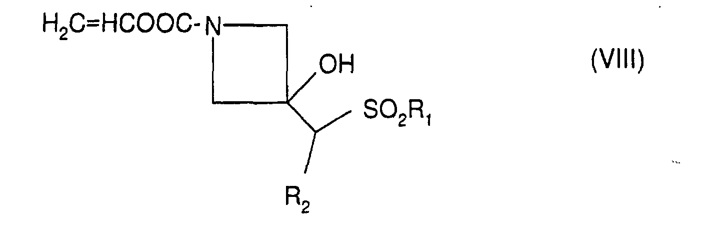 dans laquelle R₁ et R₂ ont les mêmes significations que dans la formule (I).

Cette réaction s'effectue généralement au moyen d'acide chlorhydrique, au sein d'un solvant inerte tel qu'un éther (dioxanne par exemple), à une température voisine de 20°C.

Les dérivés de formule (VIII) sont obtenus par action de chloroformiate de vinyle sur un composé de formule (I) correspondant pour R représente une chaîne de formule (B), R' représente un radical hydroxy, R₃ et R₄ sont des radicaux phényle, au sein d'un solvant inerte tel qu'un solvant chloré (dichlorométhane, chloroforme par exemple), à une température comprise entre 0°C et la température d'ébullition du mélange réactionnel.

Les composés de formule (I) pour lesquels R est une chaîne (B) dans laquelle R' est un radical -CO-alk peuvent être préparés par action d'un halogènure Hal-CO-alk dans lequel Hal représente un atome d'halogène et, de préférence, un atome de chlore et alk représente un radical alkyle sur un composé de formule (I) correspondant pour lequel R est une chaîne (B) dans laquelle R' est un atome d'hydrogène.

Cette réaction s'effectue généralement au sein d'un solvant inerte tel que le tétrahydrofuranne, le dioxanne, un solvant chloré (dichlorométhane, chloroforme par exemple), à une température comprise entre -50°C et 20°C, en présence de n-butyllithium.

Les composés de formule (I) pour lesquels R₂ représente un aromatique ou un hétéroaromatique substitué par -NR₆R₇ dans lequel R₆ et R₇ représentent chacun un atome d'hydrogène peuvent également être préparés par réduction d'un composé de formule (I) correpondant pour lequel R₂ représente un aromatique ou un hétéroaromatique substitué par nitro.

Cette réaction s'effectue par toute méthode connue permettant de réduire un nitro en amino sans toucher au reste de la molécule. De préférence, on utilise du fer en présence d'acide chlorhydrique au sein d'un alcool aliphatique 1-4C tel que l'éthanol, à la température d'ébullition du milieu réactionnel.

Les composés de formule (I) pour lesquels R₂ représente un aromatique ou hétéroaromatique substitué par -CONHR₉ et/ou R₃ et/ou R₄ représentent un aromatique ou un hétéroaromatique substitué par -CONR₁₀R₁₁ peuvent être également préparés par action d'un composé de formule (I) correspondant pour lequel R₂ et/ou R₃ et/ou R₄ représentent un aromatique ou un hétéroaromatique substitué par -COOR₅ pour lequel R₅ est alkyle ou phényle éventuellement substitué par des halogènes, avec respectivement une amine H₂NR₉ ou HNR₁₀R₁₁ pour lesquelles R₉, R₁₀ et R₁₁ ont les mêmes significations que dans la formule (I).

Cette réaction s'effectue généralement au sein d'un solvant inerte tel qu'un solvant chloré (dichlorométhane, chloroforme par exemple) ou un alcool aliphatique 1-4C (méthanol, éthanol par exemple), à une température comprise entre 0°C et la température d'ébullition du mélange réactionnel.

Les composés de formule (I) pour lesquels R₂ représente un aromatique substitué par hydroxy et/ou R₃ et/ou R₄ représentent un aromatique ou un hétéroaromatique substitué par hydroxy peuvent être également préparés par hydrolyse d'un composé de formule (I) correspondant pour lequel R₂ représente un aromatique substitué par alcoxy et/ou R₃ et/ou R₄ représentent un aromatique ou un hétéroaromatique substitué par alcoxy.

Cette réaction s'effectue par toute méthode d'hydrolyse d'un alcoxy en hydroxy sans toucher au reste de la molécule. De préférence, on hydrolyse au moyen de tribromure de bore, au sein d'un solvant chloré tel que le dichlorométhane, à une température voisine de 20°C.

Les composés de formule (I) pour lesquels R₂ représente un aromatique substitué par -NR₆R₇ pour lequel R₆ représente un radical alkyle et R₇ représente un atome d'hydrogène peuvent également être préparés par déprotection d'un composé de formule (I) correspondant pour lequel R₂ représente un aromatique substitué par un -N(alk)COOR₈ dans lequel R₈ représente un radical tertbutyle.

Cette réaction s'effectue généralement au moyen d'acide chlorhydrique, au sein d'un solvant tel que le dioxanne, à une température voisine de 20°C.

Les composés de formule (I) pour lesquels R₂ et/ou R₃ et/ou R₄ représentent un aromatique ou un hétéroaromatique substitué par -COOR₅ peuvent être également préparés par estérification d'un dérivé de formule : 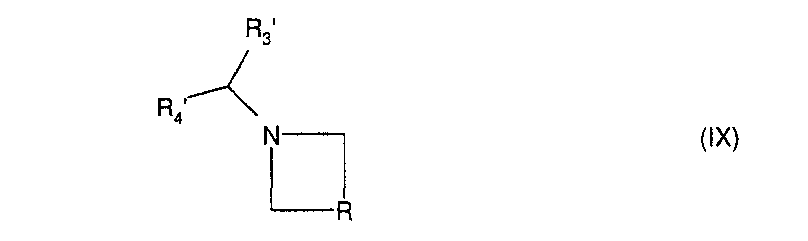 pour lequel R représente une chaîne C=C(SO₂R₁)R'₂ ou C(OR')CH(SO₂R₁)R'₂, R₁, R'₂, R'₃ et R'₄ ont les mêmes significations que les substituants R₁, R₂, R₃ et R₄ de la formule (I) sous réserve qu'au moins un des substituants R'₂, R'₃, R'₄ représente un aromatique ou un hétéroaromatique substitué par carboxyle, au moyen d'un dérivé de formule R₅OH pour lequel R₅ est alkyle ou phényle éventuellement substitué par un ou plusieurs halogène.

Lorsque R₅ est alkyle, cette réaction s'effectue généralement en présence d'un acide minéral (acide sulfurique par exemple), à une température comprise entre 20°C et la température d'ébullition du milieu réactionnel. Lorsque R₅ est phényle éventuellement substitué, cette réaction s'effectue de préférence en présence d'un carbodiimide (1-(3-diméthylaminopropyl)-3-éthylcarbodiimide, N,N'-dicyclohexyl-carbodiimide par exemple), dans un solvant inerte tel qu'un amide (diméthylformamide) ou un solvant chloré (chlorure de méthylène, dichloro-1,2 éthane, chloroforme par exemple), à une température comprise entre 0°C et la température d'ébullition du mélange réactionnel.

Les dérivés de formule (IX) pour lesquels R représente une chaîne C=C(SO₂R₁)R'₂ ou C(OR')CH(SO₂R₁)R'₂, R', R₁, R'₂, R'₃ et R'₄ ont les mêmes significations que les substituants R', R₁, R₂, R₃ et R₄ de la formule (I) sous réserve qu'au moins un des substituants R'₂, R'₃, R'₄ représente un aromatique ou un hétéroaromatique substitué par carboxyle peuvent être obtenus selon les méthodes décrites précédemment pour la préparation des composés de formule (I) à partir des intermédiaires correspondants et notamment selon la méthode décrite dans l'exemple 29.

Les composés de formule (I) pour lesquels R₂ et/ou R₃ et/ou R₄ représentent un aromatique ou un hétéroaromatique substitué par alkylthioalkyle peuvent également être préparés par action d'un dérivé de formule (IX) pour lequel R représente une chaîne C=C(SO₂R₁)R'₂ ou C(OR')CH(SO₂R₁)R'₂, R', R₁, R₂', R₃'et R₄' ont les mêmes significations que les substituants R', R₁, R₂, R₃ et R₄ de la formule (I) sous réserve qu'au moins un des substituants R₂', R₃', R₄' représente un aromatique ou un hétéroaromatique substitué par halogénoalkyle sur un alkylthiolate de sodium.

Cette réaction s'effectue généralement au sein d'un solvant inerte tel qu'un amide (diméthylformamide par exemple), à une température voisine de 20°C.

Les dérivés de formule (IX) pour lesquels R représente une chaîne C=C(SO₂R₁)R'₂ ou C(OR')CH(SO₂R₁)R'₂, R' R₁, R₂', R₃'et R₄' ont les mêmes significations que les substituants R', R₁, R₂, R₃ et R₄ de la formule (I) sous réserve qu'au moins un des substituants R₂', R₃', R₄' représente un aromatique ou un hétéroaromatique substitué par halogénoalkyle peuvent être obtenus par action d'un trihalogénure de phosphore (tribromure de phosphore de préférence) sur un composé de formule (I) correspondant pour lequel R₂ et/ou R₃ et/ou R₄ représentent un aromatique ou un hétéroaromatique substitué par hydroxyalkyle, au sein d'un solvant inerte tel qu'un solvant chloré (tétrachlorure de carbone, chloroforme par exemple, à une température voisine de 20°C.

Les composés de formule (I) pour lesquels R₂ et/ou R₃ et/ou R₄ représentent un aromatique substitué par hydroxyalkyle dans lequel l'alkyle contient un atome de carbone peuvent également être préparés par réduction d'un composé de formule (I) pour lequel au moins un des substituants R₂, R₃, R₄ représente un aromatique substitué par formyle.

Cette réaction s'effectue généralement au moyen de borohydrure de sodium, au sein d'un alcool aliphatique 1-4C (méthanol, éthanol par exemple), à une température voisine de 0°C.

Les composés de formule (I) pour lesquels R₃ et/ou R₄ représente un aromatique substitué par -alk-NR₆R₇ pour lequel alk est un alkyle contenant un atome de carbone peuvent également être préparés par action d'un composé de formule (I) pour lequel au moins un des substituants R₃, R₄ représente un aromatique substitué par formyle sur une amine HNR₆R₇ dans laquelle R₆ et R₇ ont les mêmes significations que dans la formule (I).

Cette réaction s'effectue généralement au sein d'un solvant inerte tel qu'un solvant chloré (dichloroéthane par exemple), à une température voisine de 20°C en présence de triacétoxyborohydrure de sodium ou de cyanoborohydrure de sodium.

Les composés de formule (I) pour lesquels R₂ représente un aromatique ou un hétéroaromatique substitué par -CONHR₉ et/ou R₃ et/ou R₄ représente un aromatique ou un hétéroaromatique substitué par -CO-NR₁₀R₁₁ peuvent également être préparés par action d'un dérivé de formule (IX) pour lequel R représente une chaîne C=C(SO₂R₁)R'₂ ou C(OR')CH(SO₂R₁)R'₂, R', R₁, R'₂, R'₃ et R'₄ ont les mêmes significations que les substituants R', R₁, R₂, R₃ et R₄ de la formule (I) sous réserve qu'au moins un des substituants R'₂, R'₃, R'₄ représente un aromatique ou un hétéroaromatique substitué par carboxyle sur respectivement une amine H₂NR₉ ou HNR₁₀R₁₁ dans lesquelles R₉, R₁₀ et R₁₁ ont les mêmes significations que dans la formule (I).

Cette réaction s'effectue de préférence soit en présence d'un agent de condensation utilisé en chimie peptidique tel qu'un carbodiimide (par exemple le 1-(3-diméthylaminopropyl)-3-éthylcarbodiimide, le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température d'ébullition du mélange réactionnel, soit après liaison préalable de l'acide sur une résine de type TFP de formule : 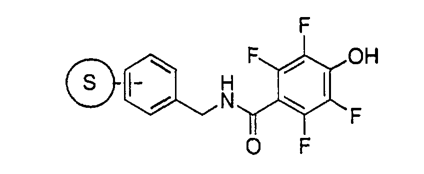 dans laquelle S représente une résine aminopolystyrène, au sein d'un solvant inerte tel que le diméthylformamide, en présence de 4-diméthylaminopyridine, à une température voisine de 20°C. La liaison sur résine s'effectue généralement au sein du diméthylformamide, en présence de 4-diméthylaminopyridine et de 1,3-diisopropylcarbodiimide, à une température voisine de 20°C.

Les composés de formule (I) pour lesquels R₂ et/ou R₃ et/ou R₄ représentent un aromatique ou un hétéroaromatique substitué par -CO-NH-NR₆R₇ peuvent également être préparés par action d'un composé de formule (I) correspondant pour lequel R₂ et/ou R₃ et/ou R₄ représentent un aromatique ou un hétéroaromatique substitué par -COOR₅ et R₅ représente un radical alkyle ou phényle éventuellement substitué par des halogènes, sur une hydrazine H₂N-NR₆R₇ pour laquelle R₆ et R₇ ont les mêmes significations que dans la formule (I).

Cette réaction s'effectue généralement au sein d'un solvant inerte tel que le diméthylformamide, à une température voisine de 20°C.

Les composés de formule (I) pour lesquels R₂ représente un aromatique ou un hétéroaromatique substitué par -CO-NHR₉ dans lequel R₉ représente un atome d'hydrogène et/ou R₃ et/ou R₄ représentent un aromatique ou un hétéroaromatique substitué par -CO-NR₁₀R₁₁ dans lequel R₁₀ et R₁₁ sont des atomes d'hydrogène peuvent également être préparés par hydrolyse d'un composé de formule (I) correspondant pour lequel R₂ et/ou R₃ et/ou R₄ représentent un aromatique ou un hétéroaromatique substitué par cyano.

Cette réaction s'effectue par toute méthode connue permettant de passer d'un nitrile au carbamoyle correspondant sans toucher au reste de la molécule. De préférence, on opère au moyen d'acide chlorhydrique, au sein de l'acide acétique, à une température voisine de 20°C.

Les composés de formule (I) pour lesquels R₂ représente un aromatique substitué par -O-alkNR₁₂R₁₃ peuvent également être préparés par action d'un dérivé de formule (IX) pour lequel R représente une chaîne C=C(SO₂R₁)R'₂ ou C(OR')CH(SO₂R₁)R'₂, R', R₁, R₂', R₃'et R₄' ont les mêmes significations que les substituants R', R₁, R₂, R₃ et R₄ de la formule (I) sous réserve qu'au moins un des substituants R₂', R₃', R₄' représente un aromatique substitué par -O-alk-Hal dans lequel alk représente un radical alkyle et Hal représente un atome d'halogène et, de préférence, un atome de chlore ou de brome, sur une amine HNR₁₂R₁₃ dans laquelle R₁₂ R₁₃ ont les mêmes significations que dans la formule (I).

Cette réaction s'effectue généralement au sein d'un solvant inerte tel que l'acétonitrile, en présence d'un carbonate de métal alcalin (carbonate de potassium par exemple), à une température voisine de 20°C.

Les dérivés de formule (IX) pour lesquels R représente une chaîne C=C(SO₂R₁)R'₂ ou C(OR')CH(SO₂R₁)R'₂, R', R₁, R₂', R₃'et R₄' ont les mêmes significations que les substituants R', R₁, R₂, R₃ et R₄ de la formule (I) sous réserve qu'au moins un des substituants R₂', R₃', R₄' représente un aromatique substitué par -O-alk-Hal dans lequel alk représente un radical alkyle et Hal représente un atome d'halogène peuvent être obtenus par action d'un composé de formule (I) correspondant pour lequel R₂ représente un aromatique substitué par hydroxy avec un dérivé Hal-alk-Hal dans lequel Hal représente un halogène.

Cette réaction s'effectue généralement au sein d'un solvant inerte tel qu'une cétone (méthyléthylcétone par exemple), en présence d'une base telle qu'un carbonate de métal alcalin (carbonate de potassium par exemple), à la température d'ébullition du milieu réactionnel.

Les composés de formule (I) pour lesquels R₃ et/ou R₄ représente un aromatique substitué par -alk-NR₆R₇ peuvent également être préparés par action d'un dérivé de formule (IX) pour lequel R représente une chaîne C=C(SO₂R₁)R'₂ ou C(OR')CH(SO₂R₁)R'₂, R', R₁, R₂', R₃' et R₄' ont les mêmes significations que les substituants R', R₁, R₂, R₃ et R₄ de la formule (I) sous réserve qu'au moins un des substituants R₃', R₄' représente un aromatique substitué -alk-Cl dans lequel alk représente un radical alkyle sur une amine HNR₆R₇ dans laquelle R₆ R₇ ont les mêmes significations que dans la formule (I).

Cette réaction s'effectue généralement au sein d'un solvant inerte tel qu'un solvant chloré (dichlorométhane par exemple), éventuellement en présence d'une base azotée telle que la diméthylaminopyridine, la diisopropyléthylamine, à une température comprise entre 5 et 25°C.

Les dérivés de formule (IX) pour lequel R représente une chaîne C=C(SO₂R₁)R'₂ ou C(OR')CH(SO₂R₁)R'₂, R', R₁, R₂', R₃'et R₄' ont les mêmes significations que les substituants R', R₁, R₂, R₃ et R₄ de la formule (I) sous réserve qu'au moins un des substituants R₃', R₄' représente un aromatique substitué par -alk-Cl peuvent être obtenus par action du chlorure de thionyle sur un composé de formule (I) correspondant pour lequel au moins un des substituants R₃, R₄ représente un aromatique substitué par un ou plusieurs radicaux hydroxyalkyle.

Cette réaction s'effectue généralement au sein d'un solvant inerte tel qu'un solvant chloré (dichlorométhane par exemple), à une température comprise entre 10 et 30°C.

Les composés de formule (I) pour lesquels R représente une chaîne B, R' représente un atome d'hydrogène et R₃ et/ou R₄ représente un aromatique substitué par hydroxyalkyle dans lequel le reste alkyle contient 1 atome de carbone peuvent également être préparés par action d'hydrure de diisobutylaluminium sur un composé de formule (I) correspondant pour lequel R représente une chaîne B, R' représente un atome d'hydrogène et R₃ et/ou R₄ représente un aromatique substitué par un ou plusieurs radicaux -COOR₅ dans lequel R₅ est un radical alkyle.

Cette réaction s'effectue généralement au sein du toluène, à une température comprise entre -30°C et 0°C.

Les composés de formule (I) pour lesquels R₂ représente un radical phényle substitué par -NR₆R₇ représentant un cycle 1-pipérazinyl substitué en position -4 par un radical alkyle peuvent également être préparés par action d'un composé de formule (I) correspondant pour lequel R₂ représente un radical phényle substitué par un radical -NR₆R₇ représentant un cycle 1-pipérazinyl sur un dérivé alk-CHO dans lequel alk représente un radical alkyle en chaîne droite ou ramifiée contenant 1 à 5 atomes de carbone.

Cette réaction s'effectue généralement au sein d'un solvant inerte tel qu'un solvant chloré (dichoroéthane, chloroforme par exemple), en présence de NaBH(OCOCH₃)₃, à une température voisine de 20°C.

Les composés de formule (I) pour lesquels R₂ représente un radical phényle substitué par -NR₆R₇ représentant un cycle 1-pipérazinyl substitué en position -4 par un radical -COOalk peuvent également être préparés par action d'un composé de formule (I) correspondant pour lequel R₂ représente un radical phényle substitué par un radical -NR₆R₇ représentant un cycle 1-pipérazinyl sur un dérivé de formule Hal-COOalk dans lequel alk représente un radical alkyle et Hal représente un atome d'halogène et, de préférence, un atome de chlore.

Cette réaction s'effectue généralement au sein de la pyridine, à une température voisine de 20°C.

Les composés de formule (I) pour lesquels R₂ représente un radical phényle substitué par -NR₆R₇ représentant un cycle 1-pipérazinyl substitué en position -4 par un radical -CO-NHalk ou -CS-NHalk peuvent également être préparés par action d'un composé de formule (I) correspondant pour lequel R₂ représente un radical phényle substitué par -NR₆R₇ représentant un cycle 1-pipérazinyl sur un dérivé de formule Y=C=Nalk dans lequel alk représente un radical alkyle en chaîne droite ou ramifiée contenant 1 à 6 atomes de carbone et Y représente un atome de soufre ou d'oxygène.

Cette réaction s'effectue généralement au sein d'un solvant inerte tel qu'un solvant chloré (dichlorométhane par exemple), à une température voisine de 20°C.

Les composés de formule (I) pour lesquels R₂ représente un radical phényle substitué par un radical -NR₆R₇ représentant un cycle 1-pipérazinyl substitué en position -4 par un radical -CO-alk-NR₁₄R₁₅ peuvent également être préparés par action d'un composé de formule (I) correspondant pour lequel R₂ représente un radical phényle substitué par un radical -NR₆R₇ représentant un cycle 1-pipérazinyl avec un acide de formule R₁₅R₁₄N-alk-COOH dans lequel alk représente un radical alkyle et R₁₄ et R₁₅ ont les mêmes significations que dans la formule (I) suivi éventuellement d'une déprotection du produit pour lequel R₁₄ est un radical tert-butoxycarbonyle pour obtenir les composés pour lesquels R₁₄ est un atome d'hydrogène.

Cette réaction s'effectue généralement au sein d'un solvant inerte tel qu'un solvant chloré (dichloroéthane par exemple, à une température voisine de 20°C. La déprotection s'effectue au moyen d'acide formique à une température voisine de 20°C.

Les composés de formule (I) pour lesquels R₂ représente un radical phényle substitué par un radical -NR₆R₇ représentant un cycle 1-pipérazinyl substitué en position -4 par un radical -CO-alk dans lequel alk représente un radical méthyle peuvent également être préparés par action d'un composé de formule (I) correspondant pour lequel R₂ représente un radical phényle substitué par un radical -NR₆R₇ représentant un cycle 1-pipérazinyl avec l'anhydride acétique.

Cette réaction s'effectue généralement en présence de pyridine, à une température voisine de 20°C.

Il est entendu pour l'homme du métier que, pour la mise en oeuvre des procédés selon l'invention décrits précédemment, il peut être nécessaire d'introduire des groupes protecteurs des fonctions amino, hydroxy et carboxy afin d'éviter des réactions secondaires. Ces groupes sont ceux qui permettent d'être éliminés sans toucher au reste de la molécule. Comme exemples de groupes protecteurs de la fonction amino on peut citer les carbamates de tert-butyle ou de méthyle qui peuvent être régénérées au moyen d'iodotriméthylsilane ou d'allyle au moyen de catalyseurs du palladium. Comme exemples de groupes protecteurs de la fonction hydroxy, on peut citer les triéthylsilyle, tert-butyldiméthylsilyle qui peuvent être régénérés au moyen de fluorure de tétrabutylammonium ou bien les acétals dissymétriques (méthoxyméthyle, tétrahydropyranyle par exemple) avec régénération au moyen d'acide chlorhydrique. Comme groupes protecteurs des fonctions carboxy, on peut citer les esters (allyle, benzyle par exemple), les oxazoles et les 2-alkyl-1,3-oxazolines. D'autres groupes protecteurs utilisables sont décrits par GREENE T.W. et coll., Protecting Groups in Organic Synthesis, second edition, 1991, Jonh Wiley & Sons.

Les composés de formule (I) peuvent être purifiés par les méthodes connues habituelles, par exemple par cristallisation, chromatographie ou extraction.

Les énantiomères des composés de formule (I) peuvent être obtenus par dédoublement des racémiques par exemple par chromatographie sur colonne chirale selon PIRCKLE W.H. et coll., asymmetric synthesis, vol. 1, Academic Press (1983) ou par formation de sels ou par synthèse à partir des précurseurs chiraux. Les diastéréoisomères peuvent être préparés selon les méthodes classiques connues (cristallisation, chromatographie ou à partir des précurseurs chiraux).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels suivants : benzènesulfonate, bromhydrate, chlorhydrate, citrate, éthanesulfonate, fumarate, gluconate, iodate, iséthionate, maléate, méthanesulfonate, méthylène-bis-β-oxynaphtoate, nitrate, oxalate, pamoate, phosphate, salicylate, succinate, sulfate, tartrate, théophyllinacétate et p-toluènesulfonate.

Les composés de formule (I) présentent des propriétés pharmacologiques intéressantes. Ces composés possèdent une forte affinité pour les récepteurs cannabinoïdes et particulièrement ceux de type CB1. Ce sont des antagonistes du récepteur CB1 et sont donc utiles dans le traitement et la prévention des désordres touchant au système nerveux central, au système immunitaire, au système cardio-vasculaire ou endocrinien, au système respiratoire, à l'appareil gastrointestinal et aux désordres de la reproduction (Hollister, Pharm. Rev.; 38, 1986, 1-20, Reny et Sinha, Prog. Drug Res., 36, 71-114 (1991), Consroe et Sandyk, in Marijuana/Cannabinoids, Neurobiology and Neurophysiology, 459, Murphy L. and Barthe A. Eds, CRC Press, 1992), des infections bactériennes, virales et parasitaires.

C'est ainsi que ces composés peuvent être utilisés pour le traitement ou la prévention des psychoses y compris la schizophrénie, des troubles anxieux, de la dépression, de l'épilepsie, de la neurodégénération, des désordres cérébelleux et spinocérébelleux, des désordres cognitifs, du trauma crânien, des attaques de panique, des neuropathies périphériques, des glaucomes, de la migraine, de la maladie de Parkinson, de la maladie d'Alzheimer, de la chorée de Huntington, du syndrome de Raynaud, des tremblements, du désordre compulso-obsessionnel, de la démence sénile, des désordres thymiques, du syndrome de Tourette, de la dyskinésie tardive, des désordres bipolaires, des cancers, des désordres du mouvement induit par les médicaments, des dystonies, des chocs endotoxémiques, des chocs hémorragiques, de l'hypotension, de l'insomnie, des maladies immunologiques, de la sclérose en plaques, des vomissements, de l'asthme, des troubles de l'appétit (boulimie, anorexie), de l'obésité, des troubles de la mémoire, dans le sevrage aux traitements chroniques et abus d'alcool ou de médicaments (opioides, barbituriques, cannabis, cocaïne, amphétamine, phencyclide, hallucinogènes, benzodiazépines par exemple), comme analgésiques ou potentialisateurs de l'activité analgésique des médicaments narcotiques et non narcotiques. Ils peuvent également être utilisés pour le traitement ou la prévention des désordres du transit intestinal, comme antibactériens, antiviraux et antiparasitaire.

L'affinité des composés de formule (I) pour les récepteurs du cannabis a été déterminée selon la méthode décrite par KUSTER J.E., STEVENSON J.I., WARD S.J., D'AMBRA T.E., HAYCOCK D.A. dans J. Pharmacol. Exp. Ther., 264 1352-1363 (1993).

Dans ce test, la CI₅₀ des composés de formule (I) est inférieure ou égale à 100 nM.

Leur activité antagonistique a été montrée au moyen du modèle d'hypothermie induite par un agoniste des récepteurs du cannabis (CP-55940) chez la souris, selon la méthode décrite par Pertwee R.G. dans Marijuana, Harvey D.J. eds, 84 Oxford IRL Press, 263-277 (1985).

Dans ce test, la DE50 des composés de formule (I) est inférieure ou égale à 50 mg/kg.

Les composés de formule (I) présentent une toxicité faible. Leur DL₅₀ est supérieure à 40 mg/kg par voie sous cutanée chez la souris.

Les composés de formule (I) préférés sont ceux pour lesquels
R représente une chaîne (A) ou (B) et R' représentant un atome d'hydrogène ou un radical -COalk,
R₁ représente un radical méthyle ou éthyle,
R₂ représente soit un aromatique choisi parmi phényle et naphtyle, ces aromatiques étant non substitués ou substitués par un ou plusieurs halogène, alkyle, alcoxy, hydroxy, -COOR₅, trifluorométhyle, trifluorométhylsulfanyle, trifluorométhoxy, -NR₆R₇, -CO-NH-NR₆R₇, cyano, -CONHR₉, alkylsulfanyle, hydroxyalkyle, nitro, -CO-NR₁₆R₁₇, -O-alkNR₁₂R₁₃ ou alkylthioalkyle ou un hétéroaromatique choisi parmi isoquinolyle, pyridyle, quinolyle, 1,2,3,4-tétrahydroisoquinolyle, 1,2,3,4-tétrahydroquinolyle, thiényle, ces hétéroaromatiques étant non substitués ou substitués par un halogène, alkyle, alcoxy, -COOR₅, trifluorométhyle, trifluorométhylsulfanyle, trifluorométhoxy, -NR₆R₇, -CO-NH-NR₆R₇, cyano, -CONHR₉, alkylsulfanyle, hydroxyalkyle, nitro ou alkylthioalkyle,
R₃ et R₄, identiques ou différents, représentent soit un aromatique choisi parmi phényle ou naphtyle , ces aromatiques étant non substitués ou substitués par un ou plusieurs halogène, alkyle, alcoxy, trifluorométhyle, trifluorométhoxy, -CONR₁₀R₁₁, -alk-NR₆R₇, hydroxyalkyle, formyle, -COOR₅, soit un hétéroaromatique choisi parmi les cycles thiazolyle ou thiényle, ces hétéroaromatiques étant non substitués ou substitués par un halogène, alkyle, alcoxy, -CONR₁₀R₁₁, -alk-NR₆R₇, hydroxyalkyle ou -COOR₅.
R₅ est alkyle ou phényle éventuellement substitué par un ou plusieurs halogène,
R₆ et R₇, identiques ou différents représentent un atome d'hydrogène ou un radical alkyle, -COOalk, cycloalkyle, alkylcycloalkyle, -alk-O-alk, hydroxyalkyle ou bien R₆ et R₇ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, -COalk, -COOalk, -CO-NHalk, -CS-NHalk, -CO-alk-NR₁₄R₁₅, oxo, hydroxyalkyle, -alk-O-alk, -CO-NH₂,
R₉ représente un atome d'hydrogène ou un radical alkyle ou alkyle substitué par dialkylamino, phényle, cycloalkyle (éventuellement substitué par -COOalk) ou un hétérocycle mono ou bicyclique saturé ou insaturé ayant 3 à 10 chaînons, contenant éventuellement un ou plusieurs hétéroatomes choisis parmi oxygène, soufre et azote et étant éventuellement substitué par un ou plusieurs radicaux alkyle,
R₁₀ et R₁₁, identiques ou différents, représentent un atome d'hydrogène ou un radical alkyle ou bien R₁₀ et R₁₁ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un radical alkyle,
R₁₂ et R₁₃, identiques ou différents, représentent un atome d'hydrogène ou un radical alkyle, cycloalkyle ou bien R₁₂ et R₁₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un radical alkyle, -COalk, -COOalk, -CO-NHalk, -CS-NHalk, -CO-alk-NR₁₄R₁₅ ou un hétérocycle mono ou bicyclique saturé ayant 3 à 10 chaînons et contenant un hétéroatome choisi parmi oxygène, soufre et azote,
R₁₄ et R₁₅, identiques ou différents, représentent un atome d'hydrogène ou un radical alkyle ou -COOalk,
R₁₆ et R₁₇ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote,
alk représente un radical alkyle ou alkylène,
leurs isomères optiques et leurs sels avec un acide minéral ou organiques.

Les composés de formule (I) particulièrement préférés sont ceux pour lesquels
R représente une chaîne (A) ou (B),
R' représentant un atome d'hydrogène ou un radical -COalk,
R₁ représente un radical méthyle ou éthyle,
R₂ représente soit un aromatique choisi parmi
naphtyle,
phényle,
phényle substitué par un ou plusieurs halogène, alkyle, alcoxy, hydroxy, -COOR₅ (dans lequel R₅ représente un radical alkyle ou phényle éventuellement substitué par plusieurs halogènes), trifluorométhyle, trifluorométhylsulfanyle, trifluorométhoxy, -NR₆R₇ (dans lequel R₆ et R₇, identiques ou différents représentent un atome d'hydrogène ou un radical alkyle ou -COOalk ou bien R₆ et R₇ forment ensemble avec l'atome d'azote auquel ils sont rattachés un hétérocycle choisi parmi pyrrolidinyle, pipéridyle, pipérazinyle ou pipérazinyle substitué par un ou plusieurs radicaux alkyle, -COalk, -COOalk, -CO-NHalk, -CS-NHalk, -CO-alk-NR₁₄R₁₅, dans lequel R₁₄ et R₁₅, identiques ou différents, représentent un atome d'hydrogène ou un radical alkyle), -CO-NH-NR₆R₇ (R₆ et R₇, identiques ou différents représentent un atome d'hydrogène ou un radical alkyle ou bien R₆ et R₇ forment ensemble avec l'atome d'azote auquel ils sont rattachés un hétérocycle choisi parmi pipéridyle, pipérazinyle ou pipérazyle substitué par un ou plusieurs radicaux alkyle), cyano, -CONHR₉ (dans lequel R₉ représente un atome d'hydrogène ou un radical alkyle ou alkyle substitué par dialkylamino, phényle, cycloalkyle (éventuellement substitué par -COOalk) ou un hétérocycle choisi parmi pyrrolidinyle (éventuellement substitué par alkyle), tétrahydrofuryle, morpholinyle ou pyrrolyle), alkylsulfanyle, hydroxyalkyle, nitro, -CO-NR₁₆R₁₇, (dans lequel R₁₆ et R₁₇ forment ensemble avec l'atome d'azote auquel ils sont rattachés un cycle pipéridyle), -O-alkNR₁₂R₁₃ (dans lequel R₁₂ et R₁₃ forment ensemble avec l'atome d'azote auquel ils sont rattachés un cycle morpholino) ou alkylthioalkyle,
soit un hétéroaromatique choisi parmi
isoquinolyle,
pyridyle,
quinolyle,
1,2,3,4-tétrahydroisoquinolyle,
1,2,3,4-tétrahydroquinolyle,
thiényle, ou
thiényle substitué par un -COOR₅ (dans lequel R₅ représente un radical alkyle) ou -CONHR₉, (dans lequel R₉ représente un radical alkyle),
R₃ et R₄, identiques ou différents, représentent soit un aromatique choisi parmi
phényle ou
phényle substitué par un ou plusieurs halogène, alkyle, alcoxy, trifluorométhyle, trifluorométhoxy, hydroxyalkyle, formyle, -COOR₅ (dans lequel R₅ est un radical alkyle), -CONR₁₀R₁₁ (dans lequel R₁₀ et R₁₁, identiques ou différents, représentent un atome d'hydrogène ou un radical alkyle), -alk-NR₆R₇, (dans lequel R₆ et R₇, identiques ou différents représentent un atome d'hydrogène ou un radical alkyle, cycloalkyle,-alk-O-alk, hydroxyalkyle ou bien R₆ et R₇ forment ensemble avec l'atome d'azote auquel ils sont rattachés un hétérocycle choisi parmi pipéridyle (éventuellement substitué par alkyle, oxo), pyrrolidinyle (éventuellement substitué par alkyle, hydroxyalkyle, -alk-O-alk, -CO-NH₂), thiomorpholinyle, morpholinyle, pyrrolyle, pipérazinyle éventuellement substitué par oxo, alkyle, hydroxyalkyle, -COOR₅ (dans lequel R₅ est un radical alkyle),
soit un hétéroaromatique choisi parmi
thiazolyle ou
thiényle,
alk représente un radical alkyle ou alkylène,
leurs isomères optiques et leurs sels avec un acide minéral ou organiques.

De préférence, lorsque R₂ est un radical phényle substitué, ce dernier est monosubstitué et, en particulier, en position 3 ou bien disubstitué et, en particulier, en positions 3,5; 2,5 ou 2,3.

De préférence, lorsque R₃ est un radical phényle substitué, ce dernier est monosubstitué et, en particulier, en position 4 ou disubstitué et, en particulier, en positions 2,4.

De préférence, lorsque R₄ est un radical phényle substitué, ce dernier est monosubstitué et, en particulier, en position 4 ou disubstitué et, en particulier, en positions 2,4.

Parmi les composés préférés, on peut citer les composés suivants :
1-benzhydryl-3-[(méthylsulfonyl)(phényl)méthylène]azétidine,
1-benzhydryl-3-[(3-méthylphényl)(méthylsulfonyl)méthylène]azétidine,
1-benzhydryl-3-[(3-chlorophényl)(méthylsulfonyl)méthylène]azétidine,
1-benzhydryl-3-[(3,5-dichlorophényl)(méthylsulfonyl)méthylène]azétidine,
1-benzhydryl-3-[(2,5-dichlorophényl)(méthylsulfonyl)méthylène]azétidine,
1-benzhydryl-3-[(2,3-dichlorophényl)(méthylsulfonyl)méthylène]azétidine,
1-benzhydryl-3-[(3-fluorophényl)(méthylsulfonyl)méthylène]azétidine,
1-benzhydryl-3-[(3,5-difluorophényl)(méthylsulfonyl)méthylène]azétidine,
1-benzhydryl-3-[(3-bromophényl)(méthylsulfonyl)méthylène]azétidine,
1-benzhydryl-3 - [(3-iodophényl)(méthylsulfonyl)méthylène]azétidine,
1-benzhydryl-3-[(méthylsulfonyl)(3-trifluorométhoxyphényl)méthylène]azétidine,
1-benzhydryl-3-[(méthylsulfonyl)(3-trifluorométhylphényl)méthylène]azétidine,
1-benzhydryl-3-{[3,5-bis(trifluorométhyl)phényl](méthylsulfonyl)méthylène}azétidine,
1-benzhydryl-3-[(3,5-dibromophényl)(méthylsulfonyl)méthylène]azétidine,
1-benzhydryl-3-[(3-méthoxycarbonylphényl)(méthylsulfonyl)méthylène]azétidine,
1-benzhydryl-3-[(3-cyanophényl)(méthylsulfonyl)méthylène]azétidine,
1-benzhydryl-3-[(3-carbamoylphényl)(méthylsulfonyl)méthylène]azétidine,
1-benzhydryl-3-[(méthylsulfonyl)(napht-1-yl)(méthylsulfonyl)méthylène]azétidine,
1-[bis(4-chlorophényl)méthyl]-3-[(3,5-difluorophényl)(méthylsulfonyl)méthylène] azétidine,
1-[bis(4-méthoxyphényl)méthyl]-3-[(3,5-difluorophényl)(méthylsulfonyl)méthylène]azétidine,
1-[bis(4-méthylphényl)méthyl]-3-[(3,5-difluorophényl)(méthylsulfonyl)méthylène] azétidine,
(RS)-3-[(3,5-difluorophényl)(méthylsulfonyl)méthylène]-1-[(4-méthoxyphényl) (phényl)méthyl)]azétidine,
(R)-3-[(3,5-difluorophényl)(méthylsulfonyl)méthylène]-1-[(4-méthoxyphényl) (phényl)méthyl]azétidine,
(S)-3-[(3,5-difluorophényl)(méthylsulfonyl)méthylène]-1-[(4-méthoxyphényl) (phényl)méthyl]azétidine,
1-[bis(4-trifluorométhoxyphényl)méthyl]-3-[(3,5-difluorophényl)(méthylsulfonyl) méthylène]azétidine,
1-[bis(4-trifluorométhylphényl)méthyl]-3-[(3,5-difluorophényl)(méthylsulfonyl)méthylène]azétidine,
1-[bis(4-chlorophényl) méthyl]3-{[3,5-bis(trifluorométhyl) phényl]méthylsulfonyl méthylène }azétidine,
(RS)-1-[(4-chlorophényl)(2,4-dichlorophényl)méthyl]-3-[(3,5-difluorophényl)(méthylsulfonyl)méthylène]azétidine,
(R)-1-[(4-chlorophényl)(2,4-dichlorophényl)méthyl]-3-[(3,5-difluorophényl)(méthylsulfonyl)méthylène]azétidine,
(S)-1-[(4-chlorophényl)(2,4-dichlorophényl)méthyl]-3-[(3,5-difluorophényl)(méthylsulfonyl)méthylène]azétidine,
(RS)-1-{(4-chlorophényl)[4-(hydroxyméthyl)phényl]méthyl}-3-[(3,5-difluorophényl)(méthylsulfonyl)méthylène]azétidine,
(R)-1-{(4-chlorophényl)[4-(hydroxyméthyl)phényl]méthyl}-3-[(3,5-difluorophényl)(méthylsulfonyl)méthylène]azétidine,
(S)-1-{(4-chlorophényl)[4-(hydroxyméthyl)phényl]méthyl}-3-[(3,5-difluorophényl)(méthylsulfonyl)méthylène]azétidine,
(RS)-1-{(4-chlorophényl)[4-(pyrrolidylméthyl)phényl]méthyl}-3-[(3,5-difluorophényl)(méthylsulfonyl)méthylène]azétidine,
(R)-1-{(4-chlorophényl)[4-(pyrrolidylméthyl)phényl]méthyl}-3-[(3,5-difluorophényl)(méthylsulfonyl)méthylène]azétidine,
(S)-1-{(4-chlorophényl)[4-(pyrrolidylméthyl)phényl]méthyl}-3- [(3,5 -difluorophényl)(méthylsulfonyl)méthylène]azétidine,
1-{(RS)-(4-chlorophényl) [4-(3,3-diméthyl-pipéridin-1-yl-méthyl) phényl]méthyl}-3-[(3,5-difluorophényl)(méthylsulfonyl)méthylène]azétidine,
1-{(R)-(4-chlorophényl) [4-(3,3-diméthyl-pipéridin-1-yl-méthyl) phényl]méthyl}-3-[(3,5-difluorophényl)(méthylsulfonyl)méthylène]azétidine,
1-{(S)-(4-chlorophényl) [4-(3,3-diméthyl-pipéridin-1-yl-méthyl) phényl]méthyl}-3-[(3,5-difluorophényl)(méthylsulfonyl)méthylène]azétidine,
1-{(RS)-(4-chlorophényl) [4-(thiomorpholin-4-yl-méthyl) phényl]méthyl}-3-[(3,5-difluorophényl)(méthylsulfonyl)méthylène]azétidine,
1-{(R)-(4-chlorophényl)[4-(thiomorpholin-4-yl-méthyl)phényl]méthyl}-3-[(3,5-difluorophényl)(méthylsulfonyl)méthylène]azétidine,
1-{(S)-(4-chlorophényl)[4-(thiomorpholin-4-yl-méthyl)phényl]méthyl}-3-[(3,5-difluorophényl)(méthylsulfonyl)méthylène]azétidine,
1-{(RS)-(4-chlorophényl)[4-(N-éthyl-N-cyclohexyl-aminométhyl)phényl]méthyl}-3-[(3,5-difluorophényl)(méthylsulfonyl)méthylène]azétidine,
1-{(R)-(4-chlorophényl)[ 4-(N-éthyl-N-cyclohexyl-aminométhyl)phényl]méthyl}-3-[(3,5-difluorophényl)(méthylsulfonyl)méthylène]azétidine,
1-{(S)-(4-chlorophényl)[ 4-(N-éthyl-N-cyclohexyl-aminométhyl)phényl]méthyl}-3-[(3,5-difluorophényl)(méthylsulfonyl)méthylène]azétidine,
1-{{(RS)-(4-chlorophényl){4-[(4-éthoxycarbonylpipérazinyl)méthyl]phényl}méthyl}}-3-[(3,5-difluorophényl)(méthylsulfonyl)méthylène]azétidine,
1-{{(R)-(4-chlorophényl){4-[(4-éthoxycarbonylpipérazinyl)méthyl]phényl} méthyl } }-3-[(3,5-difluorophényl)(méthylsulfonyl)méthylène]azétidine,
1-{{(S)-(4-chlorophényl){4-[(4-éthoxycarbonylpipérazinyl)méthyl]phényl}méthyl}}-3-[(3,5-difluorophényl)(méthylsulfonyl)méthylène]azétidine,
1-{(RS)-(4-chlorophényl)[4-(N-cyclopropyl-N-propyl-aminométhyl)phényl]méthyl}-3-[(3,5-difluorophényl)(méthylsulfonyl)méthylène]azétidine,
1-{(R)-(4-chlorophényl)[4-(N-cyclopropyl-N-propyl-aminométhyl)phényl]méthyl}-3-[(3,5-difluorophényl)(méthylsulfonyl)méthylène]azétidine,
1-{(S)-(4-chlorophényl)[4-(N-cyclopropyl-N-propyl-aminométhyl)phényl]méthyl}-3-[(3,5-difluorophényl)(méthylsulfonyl)méthylène]azétidine,
1-{(RS)-(4-chlorophényl)[4-(diisopropylaminométhyl)phényl]méthyl}-3-[(3,5-difluorophényl)(méthylsulfonyl)méthylène]azétidine,
1-{(R)-(4-chlorophényl)[4-(diisopropylaminométhyl)phényl]méthyl}-3-[(3,5-difluorophényl)(méthylsulfonyl)méthylène]azétidine,
1-{(S)-(4-chlorophényl) [4-(diisopropylaminométhyl)phényl] méthyl}-3-[(3,5-difluorophényl)(méthylsulfonyl)méthylène]azétidine,
1-{{(RS)-(4-chlorophényl) {4-[bis-(2-méthoxyéthyl)aminométhyl]phényl}méthyl}}-3-[(3,5-difluorophényl)(méthylsulfonyl)méthylène]azétidine,
1-{{(R)-(4-chlorophényl){4-[bis-(2-méthoxyéthyl)aminométhyl]phényl} méthyl}}-3-[(3,5-difluorophényl)(méthylsulfonyl)méthylène]azétidine,
1-{{(S)-(4-chlorophényl){4-[bis-(2-méthoxyéthyl)aminométhyl]phényl}méthyl}}-3-[(3,5-difluorophényl)(méthylsulfonyl)méthylène]azétidine,
1-{(RS)-(4-chlorophényl)[4-(di-n-propylaminométhyl)phényl]méthyl}-3-[(3,5-difluorophényl)(méthylsulfonyl)méthylène]azétidine,
1-{(R)-(4-chlorophényl)[4-(di-n-propylaminométhyl)phényl]méthyl}-3-[(3,5-difluorophényl)(méthylsulfonyl)méthylène]azétidine,
1-{(S)-(4-chlorophényl)[4-(di-n-propylaminométhyl)phényl]méthyl}-3-[(3,5-difluorophényl)(méthylsulfonyl)méthylène]azétidine,
1-{(RS)-(4-chlorophényl)[4-(pipéridin-1-yl-méthyl)phényl]méthyl}-3-[(3,5-difluorophényl)(méthylsulfonyl)méthylène]azétidine,
1-{(R)-(4-chlorophényl)[4-(pipéridin-1-yl-méthyl)phényl]méthyl}-3-[(3,5-difluorophényl)(méthylsulfonyl)méthylène]azétidine,
1-{(S)-(4-chlorophényl)[4-(pipéridin-1-yl-méthyl)phényl]méthyl}-3-[(3,5-difluorophényl)(méthylsulfonyl)méthylène]azétidine,
1-{(RS)-(4-chlorophényl)[4-(4-méthyl-pipérazin-1-yl-méthyl)phényl]méthyl }-3-[(3,5-difluorophényl)(méthylsulfonyl)méthylène]azétidine,
1-{(R)-(4-chlorophényl)[4-(4-méthyl-pipérazin-1-yl-méthyl)phényl]méthyl}-3-[(3,5-difluorophényl)(méthylsulfonyl)méthylène]azétidine,
1-{(S)-(4-chlorophényl)[4-(4-méthyl-pipérazin-1-yl-méthyl)phényl]méthyl}-3-[(3,5-difluorophényl)(méthylsulfonyl)méthylène]azétidine,
1-{(RS)-(4-chlorophényl)[4-(morpholin-4-yl-méthyl)phényl]méthyl}-3-[(3,5-difluorophényl)(méthylsulfonyl)méthylène]azétidine,
1-{(R)-(4-chlorophényl)[4-(morpholin-4-yl-méthyl)phényl]méthyl}-3-[(3,5-difluorophényl)(méthylsulfonyl)méthylène]azétidine,
1-{(S)-(4-chlorophényl)[4-(morpholin-4-yl-méthyl)phényl]méthyl}-3-[(3,5-difluorophényl)(méthylsulfonyl)méthylène]azétidine,
1-{(RS)-(4-chlorophényl)[4-(diéthylaminométhyl)phényl]méthyl}-3-[(3,5-difluorophényl)(méthylsulfonyl)méthylène]azétidine,
1-{(R)-(4-chlorophényl)[4-(diéthylaminométhyl)phényl]méthyl}-3-[(3,5-difluorophényl)(méthylsulfonyl)méthylène]azétidine,
1-{(S)-(4-chlorophényl)[4-(diéthylaminométhyl)phényl]méthyl}-3-[(3,5-difluorophényl)(méthylsulfonyl)méthylène]azétidine,
1-{(RS)-(4-chlorophényl)[4-(pipérazin-2-one-4-yl-méthyl)phényl]méthyl}-3-[(3,5-difluorophényl)(méthylsulfonyl)méthylène]azétidine,
1-{(R)-(4-chlorophényl)[4-(pipérazin-2-one-4-yl-méthyl)phényl]méthyl}-3-[(3,5-difluorophényl)(méthylsulfonyl)méthylène]azétidine,
1-{(S)-(4-chlorophényl)[4-(pipérazin-2-one-4-yl-méthyl)phényl]méthyl}-3-[(3,5-difluorophényl)(méthylsulfonyl)méthylène]azétidine,
1-{(RS)-(4-chlorophényl)[4-(imidazol-1-yl-méthyl)phényl]méthyl}-3-[(3,5-difluorophényl)(méthylsulfonyl)méthylène]azétidine,
1-{(R)-(4-chlorophényl)[4-(imidazol-1-yl-méthyl)phényl]méthyl}-3-[(3,5-difluorophényl)(méthylsulfonyl)méthylène]azétidine,
1-{(S)-(4-chlorophényl)[4-(imidazol-1-yl-méthyl)phényl]méthyl}-3-[(3,5-difluorophényl)(méthylsulfonyl)méthylène] azétidine,
(RS)-1-{(4-chlorophényl)[4-(N,N-diméthylcarbamoyl)phényl]méthyl}-3-[(3,5-difluorophényl)(méthylsulfonyl)méthylène]azétidine,
(R)-1-{(4-chlorophényl)[4-(N,N-diméthylcarbamoyl)phényl]méthyl}-3-[(3,5-difluorophényl)(méthylsulfonyl)méthylène]azétidine,
(S)-1-{(4-chlorophényl)[4-(N,N-diméthylcarbamoyl)phényl]méthyl}-3-[(3,5-difluorophényl)(méthylsulfonyl)méthylène]azétidine,
(RS)-1-{(4-chlorophényl)[4-(N-éthylcarbamoyl)phényl]méthyl)}-3-[(3,5-difluorophényl)(méthylsulfonyl)méthylène]azétidine,
(R)-1-{(4-chlorophényl)[4-(N-éthylcarbamoyl)phényl]méthyl}-3-[(3,5-difluorophényl)(méthylsulfonyl)méthylène]azétidine,
(S)-1-{(4-chlorophényl)[4-(N-éthylcarbamoyl)phényl]méthyl}-3-[(3,5-difluorophényl)(méthylsulfonyl)méthylène]azétidine,
(RS)-1-[(4-carbamoylphényl)(4-chlorophényl)méthyl]-3-[(3,5-difluorophényl) (méthylsulfonyl)méthylène]azétidine,
(R)-1-[(4-carbamoylphényl)(4-chlorophényl)méthyl]-3-[(3,5-difluorophényl)(méthylsulfonyl)méthylène]azétidine,
(S)-1-[(4-carbamoylphényl)(4-chlorophényl)méthyl]-3-[(3,5-difluorophényl) (méthylsulfonyl)méthylène]azétidine,
1-[bis(4-chlorophényl)méthyl]-3-[(3,5-dichlorophényl)(méthylsulfonyl)méthylène] azétidine,
1-benzhydryl-3-[(3-méthylsulfanylphényl)(méthylsulfonyl)méthylène]azétidine,
1-benzhydryl-3-[(3-méthylsulfanylméthyl)phényl)](méthylsulfonyl)méthylène]azétidine,
1-[bis(4-chlorophényl)méthyl]-3-[(3-cyanophényl)(méthylsulfonyl)méthylène]azétidine,
1-[bis(4-chlorophényl)méthyl]-3-[(3-carbamoylphényl)(méthylsulfonyl)méthylène]azétidine,
1-[bis(4-chlorophényl)méthyl]-3-[(3-méthoxyphényl)(méthylsulfonyl)méthylène] azétidine,
1-[bis(4-chlorophényl)méthyl]-3-[(3-hydroxyphényl)(méthylsulfonyl)méthylène] azétidine,
1-[bis(4-chlorophényl)méthyl]-3-[(méthylsulfonyl)(3-pyrrolidinylphényl)méthylène] azétidine,
1-[bis(4-chlorophényl)méthyl]-3-[(3-hydroxyméthylphényl)(méthylsulfonyl)méthylène]azétidine,
1-[bis(4-chlorophényl)méthyl] 3 - {(méthylsulfonyl)[3-(N-pipéridylcarbamoyl)phényl] méthylène }azétidine,
1-[bis(4-chlorophényl)méthyl]-3-[(méthylsulfonyl)(3-trifluorométhylsulfanylphényl)(méthylsulfonyl)méthylène]azétidine,
1-[bis(4-fluorophényl)méthyl]-3-[(3,5-difluorophényl)(méthylsulfonyl)méthylène] azétidine,
1-[bis(2-fluorophényl)méthyl]-3-[(3,5-difluorophényl)(méthylsulfonyl)méthylène] azétidine,
1-[bis(3-fluorophényl)méthyl] -3-[(3,5 -difluorophényl)(méthylsulfonyl)méthylène] azétidine,
(RS)-1-[(4-chlorophényl)(thiazol-2-yl)méthyl]-3-[(méthylsulfonyl)(phényl)méthylène]azétidine,
(R)-1-[(4-chlorophényl)(thiazol-2-yl)méthyl]-3-[(méthylsulfonyl)(phényl)méthylène] azétidine,
(S)-1-[(4-chlorophényl)(thiazol-2-yl)méthyl]-3-[(méthylsulfonyl)(phényl)méthylène] azétidine,
(RS)-1-[(4-chlorophényl)(thién-2-yl)méthyl]-3-[(3,5-difluorophényl)(méthylsulfonyl)méthylène]azétidine,
(R)-1-[(4-chlorophényl)(thién-2-yl)méthyl]-3-[(3,5-difluorophényl)(méthylsulfonyl) méthylène]azétidine,
(S)-1-[(4-chlorophényl)(thién-2-yl)méthyl]-3-[(3,5-difluorophényl)(méthylsulfonyl) méthylène]azétidine,
1-benzhydryl-3-[(éthylsulfonyl)(phényl)méthylène]azétidine,
1-[bis(4-chlorophényl)méthyl]-3- {{3-[N-(4-méthylpipérazinyl)carbamoyl]phényl} (méthylsulfonyl)méthylène}azétidine,
1-[bis(4-chlorophényl)méthyl]-3-{[3-(2,2-diméthylcarbohydrazido)phényl](méthylsulfonyl)méthyléne}azétidine,
1-[bis(thién-2-yl)méthyl]-3-[(3,5-difluorophényl)(méthylsulfonyl)méthylène]azétidine,
1-[bis(p-tolyl)méthyl]-3-[(méthylsulfonyl)(phényl)méthylène]azétidine,
1-[(4-chlorophényl)(4-hydroxyméthylphényl)méthyl]-3-[(3,5-difluorophényl) (méthylsulfonyl)méthylène]azétidine,
1-[bis(4-chlorophényl)méthyl]-3-[(3-méthylaminophényl)(méthylsulfonyl)méthylène]azétidine,
(RS)-1-[(4-chlorophényl)(thiazol-2-yl)méthyl]-3-[(3,5-difluorophényl)(méthylsulfonyl)méthylène]azétidine,
(R)-1-[(4-chlorophényl)(thiazol-2-yl)méthyl]-3-[(3,5-difluorophényl)(méthylsulfonyl)méthylène] azétidine,
(S)-1-[(4-chlorophényl)(thiazol-2-yl)méthyl]-3-[(3,5-difluorophényl)(méthylsulfonyl)méthylène]azétidine,
1-[bis(4-chlorophényl)méthyl]-3-[(méthylsulfonyl)(2-méthoxycarbonylthién-5-yl)méthylène]azétidine,
1-[bis(4-chlorophényl)méthyl]-3-hydroxy-3-[(méthylsulfonyl)(2-méthoxycarbonylthién-5-yl)méthyl]azétidine-(RS),
1-[bis(4-chlorophényl)méthyl]-3-[(2-isobutylaminocarbonylthién-5-yl)(méthylsulfonyl)méthylène]azétidine,
1-[bis(4-chlorophényl)méthyl]-3-[(3-méthoxycarbonylphényl)(méthylsulfonyl) méthyl-(RS)]azétidin-3-ol,
1-[bis(4-chlorophényl)méthyl]-3-[(méthylsulfonyl)(pyridin-4-yl)méthyl-(RS)]azétidin-3-ol,
1-[bis(4-chlorophényl)méthyl]-3-[(méthylsulfonyl)(pyridin-3-yl)méthyl-(RS)]azétidin-3-ol,
3-({1-[bis-(4-chlorophényl)méthyl]azétidin-3-ylidène}-méthanesulfonyl-méthyl)N-(3-morpholin-4-yl-propyl)benzamide,
3-({1-[bis-(4-chlorophényl)méthyl]azétidin-3-ylidène}-méthanesulfonyl-méthyl)N-(3 -diméthylamino-propyl)benzamide,
3-({1-[bis-(4-chlorophényl)méthyl]azétidin-3-ylidène}-méthanesulfonyl-méthyl)N-(2-pyrrolidin-1-yl-éthyl)benzamide,
3-({1-[bis-(4-chlorophényl)méthyl]azétidin-3-ylidène}-méthanesulfonyl-méthyl)N-(2-diméthylamino-1-méthyl-éthyl)benzamide,
3-({1-[bis-(4-chlorophényl)mèthyl]azétidin-3-ylidène}-méthanesulfonyl-méthyl)N-pipéridin-1-yl-benzamide,
3-({1-[bis-(4-chlorophényl)méthyl]azétidin-3-ylidène}-méthanesulfonyl-méthyl)N-isobutyl-benzamide,
3-({1-[bis-(4-chlorophényl)méthyl]azétidin-3-ylidène}-méthanesulfonyl-méthyl)N-(3-imidazol-1-yl-propyl)benzamide,
3-({1-[bis-(4-chlorophényl)méthyl]azétidin-3-ylidène}-méthanesulfonyl-méthyl)N-(2-diméthylamino-éthyl)benzamide,
N'-méthyl-hydrazide de l'acide 3-({1-[bis-(4-chlorophényl)méthyl]azétidin-3-ylidène} -méthanesulfonyl-méthyl)benzoïque,
3-({1-[bis-(4-chlorophényl)méthyl]azétidin-3-ylidène} -méthanesulfonyl-méthyl)N-(2-morpholin-4-yl-éthyl)benzamide,
3-({1-[bis-(4-chlorophényl)méthyl]azétidin-3-ylidène}-méthanesulfonyl-méthyl)N-(1-éthyl-pyrrolidin-2-ylméthyl)benzamide,
3-({1-[bis-(4-chlorophényl)méthyl]azétidin-3-ylidène} -méthanesulfonyl-méthyl)N-(2,2-diméthyl-propyl)benzamide,
3-({1-[bis-(4-chlorophényl)méthyl]azétidin-3-ylidène}-méthanesulfonyl-méthyl)N-cyclohexylméthyl-benzamide,
3-({1-[bis-(4-chlorophényl)méthyl]azétidin-3-ylidène}-méthanesulfonyl-méthyl)N-cyclopropylméthyl-benzamide,
3-({1-[bis-(4-chlorophényl)méthyl]azétidin-3-ylidène}-méthanesulfonyl-méthyl)N-(2-méthyl-butyl)benzamide,
3-({1-[bis-(4-chlorophényl)méthyl]azétidin-3-ylidène} -méthanesulfonyl-méthyl)N-(2-phényl-propyl)benzamide,
3-({1-[bis-(4-chlorophényl)méthyl]azétidin-3-ylidène}-méthanesulfonyl-méthyl)N-(tetrahydro-furan-2-ylméthyl)benzamide,
3 -( {1-[bis-(4-chlorophényl)méthyl]azétidin-3-ylidène} -méthanesulfonyl-méthyl)N-(2,2-diphényl-éthyl)benzamide,
3-({1-[bis-(4-chlorophényl)méthyl]azétidin-3-ylidène}-méthanesulfonyl-méthyl)N-(2-éthyl-butyl)benzamide,
ester méthylique de l'acide 4-{[3-({1-[bis-(4-chlorophényl)méthyl]azétidin-3-ylidène}-méthanesulfonyl-méthyl)benzoylamino]méthyl}-cyclohexanecarboxylique,
2-amino-1-{4-[3-({1-[bis-(4-chlorophényl)méthyl]azétidin-3-ylidène}-méthanesulfonyl-méthyl)phényl]pipérazin-1-yl}-éthanone,
ester tert-butylique de l'acide (2-{4-[3-({1-[bis-(4-chlorophényl)méthyl]azétidin-3-ylidène }-méthanesulfonyl-méthyl)phényl]pipérazin-1-yl}-2-oxo-éthyl)carbamique,
1-{4-[3-({1-[bis-(4-chlorophényl)méthyl]azétidin-3-ylidène}-méthanesulfonylméthyl)phényl]pipérazin- 1-yl}-2-méthylamino-éthanone,
ester ter-butylique de l'acide (2-{4-[3-({1-[bis-(4-chlorophényl)méthyl]azétidin-3-ylidène}-méthanesulfonyl-méthyl)phényl]pipérazin-1-yl}-2-oxo-éthyl)N-méthylcarbamique,
N-méthylamide de l'acide 4-[3-({1-[bis-(4-chlorophényl)méthyl]azétidin-3-ylidène}-méthanesulfonyl-méthyl)phényl]pipérazine-1-carbothioic,
N-méthylamide de l'acide 4-[3-({1-[bis-(4-chlorophényl)méthyl]azétidin-3-ylidène}-méthanesulfonyl-méthyl)phényl]pipérazine-1-carboxylique,
ester de méthyl de l'acide 4-[3-({1-[bis-(4-chlorophényl)méthyl]azétidin-3-ylidène}-méthanesulfonyl-méthyl)phényl]pipérazine-1-carboxylique,
1-[3-({1-[bis-(4-chlorophényl)méthyl]azétidin-3-ylidène}-méthanesulfonyl-méthyl)phényl]-4-isobutyl-pipérazine,
1-[3-({1-[bis-(4-chlorophényl)méthyl]azétidin-3-ylidène}-méthanesulfonyl-méthyl)phényl]-4-éthyl-pipérazine,
4-acétyl 1-[3-({1-[bis-(4-chlorophényl)méthyl]azétidin-3-ylidène}-méthanesulfonyl-méthyl)phényl]pipérazine,
1-{4-[3-({1-[bis-(4-chlorophényl)méthyl]azétidin-3-ylidène}-méthanesulfonyl-méthyl)phényl]pipérazin-1-yl}-2-diméthylamino-éthanone,
1-[3-({1-[bis-(4-chlorophényl)méthyl]azétidin-3-ylidène}-méthanesulfonyl-méthyl)phényl]pipérazine,
ester tert-butylique de l'acide 4-[3-({1-[bis-(4-chlorophényl)méthyl]azétidin-3-ylidène }-méthanesulfonyl-méthyl)phényl]pipérazine-1-carboxylique,
1-[bis(4-méthoxycarbonylphényl)méthyl]-3-[(3,5-difluorophényl)(méthylsulfonyl) méthylène]azétidine,
3-acétoxy-1-[bis(4-méthoxycarbonylphényl)méthyl]-3-[(3,5-difluorophényl) (méthylsulfonyl)méthyl-(RS)]azétidine,
(RS)-4-[4-((4-chlorophényl) {3-[(3,5-dilluorophényl)méthanesulfonyl-méthylene] azétidin-1-yl}-méthyl)benzyl]morpholine,
4-(4- {3-[(1-benzhydryl-azétidin-3-ylidene)méthanesulfonyl-méthyl]phénoxy}butyl)morpholine,
4-(4-{3-[(1-benzhydryl-azétidin-3-ylidene)méthanesulfonyl-méthyl]phénoxy}-propyl)morpholine,
leurs isomères optiques et leurs sels.

Parmi ces composés sont particulièrement préférés les composés suivants :
1-[bis(4-chlorophényl)méthyl]-3-[(3,5-difluorophényl)(méthylsulfonyl)méthylène] azétidine,
1-[bis(4-chlorophényl)méthyl]-3-[(3,5-difluorophényl)(méthylsulfonyl)méthyl-(RS)]azétidin-3-ol,
3-acétoxy-1-[bis-(4-chlorophényl)méthyl]-3-[(3,5-difluorophényl)(méthyl-ulfonyl)méthyl)méthyl sulfonylméthyl-(RS)]azétidine
leurs isomères optiques et leurs sels avec un acide minéral ou organique.

Les exemples suivants illustrent l'invention sans la limiter.

### Exemple 1

A une solution de 1 g de 1-benzhydryl-3-[(méthylsulfonyl)(phényl)méthyl-(RS)]azétidin-3-ol dans 10 cm³ de pyridine, refroidie à 5°C, on ajoute 0,3 cm³ de chlorure de méthanesulfonyle. On agite 2 heures à 5°C puis on ajoute 1 g de 4-diméthylamino pyridine dans 10 cm³ de dichlorométhane à 5°C. La solution est agitée 15 heures à température ambiante puis concentrée à sec sous pression réduite (2,7 kPa). Le résidu obtenu est chromatographié sur colonne de gel de silice (granulométrie 0,04-0,06 mm, diamètre 3 cm, hauteur 25 cm), en éluant sous une pression de 0,5 bar d'azote par du dichlorométhane et en recueillant des fractions de 80 cm³. Les fractions 17 à 20 sont réunies puis concentrées à sec sous pression réduite (2,7 kPa). Le résidu est cristallisé dans 10 cm³ d'éther éthylique. On obtient 0,14 g de 1-benzhydryl-3-[(méthylsulfonyl)(phényl)méthylène]azétidine fondant à 210°C [Spectre RMN dans DMSO-d6, T=300K, δ en ppm (300 MHz) : 2,95 (3H, s, SCH₃), 3,80 (2H, s, NCH₂), 4,20 (2H, s, NCH₂), 4,75 (1H, s, NCH), 7,20 (2H, t, J=7Hz, 2CH arom.), 7,30 (4H, t, J=7Hz, 4CH arom.), entre 7,40 et 7,60 (9H, m, 9 CH arom.)].

Le 1-benzhydryl-3-[(méthylsulfonyl)(phényl)méthyl-(RS)]azétidin-3-ol peut être obtenu de la manière suivante : à une solution de 1,4 cm³ de diisopropylamine dans 10 cm³ de tétrahydrofuranne, sous atmosphère d'argon, refroidie à 0°C, on ajoute 6,25 cm³ de n-butyl lithium 1,6N en solution dans l'hexane puis refoidie à -70°C. On ajoute ensuite un mélange de 1,7 g de benzyl méthyl sulfone dans 30 cm³ de tétrahydrofuranne et maintient l'agitation 45 minutes à -70°C. On additionne 2,4 g de 1-benzhydryl azétidin-3-one puis agite 20 minutes en laissant le mélange revenir à température ambiante. Le mélange réactionnel est filtré, puis concentré à sec sous pression réduite (2,7 kPa). Le résidu est repris par 50 cm³ d'acétate d'éthyle, 30 cm³ d'eau et 20 cm³ d'acide chlorhydrique normal. Le précipité est filtré, lavé par 30 cm³ d'eau distillée, essoré et séché. On obtient 2 g de 1-benzhydryl-3-[(méthylsulfonyl)(phényl)méthyl-(RS)]azétidin-3-ol fondant à 260°C.

La 1-benzhydryl azétidin-3-one peut être préparée selon le mode opératoire décrit par KATRITZKY A.R. et coll. dans J. Heterocycl. Chem., 271 (1994).

### Exemple 2

En opérant selon le mode opératoire de l'exemple 1 à partir de 1,9 g de 1-benzhydryl-3-[(3-méthylphényl)(méthylsulfonyl)méthyl-(RS)]azétidin-3-ol, de 0,52 cm³ de chlorure de méthanesulfonyle et de 1,7 g de 4-diméthylaminopyridine, le résidu obtenu est purifié par chromatographie sur colonne de gel de silice (granulométrie 0,04-0,06 mm, diamètre 3 cm, hauteur 17 cm) sous une pression de 0,5 bar d'azote avec du dichlorométhane puis un mélange dichlorométhane et éthanol (98,5/1,5 en volumes) comme éluants et en recueillant des fractions de 100 cm³. Les fractions 5 et 6 sont réunies et concentrées à sec sous pression réduite (2,7 kPa). Le solide obtenu est cristallisé dans 2 cm³ de dichlorométhane et 20 cm³ d'oxyde de diisopropyle. On obtient 0,9 g de 1-benzhydryl-3-[(3-méthylphényl)(méthylsulfonyl)méthylène]azétidine fondant à 180°C [Spectre RMN dans DMSO-d6, T=300K, δ en ppm (300 MHz) : 2,35 (3H, s, PhCH₃), 2,95 (3H, s, SCH₃), 3,80 (2H, s, NCH₂), 4,20 (2H, s, NCH₂), 4,75 (1H, s, NCH), 7,20 (5H, m, 5CH arom.), 7,30 (5H, t, J=7Hz, 5CH arom.), 7,50 (4H, d, J=7Hz, 4 CH arom.)].

Le 1-benzhydryl-3-[(3-méthylphényl)(méthylsulfonyl)méthyl-(RS)]azétidin-3-ol peut être obtenu de la manière suivante : en opérant selon le mode opératoire de l'exemple 1 à partir de 2,8 g de méthyl(3-méthylbenzyl)sulfone et de 3,6 g de 1-benzhydryl azétidin-3-one, on obtient après purification sur colonne de gel de silice (granulométrie 0,04-0,06 mm, diamètre 3 cm, hauteur 25 cm) sous une pression de 0,5 bar d'azote avec un mélange dichlorométhane et éthanol (98,5/1,5 en volumes) comme éluant, 2,6 g d'un solide. Celui-ci est repris par 25 cm³ d'oxyde de diisopropyle. Après filtration, essorage et séchage, on obtient 1,9 g de 1-benzhydryl-3-[(3-méthylphényl)(méthylsulfonyl)méthyl-(RS)]azétidin-3-ol fondant à 170°C.

La méthyl(3-méthylbenzyl)sulfone peut être préparée de la manière suivante : à une solution de 30 cm³ d'eau, 30 cm³ d'acide acétique et 15 cm³ d'acide sulfurique 36N, on ajoute, à température ambiante, 10,5 g d'oxone^{R} puis 2,6 g de méthyl(3-méthylbenzyl)sulfure et 30 cm³ d'éthanol. Le mélange est agité 48 heures à température ambiante puis repris par 100 cm³ d'eau et 100 cm³ d'acétate d'éthyle. La phase organique est lavée par une solution aqueuse saturée de bicarbonate de sodium, décantée, séchée sur sulfate de magnésium et concentrée à sec sous pression réduite (2,7 kPa). On obtient 2,8 g de méthyl(3-méthylbenzyl)sulfone sous forme d'une gomme.

Le méthyl(3-méthylbenzyl)sulfure peut être préparé de la manière suivante : à une solution de 3,7 g de bromure de 3-méthylbenzyle dans 25 cm³ de diméthylformamide, on ajoute en maintenant la température inférieure à 30°C, 1,7 g de méthylthiolate de sodium. Le mélange est agité 2 heures à une température proche de 20°C puis repris par 50 cm³ d'acétate d'éthyle. La phase organique est lavée par 3 fois 100 cm³ d'eau, séchée sur sulfate de magnésium et concentrée à sec sous pression réduite (2,7 kPa). On obtient 2,6 g de méthyl(3-méthylbenzyl)sulfure sous forme d'une huile.

### Exemple 3

A une solution de 3,3 g de 1-benzhydryl-3-[(4-méthylphényl)(méthylsul-fonyl)méthyl-(RS)]azétidin-3-ol dans 10 cm³ de pyridine, refroidie à 5°C, on ajoute 0,3 cm³ de chlorure de méthanesulfonyle. On agite 2 heures à 5°C puis on ajoute 1 g de 4-diméthylamino pyridine dans 10 cm³ de dichlorométhane à 5°C. La solution est agitée 15 heures à température ambiante puis concentrée à sec sous pression réduite (2,7 kPa). Le résidu obtenu est chromatographié sur colonne de gel de silice (granulométrie 0,04-0,06 mm, diamètre 3 cm, hauteur 25 cm), en éluant sous une pression de 0,5 bar d'azote avec du dichlorométhane et en recueillant des fractions de 80 cm³. Les fractions 17 à 20 sont réunies puis concentrées à sec sous pression réduite (2,7 kPa). Le solide obtenu est cristallisé dans 30 cm³ d'acétonitrile. On obtient 0,14 g de 1-benzhydryl-3-[(4-méthylphényl)(méthylsulfonyl)méthylène]azétidine fondant à 210°C [Spectre RMN dans DMSO-d6, T=300K, δ en ppm (300 MHz) : 2,30 (3H, s, PhCH₃), 2,95 (3H, s, SCH₃), 3,80 (2H, s, NCH₂), 4,20 (2H, s, NCH₂), 4,75 (1H, s, NCH), 7,20 (4H, m, 4CH arom.), 7,30 (6H, t, J=7Hz, 6CH arom.), 7,45 (4H, d, J=7Hz, 4 CH arom.)].

Le 1-benzhydryl-3-[(4-méthylphényl)(méthylsulfonyl)méthyl-(RS)]azétidin-3-ol peut être obtenu de la manière suivante : en opérant selon le mode opératoire de l'exemple 1 à partir de 4 g de méthyl(4-méthylbenzyl)sulfone et 5,1 g de 1-benzhydryl azétidin-3-one, on obtient 3 g de 1-benzhydryl-3-[(4-méthylphényl)(méthylsulfonyl)méthyl-(RS)]azétidin-3-ol fondant à 226°C.

La méthyl(4-méthylbenzyl)sulfone peut être préparée de la manière suivante : en opérant selon le mode opératoire de l'exemple 2 à partir de 3,5 g de méthyl(4-méthylbenzyl)sulfure et 12,3 g d'oxone^{R}, on obtient 3,5 g de méthyl(4-méthylbenzyl)sulfone sous forme d'un solide.

Le méthyl(4-méthylbenzyl)sulfure peut être préparé de la manière suivante : en opérant selon le mode opératoire de l'exemple 2 à partir de 5,6 g de bromure de 4-méthylbenzyle et 2,3 g de méthyl thiolate de sodium, on obtient 4,7 g de méthyl(4-méthylbenzyl)sulfure sous forme d'un solide.

### Exemple 4

A une solution de 3,3 g de 1-benzhydryl-3-[(2-méthylphényl)(méthylsulfonyl)méthyl-(RS)]azétidin-3-ol dans 50 cm³ de dichlorométhane, à température ambiante, on ajoute 0,7 cm³ de chlorure de méthanesulfonyle puis 3,8 g de 4-diméthylaminopyridine. La solution est agitée 3 heures au reflux puis reprise par 2 fois 50 cm³ d'eau. La phase organique est décantée, séchée et concentrée à sec sous pression réduite (2,7 kPa). Le résidu obtenu est chromatographié sur colonne de gel de silice (granulométrie 0,04-0,06 mm, diamètre 3 cm, hauteur 25 cm), en éluant sous une pression de 0,5 bar d'azote par du dichlorométhane puis par un mélange dichlorométhane et éthanol (mélange 99/1 en volumes) et en recueillant des fractions de 100 cm³. Les fractions 6 à 17 sont réunies puis concentrées à sec sous pression réduite (2,7 kPa). Le solide obtenu est cristallisé dans 50 cm³ d'éther éthylique. On obtient 2,6 g de 1-benzhydryl-3-[(2-méthylphényl)(méthylsulfonyl)méthylène]azétidine sous forme de meringue [Spectre RMN dans DMSO-d6, T=300K, δ en ppm (300 MHz) : 2,30 (3H, s, PhCH₃), 2,95 (3H, s, SCH₃), 3,50 (2H, s, NCH₂), 4,20 (2H, s, NCH₂), 4,70 (1H, s, NCH), entre 7,10 et 7,35 (10H, m, 10CH arom.), 7,45 (4H, m, 4CH arom.)].

Le 1-benzhydryl-3-[(2-méthylphényl)(méthylsulfonyl)méthyl-(RS)]azétidin-3-ol peut être obtenu de la manière suivante : en opérant selon le mode opératoire de l'exemple 1 à partir de 3,4 g de méthyl(2-méthylbenzyl)sulfone et 4,3 g de 1-benzhydryl azétidin-3-one, on obtient 3,4 g de 1-benzhydryl-3-[(2-méthylphényl)(méthylsulfonyl)méthyl-(RS)]azétidin-3-ol fondant à 218°C.

La méthyl(2-méthylbenzyl)sulfone peut être préparée de la manière suivante : en opérant selon le mode opératoire de l'exemple 2 à partir de 4,5 g de méthyl(2-méthylbenzyl)sulfure et 16,2 g d'oxone^{R}, on obtient 3,4 g de méthyl(2-méthylbenzyl)sulfone sous forme d'un solide.

Le méthyl(2-méthylbenzyl)sulfure peut être préparé de la manière suivante : en opérant selon le mode opératoire de l'exemple 2 à partir de 5,6 g de bromure de 2-méthylbenzyle et 2,1 g de méthyl thiolate de sodium, on obtient 4,5 g de méthyl(2-méthylbenzyl)sulfure sous forme d'un solide.

### Exemple 5

En opérant selon le mode opératoire de l'exemple 4 à partir de 2,1 g de 1-benzhydryl-3-[(2-chlorophényl)(méthylsulfonyl)méthyl-(RS)]azétidin-3-ol, de 0,55 cm³ de chlorure de méthanesulfonyle et 2,3 g de 4-diméthylaminopyridine, le résidu obtenu est purifié par chromatographie sur colonne de gel de silice (granulométrie 0,04-0,06 mm, diamètre 3 cm, hauteur 25 cm) sous une pression de 0,5 bar d'azote avec du dichlorométhane comme éluant et en recueillant des fractions de 100 cm³. Les fractions 12 à 18 sont réunies et concentrées à sec sous pression réduite (2,7 kPa). Le solide obtenu est cristallisé dans un mélange de 3 cm³ de dichlorométhane et 40 cm³ d'éther éthylique. On obtient 1,1 g de 1-benzhydryl-3-[(2-chlorophényl)(méthylsulfonyl)méthylène]azétidine fondant à 204°C [Spectre RMN dans DMSO-d6, T=300K, δ en ppm (300 MHz) : 2,95 (3H, s, SCH₃), 3,60 (2H, s, NCH₂), 4,20 (2H, s, NCH₂), 4,70 (1H, s, NCH), 7,20 (2H, t, J=7Hz, 2CH arom.), 7,30 (4H, t, J=7Hz, 4CH arom.), 7,45 (7H, m, 7CH arom.), 7,55 (1H, d, J=7Hz, CH arom.)].

Le 1-benzhydryl-3-[(2-chlorophényl)(méthylsulfonyl)méthyl-(RS)]azétidin-3-ol peut être obtenu de la manière suivante : en opérant selon le mode opératoire de l'exemple 1 à partir de 4 g de (2-chlorobenzyl)méthylsulfone et 4,6 g de 1-benzhydryl azétidin-3-one, le résidu obtenu est repris par 50 cm³ d'acétate d'éthyle, filtré et séché. On obtient 2,4 g de 1-benzhydryl-3-[(2-chlorophényl)(méthylsulfonyl)méthyl-(RS)]azétidin-3-ol sous forme d'un solide blanc.

La (2-chlorobenzyl)méthylsulfone peut être préparée de la manière suivante : en opérant selon le mode opératoire de l'exemple 2 à partir de 3,4 g de (2-chlorobenzyl)méthylsulfure et 12 g d'oxone^{R}, on obtient 4 g de (2-chlorobenzyl)méthylsulfone sous forme d'une huile qui cristallise.

Le (2-chlorobenzyl)méthylsulfure peut être préparé de la manière suivante : en opérant selon le mode opératoire de l'exemple 2 à partir de 4 g de bromure de 2-chlorobenzyle et 1,5 g de méthyl thiolate de sodium, on obtient 3,4 g de (2-chlorobenzyl)méthylsulfure sous forme d'une huile.

### Exemple 6

En opérant selon le mode opératoire de l'exemple 4 à partir de 3 g de 1-benzhydryl-3-[(3-chlorophényl)(méthylsulfonyl)méthyl-(RS)]azétidin-3-ol, de 0,79 cm³ de chlorure de méthanesulfonyle et 3,3 g de 4-diméthylaminopyridine, le résidu obtenu est purifié par chromatographie sur colonne de gel de silice (granulométrie 0,04-0,06 mm, diamètre 3 cm, hauteur 25 cm) sous une pression de 0,5 bar d'azote avec du dichlorométhane comme éluant et en recueillant des fractions de 100 cm³. Les fractions 2 à 5 sont réunies et concentrées à sec sous pression réduite (2,7 kPa). Le solide obtenu est cristallisé dans 40 cm³ d'éther éthylique. On obtient 1,7 g de 1-benzhydryl-3-[(3-chlorophényl)(méthylsulfonyl)méthylène]azétidine fondant à 205°C [Spectre RMN dans DMSO-d6, T=300K, δ en ppm (300 MHz) : 2,95 (3H, s, SCH₃), 3,80 (2H, s, NCH₂), 4,20 (2H, s, NCH₂), 4,70 (1H, s, NCH), 7,20 (2H, t, J=7Hz, 2CH arom.), 7,30 (4H, t, J=7Hz, 4CH arom.), 7,45 (8H, m, 8CH arom.)].

Le 1-benzhydryl-3-[(3-chlorophényl)(méthylsulfonyl)méthyl-(RS)]azétidin-3-ol peut être obtenu de la manière suivante : en opérant selon le mode opératoire de l'exemple 1 à partir de 3,1 g de (3-chlorobenzyl)méthylsulfone et 3,4 g de 1-benzhydryl azétidin-3-one, on obtient 3,4 g de 1-benzhydryl-3-[(3-chlorophényl)(méthylsulfonyl)méthyl-(RS)]azétidin-3-ol sous forme d'un solide blanc.

La (3-chlorobenzyl)méthylsulfone peut être préparée de la manière suivante : en opérant selon le mode opératoire de l'exemple 2 à partir de 3,2 g de (3-chlorobenzyl)méthylsulfure et 12 g d'oxone^{R}, on obtient 3,2 g de (3-chlorobenzyl)méthylsulfone sous forme d'un solide blanc.

Le (3-chlorobenzyl)méthylsulfure peut être préparé de la manière suivante : en opérant selon le mode opératoire de l'exemple 2 à partir de 4 g de bromure de 3-chlorobenzyle et 1,5 g de méthyl thiolate de sodium, on obtient 3,2 g de 3-chlorobenzyl méthyl sulfure sous forme d'une huile.

### Exemple 7

En opérant selon le mode opératoire de l'exemple 4 à partir de 3,3 g de 1-benzhydryl-3-[(4-chlorophényl)(méthylsulfonyl)méthyl-(RS)]azétidin-3-ol, de 0,87 cm³ de chlorure de méthanesulfonyle et 3,6 g de 4-diméthylaminopyridine, le résidu obtenu est purifié par chromatographie sur colonne de gel de silice (granulométrie 0,04-0,06 mm, diamètre 3 cm, hauteur 25 cm) sous une pression de 0,5 bar d'azote avec du dichlorométhane comme éluant et en recueillant des fractions de 100 cm³. Les fractions 8 à 12 sont réunies et concentrées à sec sous pression réduite (2,7 kPa). Le solide obtenu est cristallisé dans un mélange de 3 cm³ et 30 cm³ d'éther éthylique. On obtient 0,5 g de 1-benzhydryl-3-[(4-chlorophényl)(méthylsulfonyl)méthylène]azétidine fondant à 192°C [Spectre RMN dans DMSO-d6, T=300K, δ en ppm (300 MHz) : 2,95 (3H, s, SCH₃), 3,80 (2H, s, NCH₂), 4,20 (2H, s, NCH₂), 4,70 (1H, s, NCH), 7,20 (2H, t, J=7Hz, 2CH arom.), 7,30 (4H, t, J=7Hz, 4CH arom.), entre 7,40 et 7,55 (8H, m, 8CH arom.)].

Le 1-benzhydryl-3-[(4-chlorophényl)(méthylsulfonyl)méthyl-(RS)]azétidin-3-ol peut être obtenu de la manière suivante : en opérant selon le mode opératoire de l'exemple 1 à partir de 2,8 g de (4-chlorobenzyl)méthylsulfone et 3,24 g de 1-benzhydryl azétidin-3-one, on obtient après cristallisation dans 80 cm³, 3,4 g de 1-benzhydryl-3-[(4-chlorophényl)(méthylsulfonyl)méthyl-(RS)]azétidin-3-ol sous forme d'un solide blanc.

La (4-chlorobenzyl)méthylsulfone peut être préparée de la manière suivante : en opérant selon le mode opératoire de l'exemple 2 à partir de 3,5 g de (4-chlorobenzyl)méthylsulfure et 12,3 g d'oxone^{R}, on obtient 3,5 g de (4-chlorobenzyl)méthylsulfone sous forme d'un solide.

### Exemple 8

En opérant selon le mode opératoire de l'exemple 4 à partir de 3,1 g de 1-benzhydryl-3-[(3,5-dichlorophényl)(méthylsulfonyl)méthyl-(RS)]azétidin-3-ol, de 0,75 cm³ de chlorure de méthanesulfonyle et 3,1 g de 4-diméthylaminopyridine, le résidu obtenu est purifié par chromatographie sur colonne de gel de silice (granulométrie 0,04-0,06 mm, diamètre 3 cm, hauteur 25 cm) sous une pression de 0,5 bar d'azote avec du dichlorométhane comme éluant et en recueillant des fractions de 100 cm³. Les fractions 6 à 10 sont réunies et concentrées à sec sous pression réduite (2,7 kPa). Le solide obtenu est cristallisé dans un mélange de 2 cm³ de dichlorométhane et 30 cm³ d'éther éthylique. On obtient 0,8 g de 1-benzhydryl-3-[(3,5-dichlorophényl)(méthylsulfonyl)méthylène]azétidine fondant à 204°C [Spectre RMN dans DMSO-d6, T=300K, δ en ppm (300 MHz): 2,95 (3H, s, SCH₃), 3,85 (2H, s, NCH₂), 4,20 (2H, s, NCH₂), 4,75 (1H, s, NCH), 7,20 (2H, t, J=7Hz, 2CH arom.), 7,30 (4H, t, J=7Hz, 4CH arom.), 7,45 (6H, m, 6CH arom.), 7,70 (1H, s, CH arom.)].

Le 1-benzhydryl-3-[(3,5-dichlorophényl)(méthylsulfonyl)méthyl-(RS)]azétidin-3-ol peut être obtenu de la manière suivante : en opérant selon le mode opératoire de l'exemple 1 à partir de 4 g de (3,5-dichlorobenzyl)méthylsulfone et 4 g de 1-benzhydrylazétidin-3-one, on obtient 3,2 g de 1-benzhydryl-3-[(3,5-dichlorophényl)(méthylsulfonyl)méthyl-(RS)]azétidin-3-ol sous forme d'un solide blanc.

La (3,5-dichlorobenzyl)méthylsulfone peut être préparée de la manière suivante : en opérant selon le mode opératoire de l'exemple 2 à partir de 5,3 g de (3,5-dichlorobenzyl)méthylsulfure et 17g d'oxone^{R}, on obtient 5 g de (3,5-dichlorobenzyl)méthylsulfone sous forme d'un solide blanc.

Le (3,5-dichlorobenzyl)méthylsulfure peut être préparé de la manière suivante : en opérant selon le mode opératoire de l'exemple 2 à partir de 5 g de chlorure de 3,5-dichlorobenzyle et 2 g de méthyl thiolate de sodium, on obtient 5,3 g de (3,5-dichlorobenzyl)méthylsulfure sous forme d'une huile.

### Exemple 9

En opérant selon le mode opératoire de l'exemple 4 à partir de 5 g de 1-benzhydryl-3-[(3,4-dichlorophényl)(méthylsulfonyl)méthyl-(RS)]azétidin-3-ol, de 1,2 cm³ de chlorure de méthanesulfonyle et 3,8 g de 4-diméthylaminopyridine, le résidu obtenu est purifié par chromatographie sur colonne de gel de silice (granulométrie 0,04-0,06 mm, diamètre 4 cm, hauteur 35 cm) sous une pression de 0,5 bar d'azote avec un mélange cyclohexane et acétate d'éthyle (70/30 en volumes) comme éluant et en recueillant des fractions de 35 cm³. Les fractions 30 à 55 sont réunies et concentrées à sec sous pression réduite (2,7 kPa). Le solide obtenu est cristallisé dans 50 cm³ d'éther éthylique. On obtient 1,5 g de 1-benzhydryl-3-[(3,4-dichlorophényl)(méthylsulfonyl)méthylène]azétidine fondant à 170°C [Spectre RMN dans DMSO-d6, T=300K, δ en ppm (300 MHz) : 2,95 (3H, s, SCH₃), 3,80 (2H, s, NCH₂), 4,20 (2H, s, NCH₂), 4,70 (1H, s, NCH), 7,20 (2H, t, J=7Hz, 2CH arom.), 7,30 (4H, t, J=7Hz, 4CH arom.), entre 7,35 et 7,50 (5H, m, 5CH arom.), 7,65 (2H, m, 2CH arom.)].

Le 1-benzhydryl-3-[(3,4-dichlorophényl)(méthylsulfonyl)méthyl-(RS)]azétidin-3-ol peut être obtenu de la manière suivante : en opérant selon le mode opératoire de l'exemple 1 à partir de 4,5 g de (3,4-dichlorobenzyl)méthylsulfone et 4,3 g de 1-benzhydryl azétidin-3-one, on obtient 5 g de 1-benzhydryl-3-[(3,4-dichlorophényl)(méthylsulfonyl)méthyl-(RS)]azétidin-3-ol sous forme d'un solide blanc.

La (3,4-dichlorobenzyl)méthylsulfone peut être préparée de la manière suivante : en opérant selon le mode opératoire de l'exemple 2 à partir de 4,3 g de (3,4-dichlorobenzyl)méthylsulfure et 13 g d'oxone^{R}, on obtient 4,7 g de (3,4-dichlorobenzyl)méthylsulfone sous forme d'un solide blanc.

Le (3,4-dichlorobenzyl)méthylsulfure peut être préparé de la manière suivante : en opérant selon le mode opératoire de l'exemple 2 à partir de 2,8 cm³ de chlorure de 3,4-dichlorobenzyle et 1,5 g de méthyl thiolate de sodium, on obtient 4,3 g de (3,4-dichlorobenzyl)méthylsulfure sous forme d'une huile.

### Exemple 10

En opérant selon le mode opératoire de l'exemple 4 à partir de 1,8 g de 1-benzhydryl-3-[(2,5-dichlorophényl)(méthylsulfonyl)méthyl-(RS)]azétidin-3-ol, de 0,4 cm³ de chlorure de méthanesulfonyle et 1,8 g de 4-diméthylaminopyridine, le résidu obtenu est purifié par chromatographie sur colonne de gel de silice (granulométrie 0,04-0,06 mm, diamètre 3 cm, hauteur 25 cm) sous une pression de 0,5 bar d'azote avec du dichlorométhane comme éluant et en recueillant des fractions de 100 cm³. Les fractions 8 à 14 sont réunies et concentrées à sec sous pression réduite (2,7 kPa). Le solide obtenu est cristallisé dans un mélange de 2 cm³ de dichlorométhane et 30 cm³ d'éther éthylique. On obtient 1,2 g de 1-benzhydryl-3-[(2,5-dichlorophényl)(méthylsulfonyl)méthylène]azétidine fondant à 202°C [Spectre RMN dans DMSO-d6, T=300K, δ en ppm (250 MHz) : 3,00 (3H, s, SCH₃), 3,70 (2H, m, NCH₂), 4,25 (2H, m, NCH₂), 4,70 (1H, s, NCH), 7,20 (2H, t, J=7Hz, 2CH arom.), 7,30 (4H, t, J=7Hz, 4CH arom.), 7,45 (4H, d, J=7Hz, 4CH arom.), entre 7,55 et 7,70 (3H, m, 3CH arom.)].

Le 1-benzhydryl-3-[(2,5-dichlorophényl)(méthylsulfonyl)méthyl-(RS)]azétidin-3-ol peut être obtenu de la manière suivante : en opérant selon le mode opératoire de l'exemple 1 à partir de 1,2 g de (2,5-dichlorobenzyl)méthylsulfone et 1,2 g de 1-benzhydryl azétidin-3-one, on obtient 1,8 g de 1-benzhydryl-3-[(2,5-dichlorophényl)(méthylsulfonyl)méthyl-(RS)]azétidin-3-ol sous forme d'un solide blanc.

La (2,5-dichlorobenzyl)méthylsulfone peut être préparée de la manière suivante : on ajoute, à température ambiante, 1,9 g de méthanesulfinate de sodium à une solution de 2,7 g de chlorure de 2,5-dichlorobenzyle dans 30 cm³ d'éthanol. Le mélange est chauffé au reflux 5 heures, refroidi à température ambiante puis repris par 50 cm³ d'eau et 50 cm³ d'acétate d'éthyle. La phase organique est décantée, lavée par 20 cm³ d'une solution aqueuse saturée par du chlorure de sodium, séchée sur sulfate de magnésium et concentrée à sec sous pression réduite (2,7 kPa). On obtient 1,2 g de (2,5-dichlorobenzyl)méthylsulfone sous forme d'un solide blanc.

### Exemple 11

En opérant selon le mode opératoire de l'exemple 4 à partir de 9,1 g de 1-benzhydryl-3-[(2,4-dichlorophényl)(méthylsulfonyl)méthyl-(RS)]azétidin-3-ol, de 2,2 cm³ de chlorure de méthanesulfonyle et 7 g de 4-diméthylaminopyridine, le résidu obtenu est purifié par chromatographie sur colonne de gel de silice (granulométrie 0,04-0,06 mm, diamètre 5,5 cm, hauteur 30 cm) sous une pression de 0,5 bar d'azote avec du dichlorométhane comme éluant et en recueillant des fractions de 40 cm³. Les fractions 27 à 39 sont réunies et concentrées à sec sous pression réduite (2,7 kPa). Le solide obtenu est cristallisé dans 20 cm³ d'éther éthylique. On obtient 1,5 g de 1-benzhydryl-3-[(2,4-dichlorophényl)(méthylsulfonyl)méthylène]azétidine fondant à 165°C [Spectre RMN dans DMSO-d6, T=300K, δ en ppm (250 MHz) : 3,00 (3H, s, SCH₃), 3,65 (2H, m, NCH₂), 4,25 (2H, m, NCH₂), 4,75 (1H, s, NCH), 7,20 (2H, t, J=7Hz, 2CH arom.), 7,30 (4H, t, J=7Hz, 4CH arom.), 7,45 (6H, m, 6CH arom.), 7,80 (1H, s, CH arom.)].

Le 1-benzhydryl-3-[(2,4-dichlorophényl)(méthylsulfonyl)méthyl-(RS)]azétidin-3-ol peut être obtenu de la manière suivante : en opérant selon le mode opératoire de l'exemple 1 à partir de 4,8 g de (2,4-dichlorobenzyl)méthylsulfone et 4,7 g de 1-benzhydryl azétidin-3-one, on obtient 9,1 g de 1-benzhydryl-3-[(2,4-dichlorophényl)(méthylsulfonyl)méthyl-(RS)]azétidin-3-ol sous forme d'une meringue brune.

La (2,4-dichlorobenzyl)méthylsulfone peut être préparée de la manière suivante : en opérant selon le mode opératoire de l'exemple 2 à partir de 4 g de (2,4-dichlorobenzyl)méthylsulfure et 13 g d'oxone^{R}, on obtient 4,8 g de (2,4-dichlorobenzyl)méthylsulfone sous forme d'un solide blanc fondant à 111°C.

Le (2,4-dichlorobenzyl)méthylsulfure peut être préparé de la manière suivante : en opérant selon le mode opératoire de l'exemple 2 à partir de 2,8 cm³ de chlorure de 2,4-dichlorobenzyle et 1,5 g de méthylthiolate de sodium, on obtient 4 g de (2,4-dichlorobenzyl)méthylsulfure sous forme d'une huile.

### Exemple 12

En opérant selon le mode opératoire de l'exemple 4 à partir de 3 g de 1-benzhydryl-3-[(2,3-dichlorophényl)(méthylsulfonyl)méthyl-(RS)]azétidin-3-ol, de 1,1 g de chlorure de méthanesulfonyle et 3 g de 4-diméthylaminopyridine, le résidu obtenu est purifié par chromatographie sur colonne de gel de silice (granulométrie 0,04-0,06 mm, diamètre 3 cm, hauteur 30 cm) sous une pression de 0,5 bar d'azote avec un mélange dichlorométhane et éthanol (98/2 en volumes) comme éluant et en recueillant des fractions de 100 cm³. Les fractions 10 à 20 sont réunies et concentrées à sec sous pression réduite (2,7 kPa). Le solide obtenu est cristallisé dans 40 cm³ d'éther éthylique. On obtient 1,6 g de 1-benzhydryl-3-[(2,3-dichlorophényl)(méthylsulfonyl)méthylène]azétidine fondant à 201°C [Spectre RMN dans DMSO-d6, T=300K, δ en ppm (300 MHz) : 3,00 (3H, s, SCH₃), 3,60 (2H, m, NCH₂), 4,20 (2H, m, NCH₂), 4,70 (1H, s, NCH), 7,20 (2H, t, J=7Hz, 2CH arom.), 7,30 (4H, t, J=7Hz, 4CH arom.), 7,45 (6H, m, 6CH arom.), 7,70 (1H, dd, J=8 et 2Hz, CH arom.)].

Le 1-benzhydryl-3-[(2,3-dichlorophényl)(méthylsulfonyl)méthyl-(RS)]azétidin-3-ol peut être obtenu de la manière suivante : en opérant selon le mode opératoire de l'exemple 1 à partir de 3,6 g de (2,3-dichlorobenzyl)méthylsulfone et 3,6 g de 1-benzhydryl azétidin-3-one, on obtient 5,4 g de 1-benzhydryl-3-[(2,3-dichlorophényl)(méthylsulfonyl)méthyl-(RS)]azétidino-3-ol sous forme d'un solide blanc.

La (2,3-dichlorobenzyl)méthylsulfone peut être préparée de la manière suivante : en opérant selon le mode opératoire de l'exemple 10 à partir de 3 g de chlorure de 2,3-dichlorobenzyle et 2,4 g de méthanesulfinate de sodium, on obtient 3,6 g de (2,3-dichlorobenzyl)méthylsulfone sous forme d'un solide blanc.

### Exemple 13

En opérant selon le mode opératoire de l'exemple 4 à partir de 2,5 g de 1-benzhydryl-3-[(3-fluorophényl)(méthylsulfonyl)méthyl-(RS)]azétidin-3-ol, de 0,72 cm³ de chlorure de méthanesulfonyle et 2,9 g de 4-diméthylaminopyridine, le résidu obtenu est purifié par chromatographie sur colonne de gel de silice (granulométrie 0,04-0,06 mm, diamètre 3 cm, hauteur 25 cm) sous une pression de 0,5 bar d'azote avec du dichlorométhane comme éluant en recueillant des fractions de 100 cm³. Les fractions 2 à 6 sont réunies et concentrées à sec sous pression réduite (2,7 kPa). Le solide obtenu est cristallisé dans 40 cm³ d'éther éthylique. On obtient 1,5 g de 1-benzhydryl-3-[(3-fluorophényl)(méthylsulfonyl)méthylène]azétidine fondant à 210°C [Spectre

RMN dans DMSO-d6, T=300K, δ en ppm (300 MHz) : 2,95 (3H, s, SCH₃), 3,80 (2H, s, NCH₂), 4,20 (2H, s, NCH₂), 4,70 (1H, s, NCH), entre 7,10 et 7,30 (9H, m, 9CH arom.), 7,45 (5H, m, 5CH arom.)].

Le 1-benzhydryl-3-[(3-fluorophényl)(méthylsulfonyl)méthyl-(RS)]azétidin-3-ol peut être obtenu de la manière suivante : en opérant selon le mode opératoire de l'exemple 1 à partir de 2,6 g de 3-fluorobenzyl méthyl sulfone et 3,3 g de 1-benzhydryl azétidin-3-one, on obtient 2,9 g de 1-benzhydryl-3-[(3-fluorophényl)(méthylsulfonyl)méthyl-(RS)]azétidin-3-ol sous forme d'un solide blanc fondant à 200°C.

La 3-fluorobenzyl méthyl sulfone peut être préparée de la manière suivante : en opérant selon le mode opératoire de l'exemple 2 à partir de 3,1 g de 3-fluorobenzyl méthyl sulfure et 13 g d'oxone^{R}, on obtient 2,7 g de 3-fluorobenzyl méthyl sulfone sous forme d'un solide blanc.

Le 3-fluorobenzyl méthyl sulfure peut être préparé de la manière suivante : en opérant selon le mode opératoire de l'exemple 2 à partir de 2,6 cm³ de bromure de 3-fluorobenzyle et 1,6 g de méthyl thiolate de sodium, on obtient 3,1 g de 3-fluorobenzyl méthyl sulfure sous forme d'une huile.

### Exemple 14

En opérant selon le mode opératoire de l'exemple 4 à partir de 4,3 g de 1-benzhydryl-3-[(2-fluorophényl)(méthylsulfonyl)méthyl-(RS)]azétidin-3-ol, de 1,2 cm³ de chlorure de méthanesulfonyle et 3,7 g de 4-diméthylaminopyridine, le résidu obtenu est purifié par chromatographie sur colonne de gel de silice (granulométrie 0,04-0,06 mm, diamètre 4,5 cm, hauteur 30 cm) sous une pression de 0,5 bar d'azote avec du dichlorométhane comme éluant et en recueillant des fractions de 30 cm³. Les fractions 28 à 58 sont réunies et concentrées à sec sous pression réduite (2,7 kPa). Le solide obtenu est cristallisé dans 100 cm³ d'éther éthylique. On obtient 2,3 g de 1-benzhydryl-3-[(2-fluorophényl)(méthylsulfonyl)méthylène]azétidine fondant à 188°C [Spectre RMN dans DMSO-d6, T=300K, δ en ppm (300 MHz) : 3,00 (3H, s, SCH₃), 3,65 (2H, m, NCH₂), 4,20 (2H, m, NCH₂), 4,75 (1H, s, NCH), 7,20 (2H, t, J=7Hz, 2CH arom.), 7,30 (6H, m, 6CH arom.), 7,50 (6H, m, 6CH arom.)].

Le 1-benzhydryl-3-[(2-fluorophényl)(méthylsulfonyl)méthyl-(RS)]azétidin-3-ol peut être obtenu de la manière suivante : en opérant selon le mode opératoire de l'exemple 1 à partir de 3,4 g de 2-fluorobenzyl méthyl sulfone et 4,2 g de 1-benzhydryl azétidin-3-one, on obtient 4,3 g de 1-benzhydryl-3-[(3-fluorophényl)(méthylsulfonyl)méthyl-(RS)]azétidin-3-ol sous forme d'un solide blanc.

La 2-fluorobenzyl méthyl sulfone peut être préparée de la manière suivante : en opérant selon le mode opératoire de l'exemple 2 à partir de 3 g de 2-fluorobenzyl méthyl sulfure et 13 g d'oxone^{R}, on obtient 3,6 g de 3-fluorobenzyl méthyl sulfone sous forme d'un solide blanc.

Le 2-fluorobenzyl méthyl sulfure peut être préparé de la manière suivante : en opérant selon le mode opératoire de l'exemple 2 à partir de 2,4 cm³ de bromure de 2-fluorobenzyle et 1,5 g de méthyl thiolate de sodium, on obtient 3 g de 2-fluorobenzyl méthyl sulfure sous forme d'une huile.

### Exemple 15

En opérant selon le mode opératoire de l'exemple 4 à partir de 1 g de 1-benzhydryl-3-[(4-fluorophényl)(méthylsulfonyl)méthyl-(RS)]azétidin-3-ol, de 0,3 cm³ de chlorure de méthanesulfonyle et 0,9 g de 4-diméthylaminopyridine, le résidu obtenu est purifié par chromatographie sur colonne de gel de silice (granulométrie 0,04-0,06 mm, diamètre 3 cm, hauteur 35 cm) sous une pression de 0,5 bar d'azote avec du dichlorométhane comme éluant et en recueillant des fractions de 30 cm³. Les fractions 20 à 35 sont réunies et concentrées à sec sous pression réduite (2,7 kPa). Le solide obtenu est cristallisé dans 50 cm³ d'éther éthylique. On obtient 0,4 g de 1-benzhydryl-3-[(4-fluorophényl)(méthylsulfonyl)méthylène]azétidine fondant à 186°C [Spectre RMN dans DMSO-d6, T=300K, δ en ppm (300 MHz) : 2,95 (3H, s, SCH₃), 3,80 (2H, m, NCH₂), 4,20 (2H, m, NCH₂), 4,75 (1H, s, NCH), entre 7,15 et 7,35 (8H,m, 8CH arom.), 7,45 (6H, m, 6CH arom.)].

Le 1-benzhydryl-3-[(4-fluorophényl)(méthylsulfonyl)méthyl-(RS)]azétidin-3-ol peut être obtenu de la manière suivante : en opérant selon le mode opératoire de l'exemple 1 à partir de 2,8 g de 4-fluorobenzyl méthyl sulfone et 3,6 g de 1-benzhydryl azétidin-3-one, on obtient 1 g de 1-benzhydryl-3-[(4-fluorophényl)(méthylsulfonyl)méthyl-(RS)]azétidin-3-ol sous forme d'un solide blanc.

La 4-fluorobenzyl méthyl sulfone peut être préparée de la manière suivante : en opérant selon le mode opératoire de l'exemple 2 à partir de 3 g de 4-fluorobenzyl méthyl sulfure et 13 g d'oxone^{R}, on obtient 3 g de 4-fluorobenzyl méthyl sulfone sous forme d'un solide blanc fondant à 110°C.

Le 4-fluorobenzyl méthyl sulfure peut être préparé de la manière suivante : en opérant selon le mode opératoire de l'exemple 2 à partir de 2,5 cm³ de chlorure de 4-fluorobenzyle et 1,5 g de méthyl thiolate de sodium, on obtient 3 g de 4-fluorobenzyl méthyl sulfure sous forme d'une huile.

### Exemple 16

En opérant selon le mode opératoire de l'exemple 4 à partir de 3,8 g de 1-benzhydryl-3-[(3,5-difluorophényl)(méthylsulfonyl)méthyl-(RS)]azétidin-3-ol, de 1 cm³ de chlorure de méthanesulfonyle et 4,2 g de 4-diméthylaminopyridine, le résidu obtenu est purifié par chromatographie sur colonne de gel de silice (granulométrie 0,04-0,06 mm, diamètre 3 cm, hauteur 30 cm) sous une pression de 0,5 bar d'azote avec du dichlorométhane comme éluant en recueillant des fractions de 100 cm³. Les fractions 5 à 10 sont réunies et concentrées à sec sous pression réduite (2,7 kPa). Le solide obtenu est cristallisé dans 30 cm³ d'éther éthylique. On obtient 0,8 g de 1-benzhydryl-3-[(3,5-difluorophényl)(méthylsulfonyl)méthylène]azétidine fondant à 172°C [Spectre RMN dans DMSO-d6, T=300K, δ en ppm (250 MHz) : 3,00 (3H, s, SCH₃), 3,85 (2H, m, NCH₂), 4,20 (2H, m, NCH₂), 4,75 (1H, s, NCH), entre 7,10 et 7,40 (9H,m, 9CH arom.), 7,50 (4H, d, J=7Hz, 4CH arom.)].

Le 1-benzhydryl-3-[(3,5-difluorophényl)(méthylsulfonyl)méthyl-(RS)]azétidin-3-ol peut être obtenu de la manière suivante : en opérant selon le mode opératoire de l'exemple 1 à partir de 3,2 g de 3,5-difluorobenzyl méthyl sulfone et 3,7 g de 1-benzhydryl azétidin-3-one, on obtient 3,9 g de 1-benzhydryl-3-[(3,5-difluorophényl)(méthylsulfonyl)méthyl-(RS)]azétidin-3-ol sous forme d'un solide blanc.

La 3,5-difluorobenzyl méthyl sulfone peut être préparée de la manière suivante : en opérant selon le mode opératoire de l'exemple 2 à partir de 4,2 g de 3,5-difluorobenzyl méthyl sulfure et 16 g d'oxone^{R}, on obtient 3,3 g de 3,5-difluorobenzyl méthyl sulfone sous forme d'un solide blanc.

Le 3,5-difluorobenzyl méthyl sulfure peut être préparé de la manière suivante : en opérant selon le mode opératoire de l'exemple 2 à partir de 5 g de bromure de 3,5-difluorobenzyle et 2 g de méthyl thiolate de sodium, on obtient 4,9 g de 3,5-difluorobenzyl méthyl sulfure sous forme d'une huile.

### Exemple 17

En opérant selon le mode opératoire de l'exemple 4 à partir de 5,2 g de 1-benzhydryl-3-[(2,3-difluorophényl)(méthylsulfonyl)méthyl-(RS)]azétidin-3-ol, de 2,3 cm³ de chlorure de méthanesulfonyle et 7,3 g de 4-diméthylaminopyridine, le résidu obtenu est purifié par chromatographie sur colonne de gel de silice (granulométrie 0,04-0,06 mm, diamètre 6 cm, hauteur 40 cm) sous une pression de 0,5 bar d'azote avec un mélange dichlorométhane et méthanol (98/2 en volumes) comme éluant et en recueillant des fractions de 50 cm³. Les fractions 65 à 87 sont réunies et concentrées à sec sous pression réduite (2,7 kPa). Le solide obtenu est cristallisé dans 75 cm³ d'éther éthylique. On obtient 2,5 g de 1-benzhydryl-3-[(2,3-difluorophényl)(méthylsulfonyl)méthylène]azétidine fondant à 208°C [Spectre RMN dans DMSO-d6, T=300K, δ en ppm (400 MHz) : 3,05 (3H, s, SCH₃), 3,70 (2H, s, NCH₂), 4,25 (2H, s, NCH₂), 4,75 (1H, s, NCH), entre 7,15 et 7,55 (13H, m, 13CH arom.)].

Le 1-benzhydryl-3-[(2,3-difluorophényl)(méthylsulfonyl)méthyl-(RS)]azétidin-3-ol peut être obtenu de la manière suivante : en opérant selon le mode opératoire de l'exemple 1 à partir de 4 g de (2,3-difluorobenzyl)méthylsulfone et 4,8 g de 1-benzhydryl azétidin-3-one, on obtient 5,5 g de 1-benzhydryl-3-[(2,3-difluorophényl)(méthylsulfonyl)méthyl-(RS)]azétidin-3-ol sous forme d'un solide beige.

La (2,3-difluorobenzyl)méthylsulfone peut être préparée de la manière suivante ; en opérant selon le mode opératoire de l'exemple 10 à partir de 4,1 g de bromure de 2,3-difluorobenzyle et 4,1 g de méthanesulfinate de sodium, on obtient 4 g de (2,3-difluorobenzyl)méthyl sulfone sous forme d'un solide blanc.

### Exemple 18

En opérant selon le mode opératoire de l'exemple 4 à partir de 5,2 g de 1-benzhydryl-3-[(2,5-difluorophényl)(méthylsulfonyl)méthyl-(RS)]azétidin-3-ol, de 2,3 cm³ de chlorure de méthanesulfonyle et 7,3 g de 4-diméthylaminopyridine, le résidu obtenu est purifié par chromatographie sur colonne de gel de silice (granulométrie 0,04-0,06 mm, diamètre 6 cm, hauteur 40 cm) sous une pression de 0,5 bar d'azote avec un mélange dichlorométhane et méthanol (98/2 en volumes) comme éluant et en recueillant des fractions de 50 cm³. Les fractions 73 à 90 sont réunies et concentrées à sec sous pression réduite (2,7 kPa). Le solide obtenu est cristallisé dans 75 cm³ d'éther éthylique. On obtient 2,6 g de 1-benzhydryl-3-[(2,5-difluorophényl)(méthylsulfonyl)méthylène]azétidine fondant à 176°C.

Le 1-benzhydryl-3-[(2,5-difluorophényl)(méthylsulfonyl)méthyl-(RS)]azétidin-3-ol peut être obtenu de la manière suivante : en opérant selon le mode opératoire de l'exemple 1 à partir de 4 g de (2,5-difluorobenzyl)méthyl sulfone et 4,8 g de 1-benzhydryl azétidin-3-one, on obtient 5,9 g de 1-benzhydryl-3-[(2,5-difluorophényl)(méthylsulfonyl)méthyl-(RS)]azétidin-3-ol sous forme d'un solide blanc crème.

La (2,5-difluorobenzyl)méthyl sulfone peut être préparée de la manière suivante : en opérant selon le mode opératoire de l'exemple 10 à partir de 4,1 g de bromure de 2,5-difluorobenzyle et 4,1 g de méthanesulfinate de sodium, on obtient 4,8 g de (2,5-difluorobenzyl)méthyl sulfone sous forme d'un solide blanc fondant à 95°C.

### Exemple 19

En opérant selon le mode opératoire de l'exemple 4 à partir de 7,7 g de 1-benzhydryl-3-[(3-bromophényl)(méthylsulfonyl)méthyl-(RS)]azétidin-3-ol, de 1,8 cm³ de chlorure de méthanesulfonyle et 5,8 g de 4-diméthylaminopyridine, le résidu obtenu est purifié par chromatographie sur colonne de gel de silice (granulométrie 0,04-0,06 mm, diamètre 3 cm, hauteur 35 cm) sous une pression de 0,5 bar d'azote avec du dichlorométhane puis un mélange dichlorométhane et éthanol (99,5/0,5 en volumes) comme éluants et en recueillant des fractions de 100 cm³. Les fractions 17 à 28 sont réunies et concentrées à sec sous pression réduite (2,7 kPa). Le solide obtenu est cristallisé dans un mélange de 5 cm³ de dichlorométhane et 50 cm³ d'éther éthylique. On obtient 3,5 g de 1-benzhydryl-3-[(3-bromophényl)(méthylsulfonyl)méthylène]azétidine fondant à 200°C [Spectre RMN dans DMSO-d6, T=300K, δ en ppm (300 MHz) : 2,95 (3H, s, SCH₃), 3,80 (2H, s, NCH₂), 4,20 (2H, s, NCH₂), 4,75 (1H, s, NCH), 7,20 (2H, t, J=7Hz, 2CH arom.), 7,30 (4H, t, J=7Hz, 4CH arom.), entre 7,35 et 7,55 (6H, m, 6CH arom.), 7,65 (2H, m, 2CH arom.)].

Le 1-benzhydryl-3-[(3-bromophényl)(méthylsulfonyl)méthyl-(RS)]azétidin-3-ol peut être obtenu de la manière suivante : en opérant selon le mode opératoire de l'exemple 1 à partir de 8 g de 3-bromobenzyl méthyl sulfone et 7,6 g de 1-benzhydryl azétidin-3-one, on obtient 8 g de 1-benzhydryl-3-[(3-bromophényl)(méthylsulfonyl)méthyl-(RS)]azétidin-3-ol sous forme d'un solide blanc.

La 3-bromobenzyl méthyl sulfone peut être préparée de la manière suivante : en opérant selon le mode opératoire de l'exemple 2 à partir de 9 g de 3-bromobenzyl méthyl sulfure et 27 g d'oxone^{R}, on obtient 8,2 g de 3-bromobenzyl méthyl sulfone sous forme d'un solide blanc.

Le 3-bromobenzyl méthyl sulfure peut être préparé de la manière suivante : en opérant selon le mode opératoire de l'exemple 2 à partir de 10 g de bromure de 3-bromobenzyle et 3,1 g de méthyl thiolate de sodium, on obtient 9 g de 3-bromobenzyl méthyl sulfure sous forme d'une huile.

### Exemple 20

En opérant selon le mode opératoire de l'exemple 4 à partir de 1,5 g de 1-benzhydryl-3-[(3-iodophényl)(méthylsulfonyl)méthyl-(RS)]azétidin-3-ol, de 0,3 cm³ de chlorure de méthanesulfonyle et 1,4 g de 4-diméthylaminopyridine, le résidu obtenu est purifié par chromatographie sur colonne de gel de silice (granulométrie 0,04-0,06 mm, diamètre 3 cm, hauteur 35 cm) sous une pression de 0,5 bar d'azote avec du dichlorométhane puis un mélange dichlorométhane et éthanol (99,7/0,3 en volumes) comme éluants et en recueillant des fractions de 100 cm³. Les fractions 16 à 24 sont réunies et concentrées à sec sous pression réduite (2,7 kPa). Le solide obtenu est cristallisé dans un mélange de 1,5 cm³ de dichlorométhane et 25 cm³ d'éther éthylique. On obtient 0,5 g de 1-benzhydryl-3-[(3-iodophényl)(méthylsulfonyl)méthylène]azétidine fondant à 198°C [Spectre RMN dans DMSO-d6, T=300K, δ en ppm (300 MHz) : 2,95 (3H, s, SCH₃), 3,80 (2H, s, NCH₂), 4,20 (2H, s, NCH₂), 4,75 (1H, s, NCH), entre 7,10 et 7,30 (7H,m, 7CH arom.), 7,45 (5H, m, 5CH arom.), 7,80 (2H, m, 2CH arom.)].

Le 1-benzhydryl-3-[(3-iodophényl)(méthylsulfonyl)méthyl-(RS)]azétidin-3-ol peut être obtenu de la manière suivante : en opérant selon le mode opératoire de l'exemple 1 à partir de 3,7 g de 3-iodobenzyl méthyl sulfone et 3 g de 1-benzhydryl azétidin-3-one, on obtient 1,5 g de 1-benzhydryl-3-[(3-iodophényl)(méthylsulfonyl)méthyl-(RS)]azétidin-3-ol sous forme d'un solide blanc.

La 3-iodobenzyl méthyl sulfone peut être préparée de la manière suivante : en opérant selon le mode opératoire de l'exemple 2 à partir de 3,6 g de 3-iodobenzyl méthyl sulfure et 10,3 g d'oxone^{R}, on obtient 3,7 g de 3-iodobenzyl méthyl sulfone sous forme d'un solide blanc.

Le 3-iodobenzyl méthyl sulfure peut être préparé de la manière suivante : en opérant selon le mode opératoire de l'exemple 2 à partir de 5 g de bromure de 3-iodobenzyle et 1,3 g de méthyl thiolate de sodium, on obtient 4 g de 3-iodobenzyl méthyl sulfure sous forme d'une huile.

### Exemple 21

En opérant selon le mode opératoire de l'exemple 4 à partir de 2,4 g de 1-benzhydryl-3-[(méthylsulfonyl)(3-trifluorométhoxyphényl)méthyl-(RS)]azétidin-3-ol, de 0,6 cm³ de chlorure de méthanesulfonyle et 2,3 g de 4-diméthylaminopyridine, le résidu obtenu est purifié par chromatographie sur colonne de gel de silice (granulométrie 0,04-0,06 mm, diamètre 3 cm, hauteur 35 cm) sous une pression de 0,5 bar d'azote avec du dichlorométhane puis un mélange dichlorométhane et éthanol (99,7/0,3 en volumes) comme éluants et en recueillant des fractions de 100 cm³. Les fractions 12 à 25 sont réunies et concentrées à sec sous pression réduite (2,7 kPa). Le solide obtenu est cristallisé dans un mélange de 2 cm³ de dichlorométhane et 30 cm³ d'éther éthylique. On obtient 0,7 g de 1-benzhydryl-3-[(méthylsulfonyl)(3-trifluorométhoxyphényl)méthylène]azétidine fondant à 162°C [Spectre RMN dans DMSO-d6, T=300K, δ en ppm (250 MHz) : 3,00 (3H, s, SCH₃), 3,80 (2H, s, NCH₂), 4,20 (2H, s, NCH₂), 4,75 (1H, s, NCH), entre 7,15 et 7,40 (6H,m, 6CH arom.), entre 7,45 et 7,55 (7H, m, 7CH arom.), 7,60 (1H, t, J=7Hz, CH arom.)].

Le 1-benzhydryl-3-[(méthylsulfonyl)(3-trifluorométhoxyphényl)méthyl-(RS)]azétidin-3-ol peut être obtenu de la manière suivante : en opérant selon le mode opératoire de l'exemple 1 à partir de 2,4 g de méthyl(3-trifluorométhoxybenzyl)sulfone et 2,2 g de 1-benzhydryl azétidin-3-one, on obtient 2,4 g de 1-benzhydryl-3-[(méthylsulfonyl)(3-trifluorométhoxyphényl)méthyl-(RS)]azétidin-3-ol sous forme d'un solide blanc.

La méthyl(3-trifluorométhoxybenzyl)sulfone peut être préparée de la manière suivante : en opérant selon le mode opératoire de l'exemple 2 à partir de 2,6 g de méthyl (3-trifluorométhoxybenzyl)sulfure et 7,2 g d'oxone^{R}, on obtient 2,4 g de méthyl (3-trifluorométhoxybenzyl)sulfone sous forme d'un solide blanc.

Le méthyl(3-trifluorométhoxybenzyl)sulfure peut être préparé de la manière suivante : en opérant selon le mode opératoire de l'exemple 2 à partir de 3 g de bromure de 3-trifluorométhoxybenzyle et 1 g de méthyl thiolate de sodium, on obtient 3,3 g de méthyl(3-trifluorométhoxybenzyl)sulfure sous forme d'une huile.

### Exemple 22

En opérant selon le mode opératoire de l'exemple 4 à partir de 4,1 g de 1-benzhydryl-3-[(méthylsulfonyl)(3-trifluorométhylphényl)méthyl-(RS)]azétidin-3-ol, de 1 cm³ de chlorure de méthanesulfonyle et 4,2 g de 4-diméthylaminopyridine, le résidu obtenu est purifié par chromatographie sur colonne de gel de silice (granulométrie 0,04-0,06 mm, diamètre 3 cm, hauteur 35 cm) sous une pression de 0,5 bar d'azote avec du dichlorométhane comme éluant en recueillant des fractions de 100 cm³. Les fractions 10 à 14 sont réunies et concentrées à sec sous pression réduite (2,7 kPa). Le solide obtenu est cristallisé dans un mélange de 2 cm³ de dichlorométhane et 30 cm³ d'éther éthylique. On obtient 1,2 g de 1-benzhydryl-3-[(méthylsulfonyl)(3-trifluorométhylphényl)méthylène]azétidine fondant à 178°C [Spectre RMN dans DMSO-d6, T=300K, δ en ppm (300 MHz) : 3,00 (3H, s, SCH₃), 3,80 (2H, s, NCH₂), 4,15 (2H, s, NCH₂), 4,70 (1H, s, NCH), 7,20 (2H, t, J=7Hz, 2CH arom.), 7,30 (4H, t, J=7Hz, 4CH arom.), 7,45 (4H, d, J=7Hz, 4CH arom.), entre 7,60 et 7,80 (4H, m, 4CH arom.)].

Le 1-benzhydryl-3-[(méthylsulfonyl)(3-trifluorométhylphényl)méthyl-(RS)]azétidin-3-ol peut être obtenu de la manière suivante : en opérant selon le mode opératoire de l'exemple 1 à partir de 3,4 g de méthyl(3-trifluorométhylbenzyl)sulfone et 3,4 g de 1-benzhydryl azétidin-3-one, on obtient 4,2 g de 1-benzhydryl-3-[(méthylsulfonyl)(3-trifluorométhylphényl)méthyl-(RS)]azétidin-3-ol sous forme d'un solide blanc.

La méthyl(3-trifluorométhylbenzyl)sulfone peut être préparée de la manière suivante : en opérant selon le mode opératoire de l'exemple 2 à partir de 3,3 g de méthyl(3-trifluorométhylbenzyl)sulfure et 10 g d'oxone^{R}, on obtient 3,4 g de méthyl(3-trifluorométhylbenzyl)sulfone sous forme d'un solide blanc.

Le méthyl(3-trifluorométhylbenzyl)sulfure peut être préparé de la manière suivante : en opérant selon le mode opératoire de l'exemple 2 à partir de 3,9 g de bromure de 3-trifluorométhylbenzyle et 1,4 g de méthyl thiolate de sodium, on obtient 3,3 g de méthyl(3-trifluorométhylbenzyl)sulfure sous forme d'une huile.

### Exemple 23

En opérant selon le mode opératoire de l'exemple 4 à partir de 2,7 g del-benzhydryl-3-{[3,5-bis(trifluorométhyl)phényl](méthylsulfonyl)méthyl-(RS)}azétidin-3-ol, de 0,6 cm³ de chlorure de méthanesulfonyle et 2,4 g de 4-diméthylaminopyridine, le résidu obtenu est purifié par chromatographie sur colonne de gel de silice (granulométrie 0,04-0,06 mm, diamètre 6 cm, hauteur 40 cm) sous une pression de 0,5 bar d'azote avec du dichlorométhane comme éluant en recueillant des fractions de 100 cm³. Les fractions 7 à 12 sont réunies et concentrées à sec sous pression réduite (2,7 kPa). Le solide obtenu est cristallisé dans 10 cm³ d'éther éthylique. On obtient 1 g de 1-benzhydryl-3-{[3,5-bis(trifluorométhyl)phényl](méthylsulfonyl)méthylène} azétidine fondant à 192°C [Spectre RMN dans DMSO-d6, T=300K, δ en ppm (300 MHz) : 3,00 (3H, s, SCH₃), 3,85 (2H, s, NCH₂), 4,15 (2H, s, NCH₂), 4,70 (1H, s, NCH), 7,15 (2H, t, J=7Hz, 2CH arom.), 7,30 (4H, t, J=7Hz, 4CH arom.), 7,40 (4H, d, J=7Hz, 4CH arom.), 8,05 (2H, s, 2CH arom.), 8,15 (1H, s, CH arom.)].

Le 1-benzhydryl-3-{[3,5-bis(trifluorométhyl)phényl](méthylsulfonyl)méthylène} azétidin-3-ol peut être obtenu de la manière suivante : en opérant selon le mode opératoire de l'exemple 1 à partir de 3,1 g de méthyl[3,5-bis(trifluorométhyl)benzyl]sulfone et 2,4 g de 1-benzhydryl azétidin-3-one on obtient 2,8 g de 1-benzhydryl-3-{[3,5-bis(trifluorométhyl)phényl](méthylsulfonyl)méthylène}azétidin-3-ol sous forme d'un solide blanc.

La méthyl[3,5-bis(trifluorométhyl)benzyl]sulfone peut être préparée de la manière suivante : en opérant selon le mode opératoire de l'exemple 10 à partir de 3 g de chlorure de 3,5-bis(trifluorométhyl)benzyle et 2 g de méthanesulfinate de sodium, on obtient 3,1 g de méthyl [3,5-bis(trifluorométhyl)benzyl]sulfone sous forme d'un solide blanc fondant à 132°C.

### Exemple 24

En opérant selon le mode opératoire de l'exemple 4 à partir de 10,7 g de 1-benzhydryl-3-[(3,5-dibromophényl)(méthylsulfonyl)méthyl-(RS)]azétidin-3-ol, de 2,2 cm³ de chlorure de méthanesulfonyle et 7 g de 4-diméthylaminopyridine, le résidu obtenu est purifié par chromatographie sur colonne de gel de silice (granulométrie 0,04-0,06 mm, diamètre 5,5 cm, hauteur 30 cm) sous une pression de 0,5 bar d'azote avec du dichlorométhane comme éluant et en recueillant des fractions de 35 cm³. Les fractions 40 à 58 sont réunies et concentrées à sec sous pression réduite (2,7 kPa). Le solide obtenu est cristallisé dans 50 cm³ d'éther éthylique. On obtient 1,5 g de 1-benzhydryl-3-[(3,5-dibromophényl)(méthylsulfonyl)méthylène]azétidine fondant à 209°C [Spectre RMN dans DMSO-d6, T=300K, δ en ppm (250 MHz) : 3,00 (3H, s, SCH₃), 3,88 (2H, s, NCH₂), 4,22 (2H, s, NCH₂), 4,75 (1H, s, NCH), 7,22 (2H, t, J=7Hz, 2CH arom.), 7,33 (4H, t, J=7Hz, 4CH arom.), 7,48 (4H, d, J=7Hz, 4CH arom.), 7,68 (2H, s, 2CH arom.), 7,95 (1H, s, CH arom.)].

Le 1-benzhydryl-3-[(3,5-dibromophényl)(méthylsulfonyl)méthyl-(RS)]azétidin-3-ol peut être obtenu de la manière suivante : en opérant selon le mode opératoire de l'exemple 1 à partir de 6,2 g de (3,5-dibromobenzyl)méthylsulfone et 4,5 g de 1-benzhydryl azétidin-3-one, on obtient 10,7 g de 1-benzhydryl-3-[(3,5-dibromophényl)(méthylsulfonyl)méthyl-(RS)]azétidin-3-ol sous forme d'une meringue.

La (3,5-dibromobenzyl)méthylsulfone peut être préparée de la manière suivante : en opérant selon le mode opératoire de l'exemple 2 à partir de 5,8 g de (3,5-dibromobenzyl)méthylsulfure et 13 g d'oxone^{R}, on obtient 6,2 g de (3,5-dibromobenzyl)méthylsulfone sous forme d'un solide blanc.

Le (3,5-dibromobenzyl)méthylsulfure peut être préparé de la manière suivante : en opérant selon le mode opératoire de l'exemple 2 à partir de 6,6 g de bromure de 3,5-dibromobenzyle et 1,5 g de méthyl thiolate de sodium, on obtient 5,8 g de (3,5-dibromobenzyl)méthylsulfure sous forme d'une huile.

### Exemple 25

En opérant selon le mode opératoire de l'exemple 4 à partir de 4,2 g de 1-benzhydryl-3-[(méthylsulfonyl)(3-nitrophényl)méthyl-(RS)]azétidin-3-ol, de 1,1 cm³ de chlorure de méthanesulfonyle et 2,5 g de 4-diméthylaminopyridine, le résidu obtenu est purifié par chromatographie sur colonne de gel de silice (granulométrie 0,04-0,06 mm, diamètre 4 cm, hauteur 35 cm) sous une pression de 0,5 bar d'azote avec un mélange cyclohexane et acétate d'éthyle (50/50 en volumes) comme éluants en recueillant des fractions de 400 cm³. Les fractions 17 à 33 sont réunies et concentrées à sec sous pression réduite (2,7 kPa). Le solide obtenu est recristallisé dans 15 cm³ d'acétate d'éthyle. On obtient 0,6 g de 1-benzhydryl-3-[(méthylsulfonyl)(3-nitrophényl)méthylène]azétidine fondant à 184°C [Spectre RMN dans DMSO-d6, T=300K, δ en ppm (300 MHz) : 3,00 (3H, s, SCH₃), 3,85 (2H, s, NCH₂), 4,25 (2H, s, NCH₂), 4,75 (1H, s, NCH), 7,20 (2H, t, J=7Hz, 2CH arom.), 7,30 (4H, t, J=7Hz, 4CH arom.), 7,45 (4H, d, J=7Hz, 4CH arom.), 7,75 (1H, t, J=7Hz, CH arom.), 7,85 (1H, d, J=7Hz, CH arom.), 8,25 (2H, m, 2CH arom.)].

Le 1-benzhydryl-3-[(méthylsulfonyl)(3-nitrophényl)méthyl-(RS)]azétidin-3-ol peut être obtenu de la manière suivante : en opérant selon le mode opératoire de l'exemple 1 à partir de 3,9 g de méthyl (3-nitrobenzyl)sulfone et 4,2 g de 1-benzhydryl azétidin-3-one, on obtient 4,2 g de 1-benzhydryl 3-[(méthylsulfonyl)(3-nitrophényl)méthyl-(RS)]azétidin-3-ol sous forme d'une meringue.

La méthyl(3-nitrobenzyl)sulfone peut être préparée de la manière suivante : en opérant selon le mode opératoire de l'exemple 2 à partir de 18,1 g de méthyl(3-nitrobenzyl)sulfure et 68 g d'oxone^{R}, on obtient 13,9 g de méthyl (3-nitrobenzyl)sulfone sous forme d'une meringue.

Le méthyl(3-nitrobenzyl)sulfure peut être préparé de la manière suivante : en opérant selon le mode opératoire de l'exemple 2 à partir de 17,2 g de chlorure de 3-nitrobenzyle et 7,7 g de méthyl thiolate de sodium, on obtient 18,2 g de méthyl(3-nitrobenzyl)sulfure sous forme d'une huile.

### Exemple 26

Un mélange de 0,34 g de 1-benzhydryl-3-[(méthylsulfonyl)(3-nitrophényl)méthylène]azétidine, 16 cm³ d'acide chlorhydrique 1N dans 8 cm³ d'éthanol et 16 cm³ de tétrahydrofuranne est chauffé au reflux. On ajoute 0,17 g de fer en poudre et maintient le reflux durant 3 heures. Le mélange est ensuite refroidi à température ambiante, l'insoluble est filtré. La solution est reprise par 10 cm³ de soude 1N et 50 cm³ d'une solution aqueuse saturée de chlorure de sodium. La phase aqueuse est extraite par 3 fois 40 cm³ de dichlorométhane, les extraits sont réunis, séchés sur du sulfate de sodium et concentrés à sec sous pression réduite (2,7kPa). Le résidu est purifié sur colonne de gel de silice (granulométrie 0,04-0,06 mm, diamètre 3 cm, hauteur 30 cm) sous une pression de 0,5 bar d'azote avec un mélange cyclohexane et acétate d'éthyle (50/50 en volumes) comme éluant en recueillant des fractions de 20 cm³. Les fractions 13 à 31 sont réunies et concentrées à sec sous pression réduite (2,7 kPa). Le solide obtenu est cristallisé dans 15 cm³ d'éther éthylique. On obtient 0,17 g de 3-[(3-aminophényl)(méthylsulfonyl)méthylène]-1-benzhydrylazétidine fondant à 197°C [Spectre RMN dans DMSO-d6, T=300K, δ en ppm (250 MHz) : 2,95 (3H, s, SCH₃), 3,80 (2H, s, NCH₂), 4,20 (2H, s, NCH₂), 4,75 (1H, s, NCH), 5,25 (2H, s, NH₂), 6,55 (3H, m, 3CH arom.), 7,05 (1H, t, J=7Hz, CH arom.), 7,20 (2H, t, J=7Hz, 2CH arom.), 7,30 (4H, t, J=7Hz, 4CH arom.), 7,45 (4H, d, J=7Hz, 4CH arom.)].

### Exemple 27

En opérant selon le mode opératoire de l'exemple 4 à partir de 1,2 g de 1-benzhydryl-3-[(3-méthoxycarbonylphényl)(méthylsulfonyl)méthyl-(RS)]azétidin-3-ol, de 0,3 cm³ de chlorure de méthanesulfonyle et 1,3 g de 4-diméthylaminopyridine, le résidu obtenu est purifié par chromatographie sur colonne de gel de silice (granulométrie 0,04-0,06 mm, diamètre 3 cm, hauteur 30 cm) sous une pression de 0,5 bar d'azote avec du dichlorométhane puis un mélange dichlorométhane et acétate d'éthyle (99,5/0,5 en volumes) comme éluants et en recueillant des fractions de 100 cm³. La fraction 18 est concentrée à sec sous pression réduite (2,7 kPa). Le solide obtenu est précipité dans 5 cm³ d'éther éthylique. On obtient 0,13 g de 1-benzhydryl-3-[(3-méthoxycarbonylphényl)(méthylsulfonyl)méthylène]azétidine sous forme d'un solide meringué [Spectre RMN dans DMSO-d6, T=300K, δ en ppm (300 MHz): 2,95 (3H, s, SCH₃), 3,80 (2H, s, NCH₂), 4,20 (2H, s, NCH₂), 4,75 (1H, s, NCH), 7,20 (2H, t, J=7Hz, 2CH arom.), 7,30 (4H, t, J=7Hz, 4CH arom.), 7,45 (4H, d, J=7Hz, 4CH arom.), 7,60 (1H, t, J=7Hz, CH arom.), 7,70 (1H, d, J=7Hz, CH arom.), 8,00 (2H, m, 2CH arom.)].

Le 1-benzhydryl-3-[(3-méthoxycarbonylphényl)(méthylsulfonyl)méthyl-(RS)]azétidin-3-ol peut être obtenu de la manière suivante : en opérant selon le mode opératoire de l'exemple 1 à partir de 3 g de (3-méthoxycarbonylbenzyl)méthylsulfone et 3,6 g de 1-benzhydryl azétidin-3-one, on obtient après purification par chromatographie sur colonne de gel de silice (granulométrie 0,04-0,06 mm, diamètre 3 cm, hauteur 30 cm) sous une pression de 0,5 bar d'azote avec du dichlorométhane puis un mélange dichlorométhane et éthanol (99/1 en volumes) comme éluants, 1,2 g de 1-benzhydryl-3-[(3-méthoxycarbonylphényl)(méthylsulfonyl)méthyl-(RS)]azétidin-3-ol sous forme d'une meringue.

La (3-méthoxycarbonylbenzyl)méthylsulfone peut être préparée de la manière suivante : en opérant selon le mode opératoire de l'exemple 2 à partir de 4,3 g de (3-méthoxycarbonylbenzyl)méthylsulfure et 13,4 g d'oxone^{R}, on obtient 3,4 g de (3-méthoxycarbonylbenzyl)méthylsulfone sous forme d'un solide blanc.

Le (3-méthoxycarbonylbenzyl)méthylsulfure peut être préparé de la manière suivante : en opérant selon le mode opératoire de l'exemple 2 à partir de 5 g de bromure de 3-méthoxycarbonylbenzyle et 1,7 g de méthyl thiolate de sodium, on obtient 4,3 g de (3-méthoxycarbonylbenzyl)méthylsulfure sous forme d'une huile.

### Exemple 28

En opérant selon le mode opératoire de l'exemple 4 à partir de 6,2 g de 1-benzhydryl-3-[(3-cyanophényl)(méthylsulfonyl)méthyl-(RS)]azétidin-3-ol, de 1,6 cm³ de chlorure de méthanesulfonyle et 6,8 g de 4-diméthylaminopyridine, le résidu obtenu est purifié par chromatographie sur colonne de gel de silice (granulométrie 0,04-0,06 mm, diamètre 3 cm, hauteur 30 cm) sous une pression de 0,5 bar d'azote avec du dichlorométhane puis un mélange dichlorométhane et acétate d'éthyle (99,5/0,5 en volumes) comme éluants et en recueillant des fractions de 250 cm³. Les fractions 10 à 15 sont réunies et concentrées à sec sous pression réduite (2,7 kPa). Le solide obtenu est cristallisé dans un mélange de 5 cm³ de dichlorométhane et 70 cm³ d'éther éthylique. On obtient 2,9 g de 1-benzhydryl-3-[(3-cyanophényl)(méthylsulfonyl)méthylène]azétidine fondant à 152°C [Spectre RMN dans DMSO-d6, T=300K, δ en ppm (300 MHz) : 3,00 (3H, s, SCH₃), 3,80 (2H, s, NCH₂), 4,20 (2H, s, NCH₂), 4,75 (1H, s, NCH), 7,20 (2H, t, J=7Hz, 2CH arom.), 7,30 (4H, t, J=7Hz, 4CH arom.), 7,45 (4H, d, J=7Hz, 4CH arom.), 7,65 (1H, t, J=7Hz, CH arom.), 7,75 (1H, d, J=7Hz, CH arom.), 7,90 (2H, m, 2CH arom.)].

Le 1-benzhydryl-3-[(3-cyanophényl)(méthylsulfonyl)méthyl-(RS)]azétidin-3-ol peut être obtenu de la manière suivante : en opérant selon le mode opératoire de l'exemple 1 à partir de 3,9 g de (3-cyanobenzyl)méthylsulfone et 4,7 g de 1-benzhydryl azétidin-3-one, on obtient 6,2 g de 1-benzhydryl-3-[(3-cyanophényl)(méthylsulfonyl)méthyl-(RS)]azétidin-3-ol sous forme d'un solide blanc.

La (3-cyanobenzyl)méthylsulfone peut être préparée de la manière suivante : en opérant selon le mode opératoire de l'exemple 2 à partir de 6,7 g de (3-cyanobenzyl)méthylsulfure et 27,6 g d'oxone^{R}, on obtient 3,9 g de (3-cyanobenzyl)méthylsulfone sous forme d'un solide blanc.

Le (3-cyanobenzyl)méthylsulfure peut être préparé de la manière suivante : en opérant selon le mode opératoire de l'exemple 2 à partir de 8 g de bromure de 3-cyanobenzyle et 3,1 g de méthyl thiolate de sodium, on obtient 6,8 g de (3-cyanobenzyl)méthylsulfure sous forme d'une huile.

### Exemple 29

On agite à température ambiante pendant 15 heures un mélange de 3 g de chlorhydrate de 1-benzhydryl-3-[(3-carboxyphényl)(méthylsulfonyl)méthylène]azétidine, 1,3 g de pentafluorophénol, 1,4 g de chlorhydrate de 1-(3-diméthylaminopropyl)-3-éthylcarbodiimide dans 30 cm³ de diméthylformamide. Le mélange est repris par 100 cm³ d'eau et 100 cm³ d'une solution aqueuse saturée avec du chlorure de sodium et 50 cm³ d'acétate d'éthyle. La phase organique est décantée, séchée sur sulfate de magnésium et concentrée à sec sous pression réduite (2,7 kPa). Le résidu est purifié par chromatographie sur colonne de gel de silice (granulométrie 0,04-0,06 mm, diamètre 3 cm, hauteur 35 cm) sous une pression de 0,5 bar d'azote avec du dichlorométhane puis un mélange dichlorométhane et méthanol (99,4/0,6 en volumes) comme éluants et en recueillant des fractions de 100 cm³. Les fractions 13 à 16 sont réunies et concentrées à sec sous pression réduite (2,7 kPa). Le solide obtenu est cristallisé dans 10 cm³ d'éther éthylique. On obtient 0,6 g de 1-benzhydryl-3-[(méthylsulfonyl)(3-pentafluorophénoxycarbonylphényl)méthylène]azétidine fondant à 182°C [Spectre RMN dans DMSO-d6, T=300K, δ en ppm (400 MHz) : 3,00 (3H, s, SCH₃), 3,85 (2H, s, NCH₂), 4,25 (2H, s, NCH₂), 4,75 (1H, s, NCH), 7,20 (2H, t, J=7Hz, 2CH arom.), 7,30 (4H, t, J=7Hz, 4CH arom.), 7,45 (4H, d, J=7Hz, 4CH arom.), 7,70 (1H, t, J=7Hz, CH arom.), 8,20 (2H, m, 2CH arom.)].

Le chlorhydrate de 1-benzhydryl-3-[(3-carboxyphényl)(méthylsulfonyl)méthylène] azétidine peut être préparé de la manière suivante : on chauffe à 45°C pendant 7 jours un mélange de 10 g de 1-benzhydryl-3-[(3-cyanophényl)(méthylsulfonyl)méthylène] azétidine dans 40 cm³ d'acide acétique et 40 cm³ d'acide chlorhydrique concentré (d=1,18). Le milieu réactionnel est refroidi dans un bain d'eau glacée et le précipité formé est filtré sur verre fritté. Le solide est lavé par 20 cm³ d'un mélange d'acide acétique et d'acide chlorhydrique concentré (50-50 en volumes) puis par 3 fois 20 cm³ d'eau et finalement par 20 cm³ d'éthanol Le solide blanc obtenu est sous pression réduite (2,7 kPa) à 45°C et l'on obtient 2,5 g de chlorhydrate de 1-benzhydryl-3-[(3-carboxyphényl)(méthylsulfonyl)méthylène]azétidine sous forme d'un solide blanc.

### Exemple 30

On agite pendant 4 heures à température ambiante une solution de 0,65 g de 1-benzhydryl-3-[(méthylsulfonyl)(3-pentafluorophénoxycarbonylphényl)méthylène] azétidine dans 25 cm³ d'éthanol ammoniacal 6,2N. Le mélange est concentrée à sec sous pression réduite (2,7 kPa) puis le résidu est purifié par chromatographie sur colonne de gel de silice (granulométrie 0,04-0,06 mm, diamètre 3 cm, hauteur 30 cm) sous une pression de 0,5 bar d'azote avec un mélange dichlorométhane et éthanol (99/1 en volumes) puis un mélange dichlorométhane et éthanol (98/2 en volumes) comme éluants et en recueillant des fractions de 60 cm³. Les fractions 18 à 30 sont réunies et concentrées à sec sous pression réduite (2,7 kPa). On obtient 0,2 g de 1-benzhydryl-3-[(3-carbamoylphényl)(méthylsulfonyl)méthylène]azétidine fondant à 140°C [Spectre RMN dans DMSO-d6, T=300K, δ en ppm (300 MHz) : 3,00 (3H, s, SCH₃), 3,80 (2H, s, NCH₂), 4,25 (2H, s, NCH₂), 4,75 (1H, s, NCH), 7,25 (2H, t, J=7Hz, 2CH arom.), 7,30 (4H, t, J=7Hz, 4CH arom.), entre 7,45 et 7,65 (7H, m, 6CH arom. et ½ CONH₂), 7,95 (2H, m, 2CH arom.), 8,10 (1H, s, ½ CONH₂).

### Exemple 31

En opérant selon le mode opératoire de l'exemple 4 à partir de 4,6 g de 1-benzhydryl-3-[(3-méthoxyphényl)(méthylsulfonyl)méthyl-(RS)]azétidin-3-ol, de 1,2 cm³ de chlorure de méthanesulfonyle et 3,8 g de 4-diméthylaminopyridine, on obtient après recristallisation dans 150 cm³ d'acétonitrile 2,6 g de 1-benzhydryl-3-[(3-méthoxyphényl)(méthylsulfonyl)méthylène]azétidine fondant à 179°C [Spectre RMN dans DMSO-d6, T=300K, δ en ppm (250 MHz) : 2,95 (3H, s, SCH₃), 3,75 (3H, s, OCH₃), 3,80 (2H, s, NCH₂), 4,20 (2H, s, NCH₂), 4,75 (1H, s, NCH), 7,00 (3H, m, 3 CH arom.), entre 7,20 et 7,12 (11H, m, 10H Phenyles et 1 CH arom.)].

Le 1-benzhydryl-3-[(3-méthoxyphényl)(méthylsulfonyl)méthyl-(RS)]azétidin-3-ol peut être obtenu de la manière suivante : en opérant selon le mode opératoire de l'exemple 1 à partir de 3,4 g de (3-méthoxybenzyl)méthylsulfone et 4 g de 1-benzhydryl azétidin-3-one, on obtient 4,6 g de 1-benzhydryl-3-[(3-méthoxyphényl)(méthylsulfonyl)méthyl-(RS)]azétidin-3-ol sous forme d'un solide blanc.

La (3-méthoxybenzyl)méthylsulfone peut être préparée de la manière suivante : en opérant selon le mode opératoire de l'exemple 2 à partir de 3,4 g de (3-méthoxybenzyl)méthylsulfure et 13 g d'oxone^{R}, on obtient 4 g de (3-méthoxybenzyl)méthylsulfone sous forme d'un solide blanc fondant à 71°C.

Le (3-méthoxybenzyl)méthylsulfure peut être préparé de la manière suivante : en opérant selon le mode opératoire de l'exemple 2 à partir de 3,1 g de bromure de 3-méthoxybenzyle et 1,5 g de méthyl thiolate de sodium, on obtient 3,4 g de (3-méthoxybenzyl)méthylsulfure sous forme d'une huile.

### Exemple 32

On ajoute, sous agitation, 10 cm³ d'une solution 1M de tribromure de bore dans le dichlorométhane à une solution de 1,3 g de 1-benzhydryl-3-[(3-méthoxyphényl)(méthylsulfonyl)méthylène]azétidine dans 100 cm³ de dichlorométhane. L'agitation est maintenue 16 heures à température ambiante. Le milieu réactionnel est repris par 100 cm³ d'eau glacée. La phase organique est lavée par 3 fois 50 cm³ d'eau, séchée sur sulfate de magnésium et concentrée à sec sous pression réduite (2,7 kPa). Le résidu est précipité dans 150 cm³ d'éther d'isopropyle puis dissous dans 50 cm³ de dichlorométhane. La phase organique est lavée par 3 fois 30 cm³ d'une solution aqueuse saturée de bicarbonate de sodium, décantée, séchée sur sulfate de magnésium et concentrée à sec sous pression réduite (2,7 kPa). Le résidu est précipité dans 80 cm³ d'éther éthylique. On obtient 0,36 g de 1-benzhydryl-3-[(3-hydroxyphényl)(méthylsulfonyl)méthylène]azétidine d'un solide fondant à 248°C [Spectre RMN dans DMSO-d6, T=300K, δ en ppm (300 MHz) : 2,95 (3H, s, SCH₃), 3,80 (2H, s, NCH₂), 4,20 (2H, s, NCH₂), 4,75 (1H, s, NCH), 6,85 (3H, m, 3 CH arom.), 7,25 (3H, m, 3 CH arom.), 7,35 (4H, t, J=7Hz, 4CH arom.), 7,50 (4H, d, J=7Hz, 4CH arom.), 9,50 (1H, s, OH)].

### Exemple 33

En opérant selon le mode opératoire de l'exemple 32 à partir de 1,4 g de 1-benzhydryl-3-[(4-méthoxyphényl)(méthylsulfonyl)méthylène]azétidine, de 10 cm³ d'une solution 1M de tribromure de bore et 100 cm³ dichlorométhane, le résidu obtenu est purifié par chromatographie sur colonne de gel de silice (granulométrie 0,04-0,06 mm, diamètre 3,5 cm, hauteur 24 cm) sous une pression de 0,5 bar d'azote avec un mélange cyclohexane et acétate d'éthyle (50/50 en volumes) comme éluants et en recueillant des fractions de 25 cm³. Les fractions 21 à 37 sont réunies et concentrées à sec sous pression réduite (2,7 kPa). Le solide obtenu est cristallisé dans 30 cm³ d'éther éthylique. On obtient 0,6 g de 1-benzhydryl-3-[(4-hydroxyphényl)(méthylsulfonyl)méthylène]azétidine fondant à 211°C [Spectre RMN dans DMSO-d6, T=300K, δ en ppm (300 MHz) : 2,90 (3H, s, SCH₃), 3,80 (2H, s, NCH₂), 4,20 (2H, s, NCH₂), 4,75 (1H, s, NCH), 6,80 (2H, d, J=7Hz, 2CH arom.), entre 7,10 et 7,35 (8H, m, 8CH arom.), 7,48 (4H, d, J=7Hz, 4CH arom.), 9,80 (1H, s, OH)].

Le 1-benzhydryl-3-[(4-méthoxyphényl)(méthylsulfonyl)méthylène]azétidine peut être obtenu de la manière suivante : en opérant selon le mode opératoire de l'exemple 4 à partir de 3,5 g de 1-benzhydryl-3-[(4-méthoxyphényl)(méthylsulfonyl)méthyl-(RS)]azétidin-3-ol, de 0,9 cm³ de chlorure de méthanesulfonyle et 2,9 g de 4-diméthylaminopyridine, le résidu obtenu est purifié par recristallisation dans 100 cm³ d'acétonitrile. On obtient 1 g de 1-benzhydryl-3-[(4-méthoxyphényl)(méthylsulfonyl)méthylène]azétidine fondant à 181°C.

Le 1-benzhydryl-3-[(4-méthoxyphényl)(méthylsulfonyl)méthyl-(RS)]azétidin-3-ol peut être obtenu de la manière suivante : en opérant selon le mode opératoire de l'exemple 1 à partir de 3,5 g de (4-méthoxybenzyl)méthylsulfone et 4 g de 1-benzhydryl azétidin-3-one, on obtient 3,6 g de 1-benzhydryl-3-[(4-méthoxyphényl)(méthylsulfonyl)méthyl-(RS)]azétidin-3-ol sous forme d'un solide blanc.

La (4-méthoxybenzyl)méthylsulfone peut être préparée de la manière suivante : en opérant selon le mode opératoire de l'exemple 2 à partir de 3,4 g de (4-méthoxybenzyl)méthylsulfure et 13 g d'oxone^{R}, on obtient 3,5 g de (3-méthoxybenzyl)méthylsulfone sous forme d'un solide blanc fondant à 113°C.

Le (4-méthoxybenzyl)méthylsulfure peut être préparé de la manière suivante : en opérant selon le mode opératoire de l'exemple 2 à partir de 3,1 g de chlorure de 4-méthoxybenzyle et 1,5 g de méthyl thiolate de sodium, on obtient 3,4 g de (4-méthoxybenzyl)méthylsulfure sous forme d'une huile.

### Exemple 34

En opérant selon le mode opératoire de l'exemple 32 à partir de 1,4 g de 1-benzhydryl-3-[(2-méthoxyphényl)(méthylsulfonyl)méthylène]azétidine, de 10 cm³ d'une solution 1M de tribromure de bore et 100 cm³ dichlorométhane, le résidu obtenu est purifié par chromatographie sur colonne de gel de silice (granulométrie 0,04-0,06 mm, diamètre 4 cm, hauteur 30 cm) sous une pression de 0,5 bar d'azote avec du dichlorométhane comme éluant et en recueillant des fractions de 40 cm³. Les fractions 15 à 34 sont réunies et concentrées à sec sous pression réduite (2,7 kPa). Le solide obtenu est cristallisé dans 40 cm³ d'éther éthylique. On obtient 0,7 g de 1-benzhydryl-3-[(2-hydroxyphényl)(méthylsulfonyl)méthylène]azétidine fondant à 196°C [Spectre RMN dans DMSO-d6, T=300K, δ en ppm (300 MHz) : 3,00 (3H, s, SCH₃), 3,60 (2H, s, NCH₂), 4,20 (2H, s, NCH₂), 4,75 (1H, s, NCH), 6,85 (1H, t, J=7Hz, CH arom.), 6,90 (1H, d, J=7Hz, CH arom.), 7,20 (4H, m, 4CH arom.), 7,30 (4H, t, J=7Hz, 4CH arom.), 7,48 (4H, d, J=7Hz, 4CH arom.), 9,90 (1H, s, OH)].

Le 1-benzhydryl-3-[(2-méthoxyphényl)(méthylsulfonyl)méthylène]azétidine peut être obtenu de la manière suivante : en opérant selon le mode opératoire de l'exemple 4 à partir de 4,2 g de 1-benzhydryl-3-[(2-méthoxyphényl)(méthylsulfonyl)méthyl-(RS)]azétidin-3-ol, de 1,1 cm³ de chlorure de méthanesulfonyle et 3,5 g de 4-diméthylaminopyridine, le résidu obtenu est purifié par chromatographie sur colonne de gel de silice (granulométrie 0,04-0,06 mm, diamètre 4 cm, hauteur 30 cm) sous une pression de 0,5 bar d'azote avec un mélange dichlorométhane et acétate d'éthyle (50/50 en volumes) comme éluants et en recueillant des fractions de 40 cm³. Les fractions 23 à 54 sont réunies et concentrées à sec sous pression réduite (2,7 kPa). Le solide obtenu est cristallisé dans 40 cm³ d'éther éthylique. On obtient 1,9 g de 1-benzhydryl-3-[(2-méthoxyphényl)(méthylsulfonyl)méthylène]azétidine fondant à 204°C.

Le 1-benzhydryl-3-[(2-méthoxyphényl)(méthylsulfonyl)méthyl-(RS)]azétidin-3-ol peut être obtenu de la manière suivante : en opérant selon le mode opératoire de l'exemple 1 à partir de 4 g de (2-méthoxybenzyl)méthylsulfone et 4,5 g de 1-benzhydryl azétidin-3-one, on obtient 4,3 g de 1-benzhydryl-3-[(2-méthoxyphényl)(méthylsulfonyl)méthyl-(RS)]azétidin-3-ol sous forme d'une meringue brune.

La (2-méthoxybenzyl)méthylsulfone peut être préparée de la manière suivante : en opérant selon le mode opératoire de l'exemple 10 à partir de 3,1 g de chlorure de 2-méthoxybenzyle et 4,1 g de méthanesulfinate de sodium, on obtient 4 g de (2-méthoxybenzyl)méthylsulfone sous forme d'un solide blanc.

### Exemple 35

En opérant selon le mode opératoire de l'exemple 4 à partir de 2,1 g de 1-benzhydryl-3-[(méthylsulfonyl)(napht-2-yl)méthyl-(RS)]azétidin-3-ol, de 0,5 cm³ de chlorure de méthanesulfonyle et 2,2 g de 4-diméthylaminopyridine, le résidu obtenu est purifié par chromatographie sur colonne de gel de silice (granulométrie 0,04-0,06 mm, diamètre 4 cm, hauteur 30 cm) sous une pression de 0,5 bar d'azote avec du dichlorométhane comme éluant et en recueillant des fractions de 100 cm³. Les fractions 6 à 10 sont réunies et concentrées à sec sous pression réduite (2,7 kPa). Le solide obtenu est cristallisé dans 20 cm³ d'éther éthylique. On obtient 0,6 g de 1-benzhydryl-3-[(méthylsulfonyl)(napht-2-yl)méthylène]azétidine fondant à 178°C [Spectre RMN dans DMSO-d6, T=300K, δ en ppm (300 MHz) : 3,00 (3H, s, SCH₃), 3,80 (2H, s, NCH₂), 4,20 (2H, s, NCH₂), 4,75 (1H, s, NCH), 7,20 (4H, m, 4CH arom.), 7,30 (4H, t, J=7Hz, 4CH arom.), 7,45 (4H, d, J=7Hz, 4CH arom.), 7,52 (3H, m, 3CH arom.), 7,90 (4H, m, 4CH arom.)].

Le 1-benzhydryl-3-[(méthylsulfonyl)(napht-2-yl)méthyl-(RS)]azétidin-3-ol peut être obtenu de la manière suivante : en opérant selon le mode opératoire de l'exemple 1 à partir de 3,5 g de méthyl(napht-2-ylméthyl) sulfone et 3,8 g de 1-benzhydryl azétidin-3-one, on obtient 2,2 g de 1-benzhydryl-3-[(méthylsulfonyl)(napht-2-yl)méthyl-(RS)]azétidin-3-ol sous forme d'un solide blanc fondant à 196°C.

La méthyl(napth-2-ylméthyl)sulfone peut être préparée de la manière suivante : en opérant selon le mode opératoire de l'exemple 2 à partir de 4,2 g de méthyl(napth-2-ylméthyl)sulfure et 13,7 g d'oxone^{R}, on obtient 3,6 g de méthyl(napth-2-ylméthyl)sulfone sous forme d'un solide crême.

Le méthyl(napth-2-ylméthyl)sulfure peut être préparé de la manière suivante : en opérant selon le mode opératoire de l'exemple 2 à partir de 5 g de 2-bromométhyl naphtalène et 1,8 g de méthyl thiolate de sodium, on obtient 4,2 g de méthyl(napth-2-ylméthyl)sulfure sous forme d'une huile.

### Exemple 36

En opérant selon le mode opératoire de l'exemple 4 à partir de 4,3 g de 1-benzhydryl-3-[(méthylsulfonyl)(napht-1-yl)méthyl-(RS)]azétidin-3-ol, de 1,1 cm³ de chlorure de méthanesulfonyle et 4,6 g de 4-diméthylaminopyridine, le résidu obtenu est purifié par chromatographie sur colonne de gel de silice (granulométrie 0,04-0,06 mm, diamètre 4 cm, hauteur 30 cm) sous une pression de 0,5 bar d'azote avec du dichlorométhane comme éluant et en recueillant des fractions de 100 cm³. Les fractions 6 à 14 sont réunies et concentrées à sec sous pression réduite (2,7 kPa). Le solide obtenu est cristallisé dans 30 cm³ d'éther éthylique. On obtient 2,5 g de 1-benzhydryl-3-[(méthylsulfonyl)(napht-1-yl)méthylène]azétidine fondant à 196°C [Spectre RMN dans DMSO-d6, T=300K, δ en ppm (300 MHz) : 3,00 (3H, s, SCH₃), 3,35 et 3,50 (1H chacun, dd, J=16 et 3Hz, NCH₂), 4,35 (2H, m, NCH₂), 4,75 (1H, s, NCH), entre 7,10 et 7,70 (14H, m, 14CH arom.), 8,00 (3H, m, 3CH arom.)].

Le 1-benzhydryl-3-[(méthylsulfonyl)(napht-1-yl)méthyl-(RS)]azétidin-3-ol peut être obtenu de la manière suivante : en opérant selon le mode opératoire de l'exemple 1 à partir de 4,1 g de méthyl(napht-1-ylméthyl)sulfone et 4,4 g de 1-benzhydryl azétidin-3-one, on obtient 4,3 g de 1-benzhydryl-3-[(méthylsulfonyl)(1-naphtyl)méthyl-(RS)]azétidin-3-ol sous forme d'un solide.

La méthyl(napht-1-ylméthyl)sulfone peut être préparée de la manière suivante : en opérant selon le mode opératoire de l'exemple 2 à partir de 4,3 g de méthyl(napht-1-ylméthyl)sulfure et 13,9 g d'oxone^{R}, on obtient 4,1 g de méthyl(napht-1-ylméthyl)sulfone sous forme d'un solide blanc.

Le méthyl(napht-1-ylméthyl)sulfure peut être préparé de la manière suivante : en opérant selon le mode opératoire de l'exemple 2 à partir de 4 g de chlorure de 1-chlorométhyl naphtalène et 1,8 g de méthyl thiolate de sodium, on obtient 4,5 g de méthyl(napht-1-ylméthyl)sulfure sous forme d'une huile.

### Exemple 37

En opérant selon le mode opératoire de l'exemple 4 à partir de 0,6 g de 1-benzhydryl-3-[(méthylsulfonyl)(3-pyrrolidinophényl)méthyl-(RS)]azétidin-3-ol, de 0,15 cm³ de chlorure de méthanesulfonyle et 0,6 g de 4-diméthylaminopyridine, le résidu obtenu est purifié par chromatographie sur colonne de gel de silice (granulométrie 0,04-0,06 mm, diamètre 2 cm, hauteur 30 cm) sous une pression de 0,5 bar d'azote avec un mélange dichlorométhane et méthanol (98/2 en volumes) comme éluants et en recueillant des fractions de 20 cm³. Les fractions 8 à 13 sont réunies et concentrées à sec sous pression réduite (2,7 kPa). Le solide obtenu est cristallisé dans 8 cm³ d'éther éthylique. On obtient 0,36 g de 1-benzhydryl-3-[(méthylsulfonyl)(3-pyrrolidinophényl)méthylène]azétidine fondant à 153°C [Spectre RMN dans DMSO-d6, T=300K, δ en ppm (250 MHz) : 1,95 (4H, m, 2 CH₂), 2,95 (3H, s, SCH₃), 3,20 (4H, m, 2 NCH₂), 3,80 (2H, s, NCH₂), 4,20 (2H, s, NCH₂), 4,75 (1H, s, NCH), 6,60 (3H, m, 3CH arom.), 7,20 (3H, m, 3CH arom.), 7,30 (4H, t, J=7Hz, 4CH arom.), 7,48 (4H, d, J=7Hz, 4CH arom.)].

Le 1-benzhydryl-3-[(méthylsulfonyl)(3-pyrrolidinophényl)méthyl-(RS)]azétidin-3-ol peut être obtenu de la manière suivante : en opérant selon le mode opératoire de l'exemple 1 à partir de 0,77 g de 3-pyrrolidinobenzyl méthyl sulfone et 0,76 g de 1-benzhydryl azétidin-3-one, on obtient 0,6 g de 1-benzhydryl-3-[(méthylsulfonyl)(3-pyrrolidinophényl)méthyl-(RS)]azétidin-3-ol sous forme d'un solide.

La méthyl(3-pyrrolidinobenzyl)sulfone peut être préparée de la manière suivante : en opérant selon le mode opératoire de l'exemple 2 à partir de 1 g de méthyl(3-pyrrolidinobenzyl)sulfure et 3,3 g d'oxone^{R}, on obtient 0,8 g de méthyl(3-pyrrolidinobenzyl)sulfone sous forme d'un solide.

Le méthyl(3-pyrrolidinobenzyl)sulfure peut être préparé de la manière suivante : on chauffe à reflux, sous un courant d'azote, pendant 3 heures un mélange de 2g de (3-iodobenzyl)méthylsulfure, 1,3 cm³ de pyrrolidine, 1,1 g de tertiobutylate de sodium, 0,28 g de chlorure de 1,1'-bis(diphénylphosphino)ferrocènyl palladium, 0,63 g de 1,1'-bis(diphénylphosphino)ferrocène et 60 cm³ de tétrahydrofuranne. Le milieu réactionnel est refroidi à température ambiante et filtré sur verre fritté. Le précipité est lavé par 20 cm³ de tétrahydroruranne et 10 cm³ de dichlorométhane puis le filtrat est concentré à sec sous pression réduite (2,7 kPa). Le résidu est repris par 30 cm³ d'acétate d'éthyle et 30 cm³ d'acide chlorhydrique 3N. La phase aqueuse est décantée, neutralisée (pH=7-8) par 35 cm³ de soude 3N et reprise par 50 cm³ d'acétate d'éthyle. La phase organique est extraite; 4g de silice sont ajoutés puis le mélange est concentré à sec sous pression réduite (2,5 kPa). La poudre obtenue est éluée sur verre fritté contenant 20 g de silice par un mélange cyclohexane et acétate d'éthyle (90/10 en volumes). Le filtrat est concentré à sec sous pression réduite (2,7 kPa). On obtient 1,2 g de méthyl(3-pyrrolidinobenzyl)sulfure sous forme d'huile.

Le chlorure de 1,1'-bis(diphénylphosphino)ferrocènyl palladium peut être préparé selon Hayashi T. et coll., J. Am. Chem. Soc., 106, 158 (1984).

### Exemple 38

### Méthode 1

A une solution de 2,94 g de 1-[bis(4-chlorophényl)méthyl]-3-[(3,5-difluorophényl)(méthylsulfonyl)méthyl-(RS)]azétidin-3-ol dans 250 cm³ de dichlorométhane à 22°C, on ajoute 0,65 cm³ de chlorure de méthanesulfonyle puis, par petites portions en 15 minutes, 2,42 g de 4-diméthylamino pyridine; la solution orange est agitée 2 heures à température ambiante. Le mélange réactionnel est lavé 3 fois avec 150 cm³ d'eau distillée et une fois avec 150 cm³ d'une solution saturée de chlorure de sodium, puis séchée avec du sulfate de magnésium, filtrée et concentrée à sec sous pression réduite (2,7 kPa). Le résidu obtenu est chromatographié sur colonne de gel de silice (granulométrie 0,04-0,06 mm, diamètre 5,5 cm, hauteur 15 cm), sous une pression de 0,5 bar d'argon avec un mélange d'acétate d'éthyle et cyclohexane (1/9 en volumes) comme éluants et en recueillant des fractions de 70 cm³. Les fractions 15 à 36 sont réunies puis concentrées à sec sous pression réduite (2,7 kPa). On obtient 1,86 g d'une meringue blanche qui est cristallisée dans de l'éther isopropylique pour obtenir un solide fondant à 190°C. Une recristallisation dans 145 cm³ d'éthanol conduit à 1,08 g de 1-[bis(4-chlorophényl)méthyl]-3-[(3,5-difluorophényl)(méthylsulfonyl)méthylène]azétidine fondant à 206°C [Spectre RMN dans DMSO-d6, T=300K, δ en ppm (300 MHz) : 3,00 (3H,s, SCH₃), 3,87 (2H, s, NCH₂), 4,20 (2H, s, NCH₂), 4,75 (1H, s, NCH), 7,15 (2H, d, J=8Hz, 2CH arom.), 7,30 (5H, m, 5CH arom.), 7,45 (4H, d, J=7Hz, 4CH arom.)].

### Méthode 2

A une solution de 2,2 g de 3-acétoxy-1-[bis(4-chlorophényl)méthyl]-3-[(3,5-difluorophényl)(méthylsulfonyl)méthyl-(RS)]azétidine dans 25 cm³ de dioxane à température ambiante, on ajoute 0,80 g de soude broyée. Après 16 heures à température ambiante, on ajoute 50 cm³ d'eau et 100 cm³ d'acétate d'éthyle. Le mélange est décanté, la phase organique relavée avec 100 cm³ d'eau, séchée sur sulfate de magnésium, filtrée et concentrée à sec sous pression réduite (2,7 kPa). On obtient une meringue blanche qui est cristallisée dans de l'éther d'isopropyle pour obtenir 0,85 g d'un solide fondant à 190°C. Une recristallisation dans 20 cm³ d'éthanol conduit à 0,70 g de 1-[bis(4-chlorophényl)méthyl]-3-[(3,5-difluorophényl)(méthylsulfonyl)méthylène]azétidine fondant à 205°C.

### Exemple 39

A une solution de 6,8 g de bis(4-chlorophényl)bromométhane dans 300 cm³ d'acétonitrile, on ajoute 6,75 g de chlorhydrate de 3-[(3,5-difluorophényl)(méthylsulfonyl)méthyl-(RS)]azétidin-3-ol puis 2,97 g de carbonate de potassium. Le mélange réactionnel est chauffé 1 heure au reflux, refroidi à température ambiante, filtré et concentré à sec sous pression réduite (2,7 kPa). Le résidu obtenu est chromatographié sur colonne de gel de silice (granulométrie 0,04-0,06 mm, diamètre 8,5 cm, hauteur 22 cm), sous une pression de 0,5 bar d'argon avec un mélange d'acétate d'éthyle et cyclohexane (25/75 en volumes) comme éluants et en recueillant des fractions de 250 cm³. Les fractions 11 à 48 sont réunies puis concentrées à sec sous pression réduite (2,7 kPa). On obtient 5,3 g de 1-[bis(4-chlorophényl)méthyl]-3-[(3,5-difluorophényl)(méthylsulfonyl)méthyl-(RS)]azétidin-3-ol [Spectre de R.M.N ¹H (300 MHz, (CD₃)₂SO d6, δ en ppm) : 2,00 (s : 3H); 2,94 (s : 3H); 3,25 (mt : 2H); 3,48 (d, J = 9 Hz : 1H); 3,80 (d, J = 9 Hz : 1H); 4,54 (s : 1H); 5,34 (s : 1H); 7,15 (d, J = 8,5 Hz : 2H); de 7,20 à 7,40 (mt : 8H); 7,50 (t large, J = 9 Hz : 1H)].

Le bis(4-chlorophényl)bromométhane peut être préparé selon le mode opératoire décrit par BACHMANN W.E., J. Am. Chem. Soc., 2135 (1933).

Le chlorhydrate de 3-[(3,5-difluorophényl)(méthylsulfonyl)méthyl-(RS)]azétidin-3-ol peut être obtenu de la manière suivante : à une solution de 37 g de 3-[(3,5-difluorophényl)(méthylsulfonyl)méthyl-(RS)]-1-(vinyloxycarbonyl)azétidin-3-ol dans 160 cm³ de dioxane on ajoute 160 cm³ d'une solution 6,2N d'acide chlorhydrique dans le dioxane. Après 16 heures à température ambiante, le mélange réactionnel est concentré à sec sous pression réduite (2,7 kPa). Le résidu obtenu est repris avec 320 cm³ d'éthanol, chauffé 1 heure au reflux et refroidi dans un bain d'eau glacée. Le solide apparu est filtré, lavé à l'éther éthylique et séché à 40°C sous pression réduite (2,7 kPa). On obtient 29,85 g de cristaux blancs dont la température de fusion est supérieure à 260°C.

Le 3-[(3,5-difluorophényl)(méthylsulfonyl)méthyl-(RS)]-1-(vinyloxycarbonyl)azétidin-3-ol peut être obtenu de la manière suivante : à une solution de 60,18 g de 1-benzhydryl-3-[(3,5-difluorophényl)(méthylsulfonyl)méthyl-(RS)]azétidin-3-ol dans 1000 cm³ de dichlorométhane on ajoute à 5°C une solution de 14,0 cm³ de chloroformiate de vinyle dans 35 cm³ de dichlorométhane. Après 20 heures à température ambiante, le mélange réactionnel est concentré à sec sous pression réduite (2,7 kPa). Le résidu obtenu est chromatographié sur colonne de gel de silice (granulométrie 0,04-0,06 mm, diamètre 11 cm, hauteur 32 cm), sous une pression de 0,5 bar d'argon avec un mélange d'acétate d'éthyle et cyclohexane (3/7 en volumes) comme éluants et en recueillant des fractions de 1000 cm³. Les fractions 8 à 18 sont réunies puis concentrées à sec sous pression réduite (2,7 kPa). On obtient 37 g de 3-[(3,5-difluorophényl)(méthylsulfonyl)méthyl-(RS)]-1-(vinyloxycarbonyl)azétidin-3-ol sous forme de cristaux blancs fondants à 195°C

### Exemple 40

A une solution de 4,77 g de (3,5-difluorobenzyl)méthylsulfone dans 70 cm³ de tétrahydrofuranne sous atmosphère d'argon, on ajoute à -70°C, 14 cm³ d'une solution 1,6N de n butyllithium dans l'hexane. Après 1 heure à -70°C, une solution de 6,8 g de 1-[bis(4-chlorophényl)méthyl]azétidin-3-one dans 30 cm³ de tétrahydrofuranne est additionnée puis, 1 heure après, une solution de 2,34 cm³ de chlorure d'acétyle dans 20 cm³ de tétrahydrofuranne et la température du mélange réactionnel est élevée à 20°C pendant 1 heure. On ajoute 50 cm³ d'eau et 200 cm³ d'acétate d'éthyle. Le mélange est décanté, la phase organique lavée avec 100 cm³ d'eau, 100 cm³ d'une solution saturée de chlorure de sodium, séchée sur sulfate de magnésium, filtrée et concentrée à sec sous pression réduite (2,7 kPa). On obtient 14,4 g de 3-acétoxy-1-[bis-(4-chlorophényl)méthyl]-3-[(3,5-difluorophényl)(méthylsulfonyl)méthyl)méthyl sulfonylméthyl-(RS)]azétidine sous forme d'une huile jaune [Spectre de R.M.N ¹H (400 MHz, CDCl₃, δ en ppm) : 2,79 (s : 3H); 3,04 (AB, J = 9 Hz : 2H); 3,27 (d, J = 9 Hz : 1H); 3,45 (s : 1H); 3,81 (d, J = 9 Hz : 1H); 4,32 (s : 1H); 4,49 (s : 1H); 6,88 (tt, J = 9 et 2,5 Hz : 1H); de 7,20 à 7,35 (mt : 10H)].

La 1-[bis(4-chlorophényl)méthyl]azétidin-3-one peut être préparée selon le mode opératoire suivant : à une solution de 5,0 cm³ de chlorure d'oxalyle dans 73 cm³ de dichlorométhane refroidie à -78°C, on additionne une solution de 8,1 cm³ de diméthylsulfoxyde dans 17,6 cm³ de dichlorométhane. Après 0,5 heure à -78°C, on coule une solution de 16,0 g de 1-[bis(4-chlorophényl)méthyl]azétidin-3-ol dissous dans 50 cm³ de dichlorométhane. Après 5 heures à -78°C, 26,6 cm³ de triéthylamine sont ajoutés goutte à goutte et on laisse le mélange réactionnel revenir à température ambiante. Après 16 heures, le mélange réactionnel est lavé par 4 fois 200 cm³ d'eau puis par 200 cm³ d'une solution saturée de chlorure de sodium, séché sur sulfate de magnésium, filtré et concentré à sec sous pression réduite (2,7 kPa). Le résidu obtenu est chromatographié sur colonne de gel de silice (granulométrie 0,04-0,06 mm, diamètre 9,2 cm, hauteur 21 cm), sous une pression de 0,5 bar d'argon avec un mélange d'acétate d'éthyle et cyclohexane (40/60 en volumes) comme éluants et en recueillant des fractions de 200 cm³. Les fractions 15 à 25 sont réunies puis concentrées à sec sous pression réduite (2,7 kPa). On obtient 8,9 g de 1-[bis(4-chlorophényl)méthyl]azétidin-3-one sous forme de cristaux jaunes pâle fondants à 111°C.

Le 1-[bis(4-chlorophényl)méthyl]azétidin-3-ol peut être préparée selon le mode opératoire décrit par KATRITZKY A.R. et coll., J. Heterocycl. Chem., (1994), 271 en partant de 35,5 g de chlorhydrate de [bis(4-chlorophényl)méthyl]amine et 11,0 cm³ d'épichlorhydrine. On isole 9,0 g de 1-[bis(4-chlorophényl)méthyl]azétidin-3-ol.

Le chlorhydrate de [bis(4-chlorophényl)méthyl]amine peut être préparé selon la méthode décrite par GRISAR M. et coll., J. Med. Chem., 885 (1973).

### Exemple 41

En opérant selon le mode opératoire de l'exemple 38 (méthode 1), à partir de 0,72 g de 1-[bis(4-méthoxyphényl)méthyl]-3-[(3,5-difluorophényl)(méthylsulfonyl)méthyl-(RS)]azétidin-3-ol et après chromatographie sur colonne de gel de silice (granulométrie 0,04-0,06 mm, diamètre 4,0 cm, hauteur 16,5 cm), sous une pression de 0,5 bar d'argon avec un mélange d'acétate d'éthyle et cyclohexane (2/8 en volumes) comme éluant et en recueillant des fractions de 40 cm³, on obtient 0,10 g d'une meringue blanche. Après cristallisation dans un mélange d'acétate d'éthyle et cyclohexane on obtient 60 mg de 1-[bis(4-méthoxyphényl)méthyl]-3-[(3,5-difluorophényl)(méthylsulfonyl)méthylène]azétidine sous la forme d'un solide fondant à 180°C [Spectre RMN dans DMSO-d6, T=300K, δ en ppm (250 MHz) : 3,00 (3H,s, SCH₃), 3,70 (6H,s, 2 OCH₃), 3,80 (2H, s, NCH₂), 4,15 (2H, s, NCH₂), 4,58 (1H, s, NCH), 6,85 (4H, d, J=7Hz, 4CH arom.), 7,15 (2H, d, J=8Hz, 2CH arom.), 7,30 (5H, m, SCH arom.)].

Le 1-[bis(4-méthoxyphényl)méthyl]-3-[(3,5-difluorophényl)(méthylsulfonyl)méthyl-(RS)]azétidin-3-ol peut être obtenu de la manière suivante : en opérant selon l'exemple 39 à partir de 1,2 g de bis(4-méthoxyphényl)bromométhane et 1,2 g de chlorhydrate de 3-[(3,5-difluorophényl)(méthylsulfonyl)méthyl-(RS)]azétidin-3-ol et après chromatographie sur colonne de gel de silice (granulométrie 0,04-0,06 mm, diamètre 4,8 cm, hauteur 18 cm), sous une pression de 0,5 bar d'argon avec un mélange d'acétate d'éthyle et cyclohexane (25/75 en volumes) comme éluant et en recueillant des fractions de 50 cm³, les fractions 9 à 18 sont réunies puis concentrées à sec sous pression réduite (2,7 kPa). On obtient 0,55 g de 1-[bis(4-méthoxyphényl)méthyl]-3-[(3,5-difluorophényl)(méthylsulfonyl)méthyl-(RS)]azétidin-3-ol.

Le bis(4-méthoxyphényl)bromométhane peut être préparé selon le mode opératoire décrit par BACHMANN W.E., J. Am. Chem. Soc., 2135 (1933).

### Exemple 42

En opérant selon l'exemple 38 (méthode 1), à partir de 0,47 g de 1-[bis(4-méthylphényl)méthyl]-3-[(3,5-difluorophényl)(méthylsulfonyl)méthyl-(RS)]azétidin-3-ol et après chromatographie sur colonne de gel de silice (granulométrie 0,04-0,06 mm, diamètre 3,2 cm, hauteur 18,5 cm), sous une pression de 0,5 bar d'argon avec un mélange d'acétate d'éthyle et cyclohexane (1/9 en volumes) comme éluant et en recueillant des fractions de 35 cm³, on obtient 0,30 g d'un solide blanc. Après cristallisation dans de l'oxyde de diisopropyle on obtient 0,20 g de 1-[bis(4-méthylphényl)méthyl]-3-[(3,5-difluorophényl)(méthylsulfonyl)méthylène]azétidine sous la forme d'aiguilles blanches fondant à 200°C.

Le 1-[bis(4-méthylphényl)méthyl]-3-[(3,5-difluorophényl)(méthylsulfonyl)méthyl-(RS)]azétidin-3-ol peut être obtenu de la manière suivante : en opérant comme à l'exemple 39 à partir de 0,7 g de bis(4-méthylphényl)bromométhane et 0,8 g de chlorhydrate de 3-[(3,5-difluorophényl)(méthylsulfonyl)méthyl-(RS)]azétidin-3-ol et après chromatographie sur colonne de gel de silice (granulométrie 0,04-0,06 mm, diamètre 4,0 cm, hauteur 19 cm), sous une pression de 0,5 bar d'argon avec un mélange d'acétate d'éthyle et cyclohexane (2/8 en volumes) comme éluant et en recueillant des fractions de 40 cm³, les fractions 35 à 40 sont réunies puis concentrées à sec sous pression réduite (2,7 kPa). On obtient 0,47 g de 1-[bis(4-méthylphényl)méthyl]-3-[(3,5-difluorophényl)(méthylsulfonyl)méthyl-(RS)]azétidin-3-ol.

Le bis(4-méthylphényl)bromométhane peut être préparé selon le mode opératoire décrit par BACHMANN W.E., J. Am. Chem. Soc., 2135 (1933).

### Exemple 43

En opérant selon l'exemple 38 (méthode 1), à partir de 1,42 g de 3-[(3,5-difluorophényl)(méthylsulfonyl)méthyl-(RS)]-1-[(4-méthoxyphényl)(phényl)méthyl-(RS)]azétidin-3-ol, mélange des deux diastéréoisomères, et après chromatographie sur colonne de gel de silice (granulométrie 0,04-0,06 mm, diamètre 4,0 cm, hauteur 21 m), sous une pression de 0,5 bar d'argon avec un mélange d'acétate d'éthyle et cyclohexane (2/8 en volumes) comme éluant et en recueillant des fractions de 40 cm³, on obtient 0,10 g d'un solide blanc. Après cristallisation dans de l'oxyde de diisopropyle on obtient 50 mg de (RS)-3-[(3,5-difluorophényl)(méthylsulfonyl) méthylène]-1-(4-méthoxyphényl)(phényl)méthyl]azétidine sous la forme d'un solide blanc [Spectre RMN dans DMSO-d6, T=300K, δ en ppm (300 MHz) : 2,23 (6H,s, 2 PhCH₃), 3,00 (3H,s, SCH₃), 3,80 (2H, s, NCH₂), 4,12 (2H, s, NCH₂), 4,58 (1H, s, NCH), 7,08 (4H, d, J=7Hz, 4CH arom.), 7,15 (2H, d, J=8Hz, 2CH arom.), 7,25 (5H, m, 5CH arom.)].

Le mélange de diastéréoisomères 3-[(3,5-difluorophényl)(méthylsulfonyl)méthyl-(RS)]-1-[(4-méthoxyphényl)(phényl)méthyl-(RS)]azétidin-3-ol peut être obtenu de la manière suivante : en opérant selon l'exemple 39 à partir de 2,52 g (RS)-bromo(4-méthoxyphényl)(phényl)méthane et 2,85 g de chlorhydrate de 3-[(3,5-difluorophé-nyl)(méthylsulfonyl)méthyl-(RS)]azétidin-3-ol et après chromatographie sur colonne de gel de silice (granulométrie 0,04-0,06 mm, diamètre 5,6 cm, hauteur 19 cm), sous une pression de 0,5 bar d'argon avec un mélange d'acétate d'éthyle et cyclohexane (25/75 en volumes) comme éluant et en recueillant des fractions de 100 cm³, les fractions 11 à 18 sont réunies puis concentrées à sec sous pression réduite (2,7 kPa). On obtient 1,16 g du mélange de diastéréoisomères 3-[(3,5-difluorophényl)(méthylsulfonyl)méthyl-(RS)]-1-[(4-méthoxyphényl)(phényl)méthyl-(RS)]azétidin-3-ol.

Le (RS)-bromo(4-méthoxyphényl)(phényl)méthane peut être préparé selon le mode opératoire décrit par BACHMANN W.E., J. Am. Chem. Soc., 2135 (1933).

### Exemple 44

En opérant comme à l'exemple 38 (méthode 1), à partir de 0,47 g de 1-[bis(4-trifluorométhoxyphényl)méthyl]-3-[(3,5-difluorophényl)(méthylsulfonyl)méthyl-(RS)] azétidin-3-ol et après chromatographie sur colonne de gel de silice (granulométrie 0,04-0,06 mm, diamètre 4,2 cm, hauteur 14 cm), sous une pression de 0,5 bar d'argon avec un mélange d'acétate d'éthyle et cyclohexane (2/8 en volumes) comme éluant et en recueillant des fractions de 25 cm³, on obtient 0,28 g de 1-[bis(4-trifluorométhoxyphényl)méthyl]-3-[(3,5-difluorophényl)(méthylsulfonyl)méthylène]azétidine sous la forme d'un solide [Spectre RMN dans DMSO-d6, T=300K, δ en ppm (300 MHz) : 3,05 (3H,s, SCH₃), 3,95 (2H, s, NCH₂), 4,25 (2H, s, NCH₂), 4,90 (1H, s, NCH), 7,20 (2H, d, J=8Hz, 2CH arom.), 7,32 (5H, m, SCH arom.), 7,60 (4H, d, J=7Hz, 4CH arom.)].

Le 1-[bis(4-trifluorométhoxyphényl)méthyl]-3-[(3,5-difluorophényl)(méthylsulfonyl)méthyl-(RS)]azétidin-3-ol peut être obtenu de la manière suivante : en opérant comme à l'exemple 39 à partir de 1,59 g de bis(4-trifluorométhoxyphényl)bromométhane et 1,2 g de chlorhydrate de 3-[(3,5-difluorophényl)(méthylsulfonyl)méthyl-(RS)]azétidin-3-ol et après chromatographie sur colonne de gel de silice (granulométrie 0,04-0,06 mm, diamètre 4,8 cm, hauteur 17 cm), sous une pression de 0,5 bar d'argon avec un mélange d'acétate d'éthyle et cyclohexane (25/75 en volumes) comme éluant et en recueillant des fractions de 50 cm³, les fractions 15 à 23 sont réunies puis concentrées à sec sous pression réduite (2,7 kPa). On obtient 0,49 g de 1-[bis(4-trifluorométhoxyphényl)méthyl]-3-[(3,5-difluorophényl)(méthylsulfonyl)méthyl]azétidin-3-ol.

Le bis(4-trifluorométhoxyphényl)bromométhane peut être préparé selon le mode opératoire décrit par BACHMANN W.E., J. Am. Chem. Soc., 2135 (1933), en partant de 1,39 g de bis(4-trifluorométhoxyphényl)méthanol, 3 cm³ d'acide bromhydrique à 33% dans l'acide acétique et 0,6 cm³ de bromure d'acétyle. On obtient 1,59g de bis(4-trifluorométhoxyphényl)bromométhane sous la forme d'une huile marron.

Le bis(4-trifluorométhoxyphényl)méthanol est préparé selon PAVIA M.R. et coll., J. Med. Chem., 4238 (1992).

### Exemple 45

En opérant comme à l'exemple 38 (méthode 1), à partir de 0,25 g de 1-[bis(4-trifluorométhylphényl)méthyl]-3-[(3,5-difluorophényl)(méthylsulfonyl)méthyl-(RS)]azétidin-3-ol et après chromatographie sur colonne de gel de silice (granulométrie 0,04-0,06 mm, diamètre 2,4 cm, hauteur 14 cm), sous une pression de 0,5 bar d'argon avec un mélange d'acétate d'éthyle et cyclohexane (2/8 en volumes) comme éluant et en recueillant des fractions de 20 cm³, on obtient 0,12 g de 1-[bis(4-trifluorométhylphényl)méthyl]-3-[(3,5-difluorophényl)(méthylsulfonyl) méthylène]azétidine sous la forme d'un solide blanc [Spectre RMN dans DMSO-d6, T=300K, δ en ppm (300 MHz) : 3,05 (3H,s, SCH₃), 3,95 (2H, s, NCH₂), 4,25 (2H, s, NCH₂), 4,90 (1H, s, NCH), 7,20 (2H, d, J=8Hz, 2CH arom.), 7,32 (5H, m, 5CH arom.), 7,60 (4H, d, J=7Hz, 4CH arom.)].

Le 1-[bis(4-trifluorométhylphényl)méthyl]-3-[(3,5-difluorophényl)(méthylsulfonyl)méthyl-(RS)]azétidin-3-ol peut être obtenu de la manière suivante : en opérant comme à l'exemple 39 à partir de 1,46 g de bis(4-trifluorométhylphényl)bromométhane et 1,2 g de chlorhydrate de 3-[(3,5-difluorophényl)(méthylsulfonyl)méthyl-(RS)]azétidin-3-ol et après chromatographie sur colonne de gel de silice (granulométrie 0,04-0,06 mm, diamètre 4,8 cm, hauteur 17 cm), sous une pression de 0,5 bar d'argon avec un mélange d'acétate d'éthyle et cyclohexane (30/70 en volumes) comme éluant et en recueillant des fractions de 50 cm³, les fractions 9 à 14 sont réunies puis concentrées à sec sous pression réduite (2,7 kPa).

On obtient 0,25 g de 1-[bis(4-trifluorométhylphényl)méthyl]-3-[(3,5-difluorophényl)(méthylsulfonyl)méthyl-(RS)]azétidin-3-ol .

Le bis(4-trifluorométhylphényl)bromométhane peut être préparé selon le mode opératoire décrit par BACHMANN W.E., J. Am. Chem. Soc., 2135 (1933), en partant de 2,5 g de bis(4-trifluorométhylphényl)méthanol, 6 cm³ d'acide bromhydrique à 33% dans l'acide acétique et 1,2 cm³ de bromure d'acétyle. On obtient 2,9 g de bis(4-trifluorométhylphényl)bromométhane sous la forme d'une huile marron.

Le bis(4-trifluorométhylphényl)méthanol est préparé selon PAVIA M.R. et coll., J. Med. Chem., 4238 (1992).

### Exemple 46

En opérant selon l'exemple 38 (méthode 2), à partir de 3,16 g de 3-acétoxy-1-[bis(4-chlorophényl)méthyl]-3-{[3,5-bis(trifluorométhyl)phényl](méthylsulfonyl)méthyl(RS)}azétidine et 0,96 g de soude broyée, on obtient, après 16 heures à température ambiante, une meringue jaune qui est chromatographiée sur colonne de gel de silice (granulométrie 0,04-0,06 mm, diamètre 4,8 cm, hauteur 14 cm), sous une pression de 0,5 bar d'argon avec un mélange d'acétate d'éthyle et cyclohexane (15/85 en volumes) comme éluant et en recueillant des fractions de 40 cm³. On obtient ainsi 1,49 g de 1-[bis(4-chlorophényl)méthyl]-3- {[3,5-bis(trifluorométhyl)phényl](mé-thylsulfonyl)méthylène}azétidine sous la forme d'une meringue blanche [Spectre RMN dans DMSO-d6, T=300K, δ en ppm (300 MHz) : 3,05 (3H,s, SCH₃), 3,90 (2H, s, NCH₂), 4,30 (2H, s, NCH₂), 4,80 (1H, s, NCH), 7,40 (2H, d, J=7Hz, 2CH arom.), 7,50 (2H, d, J=7Hz, 2CH arom.), 8,10 (2H, s, 2CH arom.), 8,20 (1H, s, CH arom.)].

Le 3-acétoxy-1-[bis(4-chlorophényl)méthyl]-3-{[3,5-bis(trifluorométhyl)phényl] (méthylsulfonyl)méthyl-(RS)}azétidine peut être obtenu de manière suivante : en opérant comme à l'exemple 40 à partir de 2,0 g de [3,5-bis(trifluorométhyl)benzyl]méthylsulfone, 4,1 cm³ d'une solution 1,6N de nbutyllithium dans l'hexane, 2,0 g de 1-[bis(4-chlorophényl)méthyl]azétidin-3-one et 0,77 cm³ de chlorure d'acétyle dans 20 cm³ de diisopropyl oxyde anhydre, on obtient, après chromatographie sur colonne de gel de silice (granulométrie 0,04-0,06 mm, diamètre 5,6 cm, hauteur 16 cm), sous une pression de 0,5 bar d'argon avec un mélange d'acétate d'éthyle et cyclohexane (1/9 en volumes) comme éluant et en recueillant des fractions de 100 cm³, 3,56 g de 3-acétoxy-1-[bis(4-chlorophényl)méthyl]-3-{[3,5-bis(trifluorométhyl)phényl](méthylsulfonyl)méthyl-(RS)}azétidine sous forme d'une meringue blanche.

La [3,5-bis(trifluorométhyl)benzyl]méthylsulfone est préparée de la manière suivante : en opérant selon l'exemple 10 à partir de 1,8 g de chlorure de 3,5-bis(trifluorométhyl)benzyle et 1,22 g de méthanesulfinate de sodium, on obtient 1,86 g de [3,5-bis(trifluorométhyl)benzyl]méthylsulfone sous forme d'un solide blanc.

### Exemple 47

En opérant comme à l'exemple 38 (méthode 1), à partir de 0,27 g du mélange des deux diastéréoisomères 1-[(4-chlorophényl)(2,4-dichlorophényl)méthyl-(RS)]-3-[(3,5-difluorophényl)(méthylsulfonyl)méthyl-(RS)]azétidin-3-ol et après chromatographie sur colonne de gel de silice (granulométrie 0,04-0,06 mm, diamètre 2,4 cm, hauteur 7,5 cm), sous une pression de 0,5 bar d'argon avec un mélange d'acétate d'éthyle et cyclohexane (15/85 en volumes) comme éluant et en recueillant des fractions de 20 cm³, on obtient 0,10 g de (RS)-1-[(4-chlorophényl)(2,4-dichlorophényl)méthyl]-3-[(3,5-difluorophényl)(méthylsulfonyl)méthylène]azétidine sous la forme d'un solide blanc [Spectre RMN dans DMSO-d6, T=300K, δ en ppm (250 MHz) : 3,02 (3H,s, SCH₃), 3,82 (1H, dd, J=3 et 16Hz, NCHH), 4,04 (1H, dd, J=3 et 16Hz, NCHH), 4,10 (1H, dd, J=3 et 16Hz, NCHH), 4,35 (1H, dd, J=3 et 16Hz, NCHH), 5,12 (1H, s, NCH), 7,18 (2H, d, J=8Hz, 2CH arom.), 7,32 (1H, t, J=8Hz, CH arom.), 7,38 (2H, d, J=7Hz, 2CH arom.), 7,45 (2H, d, J=7Hz, 2CH arom.), 7,48 (1H, dd, J=2 et 7Hz, CH arom.), 7,58 (1H, d, J=2Hz, CH arom.), 7,80 (1H, d, J=7Hz, CH arom.)].

Le mélange des deux diastéréoisomères 1-[(4-chlorophényl)(2,4-dichlorophényl)méthyl-(RS)]-3-[(3,5-difluorophényl)(méthylsulfonyl)méthyl-(RS)]azétidin-3-ol peut être obtenu de la manière suivante : en opérant selon l'exemple 39 à partir de 0,56 g de (RS)-bromo(4-chlorophényl)(2,4-dichlorophényl)méthane et 0,50 g de chlorhydrate de 3-[(3,5-difluorophényl)(méthylsulfonyl)méthyl-(RS)]azétidin-3-ol et après chromatographie sur colonne de gel de silice (granulométrie 0,04-0,06 mm, diamètre 4,0 cm, hauteur 13 cm), sous une pression de 0,5 bar d'argon avec un mélange d'acétate d'éthyle et cyclohexane (20/80 en volumes) comme éluant et en recueillant des fractions de 40 cm³, les fractions 9 à 14 sont réunies puis concentrées à sec sous pression réduite (2,7 kPa). On obtient 0,27 g du mélange des deux diastéréoisomères 1-[(4-chlorophényl)(2,4-dichlorophényl)méthyl-(RS)]-3-[(3,5-difluorophényl)(méthylsulfonyl)méthyl-(RS)]azétidin-3-ol.

Le (RS)-bromo(4-chlorophényl)(2,4-dichlorophényl)méthane peut être préparé selon le mode opératoire décrit par BACHMANN W.E., J. Am. Chem. Soc., 2135 (1933) en partant de 4,05 g de (RS)-(4-chlorophényl)(2,4-dichlorophényl)méthanol, 10 cm³ d'acide bromhydrique à 33% dans l'acide acétique et 2,1 cm³ de bromure d'acétyle. On obtient 4,6 g de (RS)-bromo(4-chlorophényl)(2,4-dichlorophényl)méthane sous la forme d'une huile verdâtre.

Le (RS)-(4-chlorophényl)(2,4-dichlorophényl)méthanol est préparé selon PAVIA M.R. et coll., J. Med. Chem., 4238 (1992).

### Exemple 48

A une solution de 18,9 g de 1-{(4-chlorophényl)[4-(1,3-dioxolan-2-yl)phényl]méthyl-(RS)}-3-[(3,5-difluorophényl)(méthylsulfonyl)méthylène]azétidine dans 80 cm³ de tétrahydrofuranne, on ajoute 75,6 cm³ d'acide chlorhydrique 5N. Après 3 heures à température ambiante, le mélange est repris par du dichlorométhane et l'eau distillée puis amené à pH 14 par ajout de soude 30% et décanté. La phase organique est lavée 2 fois avec 100 cm³ d'eau puis 100 cm³ d'une solution aqueuse saturée de chlorure de sodium, séchée sur sulfate de magnésium, filtrée et concentrée à sec sous pression réduite (2,7 kPa). On obtient 16 g de (RS)-1-[(4-chlorophényl)(4-formylphényl)méthyl]-3-[(3,5-difluorophényl)(méthylsulfonyl)méthylène]azétidine sous forme d'une meringue blanche [Spectre dans DMSO-d6, T=300K, δ en ppm (300 MHz) : 3,06 (3H,s, SCH₃), 3,95 (2H, m, NCH₂), 4,26 (2H, m, NCH₂), 4,91 (1H, s, NCH), 7,20 (2H, d, J=8Hz, 2CH arom.), 7,36 (1H, t, J=8 Hz, 1CH arom.), 7,40 et 7,52 (4H, 2d, J=7,5Hz, 4CH arom.), 7,70 et 7,88 (4H, 2d, J=7,5Hz, 4CH arom.), 9,97(1H, s, CH aldéhydique)].

La 1-{(4-chlorophényl)[4-(1,3-dioxolan-2-yl)phényl]méthyl-(RS)}-3-[(3,5-difluorophényl)(méthylsulfonyl)méthylène]azétidine peut être préparé selon la méthode suivante : à une solution de 34,45 g du mélange des deux diastéréoisomères 3-acétoxy-1-{(4-chlorophényl)[4-(1,3-dioxolan-2-yl)phényl]méthyl-(RS)}-3-[(3,5-difluorophényl)(méthylsulfonyl)méthyl-(RS)]azétidine dans 400 cm³ de tétrahydrofuranne sous argon à 0°C, on ajoute goutte à goutte 13,0 cm³ de 1,8-diazabicyclo[5-4-0]undec-7-éne et après traitement habituel et après chromatographie sur colonne de gel de silice (granulométrie 0,04-0,06 mm, diamètre 10,2 cm, hauteur 23 cm), sous une pression de 0,5 bar d'argon avec un mélange d'acétate d'éthyle et cyclohexane (2/8 en volumes) comme éluant et en recueillant des fractions de 250 cm³, on obtient 16,6 g de 1-{(4-chlorophényl)[4-(1,3-dioxolan-2-yl)phényl]méthyl-(RS)}-3-[(3,5-difluorophényl)(méthylsulfonyl)méthy-lène]azétidine sous la forme d'un solide blanc.

Le mélange des deux diastéréoisomères 3-acétoxy-1-{(4-chlorophényl)[4-(1,3-dioxolan-2-yl)phényl]méthyl-(RS)}-3-[(3,5-difluorophényl)(méthylsulfonyl)méthyl-(RS)]azétidine peut être obtenu de la manière suivante : en opérant selon l'exemple 40, à partir de 11,6 g de (3,5-difluorobenzyl)méthylsulfone, 35,1 cm³ d'une solution 1,6N de n butyllithium dans l'hexane, 19,3 g de 1-{(4-chlorophényl)[4-(1,3-dioxolan-2-yl)phényl]méthyl-(RS)}azétidin-3-one et 8,8 cm³ de chlorure d'acétyle dans 500 cm³ de tétrahydrofuranne, on obtient 37,8 g du mélange des deux diastéréoisomères 3-acétoxy-1-{(4-chlorophényl)[4-(1,3-dioxolan-2-yl)phényl]méthyl-(RS)}-3-[(3,5-difluorophényl)(méthylsulfonyl)méthyl-(RS)]azétidine sous forme d'une meringue blanche.

La 1-{(4-chlorophényl)[4-(1,3-dioxolan-2-yl)phényl]méthyl-(RS)}azétidin-3-one peut être préparée de la manière suivante : à une solution de 28,32 g de 1-{(4-chlorophényl)[4-(1,3-dioxolan-2-yl)phényl]méthyl-(RS)}azétidin-3-ol dans 200 cm³ de diméthylsulfoxyde, on ajoute à température ambiante 46 cm³ de triéthylamine puis on additionne goutte à goutte une solution de 34 g du complexe trioxyde de soufre-pyridine dans 100 cm³ diméthylsulfoxyde. Après 0,25 heure à température ambiante, le mélange réactionnel est versé sur de la glace, extrait avec de l'acétate d'éthyle, lavé par 3 fois 400 cm³ d'eau puis par 400 cm³ d'une solution saturée de chlorure de sodium, séché sur sulfate de magnésium, filtré et concentré à sec sous pression réduite (2,7 kPa). Le résidu obtenu est chromatographié sur colonne de gel de silice (granulométrie 0,04-0,06 mm, diamètre 9,2 cm, hauteur 21 cm), sous une pression de 0,5 bar d'argon avec un mélange d'acétate d'éthyle et cyclohexane (20/80 en volumes) comme éluant et en recueillant des fractions de 250 cm³. Les fractions 9 à 18 sont réunies puis concentrées à sec sous pression réduite (2,7 kPa). On obtient 20,4 g de 1-{(4-chlorophényl)[4-(1,3-dioxolan-2-yl)phényl]méthyl-(RS)}azétidin-3-one sous la forme d'une huile jaune.

Le 1-{(4-chlorophényl)[4-(1,3-dioxolan-2-yl)phényl]méthyl-(RS)}azétidin-3-ol peut être préparé selon le mode opératoire décrit par KATRITZKY A.R. et coll., J. Heterocycl. Chem., 271 (1994) en partant de 35,0 g de {(4-chlorophényl)[4-(1,3-dioxolan-2-yl)phényl]méthyl}amine, 8,3 g d'épibromhydrine, 5,1 g d'hydrogénocarbonate de sodium et 400 cm³ d'éthanol. On isole 30,3 g de 1-{(4-chlorophényl)[4-(1,3-dioxolan-2-yl)phényl]méthyl-(RS)}azétidin-3-ol.

Le chlorhydrate de {(4-chlorophényl)[4-(1,3-dioxolan-2-yl)phényl]méthyl-(RS)}amine peut être préparé selon la méthode décrite par GRISAR M. et coll., J. Med. Chem., 885 (1973) à partir de 67,2 g de 4-(1,3-dioxolan-2-yl)benzonitrile, 88,2 g de 1-bromo-4-chlorobenzène, 11 g de magnésium et 600 cm³ d'éther éthylique. On obtient 42,3 g de {(4-chlorophényl)[4-(1,3-dioxolan-2-yl)phényl]méthyl-(RS)}amine sous forme d'une huile jaune.

### Exemple 49

A une solution de 0,50 g de (RS)-1-{(4-chlorophényl)(4-formylphényl)méthyl}-3-[(3,5-difluorophényl)(méthylsulfonyl)méthylène]azétidine dans 15 cm³ de méthanol à 0°C, on ajoute 0,020 g de borohydrure de sodium. Après 1 heure à 0°C, 40 cm³ d'eau sont additionnées et le produit extrait avec 100 cm³ de dichlorométhane. La phase organique est lavée 2 fois avec 40 cm³ d'eau puis 40 cm³ d'une solution saturée de chlorure de sodium, séchée sur sulfate de magnésium, filtrée et concentrée à sec sous pression réduite (2,7 kPa). Le résidu obtenu est chromatographié sur colonne de gel de silice (granulométrie 0,04-0,06 mm, diamètre 3,2 cm, hauteur 14 cm), sous une pression de 0,5 bar d'argon avec un mélange d'acétate d'éthyle et cyclohexane (30/70 en volumes) et en recueillant des fractions de 20 cm³. Les fractions 20 à 25 sont réunies puis concentrées à sec sous pression réduite (2,7 kPa). On obtient 0,29 g de (RS)-1-{(4-chlorophényl)[4-(hydroxyméthyl)phényl]méthyl}-3-[(3,5-difluorophényl)(méthylsulfonyl)méthylène]azétidine sous forme d'une meringue blanche [Spectre RMN dans DMSO-d6, T=300K, δ en ppm (250 MHz) : 3,02 (3H,s, SCH₃), 3,90 (2H, s, NCH₂), 4,20 (2H, s, NCH₂), 4,42 (2H, d, J=5Hz, OCH₂), 4,75 (1H, s, NCH), 5,10 (1H, t, J=5Hz, OH), entre 7,10 et 7,50 (11H, m, 11CH arom.)].

### Exemple 50

A une solution de 0,10 g de pyrrolidine dans 20 cm³ de 1,2-dichloroéthane, on ajoute 0,75 g de (RS)-1-{(4-chlorophényl)(4-formylphényl)méthyl}-3-[(3,5-difluorophényl)(méthylsulfonyl)méthylène]azétidine puis 0,68 g de triacétoxyborohydrure de sodium. Après 20 heures à température ambiante, on additionne 2 cm³ de soude 1N, le produit est extrait avec 100 cm³ de dichlorométhane, la phase organique est lavée 2 fois avec 50 cm³ d'eau puis 50 cm³ d'une solution saturée de chlorure de sodium, séchée sur sulfate de magnésium, filtrée et concentrée à sec sous pression réduite (2,7 kPa). Le résidu obtenu est chromatographié sur colonne de gel de silice (granulométrie 0,04-0,06 mm, diamètre 4,1 cm, hauteur 13 cm), sous une pression de 0,5 bar d'argon avec de l'acétate comme éluant et en recueillant des fractions de 20 cm³. Les fractions 10 à 18 sont réunies puis concentrées à sec sous pression réduite (2,7 kPa). On obtient 0,39 g de (RS)-1-{(4-chlorophényl)[4-(pyrrolidylméthyl)phényl]méthyl}-3-[(3,5-difluorophényl)(méthylsulfonyl)méthylène]azéti-dine sous forme d'une meringue blanche [Spectre RMN dans DMSO-d6, T=300K, δ en ppm (300 MHz) : 1,65 (4H,m, 2CH₂), 2,40 (4H,m, 2NCH₂), 3,02 (3H,s, SCH₃), 3,50 (2H, s, NCH₂Ph), 3,85 (2H, s, NCH₂), 4,20 (2H, s, NCH₂), 4,75 (1H, s, NCH), entre 7,15 et 7,40 (9H, m, 9CH arom.), 7,48 (2H, d, J=7Hz, 2CH arom.)].

### Exemple 51

En opérant comme à l'exemple 50 à partir de 0,93 cm³ d'une solution de diméthylamine 2M dans le méthanol, 30 cm³ de 1,2-dichloroéthane, 0,75 g de (RS)-1-{(4-chlorophényl)(4-formylphényl)méthyl}-3-[(3,5-difluorophényl)(méthylsulfonyl) méthylène]azétidine puis 0,9 g de triacétoxyborohydrure de sodium on obtient après chromatographie sur colonne de gel de silice (granulométrie 0,04-0,06 mm, diamètre 4 cm, hauteur 17,5 cm), sous une pression de 0,5 bar d'argon avec un mélange d'acétate d'éthyle et cyclohexane (30/70 en volumes) comme éluant et en recueillant des fractions de 40 cm³, 0,46 g de (RS)-1-{(4-chlorophényl)[4-(diméthylaminométhyl)phenyl]methyl} -3-[(3,5)-phényl]-difluorophényl)(méthylsulfonyl)méthylène]azétidine sous la forme d'un solide blanc [Spectre RMN dans DMSO-d6, T=300K, δ en ppm (300 MHz) : 2,12 (6H,s, N(CH₃)₂), 3,02 (3H,s, SCH₃), 3,32 (2H, s, NCH₂Ph), 3,90 (2H, s, NCH₂), 4,20 (2H, s, NCH₂), 4,75 (1H, s, NCH), 7,18 (2H, d, J=8Hz, 2CH arom.), 7,22 (2H, d, J=8Hz, 2CH arom.), 7,35 (1H, t, J=8Hz, CH arom.), 7,39 (4H, m, 4CH arom.), 7,48 (4H, d, J=7Hz, 4CH arom.)].

### Exemple 52

On agite une solution de 0,5 g de (RS)-1-{(4-carboxyphényl)(4-chlorophényl)méthyl]-3-[(3,5-difluorophényl)(méthylsulfonyl)méthylène]azétidine dans 10 cm³ de dichlorométhane à 0°C, avec 0,5 cm³ d'une solution (2M) de diméthylamine dans l'éthanol. On additionne ensuite 13 mg d'hydroxybenzotriazole, 0,2 g de chlorhydrate de 1,3-diméthylaminopropyl 3-éthyl carbodiimide et 0,18 cm³ de diisopropyléthylamine. Après 20 heures à température ambiante, le mélange réactionnel est dilué avec du dichlorométhane, lavé 2 fois avec 80 cm³ d'eau puis 80 cm³ d'une solution saturée de chlorure de sodium, séché sur sulfate de magnésium, filtré et concentré à sec sous pression réduite (2,7 kPa). Le résidu obtenu est chromatographié sur colonne de gel de silice (granulométrie 0,04-0,06 mm, diamètre 4,1 cm, hauteur 13 cm), sous une pression de 0,5 bar d'argon avec un mélange dichlorométhane/acétonitrile/méthanol (98/1/1 en volumes) comme éluant et en recueillant des fractions de 15 cm³. Les fractions 13 à 15 sont réunies puis concentrées à sec sous pression réduite (2,7 kPa). On obtient 0,16 g d'un solide crème qui, après reprise par de l'éther isopropylique et séchage, conduit à 0,11 g de (RS)-1-{(4-chlorophényl)[4-(N,N-diméthylcarbamoyl)phényl]méthyl}-3-[(3,5-difluorophényl)(méthylsulfonyl) méthylène]azétidine sous forme d'un solide [Spectre RMN dans DMSO-d6, T=300K, δ en ppm (300 MHz) : 2,85 (3H,s large, NCH₃), 2,95 (3H,s large, NCH₃), 3,00 (3H, s, SCH₃), 3,90 (2H, s, NCH₂), 4,20 (2H, s, NCH₂), 4,80 (1H, s, NCH), 7,15 (2H, d, J=8Hz, 2CH arom.), 7,30 (1H, t, J=8Hz, CH arom.), 7,35 (4H, m, 4CH arom.), 7,50 (4H, d, J=7Hz, 4CH arom.)].

Le (RS)-1-{(4-carboxyphényl)(4-chlorophényl)méthyl]-3-[(3,5-difluorophényl)(méthylsulfonyl)méthylène]azétidine peut être préparé de la façon suivante : A une solution de 0,50 g de (RS)-1-{(4-chlorophényl)(4-formylphényl)méthyl}-3-[(3,5-difluorophényl)(méthylsulfonyl)méthylène]azétidine dans 10 cm³ d'acétone à 0°C, on ajoute 1,0 cm³ du réactif de Jones. Après 5 heures, le mélange réactionnel est versé dans de l'eau distillée, le produit est extrait avec 50 cm³ d'acétate d'éthyle, la phase organique est lavée 2 fois avec 50 cm³ d'eau puis 50 cm³ d'une solution saturée de chlorure de sodium, séchée sur sulfate de magnésium, filtrée et concentrée à sec sous pression réduite (2,7 kPa). Le solide obtenu est cristallisé dans un mélange acétate d'éthyle-cyclohexane, filtré et séché. On obtient 0,50 g de (RS)-1-{(4-carboxyphényl)(4-chlorophényl)méthyl-(RS)]-3-[(3,5-difluorophényl)(méthylsulfonyl)méthylène]azétidine sous forme d'un solide blanc.

### Exemple 53

On opère comme à l'exemple 52, à partir de 1 g de (RS)-1-{(4-carboxyphényl)(4-chlorophényl)méthyl]-3-[(3,5-difluorophényl)(méthylsulfonyl) méthylène]azéti-dine, 0,38 g de chlorhydrate de 1,3-diméthylaminopropyl 3-éthyl carbodiimide, 22 mg d'hydrate d'hydroxybenzotriazole, de 30 cm³ de dichlorométhane, et 0,83 cm³ d'une solution 2M d'éthylamine dans le THF, en chromatographiant sur colonne de gel de silice (granulométrie 0,04-0,06 mm, diamètre 4,1 cm, hauteur 15 cm), sous une pression de 0,5 bar d'argon avec un mélange d'acétate d'éthyle et cyclohexane (45/55 en volumes) comme éluant et en recueillant des fractions de 30 cm³. Les fractions 22 à 32 sont réunies puis concentrées à sec sous pression réduite (2,7 kPa). On obtient 0,29 g de (RS)-1-{(4-chlorophényl)[4-(N-éthylcarbamoyl)phényl]méthyl}-3-[(3,5-difluorophényl) (méthylsulfonyl)méthylène]azétidine sous forme d'un solide blanc [Spectre RMN dans DMSO-d6, T=300K, δ en ppm (300 MHz) : 1,07 (3H, t, J=6Hz, CH₃), 3,00 (3H, s, SCH₃), 3,35 (2H, m, NCH₂), 3,90 (2H, s, NCH₂), 4,20 (2H, s, NCH₂), 4,80 (1H, s, NCH), 7,15 (2H, d, J=8Hz, 2CH arom.), 7,30 (1H, t, J=8Hz, CH arom.), 7,35 (2H, d, J=7Hz, 2CH arom.), 7,48 (4H, m, 4CH arom.), 7,74 (2H, d, J=7Hz, 2CH arom.), 8,37 (1H, t, CONH)].

### Exemple 54

On opère selon l'exemple 52, à partir de 1 g de (RS)-1-{(4-carboxyphényl)(4-chlorophényl)méthyl]-3-[(3,5-difluorophényl)(méthylsulfonyl)méthylène]azétidine, 0,38 g de chlorhydrate de 1,3-diméthylaminopropyl 3-éthyl carbodiimide, 22 mg d'hydrate d'hydroxybenzotriazole, de 40 cm³ de dichlorométhane et 0,24 cm³ d'une solution 7N d'ammoniaque dans le méthanol et en chromatographiant sur colonne de gel de silice (granulométrie 0,04-0,06 mm, diamètre 4,1 cm, hauteur 15 cm), sous une pression de 0,5 bar d'argon avec un mélange d'acétate d'éthyle et cyclohexane (60/40 en volumes) comme éluant et en recueillant des fractions de 35 cm³. Les fractions 38 à 48 sont réunies puis concentrées à sec sous pression réduite (2,7 kPa). On obtient 0,29 g d'un solide qui, après reprise par de l'éther isopropylique et séchage, conduit à 0,22 g de (RS)-1-[(4-carbamoylphényl)(4-chlorophényl)méthyl-(RS)]-3-[(3,5-difluorophényl)(méthylsulfonyl)méthylène]azé-tidine sous forme d'un solide blanc [Spectre RMN dans DMSO-d6, T=300K, δ en ppm (300 MHz) : 3,00 (3H, s, SCH₃), 3,90 (2H, s, NCH₂), 4,20 (2H, s, NCH₂), 4,82 (1H, s, NCH), 7,17 (2H, d, J=8Hz, 2CH arom.), 7,30 (1H, t, J=8Hz, CH arom.), 7,38 (2H, d, J=7Hz, 2CH arom.), 7,50 (5H, m, 4CH arom. et ½ CONH₂), 7,80 (2H, d, J=7Hz, 2CH arom.), 7,90 (1H, s, ½ CONH₂)].

### Exemple 55

On opère selon le mode opératoire de l'exemple 4 à partir de 1,7 g de 1-[bis(4-chlorophényl)méthyl]-3-[(3,5-dichlorophényl)(méthylsulfonyl)méthyl-(RS)]azétidin-3-ol, de 0,35 cm³ de chlorure de méthanesulfonyle et 1,5 g de 4-diméthylaminopyridine. Le résidu obtenu est purifié par chromatographie sur colonne de gel de silice (granulométrie 0,04-0,06 mm, diamètre 3 cm, hauteur 35 cm) sous une pression de 0,5 bar d'azote avec un mélange dichlorométhane et éthanol (99,5/0,5 en volumes) comme éluant et en recueillant des fractions de 100 cm³. Les fractions 7 à 10 sont réunies et concentrées à sec sous pression réduite (2,7 kPa). Le solide est cristallisé dans 15 cm³ d'éther éthylique. On obtient 0,2 g de 1-[bis(4-chlorophényl)méthyl]-3-[(3,5-dichlorophényl)(méthylsulfonyl)méthylène]azétidine fondant à 200°C [Spectre RMN dans DMSO-d6, T=300K, δ en ppm (300 MHz) : 3,00 (3H, s, SCH₃), 3,80 (2H, s, NCH₂), 4,20 (2H, s, NCH₂), 4,75 (1H, s, NCH), 7,35 (4H, d, J=7Hz, 4CH arom.), 7,45 (6H,m, 6CH arom.), 7,67 (1H, s, CH arom.)].

Le 1-[bis(4-chlorophényl)méthyl]-3-[(3,5-dichlorophényl)(méthylsulfonyl)méthyl-(RS)]azétidin-3-ol peut être obtenu de la manière suivante : en opérant selon le mode opératoire de l'exemple 39 à partir de 4 g de bis(4-chlorophényl)bromométhane et 3 g de chlorhydrate de 3-[(3,5-dichlorophényl)(méthylsulfonyl)méthyl-(RS)]azétidin-3-ol, le résidu obtenu est purifié par chromatographie sur colonne de gel de silice (granulométrie 0,04-0,06 mm, diamètre 3 cm, hauteur 40 cm) sous une pression de 0,5 bar d'azote avec du dichlorométhane puis un mélange dichlorométhane et éthanol (99/1 en volumes) comme éluant et en recueillant des fractions de 100 cm³. Les fractions 15 à 19 sont réunies et concentrées à sec sous pression réduite (2,7 kPa). On obtient 1,7 g de 1-[bis(4-chlorophényl)méthyl]-3-[(3,5-dichlorophényl)(méthylsulfonyl)méthyl-(RS)]azéti-din-3-ol sous forme d'une meringue.

Le bis(4-chlorophényl)bromométhane peut être préparé selon le mode opératoire décrit par BACHMANN W.E., J. Am. Chem. Soc., 2135 (1933).

Le chlorhydrate de 3-[(3,5-dichlorophényl)(méthylsulfonyl)méthyl-(RS)]azétidin-3-ol peut être obtenu de la manière suivante : en opérant selon le mode opératoire de l'exemple 39 à partir de 5,6 g de 3-[(3,5-dichlorophényl)(méthylsulfonyl)méthyl-(RS)]-1-(vinyloxycarbonyl)azétidin-3-ol et 56 cm³ d'une solution de dioxane chlorhydrique 6,2N dans 56 cm³ de dioxane, on obtient 5,1 g de chlorhydrate de 3-[(3,5-dichlorophényl)(méthylsulfonyl)méthyl-(RS)]azétidin-3-ol sous forme d'une meringue.

Le 3-[(3,5-dichlorophényl)(méthylsulfonyl)méthyl-(RS)]-1-(vinyloxycarbonyl)azétidin 3-ol peut être préparé de la manière suivante : en opérant selon le mode opératoire de l'exemple39 à partir de 7,4 g de 1-benzhydryl-3-[(3,5-dichlorophényl)(méthylsulfonyl)méthyl-(RS)]azétidin-3-ol et 1,6 cm³ de chloroformiate de vinyle dans 75 cm³ de dichlorométhane, le résidu obtenu est purifié par chromatographie sur colonne de gel de silice (granulométrie 0,04-0,06 mm, diamètre 3 cm, hauteur 40 cm) sous une pression de 0,5 bar d'azote avec un mélange d'acétate d'éthyle et cyclohexane (30/70 en volumes) comme éluant et en recueillant des fractions de 100 cm³. Les fractions 4 à 10 sont réunies et concentrées à sec sous pression réduite (2,7 kPa). On obtient 5,6 g de 3-[(3,5-dichlorophényl)(méthylsulfonyl)méthyl-(RS)]-1-(vinyloxycarbonyl)azétidin-3-ol sous forme d'une meringue.

### Exemple 56

En opérant selon le mode opératoire de l'exemple 4 à partir de 0,5 g de 1-benzhydryl-3-[(3-diméthylaminophényl)(méthylsulfonyl)méthyl-(RS)]azétidin-3-ol, de 0,1 cm³ de chlorure de méthanesulfonyle et 0,5 g de 4-diméthylaminopyridine, le résidu obtenu est purifié par chromatographie sur colonne de gel de silice (granulométrie 0,04-0,06 mm, diamètre 2 cm, hauteur 30 cm) sous une pression de 0,5 bar d'azote avec un mélange dichlorométhane et éthanol (98/2 en volumes) comme éluant et en recueillant des fractions de 20 cm³. Les fractions 8 à 13 sont réunies et concentrées à sec sous pression réduite (2,7 kPa). Le solide obtenu est cristallisé dans 8 cm³ d'éther éthylique. On obtient 0,3 g de 1-benzhydryl-3-[(3-diméthylaminophényl)(méthylsulfonyl)méthylène]azétidine fondant à 176°C [Spectre RMN dans DMSO-d6, T=300K, δ en ppm (250 MHz) : 2,90 (6H, s, N(CH₃)₂), 2,95 (3H, s, SCH₃), 3,80 (2H, s, NCH₂), 4,20 (2H, s, NCH₂), 4,75 (1H, s, NCH), 6,70 (3H, m, 3CH arom.), 7,20 (3H, m, 3CH arom.), 7,30 (4H, t, J=7Hz, 4CH arom.), 7,48 (4H, d, J=7Hz, 4CH arom.)].

Le 1-benzhydryl-3-[(3-diméthylaminophényl)(méthylsulfonyl)méthyl-(RS)]azétidin-3-ol peut être obtenu de la manière suivante : en opérant selon le mode opératoire de l'exemple 1 à partir de 0,4 g de (3-diméthylaminobenzyl)méthylsulfone, de 0,4 g de 1-benzhydryl azétidin-3-one et 1,2 cm³ d'une solution 1,6M de nbutyllithium dans l'hexane, on obtient 0,5 g de 1-benzhydryl-3-[(3-diméthylaminophényl)(méthylsulfonyl)méthyl-(RS)]azétidin-3-ol sous forme d'un solide fondant à 185°C.

La (3-diméthylaminobenzyl)méthylsulfone peut être préparée de la manière suivante : en opérant selon le mode opératoire de l'exemple 2 à partir de 1,4 g de (3-diméthylaminobenzyl)méthylsulfure et 5,1 g d'oxone^{R}, on obtient 1,1 g de (3-diméthylaminobenzyl)méthylsulfone sous forme d'un solide blanc fondant à 195°C.

Le (3-diméthylaminobenzyl)méthylsulfure peut être préparé de la manière suivante : en opérant selon le mode opératoire de l'exemple 37 à partir de 4g de (3-iodobenzyl)méthylsulfure, de 1,4 g de diméthylamine en solution dans 5 cm³ de tétrahydrofuranne, de 2,9 g de tertiobutylate de sodium, de 0,56 g de chlorure de 1,1'-bis(diphénylphosphino)ferrocènyl palladium et 1,3 g de 1,1'-bis(diphénylphosphino)ferrocène dans 35 cm³ de tétrahydrofuranne, on obtient 0,9 g de (3-diméthylaminobenzyl)méthylsulfure sous forme d'une huile.

### Exemple 57

En opérant selon le mode opératoire de l'exemple 4 à partir de 1,3 g de 1-benzhydryl-3-[(3-méthylsulfanylphényl)(méthylsulfonyl)méthyl-(RS)]azétidin-3-ol, de 0,3 cm³ de chlorure de méthanesulfonyle et 1,4 g de 4-diméthylaminopyridine, le résidu obtenu est purifié par chromatographie sur colonne de gel de silice (granulométrie 0,04-0,06 mm, diamètre 2 cm, hauteur 30 cm) sous une pression de 0,5 bar d'azote avec un mélange dichlorométhane et éthanol (98/2 en volumes) comme éluant et en recueillant des fractions de 20 cm³. Les fractions 11 à 13 sont réunies et concentrées à sec sous pression réduite (2,7 kPa). Le solide obtenu est cristallisé dans 15 cm³ d'éther éthylique. On obtient 0,6 g de 1-benzhydryl-3-[(3-méthylsulfanylphényl)(méthylsulfonyl)méthylène]azétidine fondant à 146°C [Spectre RMN dans DMSO-d6, T=300K, δ en ppm (300 MHz) : 2,45 (3H, s, PhSCH₃), 2,95 (3H, s, SCH₃), 3,80 (2H, s, NCH₂), 4,20 (2H, s, NCH₂), 4,75 (1H, s, NCH), entre 7,10 et 7,50 (14H, m, 14CH arom.)].

Le 1-benzhydryl-3-[(3-méthylsulfanylphényl)(méthylsulfonyl)méthyl-(RS)]azétidin-3-ol peut être obtenu de la manière suivante : en opérant selon le mode opératoire de l'exemple 1 à partir de 1,1 g de méthyl(3-méthylsulfanylbenzyl)sulfone, de 1,2 g de 1-benzhydryl azétidin-3-one, on obtient 1,3 g de 1-benzhydryl-3-[(3-méthylsulfanylphényl)(méthylsulfonyl)méthyl-(RS)]azétidin-3-ol sous forme d'un solide.

La méthyl(3-méthylsulfanylbenzyl)sulfone peut être préparée de la manière suivante : on chauffe à une température proche de 100°C, sous un courant d'argon, pendant 1 heure un mélange de 5g de (3-iodobenzyl)méthylsulfone et 1g de tétrakistriphénylphosphine palladium dans 250 cm³ de diméthylsulfoxyde. 2,5 g de méthylthiolate de sodium sont ajoutés puis le chauffage à 100°C est maintenu 18 heures. Le milieu réactionnel est refroidi à température ambiante, repris par 700 cm³ d'acétate d'éthyle et 500 cm³ d'eau. La phase organique est décantée, lavée par 10 fois 500 cm³ d'eau, 500 cm³ d'une solution aqueuse saturée en chlorure de sodium, filtrée sur verre fritté et concentrée à sec sous pression réduite (2,7 kPa). Le résidu obtenu est purifié par chromatographie sur colonne de gel de silice (granulométrie 0,04-0,06 mm, diamètre 3 cm, hauteur 30 cm) sous une pression de 0,5 bar d'azote avec un mélange cyclohexane et acétate d'éthyle (70/30 puis 60/40 puis 50/50 en volumes) comme éluant et en recueillant des fractions de 30 cm³, Les fractions 26 à 30 sont réunies et concentrées à sec sous pression réduite (2,7 kPa). On obtient 1,2 g de méthyl(3-méthylsulfanylbenzyl)méthylsulfone sous forme d'huile.

### Exemple 58

A une solution refroidie à 5°C de 1,1 g 1-benzhydryl-3-{[3-(tert-butyldiméthylsilyloxyméthyl)phényl](méthylsulfonyl)méthylène}azétidine dans 10 cm³ de tétrahydrofuranne, on ajoute 4 cm³ d'une solution 1M de fluorure de tétrabutylammmonium dans le tétrahydrofuranne. Le mélange est agité 3 heures à une température proche de 20°C puis est repris par 100 cm³ d'acétate d'éthyle et 2 fois 50 cm³ d'eau. La phase organique est décantée, extraite, séchée sur du sulfate de magnésium anhydre et concentrée à sec sous pression réduite (2,7 kPa). Le résidu obtenu est purifié par chromatographie sur colonne de gel de silice (granulométrie 0,04-0,06 mm, diamètre 2 cm, hauteur 30 cm) sous une pression de 0,5 bar d'azote avec un mélange dichlorométhane et éthanol (95/5 en volumes) comme éluant et en recueillant des fractions de 60 cm³. Les fractions 4 à 6 sont réunies et concentrées à sec sous pression réduite (2,7 kPa). On obtient 0,5 g de 1-benzhydryl-3-[(3-hydroxyméthylphényl)(méthylsulfonyl)méthylène]azétidine sous forme d'un solide blanc fondant à 152°C [Spectre RMN dans DMSO-d6, T=300K, δ en ppm (300 MHz) : 2,95 (3H, s, SCH₃), 3,80 (2H, s, NCH₂), 4,20 (2H, s, NCH₂), 4,50 (2H, d, J=5Hz, OCH₂), 4,75 (1H, s, NCH), 5,25 (1H, t, J=5Hz, OH), 7,20 (2H, t, J=7Hz, 2CH arom.), 7,30 (8H, m, 8CH arom.), 7,45 (4H, d, J=7Hz, 4 CH arom.)].

Le 1-benzhydryl-3-{[3-(tert-butyldiméthylsilyloxyméthyl)phényl](méthylsulfonyl)méthylène}azétidine peut être préparé de la manière suivante : en opérant selon le mode opératoire de l'exemple 4 à partir de 1,6 g de 1-benzhydryl-3-{[3-(tert-butyldiméthylsilyloxyméthyl)phényl](méthylsulfonyl)méthyl-(RS)]azétidin-3-ol, de 0,3 cm³ de chlorure de méthanesulfonyle et 1,4 g de 4-diméthylaminopyridine, le résidu obtenu est purifié par chromatographie sur colonne de gel de silice (granulométrie 0,04-0,06 mm, diamètre 3 cm, hauteur 30 cm) sous une pression de 0,5 bar d'azote avec du dichlorométhane comme éluant et en recueillant des fractions de 60 cm³. Les fractions 15 à 30 sont réunies et concentrées à sec sous pression réduite (2,7 kPa). On obtient 1,1 g de 1-benzhydryl-3-{[3-(tert-butyldiméthylsilyloxyméthyl)phényl](méthylsulfonyl)méthylène]azétidine sous forme d'un solide blanc fondant à 148°C.

Le 1-benzhydryl-3-{[3-(tert-butyldiméthylsilyloxyméthyl)phényl](méthylsulfonyl) méthyl-(RS)]azétidin-3-ol peut être obtenu de la manière suivante : en opérant selon le mode opératoire de l'exemple 1 à partir de 2 g de [3-(tert-butyldiméthylsilyloxyméthyl)benzyl]méthylsulfone et 1,5 g de 1-benzhydryl azétidin-3-one, on obtient 1,6 g de 1-benzhydryl-3-{[3-(tert-butyldiméthylsilyloxyméthyl)phényl](méthylsulfonyl)méthyl-(RS)]azétidin-3-ol sous forme d'un solide blanc fondant à 175°C.

La [3-(tert-butyldiméthylsilyloxyméthyl)benzyl]méthylsulfone peut être préparée de la manière suivante : on agite 18 heures à une température proche de 20°C un mélange de 13,4 g de (3-hydroxyméthylbenzyl)méthylsulfone, 11 g d'imidazole et 12 g de chlorure de tert-butyldiméthylsilane. La solution est concentrée à sec sous pression réduite (2,7 kPa). le résidu obtenu est purifié par chromatographie sur colonne de gel de silice (granulométrie 0,04-0,06 mm, diamètre 5 cm, hauteur 50 cm) sous une pression de 0,5 bar d'azote avec du dichlorométhane comme éluant et en recueillant des fractions de 100 cm³. Les fractions 7 à 14 sont réunies et concentrées à sec sous pression réduite (2,7 kPa). On obtient 5,7 g de [3-(tert-butyldiméthylsilyloxyméthyl)benzyl]méthylsulfone sous forme d'un solide blanc fondant à 80°C.

La (3-hydroxyméthylbenzyl)méthylsulfone peut être préparée de la manière suivante : on agite 18 heures à une température proche de 20°C un mélange de 26 g d'acide 3-(méthylsulfonylméthyl)benzoïque et 4,6 g d'hydrure de lithium et d'aluminium dans 600 cm³ de tétrahydrofuranne. La solution est refroidie à 0°C puis on ajoute successivement 15 cm³ d'acétate d'éthyle, 5 cm³ d'eau, 5 cm³ d'une solution aqueuse à 15% de soude et enfin 30 cm³ d'eau. Le mélange est filtré sur célite, le filtrat repris par 600 cm³ d'acétate d'éthyle. La phase organique est reprise par 500 cm³ d'eau puis 200 cm³ d'une solution aqueuse saturée par du chlorure de sodium, décantée, séchée sur du sulfate de magnésium anhydre, filtrée et concentrée à sec sous pression réduite (2,7 kPa). On obtient 10,4 g de (3-hydroxyméthylbenzyl)méthylsulfone sous forme d'une gomme.

L'acide 3-(méthylsulfonylméthyl)benzoïque peut être préparé de la manière suivante : en opérant selon le mode opératoire de l'exemple 10 à partir de 23,3 g d'acide 3-chlorométhylbenzoïque et 23,3 g de méthanesulfinate de sodium, on obtient 26 g d'acide 3-(méthylsulfonylméthyl)benzoïque sous forme d'un solide blanc fondant à 210°C.

### Exemple 59

A une solution de 0,8 g de 1-benzhydryl-3-[(3-bromométhylphényl)(méthylsulfonyl)méthylène]azétidine dans 8 cm³ de diméthylformamide, on ajoute en maintenant la température inférieure à 30°C, 0,13 g de méthylthiolate de sodium. Le mélange est agité 18 heures à une température proche de 20°C puis repris par 30 cm³ d'acétate d'éthyle et 50 cm³ d'eau. La phase organique est décantée, extraite et lavée par 3 fois 50 cm³ d'eau, séchèe sur sulfate de magnésium et concentrée à sec sous pression réduite (2,7 kPa). Le résidu obtenu est purifié par chromatographie sur colonne de gel de silice (granulométrie 0,04-0,06 mm, diamètre 2 cm, hauteur 28 cm) sous une pression de 0,5 bar d'azote avec un mélange cyclohexane et acétate d'éthyle (90/10 en volumes) comme éluant et en recueillant des fractions de 50 cm³. Les fractions 8 à 14 sont réunies et concentrées à sec sous pression réduite (2,7 kPa). On obtient 0,3 g de 1-benzhydryl-3-{[3-(méthylsulfanylméthyl)phényl](méthylsulfonyl)méthylène}azétidine sous forme d'un solide blanc fondant à 150°C [Spectre RMN dans DMSO-d6, T=300K, δ en ppm (300 MHz) : 1,95 (3H, s, SCH₃), 2,95 (3H, s, SCH₃), 3,75 (2H, s, SCH₂), 3,80 (2H, s, NCH₂), 4,20 (2H, s, NCH₂), 4,75 (1H, s, NCH), 7,20 (2H, t, J=7Hz, CH arom.), 7,30 (8H, d, J=7Hz, 8CH arom.), 7,45 (4H, d, J=7Hz, 4 CH arom.)].

La 1-benzhydryl-3-[(3-bromométhylphényl)(méthylsulfonyl)méthylène]azétidine peut être préparée de la manière suivante : à un mélange de 1 g de 1-benzhydryl-3-[(3-hydroxyméthylphényl)(méthylsulfonyl)méthylène]azétidine dans 10 cm³ de dichlorométhane, on ajoute à une température proche de 20°C, 0,23 cm³ de tribromure de phosphore puis une goutte de pyridine. L'agitation est maintenue 18 heures à la même température. Le milieu réactionnel est repris par 20 cm³ d'eau et 10 cm³ d'une solution aqueuse saturée avec du chlorure de sodium. La phase organique est décantée, extraite, séchée sur du sulfate de magnésium anhydre, filtrée et concentrée à sec sous pression réduite (2,7 kPa). On obtient 1g de 1-benzhydryl-3-[(3-bromométhylphényl)(méthylsulfonyl)méthylène]azétidine sous forme d'une meringue utilisée à l'état brut dans les synthèses ultérieures.

### Exemple 60

En opérant selon le mode opératoire de l'exemple 4 à partir de 6,6 g de 1-benzhydryl-3-[(méthylsulfonyl)(quinol-8-yl)méthyl-(RS)]azétidin-3-ol, de 1,7 cm³ de chlorure de méthanesulfonyle et 5,2 g de 4-diméthylaminopyridine, le résidu obtenu est purifié par chromatographie sur colonne de gel de silice (granulométrie 0,04-0,06 mm, diamètre 6,5 cm, hauteur 35 cm) sous une pression de 0,5 bar d'azote avec un mélange dichlorométhane et méthanol (95/5 en volumes) comme éluant et en recueillant des fractions de 40 cm³. Les fractions 7 à 15 sont réunies et concentrées à sec sous pression réduite (2,7 kPa). Le solide obtenu est cristallisé dans 100 cm³ d'éther éthylique. On obtient 4,4 g de 1-benzhydryl-3-[(méthylsulfonyl)(quinol-8-yl)méthylène]azétidine fondant à 212°C [Spectre RMN dans DMSO-d6, T=300K, δ en ppm (250 MHz) : 3,15 (3H, s, SCH₃), 3,55 (2H, s large, NCH₂), 4,30 (2H, s, NCH₂), 4,70 (1H, s, NCH), 7,18 (2H, t, J=7Hz, 2CH arom.), 7,25 (4H, t, J=7Hz, 4CH arom.), 7,43 (4H, d, J=7Hz, 4 CH arom.), 7,62 (2H, m, 2CH quinoleine), 7,75 (1H, dd, J=2 et 7Hz, CH quinoleine), 8,05 (1H, dd, J=2 et 7Hz, CH quinoleine), 8,43 (1H, dd, J=2 et 8Hz, CH quinoleine), 9,00 (1H, dd, J=2 et 5Hz, CH quinoleine)].

Le 1-benzhydryl-3-[(méthylsulfonyl)(quinol-8-yl)méthyl-(RS)]azétidin-3-ol peut être obtenu de la manière suivante : en opérant selon le mode opératoire de l'exemple 1 à partir de 5,5 g de méthyl(quinol-8-ylméthyl)sulfone, de 5,9 g de 1-benzhydryl azétidin-3-one et 18,8 cm³ d'une solution 1,6 M de nbutyllithium dans l'hexane, on obtient 6,6 g de 1-benzhydryl-3-[(méthylsulfonyl)(quinol-8-yl)méthyl-(RS)]azétidin-3-ol sous forme d'un solide beige.

La méthyl(quinol-8-ylméthyl)sulfone peut être préparée de la manière suivante : en opérant selon le mode opératoire de l'exemple 10 à partir de 4,5 g de 8-chlorométhylquinoléine et 4,4 g de méthanesulfinate de sodium, on obtient 5,7 g de méthyl(quinol-8-ylméthyl)sulfone sous forme d'un solide beige.

La 8-chlorométhylquinoléine peut être préparée de la manière suivante : à un mélange de 7,1 g de 8-méthylquinoléine dans 250 cm³ de tétrachlorure de carbone, on ajoute à une température proche de 20°C, 6,7 g de N-chlorosuccinimide puis 250 mg de peroxyde de benzoyle. Le milieu réactionnel est chauffé à reflux du solvant 36 heures puis refroidi à 20°C. Le mélange est filtré sur verre fritté, le filtrat est concentré à sec sous pression réduite (2,7 kPa). le résidu obtenu est purifié par chromatographie sur colonne de gel de silice (granulométrie 0,04-0,06 mm, diamètre 5,5 cm, hauteur 32 cm) sous une pression de 0,5 bar d'azote avec du dichlorométhane comme éluant et en recueillant des fractions de 40 cm³. Les fractions 21 à 40 sont réunies et concentrées à sec sous pression réduite (2,7 kPa). On obtient 4,5 g de 8-chlorométhylquinoléine sous forme d'une huile brune utilisée à l'état brut dans les synthèses ultérieures.

### Exemple 61

En opérant selon le mode opératoire de l'exemple 4 à partir de 6,2 g de 1-[bis(4-chlorophényl)méthyl]-3-[(3-cyanophényl)(méthylsulfonyl)méthyl-(RS)]azétidin-3-ol, de 1,4 cm³ de chlorure de méthanesulfonyle et 6,1 g de 4-diméthylaminopyridine, le résidu obtenu est purifié par chromatographie sur colonne de gel de silice (granulométrie 0,04-0,06 mm, diamètre 4 cm, hauteur 60 cm) sous une pression de 0,5 bar d'azote avec du dichlorométhane comme éluant et en recueillant des fractions de 100 cm³. Les fractions 4 à 7 sont réunies et concentrées à sec sous pression réduite (2,7 kPa). Le solide obtenu est cristallisé dans 25 cm³ d'éther éthylique. On obtient 0,7 g de 1-[bis(4-chlorophényl)méthyl]-3-[(3-cyanophényl)(méthylsulfonyl)méthylène]azétidine sous forme d'un solide fondant à 178°C [Spectre RMN dans DMSO-d6, T=300K, δ en ppm (300 MHz) : 3,00 (3H, s, SCH₃), 3,80 (2H, s, NCH₂), 4,20 (2H, s, NCH₂), 4,75 (1H, s, NCH), 7,30 (4H, d, J=7Hz, 4CH arom.), 7,40 (4H, d, J=7Hz, 4 CH arom.), 7,60 (1H, t, J=7Hz, CH arom), 7,70 (1H, d, J=7Hz, CH arom.), 7,85 (2H, m, 2CH arom.)].

Le 1-[bis(4-chlorophényl)méthyl]-3-[(3-cyanophényl)(méthylsulfonyl)méthyl-(RS)]azétidin-3-ol peut être obtenu de la manière suivante : en opérant selon le mode opératoire de l'exemple 1 à partir de 5,5 g de (3-cyanophényl)méthylsulfone, de 6,1 g de 1-[bis(4-chlorophényl)méthyl]azétidin-3-one et 13,8 cm³ d'une solution 1,6 M de nbutyllithium dans l'hexane, on obtient 6,3 g de 1-[bis(4-chlorophényl)méthyl]-3-[(3-cyanophényl)(méthylsulfonyl)méthyl-(RS)]azétidin-3-ol sous forme d'une meringue.

### Exemple 62

On chauffe à 50°C pendant 20 heures un mélange de 4,5 g de 1-[bis(4-chlorophényl)méthyl]-3-[(3-cyanophényl)(méthylsulfonyl)méthylène]azétidine dans 50 cm³ d'acide acétique et 50 cm³ d'acide chlorhydrique concentré (d=1,18). Le milieu réactionnel est refroidi à température ambiante et concentré à sec sous pression réduite (2,7 kPa). L'huile obtenue est reprise par 100 cm³ d'éthanol puis la solution est concentrée à sec sous pression réduite (2,7 kPa). Le résidu est précipité dans 60 cm³ d'éther éthylique. Le solide obtenu est purifié par chromatographie sur colonne de gel de silice (granulométrie 0,04-0,06 mm, diamètre 25 cm, hauteur 40 cm) sous une pression de 0,5 bar d'azote avec du dichlorométhane puis un mélange dichlorométhane et éthanol (99,5/0,5 en volumes) comme éluant et en recueillant des fractions de 30 cm³. Les fractions 35 à 46 sont réunies et concentrées à sec sous pression réduite (2,7 kPa). Le solide obtenu est cristallisé dans 15 cm³ d'éther éthylique. On obtient 0,2 g de 1-[bis(4-chlorophényl)méthyl]-3-[(3-carbamoylphényl)(méthylsulfonyl)méthylène]azétidine sous forme d'un solide fondant à 192°C [Spectre RMN dans DMSO-d6, T=300K, δ en ppm (300 MHz) : 2,95 (3H, s, SCH₃), 3,80 (2H, s, NCH₂), 4,20 (2H, s, NCH₂), 4,80 (1H, s, NCH), 7,35 (4H, d, J=7Hz, 4CH arom.), 7,45 (5H, d, J=7Hz, 4 CH arom. et ½ CONH₂), 7,50 (2H, m, 2CH arom.), 7,85 (2H, m, 2CH arom.)].

### Exemple 63

En opérant selon le mode opératoire de l'exemple 1 à partir de 0,8 g de 1-benzhydryl-3-{[3-(N-tert-butyloxycarbonyl-N-méthylamino)phényl](méthylsulfonyl)méthyl-(RS)}azétidin-3-ol, de 0,2 cm³ de chlorure de méthanesulfonyle et 0,7 g de 4-diméthylaminopyridine, le résidu obtenu est purifié par chromatographie sur colonne de gel de silice (granulométrie 0,04-0,06 mm, diamètre 2 cm, hauteur 30 cm) sous une pression de 0,5 bar d'azote avec un mélange dichlorométhane et éthanol (98/2 en volumes) comme éluant en recueillant des fractions de 20 cm³. Les fractions 4 à 8 sont réunies et concentrées à sec sous pression réduite (2,7 kPa). Le solide obtenu est recristallisé dans 10 cm³ d'acétate d'éthyle. On obtient 0,5 g de 1-benzhydryl-3-{[3-(N-tert-butyloxycarbonyl-N-méthylamino)phényl](méthyl-sulfonyl)méthylène}azétidine sous forme d'un solide fondant à 161°C [Spectre RMN dans DMSO-d6, T=300K, δ en ppm (300 MHz) : 1,30 (9H, s, (CH₃)₃), 2,95 (3H, s, SCH₃), 3,15 (3H, s, NCH₃), 3,75 (2H, s, SCH₂), 3,80 (2H, s, NCH₂), 4,20 (2H, s, NCH₂), 4,75 (1H, s, NCH), entre 7,15 et 7,50 (14H, m, 14CH arom.)].

Le 1-benzhydryl-3-{[3-(N-tert-butyloxycarbonyl-N-méthylamino)phényl](méthylsulfonyl)méthyl-(RS)}azétidin-3-ol peut être obtenu de la manière suivante : en opérant selon le mode opératoire de l'exemple 1 à partir de 1,6 g de [3-(N-tert-butyloxycarbonyl-N-méthylamino)benzyl]méthylsulfone, de 1,3 g de 1-benzhydryl azétidin-3-one et 3,8 cm³ d'une solution 1,6 M de nbutyllithium dans l'hexane, on obtient 0,8 g 1-benzhydryl-3-{[3-(N-tert-butyloxycarbonyl-N-méthylamino)phényl](méthylsulfonyl)méthyl-(RS)}azétidin-3-ol sous forme d'un solide blanc.

La [3-(N-tert-butyloxycarbonyl-N-méthylamino)benzyl]méthylsulfone peut être préparée de la manière suivante : à une solution refroidie à 0°C de méthyl(3-méthylaminobenzyl)sulfone dans 30 cm³ de dioxane, on ajoute 2,5 g de ditertiobutyldicarbonate dans 40 cm³ de dioxane. L'agitation est maintenue 18 heures à température ambiante. Le milieu réactionnel est repris par 75 cm³ de dichlorométhane; la phase organique est lavée par 75 cm³ d'eau puis par 75 cm³ d'une solution aqueuse saturée par du chlorure de sodium. La phase organique est décantée, extraite, séchée sur du sulfate de sodium anhydre, filtrée et concentrée à sec sous pression réduite (2,7 kPa). Le résidu obtenu est purifié par chromatographie sur colonne de gel de silice (granulométrie 0,04-0,06 mm, diamètre 2 cm, hauteur 35 cm) sous une pression de 0,5 bar d'azote avec un mélange cyclohexane et acétate d'éthyle (50/50 en volumes) comme éluant en recueillant des fractions de 20 cm³. Les fractions 5 à 10 sont réunies et concentrées à sec sous pression réduite (2,7 kPa). On obtient 1,8 g de [3-(N-tert-butyloxycarbonyl-N-méthylamino)benzyl]méthylsulfone sous forme d'une huile incolore.

La méthyl(3-méthylaminobenzyl)sulfone peut être préparée de la manière suivante : on chauffe pendant 3 heures à 50°C un mélange de 9,7 cm³ d'acide formique (d=1,22) et 19,6 cm³ d'anhydride acétique (d=1,08) puis laisse revenir à température ambiante la solution. On ajoute 40 cm³ de tétrahydrofuranne et refroidi à -20°C. On ajoute ensuite 14,8 g de (3-aminobenzyl)méthylsulfone et 200 cm³ de tétrahydrofuranne. L'agitation est maintenue 2 heures à -20°C puis 48 heures à température ambiante. Le mélange est filtré sur verre fritté, le précipité est lavé par 3 fois 50 cm³ d'oxyde de diisopropyle puis séché. Le filtrat est concentré de moitié en volume (2,7 kPa), le précipité obtenu est filtré sur verre fritté et lavé par 3 fois 30 cm³ d'oxyde de diisopropyle puis séché. Les deux précipités sont réunis et dissous dans 375 cm³ de tétrahydrofuranne. La solution est refroidie à 0°C; on ajoute 100 cm³ d'une solution 2 M de borane diméthylsulfure dans le tétrahydrofuranne puis chauffe à reflux 3 heures. Le mélange est refroidi à 5°C puis on ajoute en 20 minutes 60 cm³ de méthanol. L'agitation est maintenue 1 heure à température ambiante. On fait barbotter un courant de chlorure d'hydrogène dans la solution pendant 5 minutes. Le milieu réactionnel est ensuite chauffé à reflux 1 heure, refroidi à température ambiante et repris par 300 cm³ d'eau. La solution est alcalinisée par de la soude 3 N puis par une solution aqueuse saturée de bicarbonate de sodium. La phase organique est extraite par 2 fois 250 cm³ d'acétate d'éthyle, lavée par 300 cm³ d'une solution aqueuse saturée de bicarbonate de sodium et 2 fois 300 cm³. Elle est concentrée à sec sous pression réduite (2,7 kPa). L'huile obtenue est reprise par 100 cm³ d'acide chlorhydrique 4N, puis par 100 cm³ d'acétate d'éthyle. La phase aqueuse est alcalinisée par 120 cm³ de soude 3 N, puis par une solution aqueuse de bicarbonate de sodium. La phase organique est extraite par 2 fois 75 cm³ d'acétate d'éthyle, séchée sur du sulfate de magnésium anhydre, filtrée et concentrée à sec sous pression réduite (2,7 kPa). On obtient 9 g de méthyl(3-méthylaminobenzyl)sulfone sous forme d'un solide rose.

La (3-aminobenzyl)méthylsulfone peut être préparée de la manière suivante : on chauffe à reflux 15 minutes un mélange de 23,7 g de méthyl(3-nitrobenzyl)sulfone, 65 cm³ d'acide chlorhydrique (d=1,18) et 150 cm³ de méthanol. On ajoute en 10 minutes 18,5 g de fer et maintient au reflux pendant 4 heures puis 18 heures à température ambiante. Le milieu réactionnel est alcalinisé par une solution aqueuse d'ammoniaque puis par une solution aqueuse de bicarbonate de sodium. La phase organique est extraite par 3 fois 250 cm³ d'acétate d'éthyle, séchée sur du sulfate de magnésium, filtrée sur verre fritté et concentrée à sec sous pression réduite (2,7 kPa). On obtient 14,9 g de (3-aminobenzyl)méthylsulfone sous forme d'un solide beige utilisé à l'état brut dans les synthèses ultérieures.

### Exemple 64

On agite 18 heures à température ambiante un mélange de 0,3 g de 1-benzhydryl-3-{[3-(N-tert-butyloxycarbonyl-N-méthylamino)phényl] (méthylsulfonyl)méthylène} azétidine, 4 cm³ d'une solution 4,7 N de dioxane chlorhydrique et 4 cm³ de dioxane. Le milieu réactionnel est concentré à sec sous pression réduite (2,7 kPa). Le résidu est repris par 100 cm³ d'eau et 20 cm³ d'oxyde de diéthyle. La phase aqueuse est alcalinisée par 30 cm³ d'une solution aqueuse de bicarbonate de sodium. La phase organique est extraite par 2 fois 40 cm³ d'acétate d'éthyle, lavée par 2 fois 30 cm³ d'eau, décantée, séchée sur du sulfate de magnésium anhydre, filtrée et concentrée à sec sous pression réduite (2,7 kPa). Le résidu est cristallisé dans 20 cm³ d'oxyde de diéthyle. On obtient 0,16 g de 1-benzhydryl-3-[(3-méthylaminophényl)(méthylsulfonyl)méthylène]azétidine sous forme d'un solide fondant à 161°C [Spectre RMN dans DMSO-d6, T=300K, δ en ppm (250 MHz) : 2,65 (3H, d, J=5Hz, NCH₃), 2,95 (3H, s, SCH₃), 3,80 (2H, s, NCH₂), 4,20 (2H, s, NCH₂), 4,75 (1H, s, NCH), 5,80 (1H, q, J=5Hz, NH), 6,60 (3H, m, 3CH arom.), 7,15 (1H, t, J=7Hz, CH arom.), 7,22 (2H, t, J=7Hz, 2CH arom.), 7,30 (4H, t, J=7Hz, 4CH arom.), 7,48 (4H, d, J=7Hz, 4 CH arom.)].

### Exemple 65

En opérant selon le mode opératoire de l'exemple 4 à partir de 11,3 g de 1-[bis(4-chlorophényl)méthyl]-3-[(3-méthoxyphényl)(méthylsulfonyl)méthyl-(RS)]azétidin-3-ol, de 2,6 cm³ de chlorure de méthanesulfonyle et 10,9 g de 4-diméthylaminopyridine, on obtient après recristallisation dans 20 cm³ d'oxyde de diéthyle 5 g 1-[bis(4-chlorophényl)méthyl]-3-[(3-méthoxyphényl)(méthylsulfonyl) méthylène]azétidine fondant à 181°C [Spectre RMN dans DMSO-d6, T=300K, δ en ppm (300 MHz) : 2,95 (3H, s, SCH₃), 3,77 (3H, s, OCH₃), 3,80 (2H, s, NCH₂), 4,20 (2H, s, NCH₂), 4,80 (1H, s, NCH), 6,95 (3H, m, 3CH arom.), 7,35 (5H, m, 5CH arom.), 7,45 (4H, d, J=7Hz, 4 CH arom.)].

Le 1-[bis(4-chlorophényl)méthyl]-3-[(3-méthoxyphényl)(méthylsulfonyl)méthyl-(RS)]azétidin-3-ol peut être obtenu de la manière suivante : en opérant selon le mode opératoire de l'exemple 1 à partir de 6,6 g de (3-méthoxybenzyl)méthylsulfone, de 10 g de 1-[bis(4-chlorophényl)méthyl]azétidin-3-one et 23 cm³ d'une solution 1,6 N de nbutyllithium dans l'hexane, on obtient 11,4 g de 1-[bis(4-chlorophényl)méthyl]-3-[(3-méthoxyphényl)(méthylsulfonyl)méthyl-(RS)]azétidin-3-ol sous forme d'un solide blanc fondant à 130°C.

### Exemple 66

En opérant selon le mode opératoire de l'exemple 32 à partir de 4,8 g de 1-[bis(4-chlorophényl)méthyl]-3-[(3-méthoxyphényl)(méthylsulfonyl)méthylène]azétidine, de 32 cm³ d'une solution 1 M de tribromure de bore dans le dichlorométhane, le résidu obtenu est purifié par chromatographie sur colonne de gel de silice (granulométrie 0,04-0,06 mm, diamètre 3 cm, hauteur 30 cm) sous une pression de 0,5 bar d'azote avec un mélange dichlorométhane et éthanol comme éluant (98/2 en volumes) et en recueillant des fractions de 20 cm³. Les fractions 16 et 17 sont concentrées à sec sous pression réduite (2,7 kPa). on obtient après recristallisation dans 5 cm³ d'oxyde de diéthyle 0,1 g 1-[bis(4-chlorophényl)méthyl]-3-[(3-hydroxyphényl)(méthylsulfonyl)méthylène]azétidine sous forme d'un solide fondant à 114°C [Spectre RMN dans DMSO-d6, T=300K, δ en ppm (250 MHz) : 2,92 (3H, s, SCH₃), 3,80 (2H, s, NCH₂), 4,20 (2H, s, NCH₂), 4,80 (1H, s, NCH), 6,80 (3H, m, 3CH arom.), 7,20 (1H, t, J=7Hz, CH arom.), 7,37 (4H, t, J=7Hz, 4CH arom.), 7,47 (4H, d, J=7Hz, 4 CH arom.)].

### Exemple 67

En opérant selon le mode opératoire de l'exemple 4 à partir de 0,6 g de 1-[bis(4-chlorophényl)méthyl]-3-[(méthylsulfonyl)(3-pyrrolidinylphényl)méthyl-(RS)]azétidin-3-ol, de 0,1 cm³ de chlorure de méthanesulfonyle et 0,5 g de 4-diméthylaminopyridine, le résidu obtenu est purifié par chromatographie sur colonne de gel de silice (granulométrie 0,04-0,06 mm, diamètre 2 cm, hauteur 30 cm) sous une pression de 0,5 bar d'azote avec un mélange dichlorométhane et éthanol comme éluant (98,5/1,5 en volumes) et en recueillant des fractions de 10 cm³. La fraction 4 est concentrée à sec sous pression réduite (2,7 kPa). On obtient après recristallisation dans 5 cm³ d'oxyde de diéthyle 0,5 g 1-[bis(4-chlorophényl)méthyl]-3-[(méthylsulfonyl)(3-pyrrolidinylphényl)méthylène]azétidine sous forme d'un solide fondant à 133°C [Spectre RMN dans DMSO-d6, T=300K, δ en ppm (400 MHz) : 2,00 (4H, m, 2 CH₂), 2,95 (3H, s, SCH₃), 3,20 (4H, m, 2 NCH₂), 3,80 (2H, s, NCH₂), 4,20 (2H, s, NCH₂), 4,80 (1H, s, NCH), 6,50 (1H, s, CH arom.), 6,60 (1H, d, J=7Hz, CH arom.), 6,65 (1H, d, J=7Hz, CH arom), 7,20 (1H, t, J=7Hz, CH arom.), 7,40 (4H, d, J=7Hz, 4 CH arom.), 7,50 (4H, d, J=7Hz, 4 CH arom.)].

Le 1-[bis(4-chlorophényl)méthyl]-3-[(méthylsulfonyl)(3-pyrrolidinylphényl)méthyl-(RS)]azétidin-3-ol peut être obtenu de la manière suivante : en opérant selon le mode opératoire de l'exemple 1 à partir de 0,5 g de méthyl(3-pyrrolidinylbenzyl)sulfone, de 0,6 g de 1-[bis(4-chlorophényl)méthyl]azétidin-3-one et 1,4 cm³ d'une solution 1,6 N de n butyllithium dans l'hexane, on obtient 0,6 g de 1-[bis(4-chlorophényl)méthyl]-3-[(méthylsulfonyl)(3-pyrrolidinylphényl)méthyl-(RS)]azétidin-3-ol sous forme d'un solide crême.

### Exemple 68

En opérant selon le mode opératoire de l'exemple 58 à partir de 5,1 g de 1-[bis(4-chlorophényl)méthyl]-3-{[3-(tert-butyldiméthylsilyloxyméthyl)phényl](méthylsulfonyl)méthylène}azétidine et 17 cm³ d'une solution 1M de fluorure de tétrabutylammmonium dans le tétrahydrofuranne, le résidu obtenu est purifié par chromatographie sur colonne de gel de silice (granulométrie 0,04-0,06 mm, diamètre 2 cm, hauteur 30 cm) sous une pression de 0,5 bar d'azote avec un mélange dichlorométhane et éthanol (97/3 en volumes) comme éluant et en recueillant des fractions de 100 cm³. Les fractions 10 à 14 sont réunies, concentrées à sec sous pression réduite (2,7 kPa). Le solide jaune obtenu est repris par 2 cm³ de dichlorométhane et 10 cm³ d'acétate d'éthyle puis filtré sur verre fritté et lavé par 2 cm³ d'acétate d'éthyle. On obtient 1,6 g de 1-[bis(4-chlorophényl)méthyl]-3-[(3-hydroxyméthylphényl)(méthylsulfonyl)méthylène]azétidine sous forme d'un solide blanc fondant à 214°C [Spectre RMN dans DMSO-d6, T=300K, δ en ppm (400 MHz) : 2,95 (3H, s, SCH₃), 3,80 (2H, s, NCH₂), 4,20 (2H, s, NCH₂), 4,50 (2H, d, J=5Hz, OCH₂), 4,80 (1H, s, NCH), 5,25 (1H, t, J=5Hz, OH), 7,30 (1H, d, J=7Hz, CH arom.), entre 7,35 et 7,45 (7H, m, 7CH arom.), 7,50 (4H, d, J=7Hz, 4CH arom.)].

Le 1-[bis(4-chlorophényl)méthyl]-3-{[3-(tert-butyldiméthylsilyloxyméthyl)phényl](méthylsulfonyl)méthylène}azétidine peut être préparé de la manière suivante : en opérant selon le mode opératoire de l'exemple 4 à partir de 10,8 g de 1-[bis(4-chlorophényl)méthyl]-3-{[3-(tert-butyldiméthylsilyloxyméthyl)phényl](méthylsulfo-nyl)méthyl-(RS)}azétidin-3-ol, de 2 cm³ de chlorure de méthanesulfonyle et 8,5 g de 4-diméthylaminopyridine, le résidu obtenu est purifié par chromatographie sur colonne de gel de silice (granulométrie 0,04-0,06 mm, diamètre 4 cm, hauteur 40 cm) sous une pression de 0,5 bar d'azote avec du dichlorométhane comme éluant et en recueillant des fractions de 100 cm³. Les fractions 12 à 29 sont réunies, concentrées à sec sous pression réduite (2,7 kPa). On obtient 5,2 g de 1-[bis(4-chlorophényl)méthyl]-3-{[3-(tert-butyldiméthylsilyloxyméthyl)phényl](méthylsul-fonyl)méthyléne}azétidine sous forme d'une gomme.

Le 1-[bis(4-chlorophényl)méthyl]-3-{[3-(tert-butyldiméthylsilyloxyméthyl)phényl] (méthylsulfonyl)méthyl-(RS)}azétidin-3-ol peut être obtenu de la manière suivante : en opérant selon le mode opératoire de l'exemple 1 à partir de 5,8 g de [3-(tert-butylsilyloxyméthyl)benzyl]méthylsulfone et 5,6 g de 1 -[bis(4-chlorophényl)méthyl]azétidin-3-one, on obtient 10,8 g de 1-[bis(4-chlorophényl)méthyl]-3-{[3-(tert-butyldiméthylsilyloxyméthyl)phényl](méthylsulfonyl)méthyl-(RS)} azétidin-3-ol sous forme d'une gomme.

### Exemple 69

On agite pendant 18 heures à température ambiante un mélange de 0,45 g de 1-[bis(4-chlorophényl)méthyl]-3-{(méthylsulfonyl)[3-(pentafluorophénoxycarbonyl)phényl] méthylène}azétidine, 0,07 cm³ de 1-aminopipéridine dans 4 cm³ de diméthylformamide. Le mélange est repris par 30 cm³ d'acétate d'éthyle. La phase organique est lavée par 3 fois 50 cm³ d'eau, séchée sur du sulfate de magnésium, filtrée et concentrée à sec sous pression réduite (2,7 kPa). On obtient 0,2 g de 1-[bis(4-chlorophényl)méthyl]-3-{(méthylsulfonyl)[3-(N-pipéridylcarbamoyl)phényl]méthylène} azétidine fondant à 175°C [Spectre RMN dans DMSO-d6, T=300K, δ en ppm (400 MHz) : 1,40 (2H, m, CH₂), 1,60 (4H, m, 2CH₂), 2,85 (4H, m, 2NCH₂), 3,00 (3H, s, SCH₃), 3,80 (2H, s, NCH₂), 4,20 (2H, s, NCH₂), 4,80 (1H, s, NCH), entre 7,45 et 7,60 (10H, m, 10CH arom.), 7,75 (2H, m, 2CH arom.), 9,45 (1H, s, NH)].

Le 1-[bis(4-chlorophényl)méthyl]-3- {(méthylsulfonyl)[3-(pentafluorophénoxy-carbonyl)phényl]méthylène} azétidine peut être préparé de la manière suivante : en opérant selon le mode opératoire de l'exemple 29 à partir de 2,6 g de chlorhydrate de 1-[bis(4-chlorophényl)méthyl]-3-[(3-carboxyphényl)(méthylsulfonyl)méthylène]azétidine, 0,9 g de pentafluorophénol, 0,9 g de chlorhydrate de 1-(3-diméthylaminopropyl)3-éthylcarbodiimide dans 25 cm³ de diméthylformamide, le résidu obtenu est purifié par chromatographie sur colonne de gel de silice (granulométrie 0,04-0,06 mm, diamètre 2 cm, hauteur 30 cm) sous une pression de 0,5 bar d'azote avec un mélange dichlorométhane et éthanol (99/1 en volumes) comme éluant et en recueillant des fractions de 30 cm³. Les fractions 7 à 12 sont réunies et concentrées à sec sous pression réduite (2,7 kPa). On obtient 0,9 g de 1-[bis(4-chlorophényl)méthyl]-3-{(méthylsulfonyl)[3-(pentafluorophénoxycarbonyl)phényl]méthylène}azéti-dine sous forme d'une meringue.

La 1-[bis(4-chlorophényl)méthyl]-3-[(3-carboxyphényl)(méthylsulfonyl) méthylène]azétidine peut être préparée de la manière suivante : à un mélange de 0,5 g de 1-[bis(4-chlorophényl)méthyl]-3-[(3-hydroxyméthylphényl)(méthylsulfonyl)méthylène]azétidine dans 9 cm³ d'acétone, refroidie à 5°C, on ajoute 2 cm³ de réactif de Jones. L'agitation est maintenue 2 heures à cette température puis on ajoute 50 cm³ d'un mélange d'eau et glace et 50 cm³ d'acétate d'éthyle. La phase organique est décantée, lavée par 50 cm³ d'une solution aqueuse saturée de chlorure de sodium, séchée sur du sulfate de magnésium, filtrée et concentrée à sec sous pression réduite (2,7 kPa). Le résidu obtenu est purifié par chromatographie sur colonne de gel de silice (granulométrie 0,04-0,06 mm, diamètre 3 cm, hauteur 25 cm) sous une pression de 0,5 bar d'azote avec un mélange dichlorométhane et éthanol comme éluant et en recueillant des fractions de 60 cm³. Les fractions 12 à 14 sont réunies, concentrées à sec sous pression réduite (2,7 kPa). Le solide obtenu est cristallisé dans 10 cm³ d'éther éthylique. On obtient 32 mg de 1-[bis(4-chlorophényl)méthyl]-3-[(3-carboxyphényl)(méthylsulfonyl)méthylène]azétidine sous forme d'un solide fondant à 205°C [Spectre RMN dans DMSO-d6, T=300K, δ en ppm (400 MHz) : 2,90 (3H, s, SCH₃), 3,80 (2H, s, NCH₂), 4,20 (2H, s, NCH₂), 4,80 (1H, s, NCH), 7,33 (4H, d, J=7Hz, 4CH arom.), 7,39 (1H, d, J=7Hz, CH arom.), 7,42 (4H, d, J=7Hz, 4CH arom.), 7,49 (1H, t, J=7Hz, CH arom.), 7,57 (1H, d, J=7Hz, CH arom.), 7,90 (2H, s, CH arom. et NH⁺)].

### Exemple 70

En opérant selon le mode opératoire de l'exemple 4 à partir de 0,8 g de 1-[bis(4-chlorophényl)méthyl]-3-[(méthylsulfonyl)(3-trifluorométhylsulfanylphényl)méthyl-(RS)]azétidin-3-ol, de 0,24 g de chlorure de méthanesulfonyle et 0,7 g de 4-diméthylaminopyridine, le résidu obtenu est purifié par chromatographie sur colonne de gel de silice (granulométrie 0,04-0,06 mm, diamètre 2 cm, hauteur 18 cm) sous une pression de 0,5 bar d'azote avec du dichlorométhane comme éluant et en recueillant des fractions de 50 cm³. Les fractions 12 à 17 sont réunies, concentrées à sec sous pression réduite (2,7 kPa). Le résidu obtenu est purifié à nouveau par chromatographie sur colonne de gel de silice (granulométrie 0,04-0,06 mm, diamètre 2 cm, hauteur 20 cm) sous une pression de 0,5 bar d'azote avec du dichlorométhane comme éluant et en recueillant des fractions de 30 cm³. Les fractions 15 à 28 sont réunies, concentrées à sec sous pression réduite (2,7 kPa). On obtient 0,25 g de 1-[bis(4-chlorophényl)méthyl]-3-[(méthylsulfonyl)(3-trifluorométhylsulfanylphényl)méthylène]azétidine fondant à 70°C [Spectre RMN dans DMSO-d6 + CD₃CO₂D, T=300K, δ en ppm (300 MHz) : 3,00 (3H, s, SCH₃), 3,80 (2H, s, NCH₂), 4,20 (2H, s, NCH₂), 4,80 (1H, s, NCH), 7,35 (4H, d, J=7Hz, 4CH arom.), 7,45 (4H, d, J=7Hz, 4 CH arom.), 7,60 (2H, m, 2CH arom), 7,75 (2H, m, 2CH arom.)].

Le 1-[bis(4-chlorophényl)méthyl]-3-[(méthylsulfonyl)(3-trifluorométhylsulfanylphényl)méthyl-(RS)]azétidin-3-ol peut être obtenu de la manière suivante : en opérant selon le mode opératoire de l'exemple 1 à partir de 2 g de méthyl(3-trifluorométhylsulfanylbenzyl)sulfone, de 2,3 g de 1-[bis(4-chlorophényl)méthyl]azétidin-3-one et 5,5 cm³ d'une solution de n butyllithium 1,6M dans l'hexane, on obtient 0,9 g de 1-benzhydryl-3-[(méthylsulfonyl)(3-trifluorométhylsulfanylphényl)méthyl-(RS)]azétidino-3-ol sous forme d'un solide blanc.

La méthyl(3-trifluorométhylsulfanylbenzyl)sulfone peut être préparée de la manière suivante : en opérant selon le mode opératoire de l'exemple 10 à partir de 5 g de chlorure de 3-trifluorométhylsulfanylbenzyle et 3,2 g de méthanesulfinate de sodium, on obtient 5,2 g de méthyl(3-trifluorométhylsulfanylbenzyl)sulfone sous forme d'un solide blanc fondant à 125°C.

### Exemple 71

En opérant comme à l'exemple 38 (méthode 1), à partir de 0,72 g de 1-[bis(4-fluorophényl)méthyl]-3-[(3,5-difluorophényl)(méthylsulfonyl)méthyl-(RS)]azétidin-3-ol, de 0,18 cm³ de chlorure de méthanesulfonyle et 0,66 g de 4-diméthylaminopyridine, on obtient après chromatographie sur colonne de gel de silice (granulométrie 0,04-0,06 mm, diamètre 2,5 cm, hauteur 15 cm), sous une pression de 1 bar d'argon avec un mélange d'acétate d'éthyle et cyclohexane (15/85 en volumes) comme éluant et en recueillant des fractions de 25 cm³, 0,42 g de 1-[bis(4-fluorophényl)méthyl]-3-[(3,5-difluorophényl)(méthylsulfonyl)méthylène]azétidine sous la forme d'une meringue blanche [Spectre RMN dans DMSO-d6, T=300K, δ en ppm (250 MHz) : 3,05 (3H, s, SCH₃), 3,90 (2H, s, NCH₂), 4,20 (2H, s, NCH₂), 4,80 (1H, s, NCH), 7,15 (6H, m, 6CH arom.), 7,35 (1H, t, J=8Hz, CH arom.), 7,50 (4H, dd, J=6 et 8Hz, 4CH arom.)].

Le 1-[bis(4-fluorophényl)méthyl]-3-[(3,5-difluorophényl)(méthylsulfonyl)méthyl-(RS)]azétidin-3-ol peut être obtenu de la manière suivante : on opère comme à l'exemple 39 à partir de 2,25 g de bis(4-fluorophényl)bromométhane, de 1,1 g de carbonate de potassium, et 2,5 g de chlorhydrate de 3-[(3,5-difluorophényl)(méthylsulfonyl)méthyl-(RS)]azétidin-3-ol. Après chromatographie sur colonne de gel de silice (granulométrie 0,04-0,06 mm, diamètre 4,4 cm, hauteur 25 cm), sous une pression de 0,9 bar d'argon avec un mélange d'acétate d'éthyle et cyclohexane (2/8 en volumes) comme éluant et en recueillant des fractions de 60 cm³, les fractions 23 à 39 sont réunies puis concentrées à sec sous pression réduite (2,7 kPa). On obtient 0,72 g de 1-[bis(4-fluorophényl)méthyl]-3-[(3,5-difluorophényl)(méthylsulfonyl) méthyl-(RS)]azétidin-3-ol, sous la forme d'un solide blanc.

Le bis(4-fluorophényl)bromométhane peut être préparé selon le mode opératoire décrit par BACHMANN W.E., J. Am. Chem. Soc., 2135 (1933) à partir de 4 g de 4,4'-difluorobenzydrol, de 2,70 cm³ de bromure d'acétyle et 14 cm³ d'une solution d'acide bromhydrique à 33% dans l'acide acétique.

### Exemple 72

En opérant comme à l'exemple 38 (méthode 1), à partir de 1,22 g de 1-[bis(2-fluorophényl)méthyl]-3-[(3,5-difluorophényl)(méthylsulfonyl)méthyl-(RS)]azétidin-3-ol, de 0,29 cm³ de chlorure de méthanesulfonyle et 1,1 g de 4-diméthylaminopyridine, on obtient après chromatographie sur colonne de gel de silice (granulométrie 0,04-0,06 mm, diamètre 3 cm, hauteur 23 cm), sous une pression de 1 bar d'argon avec un mélange d'acétate d'éthyle et cyclohexane (15/85 en volumes) comme éluant et en recueillant des fractions de 60 cm³, 0,177 g de 1-[bis(2-fluorophényl)méthyl]-3-[(3,5-difluorophényl)(méthylsulfonyl)méthylène]azétidine sous la forme d'une meringue blanche [Spectre RMN dans DMSO-d6, T=300K, δ en ppm (300 MHz) : 3,05 (3H, s, SCH₃), 3,95 (2H, s, NCH₂), 4,25 (2H, s, NCH₂), 5,35 (1H, s, NCH), 7,20 (6H, m, 6CH arom.), 7,35 (3H, m, 3CH arom.), 7,55 (2H, m, 2CH arom.)].

Le 1-[bis(2-fluorophényl)méthyl]-3-[(3,5-difluorophényl)(méthylsulfonyl)méthyl-(RS)]azétidin-3-ol peut être obtenu de la manière suivante : on opère comme à l'exemple 39 à partir de 2 g de bis(2-fluorophényl)bromométhane, de 1,0 g de carbonate de potassium et 2,22 g de chlorhydrate de 3-[(3,5-difluorophényl)(méthylsulfonyl)méthyl-(RS)]azétidin-3-ol. Après chromatographie sur colonne de gel de silice (granulométrie 0,04-0,06 mm, diamètre 3 cm, hauteur 17 cm), sous une pression de 1 bar d'argon avec un mélange d'acétate d'éthyle et de cyclohexane (2/8 en volumes) comme éluant et en recueillant des fractions de 60 cm³, les fractions 6 à 10 sont réunies puis concentrées à sec sous pression réduite (2,7 kPa). On obtient 1,22 g de 1-[bis(2-fluorophényl)méthyl]-3-[(3,5-difluorophényl)(méthylsulfonyl)méthyl-(RS)]azétidin-3-ol, sous la forme d'un solide blanchâtre.

Le bis(2-fluorophényl)bromométhane peut être préparé selon le mode opératoire décrit par BACHMANN W.E., J. Am. Chem. Soc., 2135 (1933), à partir de 1,80 g de 2,2'-difluorobenzydrol, de 1,22 cm³ de bromure d'acétyle et 6,5 cm³ d'une solution d'acide bromhydrique à 33% dans l'acide acétique.

Le 2,2'-difluorobenzydrol peut être préparé selon la méthode suivante : à une solution refroidie à -70°C sous argon de 8,8 g de 2-bromofluorobenzène dans 100 cm³ de tétrahydrofuranne, on coule goutte à goutte 32 cm³ de n-butyllithium en solution 1,6M dans l'hexane. Après 10 minutes d'agitation à -70°C, on ajoute lentement 2,1 cm³ de formiate d'éthyle puis agite le mélange à -70°C pendant 30 minutes. Le milieu réactionnel est ensuite amené à 0°C puis additionné de 50 cm³ d'acétate d'éthyle et 100 cm³ de solution saturée de chlorure d'ammonium. Après agitation, la phase organique est séparée, séchée sur sulfate de magnésium, concentrée à sec à 55°C, sous pression réduite, (2,7 Kpa). On obtient 3,63 g de 2,2'-difluorobenzydrol, sous la forme d'une huile jaune.

### Exemple 73

En opérant comme à l'exemple 38 (méthode 1), à partir de 1,15 g de 1-[bis(3-fluorophényl)méthyl]-3-[(3,5-difluorophényl)(méthylsulfonyl)méthyl-(RS)]azétidin-3-ol, de 0,264 cm³ de chlorure de méthanesulfonyle, et 0,98 g de 4-diméthylaminopyridine, on obtient après chromatographie sur colonne de gel de silice (granulométrie 0,06-0,200 mm, diamètre 2,8 cm, hauteur 25 cm), sous une pression de 1 bar d'argon avec un mélange d'acétate d'éthyle et cyclohexane (15/85 en volumes) comme éluant et en recueillant des fractions de 60 cm³, 0,55 g de 1-[bis(3-fluorophényl)méthyl]-3-[(3,5-difluorophényl)(méthylsulfonyl)méthylène] azétidine sous la forme d'un solide blanc fondant à 178°C [Spectre RMN dans DMSO-d6, T=300K, δ en ppm (250 MHz) : 3,05 (3H, s, SCH₃), 3,95 (2H, s, NCH₂), 4,25 (2H, s, NCH₂), 4,80 (1H, s, NCH), 7,10 (2H, m, 2CH arom.), 7,20 (2H, m, 2CH arom.), entre 7,30 et 7,50 (7H, m, 7CH arom.)].

Le 1-[bis(3-fluorophényl)méthyl]-3-[(3,5-difluorophényl)(méthylsulfonyl)méthyl-(RS)]azétidin-3-ol peut être préparé de la manière suivante : en opérant selon l'exemple 1 à partir de 1,2 g de (3,5-difluorobenzyl)méthylsulfone et 1,5 g de 1-[bis(3-fluorophényl)méthyl]azétidin-3-one, on obtient après purification sur colonne de gel de silice (granulométrie 0,06-0,200 mm, diamètre 3,2 cm, hauteur 30 cm), sous une pression de 1 bar d'argon avec un mélange d'acétate d'éthyle et cyclohexane (2/8 en volumes) comme éluant et en recueillant des fractions de 60 cm³, 1,95 g de 1-[bis(3-fluorophényl)méthyl]-3-[(3,5-difluorophényl)(méthylsulfonyl)méthyl-(RS)]azétidin-3-ol, sous la forme d'un solide blanc, fondant à 170°C (décomposition).

La 1-[bis(3-fluorophényl)méthyl]azétidin-3-one peut être préparée en opérant de façon identique au mode opératoire décrit par KATRITZKY A.R. et coll., J. Heterocycl. Chem., 271 (1994), à partir de 4,9 g de [bis(3-fluorophényl)méthyl]amine et 1,78 cm³ d'épichlorhydrine.

La [bis(3-fluorophényl)méthyl]amine peut être préparée de la façon suivante : à une suspension de 1,27 g d'hydrure de lithium et d'aluminium dans 80 cm³ de tétrahydrofuranne, on coule sous atmosphère d'argon en 30 minutes une solution de 5,17 g de 3,3'-difluorobenzophénone oxime dans 30 cm³ de tétrahydrofuranne. Après 5 heures d'agitation au reflux, on ajoute successivement 1,3 cm³ d'eau, 1,3 cm³ de soude 4N, 2,6 cm³ d'eau puis 50 cm³ d'acétate d'éthyle. Après séchage sur sulfate de magnésium et concentration à sec sous pression réduite (2,7 kPa), on obtient 4,9 g de [bis(3-fluorophényl)méthyl]amine, sous la forme d'une huile jaune.

La 3-3'-difluorobenzophénone oxime peut être préparée selon le mode opératoire suivant : dans une solution de 5,0 g de 3,3'-difluorobenzophénone dans 10 cm³ d'éthanol, on coule goutte à goutte une solution de 1,6 g de chlorhydrate d'hydroxylamine dans 8 cm³ d'eau, puis ajoute par petites fractions 1,2 g de soude en pastilles. Le mélange réactionnel, porté au reflux pendant 10 minutes est refroidi à 20°C puis acidifié par 7,5 cm³ d'acide chlorhydrique 4N. Le précipité huileux obtenu une fois trituré devient un solide blanc que l'on filtre, lave par de l'eau puis sèche à 35°C sous pression réduite (2,7 kPa). On obtient 5,17 g de 3,3'-difluorobenzophénone oxime, sous la forme d'un solide blanc.

### Exemple 74

En opérant comme à l'exemple 1, à partir de 1,30 g d'un mélange de deux diastéréoisomères 1-[(4-chlorophényl)(thiazol-2-yl)méthyl-(RS)]-3-[(méthylsulfonyl)(phényl) méthyl-(RS)]azétidin-3-ol, de 0,35 cm³ de chlorure de méthanesulfonyle et 1,22 g de 4-diméthylaminopyridine, on obtient après chromatographie sur colonne de gel de silice (granulométrie 0,06-0,200 mm, diamètre 2,4 cm, hauteur 25 cm), sous une pression de 1 bar d'argon avec un mélange d'acétate d'éthyle et cyclohexane (1/1 en volumes) comme éluant et en recueillant des fractions de 30 cm³, 0,7 g de (RS)-1-[(4-chlorophényl)(thiazol-2-yl)méthyl]-3-[(méthylsulfonyl)(phényl)méthylène]azétidine, sous la forme d'un solide rosâtre [Spectre RMN dans DMSO-d6, T=300K, δ en ppm (300 MHz) : 2,95 (3H, s, SCH₃), 3,95 (2H, m, NCH₂), 4,35 (2H, m, NCH₂), 5,25 (1H, s, NCH), 7,45 (9H, m, 9CH arom.), 7,65 (1H, d, J=2Hz, CH thiazole), 7,70 (1H, d, J=2Hz, CH thiazole)].

Le mélange des deux diastéréoisomères 1-[(4-chlorophényl)(thiazol-2-yl)méthyl-(RS)]-3-[(méthylsulfonyl)(phényl)méthyl-(RS)]azétidin-3-ol, peut être obtenu de la manière suivante : en opérant comme à l'exemple 39 à partir de 4,47 g de (RS)-bromo(4-chlorophényl)(thiazol-2-yl)méthane et 4,31 g de chlorhydrate de 3-[(méthylsulfonyl)(phényl)méthyl-(RS)]azétidin-3-ol et après chromatographie sur colonne de gel de silice (granulométrie 0,06-0,200 mm, diamètre 5,6 cm, hauteur 40 cm), sous une pression de 0,5 bar d'argon avec un mélange d'acétate d'éthyle et cyclohexane (25/75 en volumes) jusqu'à la fraction 35 puis par de l'acétate d'éthyle pur comme éluant et en recueillant des fractions de 60 cm³, les fractions 38 à 40 sont réunies puis concentrées à sec sous pression réduite (2,7 kPa). On obtient 1,3 g du mélange des deux diastéréoisomères 1-[(4-chlorophényl)(thiazol-2-yl)méthyl-(RS)]-3-[(méthylsulfonyl)(phényl)méthyl-(RS)]azétidin-3-ol, sous la forme d'un solide blanchâtre.

Le (RS)-bromo(4-chlorophényl)(thiazol-2-yl)méthane peut être préparé selon le mode opératoire décrit par BACHMANN W.E., J. Am. Chem. Soc., 2135 (1933), à partir de 3,5g de (RS)-(4-chlorophényl)(2-thiazolyl)méthanol, de 3,81 g de bromure d'acétyle et 12,0 cm³ d'une solution d'acide bromhydrique à 33% dans l'acide acétique.

Le (RS)-(4-chlorophényl)(thiazol-2-yl)méthanol peut être préparé selon le mode opératoire décrit par G. EVAN BOSWELL et coll, J. Heterocyclic Chem., 32, 1801 (1995), à partir de 4,22 g de 4-chlorobenzaldéhyde et 4,92 g de 2-bromothiazole.

### Exemple 75

En opérant comme à l'exemple 1, à partir de 0,52 g d'un mélange des deux diastéréoisomères 1-[(4-chlorophényl)(thién-2-yl)méthyl-(RS)]-3-[(3,5-difluorophényl)(méthylsulfonyl)méthyl-(RS)]azétidin-3-ol, de 0,14 cm³ de chlorure de méthanesulfonyle et 0,49 g de 4-diméthylaminopyridine, on obtient après chromatographie sur colonne de gel de silice (granulométrie 0,06-0,200 mm, diamètre 2,4 cm, hauteur 20 cm), sous une pression de 0,5 bar d'argon avec un mélange d'acétate d'éthyle et cyclohexane (20/80 en volumes) comme éluant et en recueillant des fractions de 30 cm³, 0,32 g de (RS)-1-[(4-chlorophényl)(thién-2-yl)méthyl]-3-[(3,5-difluorophényl)(méthylsulfonyl)méthylène)]azétidine, sous la forme d'un solide blanc, fondant à 176°C [Spectre RMN dans DMSO-d6, T=300K, δ en ppm (300 MHz) : 2,98 (3H, s, SCH₃), 3,90 (2H, m, NCH₂), 4,20 (2H, s, NCH₂), 5,03 (1H, s, NCH), 6,85 (1H, dd, J=3 et 5Hz, CH thiophene), 7,08 (3H, m, 2CH arom. et 1CH thiophene), 7,22 (1H, t, J=8Hz, CH arom.), 7,32 (3H, m, 2CH arom. et 1CH thiophene), 7,40 (2H, d, J=7Hz, 2CH arom.)].

Le mélange des deux diastéréoisomères 1-[(4-chlorophényl)(thién-2-yl)méthyl-(RS)]-3-[(3,5-difluorophényl)(méthylsulfonyl)méthyl-(RS)]azétidin-3-ol peut être préparé de la manière suivante : en opérant comme à l'exemple 1 à partir de 1,60 cm³ de n-butyllithium 1,6N en solution dans l'hexane, de 0,83 g de (3,5-difluorobenzyl)méthylsulfone et 1,06 g de 1-[(4-Chlorophényl)(thién-2-yl)méthyl-(RS)]azétidin-3-one, on obtient après purification sur colonne de gel de silice (granulométrie 0,06-0,200 mm, diamètre 2,8 cm, hauteur 30 cm), sous une pression de 0,5 bar d'argon avec un mélange d'acétate d'éthyle et cyclohexane (25/75 en volumes) comme éluant et en recueillant des fractions de 40 cm³, 0,55 g du mélange de diastéréoisomères 1-[(4-chlorophényl)(thién-2-yl)méthyl-(RS)]-3-[(3,5-difluorophényl)(méthylsulfonyl)méthyl-(RS)]azétidin-3-ol, sous la forme d'un solide blanc cassé.

La 1-[(4-chlorophényl)(thién-2-yl)méthyl-(RS)]azétidin-3-one peut être préparé en opérant de la façon suivante : à une solution refroidie à -70°C de 1,83 cm³ de chlorure d'oxalyle dans 20 cm³ de dichlorométhane sous argon, on coule en 10 minutes 3,04 cm³ de diméthylsulfoxyde. Après 30 minutes d'agitation à -60°C, on coule en 20 minutes une solution de 5,2 g de 1-[(4-chlorophényl)(thién-2-yl)méthyl-(RS)]azétidin-3-ol dans 80 cm³ de dichlorométhane, agite le mélange pendant 3 heures à une température comprise entre -60° et -70°C, puis ajoute 9,12 cm³ de triéthylamine. Le mélange est alors laissé revenir à température ambiante, puis dilué avec de l'eau. La phase organique est séparée, séchée sur sulfate de magnésium, puis concentrée sous pression réduite à sec. Le résidu est chromatographié sur une colonne de gel de silice (granulométrie 0,06-0,200 mm, diamètre 4 cm, hauteur 36 cm), sous une pression de 0,5 bar d'argon avec un mélange d'acétate d'éthyle et cyclohexane (1/9 en volumes) comme éluant et en recueillant des fractions de 60 cm³. On obtient 3,3 g de 1-[(4-chlorophényl)(thién-2-yl)méthyl-(RS)]zétidin-3-one, sous la forme d'une huile jaune qui cristallise à température ordinaire.

Le 1-[(4-chlorophényl)(thién-2-yl)méthyl-(RS)]azétidin-3-ol peut être préparé de la façon suivante : à une solution de 11,0 g de [(4-chlorophényl)(thién-2-yl)méthyl-(RS)]amine dans 80 cm³ d'éthanol on ajoute 4,12 g de bicarbonate de sodium. Le mélange chauffé à 65°C est additionné de 4,03 cm³ d'épibromhydrine. Après 20 heures d'agitation à 65°C, le mélange refroidi est filtré et le filtrat concentré à sec sous pression réduite (2,7 Kpa). Le résidu est chromatographié sur une colonne de gel de silice (granulométrie 0,06-0,200 mm, diamètre 3,6 cm, hauteur 32 cm), sous une pression de 0,5 bar d'argon avec un mélange d'acétate d'éthyle et cyclohexane (25/75 en volumes) comme éluant et en recueillant des fractions de 60 cm³. On obtient 6,3 g de 1-[(4-chlorophényl)(thién-2-yl)méthyl-(RS)]azétidin-3-ol, sous la forme d'une huile jaune pâle.

La [(4-chlorophényl)(thién-2-yl)méthyl-(RS)]amine peut être préparée de la façon suivante : à une suspension refroidie à 10°C de bromure de 4-chlorophénylmagnésien (préparée à partir de 19,15 g de 4-bromochlorobenzène et 2,43 g de magnésium) dans 120 cm³ d'éther éthylique anhydre, on coule lentement une solution de 10,92 g de 2-thiophénecarbonitrile dans 80 cm³ d'éther éthylique. Après une heure de reflux, le mélange est refroidi à 10°C, additionné lentement de 40 cm³ de méthanol et ensuite filtré sur supercel. On ajoute sous argon et par petites fractions en 15 minutes 4,54 g de borohydrure de sodium puis agite le milieu réactionnel pendant 20 heures à 20°C. Le mélange obtenu est dilué avec de l'acétate d'éthyle, puis lavé avec de l'eau. La phase organique est séchée sur sulfate de magnésium, concentrée à sec à 50°C sous pression réduite (2,7 kPa). Le résidu est chromatographié sur une colonne de gel de silice (granulométrie 0,06-0,200 mm, diamètre 5 cm, hauteur 42 cm), sous une pression de 0,5 bar d'argon avec un mélange d'acétate d'éthyle et cyclohexane (4/6 en volumes) comme éluant et en recueillant des fractions de 100 cm³. Les fractions 6 à 12 concentrées à sec correspondent à 13 g d'imine sous la forme d'une huile jaune que l'on reprend dans 100 cm³ de méthanol. La solution obtenue est additionnée de 2,4 g de borohydrure de sodium, et agitée pendant une heure à 5°C. Le mélange obtenu est dilué avec de l'acétate d'éthyle, puis lavé avec de l'eau. La phase organique est séchée sur sulfate de magnésium, concentrée à sec à 50°C sous pression réduite (2,7 Kpa). Le résidu est chromatographié sur une colonne de gel de silice (granulométrie 0,06-0,200 mm, diamètre 3,2 cm, hauteur 40 cm), sous une pression de 0,5 bar d'argon avec un mélange d'acétate d'éthyle et cyclohexane (4/6 en volumes) comme éluant et en recueillant des fractions de 60 cm³ On obtient 11,0 g de [(4-chlorophényl)(thién-2-yl)méthyl-(RS)]amine, sous la forme d'une huile jaune.

### Exemple 76

En opérant comme décrit dans l'exemple 75, à partir de 1,66 g du mélange des deux diastéréoisomères chiraux 1-[(4-chlorophényl)(thièn-2-yl)méthyl-(R*)]-3-[(3,5-difluorophényl)(méthylsulfonyl)méthyl-(R*)]azétidin-3-ol et 1-[(4-chlorophényl)(thièn-2-yl)méthyl-(R*)]-3-[(3,5-difluorophényl)(méthylsulfony])méthyl-(S*)]azétidin-3-ol, 50 cm³ de dichlorométhane, 0,45 cm³ de chlorure de méthanesulfonyle, et 1,64 g de 4-diméthylaminopyridine, on obtient 0,6 g de (+)-1-[(4-chlorophényl)(thièn-2-yl)méthyl]-3-[(3,5-difluorophényl)(méthylsulfonyl)méthylène]azétidine, sous forme de cristaux blancs fondant à 136°C, [α]²⁰_{D} = +3,2° (c = 0,5% dans le dichlorométhane).

Le mélange des deux diastéréoisomères chiraux 1-[(4-chlorophényl)(thièn-2-yl)méthyl-(R*)]-3-[(3,5-difluorophényl)(méthylsulfonyl)méthyl-(R*)]azétidin-3-ol et 1-[(4-chlorophényl)(thièn-2-yl)méthyl-(R*)]-3-[(3,5-difluorophényl)(méthylsulfonyl)méthyl-(S*)]azétidin-3-ol peut être préparé comme il est décrit dans l'exemple 75, à partir de 1,06 g de (+)-1-[(4-chlorophényl)(thièn-2-yl)méthyl]azétidin-3-one, 0,82 g de (3,5-difluorobenzyl)méthylsulfone, 2,5 cm³ de solution de nbutyllithium 1,6N dans l'hexane, et 25 cm³ de tétrahydrofuranne. On obtient après purification par chromatographie, 1,7 g du mélange des deux diastéréoisomères chiraux 1-[(4-chlorophényl)(thièn-2-yl)méthyl-(R*)]-3-[(3,5-difluorophényl)(méthylsulfonyl)méthyl-(R*)]azétidin-3-ol et 1-[(4-chlorophényl)(thièn-2-yl)méthyl-(R*)]-3-[(3,5-difluorophényl)(méthylsulfonyl)méthyl-(S*)]azétidin-3-ol, sous la forme d'un solide blanc.

Le (+)-1-[(4-chlorophényl)(thièn-2-yl)méthyl]azétidin-3-one peut être préparé comme il est décrit dans l'exemple 75, à partir de 12,4 g de (+)-1-[(4-chlorophényl)(thièn-2-yl)méthyl]azétidin-3-ol, 220 cm³ de dichlorométhane, 7,1 cm³ de diméthylsulfoxyde, 4,4 cm³ de chlorure d'oxalyle, et 21,5 cm³ de triéthylamine. On obtient 9,2 g de (+)-1-[(4-chlorophényl)(thièn-2-yl)méthyl]azétidin-3-one, sous la forme d'une huile jaune pâle cristallisant à 20°C.

Le (+)-1-[(4-chlorophényl)(thièn-2-yl)méthyl]azétidin-3-ol peut être préparé comme il est décrit dans l'exemple 75, à partir de 16,1 g de (+)-[(4-chlorophényl)(thièn-2-yl)méthyl]amine, 130 cm³ d'éthanol, 5,9 cm³ d'épibromhydrine, et 6,05 g de bicarbonate de sodium. On obtient après purification par chromatographie, 11,5 g de (+)-1-[(4-chlorophényl)(thièn-2-yl)méthyl]azétidin-3-ol, sous la forme d'une huile de couleur crème.

La (+)-4-[(chlorophényl)(thièn-2-yl)méthyl]amine peut être préparée de la façon suivante : à une solution de 109 g de [(4-chlorophényl)(thièn-2-yl)méthyl-(RS)]amine dans 500 cm³ de méthanol, on ajoute 73 g d'acide D-(-)-tartrique. Le mélange est concentré à sec sous pression réduite (2,7 kPa). La meringue obtenue est reprise par 2,05 litres d'un mélange éthanol-eau 90/10 en volumes. Après 20 heures d'agitation lente à 20°C, la suspension cristalline obtenue est filtrée, les cristaux lavés avec le minimum du même mélange de solvants, puis séchés. Une nouvelle recristallisation est effectuée dans les mêmes conditions avec 1,5 litres du même mélange de solvants. On obtient 44,9 g de cristaux du tartrate acide de l'amine. [α]²⁰_{D} = +10,3° (c = 0,5% dans le diméthylformamide). Ce composé est recristallisé dans 600 cm³ d'un mélange éthanol-eau 80/20 en volumes (les cristaux sont filtrés et lavés par 2 fois 30 cm³ du même mélange de solvants puis essorés), puis recristallisé dans les mêmes conditions avec 400 cm³ d'un mélange éthanol-eau 78/22. On obtient 28,2 g de D-(-)-tartrate acide de (+)-[(4-chlorophényl)thièn-2-yl)méthyl]amine, sous la forme de cristaux blancs [α]²⁰_{D} = +10,8° (c = 0,5% dans le diméthylformamide).

Ce sel est repris par 400 cm³ d'une solution aqueuse d'hydroxyde de sodium 1N et par 100 cm³ d'acétate d'éthyle. La phase organique est séparée, lavée avec 100 cm³ d'eau, séchée sur sulfate de magnésium, puis concentrée à sec sous pression réduite (2,7 kPa). On obtient 16,1 g de (+)-[(4-chlorophényl)(thièn-2-yl)méthyl]amine, sous la forme d'une huile qui cristallise à 20°C, [α]²⁰_{D} = +32,7° (c = 0,5% dans le dichlorométhane).

### Exemple 77

En opérant comme décrit dans l'exemple 75, à partir de 1,30 g du mélange des deux diastéréoisomères chiraux 1-[(4-chlorophényl)(thièn-2-yl)méthyl-(S*)]-3-[(3,5-difluorophényl)(méthylsulfonyl)méthyl-(R*)]azétidin-3-ol et 1-[(4-chlorophényl)(thièn-2-yl)méthyl-(S*)]-3-[(3,5-difluorophényl)(méthylsulfonyl)méthyl-(S*)] azétidin-3-ol, 40 cm⁵ de dichlorométhane, 0,35 cm³ de chlorure de méthanesulfonyle et 1,28 g de 4-diméthylaminopyridine, on obtient 0,97 g de (-)-1-[(4-chlorophényl)(thièn-2-yl)méthyl]-3-[(3,5-difluorophényl)(méthylsulfonyl)méthylène] zétidine, sous la forme de cristaux blancs fondant à 135°C, [α]²⁰_{D} = -3,4° (c = 0,5% dans le dichlorométhane).

Le mélange des deux diastéréoisomères chiraux 1-[(4-chlorophényl)(thièn-2-yl)méthyl-(S*)]-3-[(3,5-difluorophényl)(méthylsulfonyl)méthyl-(R*)]azétidin-3-ol et 1-[(4-chlorophényl)(thièn-2-yl)méthyl-(S*)]-3-[(3,5-difluorophényl)(méthyl-sulfonyl)méthyl-(S*)]azétidin-3-ol peut être préparé comme il est décrit dans l'exemple 75, à partir de 1,06 g de (-)-1-[(4-chlorophényl)(thièn-2-yl)méthyl]azétidin-3-one, 0,82 g de (3,5-difluorobenzyl)méthylsulfone, 2,5 cm³ de solution de nbutyllithium 1,6N dans l'hexane, et 25 cm³ de tétrahydrofuranne. On obtient après purification par chromatographie, 1,3 g du mélange des deux diastéréoisomères chiraux 1-[(4-chlorophényl)(thièn-2-yl)méthyl-(S*)]-3-[(3,5-difluorophényl)(méthylsulfonyl)méthyl-(R*)]azétidin-3-ol et 1-[(4-chlorophényl)(thièn-2-yl)méthyl-(S*)]-3-[(3,5-difluorophényl)(méthylsulfonyl)méthyl-(S*)]azétidin-3-ol, sous la forme d'un solide blanc.

Le (-)-1-[(4-chlorophényl)(thièn-2-yl)méthyl]azétidin-3-one peut être préparé comme il est décrit dans l'exemple 75, à partir de 11,4 g de (-)-1-[(4-chlorophényl)(thièn-2-yl)méthyl]azétidin-3-ol, 200 cm³ de dichlorométhane, 4,0 cm³ de diméthylsulfoxyde, 4,0 cm³ de chlorure d'oxalyle, et 19,5 cm³ de triéthylamine. On obtient 8,3 g de (-)-1-[(4-chlorophényl)(thièn-2-yl)méthyl]azétidin-3-one, sous la forme d'une huile jaune pâle cristallisant à 20°C.

Le (-)-1-[(4-chlorophényl)(thièn-2-yl)méthyl]azétidin-3-ol peut être préparé comme il est décrit dans l'exemple 75, à partir de 15,4 g de (-)-[(4-chlorophényl)(thièn-2-yl)méthyl]amine, 120 cm³ d'éthanol, 5,8 cm³ d'épibromhydrine, et 5,8 g de bicarbonate de sodium. On obtient après purification par chromatographie, 10,7 g de (-)-1-[(4-chlorophényl)(thièn-2-yl)méthyl]azétidin-3-ol, sous la forme d'une huile de couleur crème.

La (-)-[(4-chlorophényl)(thièn-2-yl)méthyl]amine peut être préparée de la façon suivante: à une solution de 43 g de [(4-chlorophényl)(thièn-2-yl)méthyl-(RS)]amine dans 200 cm³ de méthanol, on ajoute 29 g d'acide L-(+)-tartrique. Le mélange obtenu cristallise en 2 heures à température ambiante. Les cristaux sont filtrés, lavés par 2 fois 10 cm³ de méthanol. Une recristallisation est effectuée avec 500 cm³ d'un mélange d'éthanol-eau 80/20 en volumes, les cristaux sont filtrés, lavés par 2 fois avec 30 cm³ du même mélange de solvants, puis séchés sous vide à 45°C. Une dernière recristallisation est effectuée avec 350 cm³ d'un mélange éthanol-eau 78/22 en volumes, en laissant agiter 20 heures à 20°C. Les cristaux obtenus sont essorés, séchés sous pression réduite (2,7 kPa). On obtient 26 g de L-(+)-tartrate acide de (-)-[(4-chlorophényl)(thièn-2-yl)méthyl]amine. [α]²⁰_{D} = -10,7° (c = 0,5% dans le diméthylformamide).

Ce sel est repris par 400 cm³ d'une solution aqueuse d'hydroxyde de sodium 1N et par 100 cm³ d'acétate d'éthyle. La phase organique est séparée, lavée avec 100 cm³ d'eau, séchée sur sulfate de magnésium, puis concentrée à sec sous pression réduite (2,7 kPa). On obtient 15,4 g de (-)-[(4-chlorophényl)(thièn-2-yl)méthyl]amine, sous la forme d'une huile qui cristallise à 20°C. [α]²⁰_{D} = -31,7° (c = 0,5% dans le dichlorométhane).

### Exemple 78

En opérant selon le mode opératoire de l'exemple 1 à partir de 3,4 g de 1-benzhydryl-3-[(éthylsulfonyl)(phényl)méthyl-(RS)]azétidin-3-ol, de 0,72 cm ³ de chlorure de methanesulfonyle et 3,8g de 4-diméthylaminopyridine, on obtient, après recristallisation dans 40 cm³ d'acétonitrile, 1,9 g de 1-benzhydryl-3-[(éthylsulfonyl)(phényl)méthylène]azétidine sous la forme de cristaux fondant à 210°C [Spectre RMN dans DMSO-d6, T=300K, δ en ppm (300 Mhz) : 1,15 (3H, t, J=6Hz, CH₃), 2,92 (2H, q, J=6Hz, CH₂), 3,83 (2H, s, NCH₂), 4,20 (2H, s, NCH₂), 4,75 (1H, s, NCH), entre 7,20 et 7,50 (15H, m, 3 phényles)].

Le 1-benzhydryl-3-[(éthylsulfonyl)(phényl)méthyl-(RS)]azétidin-3-ol peut-être obtenu en opérant selon le mode opératoire décrit dans l'exemple 1 à partir de 2,4g de benzyléthylsulfone, de 2,2 cm³ de diisopropylamine, de 10 cm³ de n-butyl lithium 1,6N en solution dans l'hexane, 65 cm³ de tétrahydrofuranne et 3,1 g de 1-benzhydryl azétidin-3-one. On obtient après recristallisation dans 30cm³ d'acétonitrile, 3,6 g de 1-benzhydryl-3-[(éthylsulfonyl)(phényl)méthyl-(RS)]azétidin-3-ol sous la forme de cristaux blancs fondant à 222°C.

La benzyléthylsulfone peut-être préparée en opérant selon le mode opératoire de l'exemple 2 à partir de 6,3 g de benzyléthylsulfure, de 50 cm³ d'acide acétique, 50 cm³ d'eau, 25 cm³ d'acide sulfurique 36N et 24,8 g d'oxone^{R}. On obtient 3,2 g de benzyléthylsulfone, par recristallisation dans 20cm³ d'éther éthylique, sous la forme d'un solide fondant à 86°C.

Le benzyléthylsulfure peut-être préparé de la manière suivante : à une solution de 5 g de benzylmercaptan dans 50 cm³ de diméthylformamide sous argon, on ajoute par petites portions 1,2 g d'hydrure de sodium puis coule 3,36 cm³ d'iodure d'éthyle, en maintenant une température inférieure à 45°C. Le mélange est agité pendant 2 heures puis repris par 200 cm³ d'éther éthylique. La phase organique est lavée par 200 cm³ d'eau puis par 3 fois 100 cm³ d'eau, séchée sur sulfate de magnésium et concentrée à sec sous pression réduite (2,7kPa). On obtient 6,3 g de benzyléthylsulfure sous la forme d'un liquide jaune pâle.

### Exemple 79

A une solution de 0,45 g de 1-[bis(4-chlorophényl)méthyl]-3-{(méthylsulfonyl)[3-(pentafluorophénoxycarbonyl)phényl]méthylène]azétidine dans 5 cm³ de diméthylformamide, on ajoute 0,083 g de 1-amino-4-méthylpipérazine. On agite 20 heures à température ambiante puis on ajoute 40 cm³ d'acétate d'éthyle. La phase organique est lavée par 4 fois 20 cm³ d'eau, séchée sur du sulfate de magnésium et concentrée à sec sous pression réduite (2,7kPa). Le résidu est trituré avec 10cm³ d'éther éthylique, filtré, puis séché. On obtient 0,2 g de 1-[bis(4-chlorophényl)méthyl]-3-{(méthylsulfonyl)[(N-4-méthylpipérazinylcarbamoyl)phényl]méthylène}azétidine sous la forme d'un solide jaune, fondant à 162°C [Spectre RMN dans DMSO-d6, T=300K, δ en ppm (300 MHz) : 2,20 (3H, s, NCH₃), 2,40 (4H, m, 2 NCH₂), 2,90 (4H, m, 2 NCH₂), 2,95 (3H, s, SCH₃), 3,80 (2H, s, NCH₂), 4,20 (2H, s, NCH₂), 4,80 (1H, s, NCH), 7,40 (4H, d, J=7Hz, 4CH arom.), 7,50 (4H, d, J=7Hz, 4CH arom.), 7,55 (2H, m, 2CH arom.), 7,80 (2H, m, 2CH arom.), 9,50 (1H, s, CONH)].

Le 1-[bis(4-chlorophényl)méthyl]-3-{(méthylsulfonyl)[3-(pentafluorophénoxycarbonyl)phényl]méthylène]azétidine peut être préparé de la façon suivante : A une solution de 2,9g de 1-[bis(4-chlorophényl)méthyl]-3-[(3-carboxyphényl)(méthylsulfonyl)méthylène]azétidine dans 25 cm³ de diméthylformamide, on ajoute 0,94 g de chlorhydrate de N-(3-diméthylaminopropyl)N'-éthylcarbodiimide et 0,89 g de pentafluorophénol. On agite 20 heures à température ambiante puis on reprend par 50 cm³ d'acétate d'éthyle. La phase organique est lavée par 100 cm³ d'eau, 200 cm³ d'une solution aqueuse saturée de bicarbonate de sodium puis par deux fois 50 cm³ d'eau distillée, séchée sur sulfate de magnésium et concentrée à sec sous pression réduite (2,7kpa). Le résidu est chromatographié sur une colonne de silice (gralunométrie 0,04-0,006mm, diamètre 2 cm), en éluant par un mélange dichlorométhane et éthanol (99/1 en volume). On obtient 0,92 g de 1-[bis(4-chlorophényl)méthyl]-3- {(méthylsulfonyl)[3-(pentafluorophénoxycarbonyl)phényl]méthylène]azétidine, sous la forme d'une meringue blanche.

Le 1-[bis(4-chlorophényl)méthyl]-3-[(3-carboxyphényl)(méthylsulfonyl)méthylène] azétidine peut être préparé de la manière suivante : A une solution de 3,8 g 1-[bis(4-chlorophényl)méthyl]-3-[(3-cyanophényl)(méthylsulfonyl)méthylène]azétidine dans 5 cm³ d'acide acétique est ajoutée une solution d'acide chlorhydrique à 36% à une température de 50°C. Le chauffage est poursuivi 48 heures puis le mélange est évaporé à sec sous pression réduite (2,7 Kpa). Le résidu est repris par 30 cm³ d'éthanol et évaporé à sec de nouveau. Le résidu est trituré dans 35 cm³ d'éther éthylique. On obtient 3,8 g de 1-[bis(4-chlorophényl)méthyl]-3-[(3-carboxyphényl)(méthylsulfonyl)méthylène]azétidine, sous la forme d'un solide beige.

Le 1-[bis(4-chlorophényl)méthyl]-3-[(3-cyanophényl)(méthylsulfonyl)méthylène] azétidine peut être préparé selon le mode opératoire de l'exemple 4, à partir de 11 g de 1-[bis(4-chlorophényl)méthyl]-3-[(3-cyanophényl)(méthylsulfonyl)méthyl-(RS)] azétidin-3-ol, de 150 cm³ de dichlorométhane, de 2,54 cm³ de chlorure de méthanesulfonyle et 10,7 g de 4-diméthylaminopyridine, à température ambiante pendant 3 heures. Le résidu obtenu est purifié par chromatographie sur colonne de gel de silice (granulométrie 0,04-0,06mm, diamètre 4,5 cm) et élué avec du dichlorométhane puis avec un mélange dichlorométhane et éthanol (99,6/0,4 en volume). Les fractions sont évaporées à sec sous pression réduite (2,7 Kpa). On obtient 3,8 g de 1-[bis(4-chlorophényl)méthyl]-3-[(3-cyanophényl)(méthylsulfonyl)méthylène]azétidine, sous la forme d'une meringue blanche .

Le 1-[bis(4-chlorophényl)méthyl]-3-[(3-cyanophényl)(méthylsulfonyl)méthyl-(RS)]azétidin-3-ol peut-être préparé de la façon suivante : A une solution de 17,6 cm³ de n-butyllithium à 1,6M dans l'hexane, dans 30 cm³ de tétrahydrofuranne sous argon, et refroidie à -70°C, est ajoutée une solution de 5 g de 3-cyanobenzyl méthyl sulfone dans 500 cm³ de tétrahydrofuranne en 15minutes. Le mélange est agité pendant 1 heure 30 minutes. Ensuite on coule une solution de 7,8 g de 1-[bis(4-chlorophényl)méthyl]azétidin-3-one dans 80 cm³ de tétrahydrofuranne en 10 minutes. Après 1 heure 30 minutes d'agitation, on coule 60 cm³ d'une solution aqueuse saturée de chlorure d'ammonium, puis laisse revenir à température ambiante. Le mélange est repris par 300 cm³ d'acétate d'éthyle, la phase organique lavée avec 200 cm³ d'une solution aqueuse saturée de chlorure de sodium, sèchée sur sulfate de magnésium et évaporée sous pression réduite (2,7 Kpa). On obtient 11 g de 1-[bis(4-chlorophényl)méthyl]-3-[(3-cyanophényl)(méthylsulfonyl)méthyl-(RS)]azétidin-3-ol sous la forme d'une meringue.

La (3-cyanobenzyl)méthylsulfone peut -être préparée de la façon suivante : A partir d'une solution de 20,2 g de 3-chlorométhylbenzonitrile dans 200 cm³ d'éthanol, on ajoute 17,4 g de méthanesulfinate de sodium à 85%. On agite 20 heures au reflux, puis on reprend par 500 cm³ d'acétate d'éthyle et 500 cm³ d'eau. L'insoluble est filtré, la phase organique dans le filtrat est séchée sur sulfate de magnésium et évaporée à sec sous pression réduite (2,7 Kpa). Le solide obtenu est trituré avec 100 cm³ d'éther éthylique. Après filtration et séchage du solide, on obtient 21 g de (3-cyanobenzyl)méthylsulfone sous la forme de cristaux blancs fondant à 165°C.

Le 3-chlorométhylbenzonitrile peut-être préparé de la façon suivante : Pendant 3 heures on chauffe à 95°C 32 g de 3-chlorométhylbenzoamide dans 200 cm³ d'oxychlorure de phosphore, puis charge 1 litre de glace, agite 1 heure, extrait le mélange par 500 cm³ de dichlorométhane. La phase organique est lavée par 200 cm³ d'eau, séchée sur sulfate de magnésium et évaporée à sec sous préssion réduite (2,7 Kpa). On obtient 20,2g de 3-chlorométhylbenzonitrile sous la forme d'un solide blanc.

Le 3-chlorométhylbenzoamide peut-être préparé de la façon suivante : A une solution de 50 g de chlorure de 3-chlorométhylbenzoyle dans 150 cm³ d'éther éthylique, on coule 150 cm³ d'une solution d'ammoniaque (d=0,90), refroidit, agite 1 heure, filtre et lave avec 2 fois 200 cm³ d'éther éthylique. On obtient 32 g de 3-chlorométhylbenzoamide sous la forme de cristaux blancs.

### Exemple 80

En opérant selon le mode opératoire de l'exemple 79 à partir de 0,5g de 1-[bis(4-chlorophényl)méthyl]-3-{(méthylsulfonyl)[3-(pentafluorophénoxycarbonyl)phényl] méthylène}azétidine, 0,06 cm³, 1,1-diméthylhydrazine et 5 cm³ de diméthylformamide, on obtient 0,125 g de 1-[bis(4-chlorophényl)méthyl]-3-{[3-(2,2-diméthylcarbohydrazido)phényl](méthylsulfonyl)méthylène}azétidine, sous la forme d'un solide blanc, fondant à 134°C [Spectre RMN dans DMSO-d6, T=300K, δ en ppm (300 Mhz) : 2,60 (6H, s, N(CH₃)₂), 2,95 (3H, s, SCH₃), 3,80 (2H, s, NCH₂), 4,20 (2H, s, NCH₂), 4,80 (1H, s, NCH), 7,35 (4H, d, J=7Hz, 4CH arom.), 7,45 (4H, d, J=7Hz, 4CH arom.), 7,50 (2H, m, 2CH arom.), 7,80 (2H, m, 2CH arom.), 9,50 (1H, s, CONH)].

### Exemple 81

En opérant selon le mode opératoire décrit dans l'exemple 1 à partir de 2,2 g de 1-[bis(thién-2-yl)méthyl]-3-[(3,5-difluorophényl)(méthylsulfonyl)méthyl-(RS)]azétidin-3-ol, 0,64 cm³ de chlorure de méthanesulfonyle, 2,3 g de 4-diméthylaminopyridine et 75 cm³ de dichlorométhane, on obtient, après purification par chromatographie et cristallisation dans le diisopropyl oxyde, 1,3 g de 1-[bis(thién-2-yl)méthyl]-3-[(3,5-difluorophényl)(méthylsulfonyl)méthylène]azétidine, sous la forme de cristaux blancs fondant à 165°C [Spectre RMN dans DMSO-d6, T=300K, δ en ppm (300 Mhz) : 3,00 (3H, s, SCH₃), 3,92 (2H, s, NCH₂), 4,28 (2H, s, NCH₂), 5,40 (1H, s, NCH), 6,95 (2H, dd, J=5 et 2Hz, 2CH thio.), 7,15 (2H, d, J=2Hz, 2CH thio.), 7,20 (2H, m, 2CH arom.), 7,35 (1H, t, J=8Hz, CH arom.), 7,50 (2H, d, J=5Hz, 2CH thio.)].

Le 1-[bis(thién-2-yl)méthyl]-3-[(3,5-difluorophényl)(méthylsulfonyl)méthyl-(RS)]azétidin-3-ol peut-être obtenu selon le mode opératoire décrit dans l'exemple 1, à partir de 1,5 g de 1-[bis(thién-2-yl)méthyl]azétidin-3-one, 4 cm³ de n-butyl lithium à 1,6 N dans l'hexane, 1,3 g de (3,5-difluorobenzyl)méthylsulfone et 40 cm³ de tétrahydrofuranne. On obtient, après purification par chromatographie, 2,2 g de 1-[bis(thién-2-yl)méthyl]-3-[(3,5-difluorophényl)(méthylsulfonyl)méthyl-(RS)]azétidin-3-ol, sous la forme de cristaux blancs fondant à 145°C

Le 1-[bis-thién-2-yl)méthyl]azétidin-3-one peut-être préparé en opérant comme il est décrit dans l'exemple 75, à partir de 4 g de 1-[bis(thién-2-yl)méthyl]azétidin-3-ol, 2,6 cm³ de diméthylsulfoxyde, 7,7 cm³ de triéthylamine, 7,7 cm³ de chlorure d'oxalyle, et 100 cm³ de dichlorométhane. Le résidu obtenu est purifié par chromatographie sur colonne de gel de silice (granulométrie 0,04-0,06mm, diamètre 3 cm, hauteur 30 cm) avec comme éluant un mélange cyclohexane et acétate d'éthyle (1/1 en volume). Les fractions obtenues sont évaporées à sec sous pression réduite (2,7 Kpa). On obtient 3,2 g de 1-[bis(thién-2-yl)méthyl]azétidin-3-one, sous la forme de cristaux crèmes fondant à 70°C.

Le 1-[bis(thién-2-yl)méthyl]azétidin-3-ol peut-être préparé en opérant comme il est décrit dans l'exemple 75, à partir de 6 g de 1-[bis(thién-2-yl)méthyl]amine, 2,5 cm³ d'épibromhydrine, 2,6g de bicarbonate de sodium et 50 cm³ d'éthanol. On obtient 4 g de 1-[bis(thién-2-yl)méthyl]azétidin-3-ol sous la forme de cristaux beiges fondant à 115°C.

La 1-[bis(thién-2-yl)méthyl]amine peut -être préparée de la manière suivante : à une suspension refroidie sous argon à 10°C de bromure de thién-2-ylmagnésien (préparée à partir de 1,29 g de magnésium et 3,22 cm³ 2-bromothiophène dans 75 cm³ de diéthyl oxyde), on coule goutte à goutte une solution de 5cm³ de thién-2-ylcarbonitrile dans 50 cm³ de diéthyle oxyde. Après 1 heure et 30 minutes de reflux, le milieu réactionnel est refroidi à 5°C puis on coule goutte à goutte 20 cm³ de méthanol, filtre la suspension, lave le solide avec du méthanol. Le filtrat obtenu une solution marron. Sous argon, on ajoute à cette solution 2,45 g de borohydrure de sodium, en plusieurs fois. Le mélange est agité à température ambiante pendant 16 heures, puis est dilué par de l'éthyle acétate et additionné d'eau lentement. la phase organique est extraite, lavée avec de l'eau, sèchée sur sulfate de magnésium et évaporée à sec sous pression réduite (2,7 kpa) à 55°C. On obtient une huile marron qui est chromatographiée sur colonne de gel de silice(granulométrie 0,2-0,063 mm, diamètre 8 cm, hauteur 25 cm) et élué par un mélange cyclohexane et acétate d'éthyle (90/10 puis 85/15 en volume). Les fractions 21à 30 sont réunies et évaporées à sec sous pression réduite (2,7 kpa). On obtient 11g de 1-[bis(thién-2-yl)méthyl]amine sous la forme d'un solide cristallisé.

### Exemple 82

En opérant selon le mode opératoire décrit dans l'exemple 1 à partir de 0,47 g de 4-diméthylaminopyridine, 0,13 cm³ de chlorure de méthanesulfonyle, 25 cm³ de dichlorométhane et 0,48 g de 1-(bis-p-tolylméthyl)-3-[(méthylsulfonyl)(phényl)méthyl-(RS)]azétidin-3-ol, on obtient, après purification par chromatographie et cristallisation dans l'oxyde de diisopropyle, 0,25 g de 1-(bis-p-tolylméthyl)-3-[(méthylsulfonyl)(phényl)méthylène]azétidine, sous la forme d'un solide blanc [Spectre RMN dans DMSO-d6, T=300K, δ en ppm (250 Mhz) : 2,23 (6H, s, 2 Ph-CH₃), 2,98 (3H, s, SCH₃), 3,76 (2H, s, NCH₂), 4,20 (2H, s, NCH₂), 5,55 (1H, s, NCH), 7,10 (4H, d, J=7Hz, 4 CH arom.), 7,32 (4H, d, J=7Hz, 4 CH arom.), 7,43 (5H, s, Phényle)].

1-(bis-p-tolylméthyl)-3-[(méthylsulfonyl)(phényl)méthyl-(RS)]azétidin-3-ol peut-être préparé selon le mode opératoire décrit dans l'exemple 39, à partir de 0,59 g de bromo(bis-p-tolyl)méthane, 20 cm³ d'acétonitrile, 0,3 g de carbonate de potassium et 0,6 g de chlorhydrate de 3-[(méthylsulfonyl)(phényl)méthyl-(RS)]azétidin-3-ol. Le résidu obtenu est chromatographié sur colonne de gel de silice (granulométrie 0,04-0,06mm, diamètre 4 cm, hauteur 16 cm) avec comme éluant un mélange cyclohexane/acétate d'éthyle (7/3 en volume). Les fractions sont concentrées à sec sous pression réduite (2,7 Kpa). On obtient 0,48 g de 1-(bis-p-tolylméthyl)-3-[(méthylsulfonyl)(phényl)méthyl-(RS)]azétidin-3-ol sous la forme d'un solide blanc .

Le bromo(di-p-tolyl)méthane peut-être préparé selon le mode opératoire décrit par BACHMANN W.E., J.Am.Chem.Soc., 2135, (1933).

Le chlorhydrate de 3-[(méthylsulfonyl)(phényl)méthyl-(RS)]azétidin-3-ol peut-être préparé selon le mode opératoire décrit dans l'exemple 39 à partir de 7 g de 3-[(méthylsulfonyl)(phényl)méthyl-(RS)]-1-(vinyloxycarbonyl)azétidin-3-ol, 35 cm³ de dioxane, 35 cm³ d'une solution 6,2N d'acide chlorhydrique dans le dioxane. On obtient 5 g de chlorhydrate de 3-[(méthylsulfonyl)(phényl)méthyl-(RS)]azétidin-3-ol, sous la forme d'un solide blanc.

Le 3-[(méthylsulfonyl)(phényl)méthyl-(RS)]-1-(vinyloxycarbonyl)azétidin-3-ol peut-être préparé selon le mode opératoire décrit dans l'exemple 38 (méthode 1), à partir de 10 g de 1-benzhydryl-3-[(méthylsulfonyl)(phényl)méthyl-(RS)]azétidin-3-ol, 600 cm³ de dichlorométhane et 2,52 cm³ de chloroformiate de vinyle. Le résidu est chromatographié sur colonne de gel de silice (granulométrie 0,06-0,2mm, diamètre 5,2 cm, hauteur 36 cm avec comme éluant un mélange cyclohexane et acétate d'éthyle (7/3 en volume). Les fractions sont évaporées à sec sous pression réduite (2,7 Kpa). On obtient 7 g de 3-[(méthylsulfonyl)(phényl)méthyl-(RS)]-1-(vinyloxycarbonyl)azétidin-3-ol sous la forme d'un solide blanc.

### Exemple 83

A une solution de 0,77 g de (-)-1-[(4-chlorophényl)(4-formylphényl)méthyl]-3-[(3,5-difluorophényl)(méthylsulfonyl)méthylène]azétidine dans 20 cm³ de méthanol à 0°C sous argon, on coule une solution de 30 mg de borohydrure de sodium dans 2 cm³ de méthanol. Après agitation pendant 4 heures à 0°C on ajoute de l'eau puis extrait par du dichlorométhane. La phase organique est lavée avec une solution aqueuse saturée de chlorure de sodium, séchée sur sulfate de magnésium, puis évaporée à sec sous pression réduite (2,7 kpa). La meringue blanche obtenue est purifiée sur une colonne de gel de silice (granulométrie 0,04-0,06 mm, diamètre 3,2 cm, hauteur 17 cm) avec comme éluant un mélange cyclohexane et acétate d'éthyle (60/40 en volume) . On obtient après cristallisation dans 1,5 cm³ d'éthanol absolu 0,1 g de (+)-1-[(4-chlorophényl)(4-hydroxyméthylphényl)méthyl]-3-[(3,5-difluorophényl)(méthylsulfonyl)méthylène]azétidine, sous la forme de cristaux blancs fondant à 190°C, [α]²⁰_{D} = +4.2° (c = 0,5% dans le méthanol) [Spectre RMN dans DMSO-d6, T=300K, δ en ppm (300 MHz) : 3,05 (3H,s, SCH₃), 3,95 (2H, s, NCH₂), 4,22 (2H, s, NCH₂), 4,48 (2H, d, J=6Hz, CH₂O), 4,75 (1H, s, NCH), 5,15 (1H, t, J=6Hz, OH), 7,20 (2H, m, 2CH arom.), 7,28 (2H, d, J=7Hz, 2CH arom.), 7,40 (5H, m, 5 CH arom.), 7,50 (2H, d, J=7Hz, 2CH arom.)].

Le (-)-1-[(4-chlorophényl)(4-formylphényl)méthyl]-3-[(3,5-difluorophényl)méthylsulfonylméthylène]azétidine peut-être préparé de la manière suivante : A une solution de 0,83 g de (+)-1-{(4-chlorophényl)[4-(1,3-dioxolan-2-yl)phényl]méthyl}-3-[(3,5-difluorophényl)(méthylsulfonyl)méthylène]azétidine dans 5 cm³ de tétrahydrofuranne, on coule 3,32 cm³ d'une solution d'acide chlorhyrique 5N puis laisse agiter pendant 20 heures. Au milieu réactionnel sont ajoutés du dichlorométhane et l'eau puis une solution aqueuse d'hydroxyde de sodium à 30% jusqu'à l'obtention d'un pH 14. La phase aqueuse est extraite par du dichlorométhane, la phase organique est lavée successivement avec de l'eau, avec une solution aqueuse saturée de chlorure de sodium, séchée sur sulfate de magnésium, filtrée et évaporée à sec sous pression réduite (2,7 kpa). On obtient 0,8 g de (-)-1-[(4-chlorophényl)(4-formylphényl)méthyl]-3-[(3,5-difluorophényl)(méthylsulfonyl)méthylène]azétidine, sous la forme d'une meringue blanche.

Le (+)-1-{(4-chlorophényl)[4-(1,3-dioxolan-2-yl)phényl]méthyl}-3-[(3,5-difluorophényl)(méthylsulfonyl)méthylène]azétidine peut-être obtenu de la manière suivante : A une solution de 2,42 g du mélange des deux diastéréoisomères 3-acétoxy-1-{(4-chlorophényl)[4-(1,3-dioxolan-2-yl)phényl]méthyl-(R*)}-3-[(3,5-difluorophényl)(méthylsulfonyl)méthyl-(R*)]azétidine et 3-acétoxy-1-{(4-chlorophényl)[4-(1,3-dioxolan-2-yl)phényl]méthyl-(R*)}-3-[(3,5-difluorophényl)(méthylsulfonyl)méthyl-(S*)]azétidine dans 25 cm³ de tétrahydrofuranne sous argon à 0°C, on coule goutte à goutte 0,93 g de 1,8-diazabicyclo[5-4-0]undec-7-ène. Après agitation 1 heure et 30 minutes à 0°C, le milieu réactionnel est dilué avec de l'acétate d'éthyle, lavé avec de l'eau et avec une solution aqueuse saturée de chlorure de sodium. La phase organique est séchée sur sulfate de magnésium, filtrée et évaporée à sec sous pression réduite. Le produit brut est purifié sur une colonne de gel de silice (granulométrie 0,04-0,06mm, diamètre 4,8 cm, hauteur 17,5 cm) avec comme éluant un mélange cyclohexane et acétate d'éthyle (80/20 en volumes). On obtient 1,21g de (+)-1-{(4-chlorophényl)[4-( 1,3-dioxolan-2-yl)phényl]méthyl}-3-[(3,5-difluorophényl)(méthylsulfonyl)méthylène]azétidine, sous la forme d'une meringue jaune.

Le mélange des deux diastéréoisomères 3-acétoxy-1-{(4-chlorophényl)[4-(1,3-dioxolan-2-yl)phényl]méthyl-(R*)}-3-[(3,5-difluorophényl)(méthylsulfonyl)méthyl-(R*)]azétidine et 3-acétoxy-1-{(4-chlorophényl)[4-(1,3-dioxolan-2-yl)phényl]méthyl-(R*)}-3-[(3,5-difluorophényl)(méthylsulfonyl)méthyl-(S*)]azétidine peut-être préparé de la manière suivante : à une solution de 1,08 g de 3-5 difluorobenzyl méthyl sulfone sous argon refroidie à -70°C, on coule goutte à goutte 3,27 cm³ de n-butyllithium, laisse agiter 1 heure à -70°C puis coule goutte à goutte une solution de 1,80 g de (+)-1-{(4-chlorophényl)[4-(1,3-dioxolan-2-yl)phényl]méthyl}azétidin-3-one dans 10 cm³ de tétrahydrofuranne. Après agitation 3 heures à -70°C et 1 heure à -20°C, on coule une solution de 0,74 cm³ de chlorure d'acétyle dans 10 cm³ de diéthyl oxyde anhydre à -20°C et agite 2 heures à -20°C. Le milieu réactionnel est jetté sur de l'eau, le mélange est extrait par de l'acétate d'éthyle, la phase organique lavée avec de l'eau et avec une solution aqueuse saturée de chlorure de sodium, séchée sur sulfate de magnésium et concentrée à sec sous pression réduite (2,7 kpa). On obtient 2,42 g du mélange des deux diastéréoisomères 3-acétoxy-1-{(4-chlorophényl)[4-(1,3-dioxolan-2-yl)phényl]méthyl-(R*)}-3-[(3,5-difluorophényl)(méthylsulfonyl)méthyl-(R*)]azétidine et 3-acétoxy-1-{(4-chlorophényl)[4-(1,3-dioxolan-2-yl)phényl]méthyl-(R*)}-3-[(3,5-difluorophényl)(méthylsulfonyl)méthyl-(S*)]azétidine, sous la forme d'une huile jaune.

Le (+)-1-{(4-chlorophényl)[4-(1,3-dioxolan-2-yl)phényl]méthyl}azétidin-3-one peut-être préparé de la manière suivante : à une solution de 1,38 g de (+)-1-{(4-chlorophényl)[4-(1,3-dioxolan-2-yl)phényl]méthyl}azétidin-3-ol dans 20 cm³ de diméthylsulfoxide anhydre sous argon, on coule 2,24 cm³ de triéthylamine puis goutte à goutte 1,65 g une solution de complexe sulfure trioxyde pyridine dans 20 cm³ de diméthylsulfoxide anhydre. Après 1 heure et 15 minutes d'agitation à température ambiante, le milieu réactionnel est jetté sur de la glace, extrait avec de l'acétate d'éthyle, la phase organique est lavée avec de l'eau, avec une solution aqueuse saturée de chlorure de sodium, séchée sur sulfate de magnésium, filtrée et concentrée à sec sous pression réduite (2,7 kpa). Le résidu huileux obtenu (1,31 g), réuni avec un autre lot du même composé brut (1,21 g) sont purifiés ensemble sur une colonne de gel de silice (granulométrie 0,04-0,06mm, diamètre 4,8 cm, hauteur 18 cm) avec comme éluant un mélange cyclohexane et acétate d'éthyle (80/20 en volume). On obtient 1,87 g de (+)-1-{(4-chlorophényl)[4-(1,3-dioxolan-2-yl)phényl]méthyl}azétidin-3-one, sous la forme d'une huile jaune. [α]²⁰365nm = +5,9° (c = 0,5; méthanol)

Le (+)-1-{(4-chlorophényl)[4-(1,3-dioxolan-2-yl)phényl]méthyl}azétidin-3-ol peut être décrit selon le mode opératoire décrit dans l'exemple 75, à partir de 4,43 g de (+)-{(4-chlorophényl)[4-(1,3-dioxolan-2-yl)phényl]méthyl}amine, 40 cm³ d'éthanol absolu, 1,25 cm³ d'épibromhydrine, et 1,28 g de bicarbonate de sodium. On obtient 1,66 g de (+)-1-{(4-chlorophényl)[4-(1,3-dioxolan-2-yl)phényl]méthyl}azétidin-3-ol, sous la forme d'une huile jaune.

La (+)-{(4-chlorophényl)[4-(1,3-dioxolan-2-yl)phényl]méthyl}amine chirale, peut-être obtenue de la manière suivante : à une suspension de 18,16 g de chlorhydrate de (R*)-[(4-chlorophényl)(4-formylphényl)méthyl]amine chirale, dans 1000 cm³ de toluène, on coule 3,95 cm³ d'éthylène glycol et ajoute 0,82 g d'acide paratoluènesulfonique monohydrate. Après 20 heures d'agitation à la température du reflux, le milieu réactionnel est refroidi, lavé avec une solution aqueuse saturée de bicarbonate de sodium, avec de l'eau et avec une solution aqueuse saturée de chlorure de sodium. La phase organique est séchée sur sulfate de magnésium, filtrée et concentrée à sec sous pression réduite. Le résidu obtenu est purifié sur une colonne de gel de silice (granulométrie 0,04-0,06 mm, diamètre 8,4 cm, hauteur 21,5 cm) avec comme éluant un mélange cyclohexane et acétate d'éthyle (30/70 en volume), en recueillant des fractions de 250 cm³. les fractions 23 à 30 sont concentrées à sec sous pression réduite (2,7 kpa). On obtient 1,39 g de (+)-{(4-chlorophényl)[4-(1,3-dioxolan-2-yl)phényl]méthyl}amine chirale, sous la forme d'une huile jaune.

Le chlorhydrate de (R*)-[(4-chlorophényl)(4-formylphényl)méthyl]amine chirale, peut-être préparé de la manière suivante : à une solution de 51,4 g du diastéréoisomère N-{(4-chlorophényl)[4-(diéthoxyméthyl)phényl]méthyl-(R*)}-(R)-2-phénylglycinol dans 660 cm³ de dichlorométhane anhydre, on coule 330 cm³ de méthanol, refroidit le mélange avec un bain de glace, ajoute 60,96 g de tétraacétate de plomb, agite 5 minutes puis coule llitre d'une solution tampon phosphate pH 7. Après agitation 30 minutes à température ambiante, le mélange est filtré, la phase aqueuse extraite avec du dichlorométhane. La phase organique est concentrée à sec sous pression réduite (2,7 kpa). Le résidu est repris avec 1litre de diéthyl oxyde et additionné de 1litre d'une solution aqueuse d'acide chlorhydrique 3N, le mélange est agité 15 minutes à température ambiante, la phase aqueuse est séparée, lavée avec de l'acétate d'éthyle, puis concentrée à sec sous pression réduite (2,7 kpa). On obtient 18,16 g de chlorhydrate de (R*)-[(4-chlorophényl)(4-formylphényl)méthyl]amine chirale, sous la forme d'un solide blanc.

Le N-{(4-chlorophényl)[4-(diéthoxyméthyl)phényl]méthyl-(R*)}-(R)-2-phénylglycinol peut être préparé de la façon suivante : à une solution refroidie à -70°C, sous argon, de 87,7 g de 4-bromochlorobenzène, on coule goutte à goutte 286 cm³ de n butyl lithium à 1,6M dans l'hexane, agite 15 minutes à -70°C. Cette solution obtenue est ensuite ajoutée goutte à goutte à la solution refroidie à 0°C suivante : 30 g de (R)-N-[4-(diéthoxyméthyl)benzylidène]-2-phénylglycinol dans 300 cm³ de diéthyl oxyde. Le mélange est agité 2 heures à 0°C puis jetté sur de l'eau. La phase organique est lavée avec de l'eau, puis avec une solution aqueuse saturée de chlorure de sodium, séchée sur sulfate de magnésium, filtrée et concentrée à sec sous pression réduite (2,7 kpa). On obtient 71,5 g d'une huile rougeâtre qui est purifiée sur une colonne de gel de silice (granulométrie 0,04-0,06 mm, diamètre 11 cm, hauteur 45 cm), avec comme éluant un mélange cyclohexane et acétate d'éthyle (85/15 en volume, puis 80/20 et 75/25), en recueillant des fractions de 1 litre. Les fractions 11 à 17 sont concentrées à sec sous pression réduite (2,7kpa). On obtient 39,85g du seul diastéréoisomère N-{(4-chlorophényl)[4-(diéthoxyméthyl)phényl]méthyl-(R*)}-(R)-2-phénylglycinol, sous la forme d'une huile orangée rouge.

Le (R)-N-[4-(diéthoxyméthyl)benzylidène]-2-phénylglycinol peut être préparé de la façon suivante : A une suspension blanche de 24,7g de (R)-(-)-2-phényl glycinol dans 500 cm³ de toluène on coule 35,9 cm³ de 4-(diéthoxyméthyl)benzaldéhyde. La solution jaune trouble est chauffée au reflux pendant 6 heures 30 minutes, puis agitée à température ambiante pendant 20 heures. Après concentration à sec du milieu réactionnel sous pression réduite (2,7 kpa), on obtient 61,6 g de (R)-N-[4-(diéthoxyméthyl)benzylidène]-2-phénylglycinol, sous la forme d'une huile jaune.

### Exemple 84

En opérant selon le mode opératoire de l'exemple 1, à partir de 5,6g de 1-[bis(4-chlorophényl)méthyl]-3-{[3-(N-tertbutyloxycarbonyl-N-méthylamino)phényl](méthylsulfonyl)méthyl-(RS)}azétidin-3-ol, 100 cm³ de dichlorométhane, 1,59 g de chlorure de méthanesulfonyle et 4,5 g de 4-diméthylaminopyridine. On laisse agiter 3 heures à température ambiante. Le produit brut obtenu est purifié par chromatographie sur une colonne de gel de silice (granulométrie 0,04-0,06 mm, diamètre 4 cm et poids en silice 250g), en éluant sous une pression de 0,5 bar d'azote avec un mélange d'acétate d'éthyle/ cyclohexane (30/70 en volumes) et en recueillant des fractions de 100 cm³. Les fractions 12 à 18 sont réunies, concentrées à sec sous pression réduite (2,7 kpa). On obtient 3,2g de 1-[bis(4-chlorophényl)méthyl]-3-{[3-(N-tertbutyloxycarbonyl-N-méthylamino)phényl](méthylsulfonyl)méthyléne} azétidine sous la forme d'une meringue blanche [Spectre RMN dans DMSO-d6, T=300K, δ en ppm (300 MHz) : 1,30 (9H, s, OC(CH₃)₃), 2,65 (3H, s, J=6Hz, NCH₃), 2,85 (3H, s, SCH₃), 3,50 (2H, s, NCH₂), 3,90 (2H, s, NCH₂), 4,45 (1H, s, NCH), entre 6,85 et 7,05 (8H, m, 8 CH arom.), 7,10 (4H, d, J=7Hz, 4 CH arom.)].

Le 1-[bis(4-chlorophényl)méthyl]-3-{[3-(N-tertbutyloxycarbonyl-N-méthylamino)phényl](méthylsulfonyl)méthyl-(RS)}azétidin-3-ol peut-être préparé selon le mode opératoire décrit dans l'exemple 1 à partir de 3,8 g de [3-(N-tertbutyloxycarbonyl-N-méthylamino)benzyl]méthylsulfone, 50 cm³ de tétrahydrofuranne, 9,5 cm³ d'une solution de n-butyl lithium 1,6N dans l'hexane, 3,82 g de 1-[bis(4-chlorophényl)méthyl]azétidin-3-one. Le produit brut est purifié par chromatographie sur une colonne de gel de silice (granulométrie 0,04-0,06 mm, diamètre 4 cm, poids en silice 250 g), en éluant sous une pression de 0,5 bar d'azote avec du dichlorométhane puis avec un mélange dichlorométhane et éthanol (99/1 en volumes) et en recueillant des fractions de 500 cm³. Les fractions 10 à 16 sont réunies, concentrées à sec sous pression réduite (2,7 kPa). On obtient 5,6 g de 1-[bis(4-chlorophényl)méthyl]-3-{[3-(N-tertbutyloxycarbonyl-N-méthylamino)phényl](méthyl-sulfonyl)méthyl-(RS)}azétidin-3-ol, sous la forme d'une meringue.

### Exemple 85

On agite pendant 20 heures 2,7 g de 1-[bis(4-chlorophényl)méthyl]-3-{[3-(N-tertbutyloxycarbonyl-N-méthylamino)phényl](méthylsulfonyl)méthylène}azétidine dans 30 cm³ de dioxane et 30 cm³ d'une solution de dioxane chlorhydrique 4,7N . Le milieu réactionnel est évaporé à sec sous pression réduite (2,7 kpa), repris par 50 cm³ d'eau et 50 cm³ d'acétate d'éthyle, agité et neutralisé avec précaution par une solution aqueuse saturée de bicarbonate de sodium. La phase organique est séparée, séchée sur sulfate de magnésium, traitée au noir animal puis concentrée sous pression réduite (2,7 kpa) jusqu'à un volume d'environ 25 cm³, ensuite filtrée, concentrée à sec sous pression réduite. On ontient 1,3 g de 1-[bis(4-chlorophényl)méthyl]-3-[(3-méthylaminophényl)(méthylsulfonyl)méthylène]azétidine, sous la forme de cristaux blancs fondant à 228°C [Spectre RMN dans DMSO-d6, T=300K, δ en ppm (300 MHz) : 2,65 (3H, s, J=6Hz, NCH₃), 2,95 (3H, s, SCH₃), 3,80 (2H, s, NCH₂), 4,20 (2H, s, NCH₂), 4,80 (1H, s, NCH), 5,85 (1H, q, J=6Hz, NH), 6,55 (3H, m, 3 CH arom.), 7,15 (1H, t, J=7Hz, CH arom.), 7,40 (4H, d, J=7Hz, 4CH arom.), 7,50 (4H, m, 4CH arom.)].

### Exemple 86

On opère comme à l'exemple 1, à partir de 0,40 g d'un mélange de deux diastéréoisomères 1-[(4chlorophényl)(thiazol-2-yl)méthyl-(RS)]-3-[(3,5-difluorophényl)(méthylsulfonyl)méthyl-(RS)]azétidin-3-ol, de 0,10 cm³ de chlorure de méthanesulfonyle et 0,37 g de 4-diméthylaminopyridine, on obtient après chromatographie sur colonne de gel de silice (granulométrie 0,06-0,200 mm, diamètre 1,2 cm, hauteur 20 cm), sous une pression de 1 bar d'argon avec un mélange d'acétate d'éthyle et cyclohexane (40/60 en volumes) comme éluant et en recueillant des fractions de 20 cm³, 0,13 g de (RS)-1-[(4-chlorophényl)(thiazol-2-yl)méthyl]-3-[(3,5-difluorophényl)(méthylsulfonyl)méthylène]azétidine, sous la forme d'un solide rosâtre [Spectre RMN dans DMSO-d6, T=300K, δ en ppm (300 MHz) : 3,05 (3H,s, SCH₃), 4,05 (2H, s, NCH₂), 4,35 (2H, m, NCH₂), 5,25 (1H, s, NCH), 7,20 (2H, d, J=8Hz, 2CH arom.), 7,35 (1H, t, J=8Hz, CH arom.), 7,45 (2H, d, J=7Hz, 2CH arom.), 7,50 (2H, d, J=7Hz, 2CH arom.), 7,70 (1H, d, J=2Hz, CH thiazole), 7,75 (1H, d, J=2Hz, CH thiazole)].

Le mélange des deux diastéréoisomères 1-[(4-chlorophényl)(thiazol-2-yl)méthyl-(RS)]-3-[(3,5-difluorophényl)(méthylsulfonyl)méthyl-(RS)]azétidin-3-ol, peut être obtenu de la manière suivante : en opérant comme à l'exemple 72, à partir de 1,01 g de (RS)-bromo(4-chlorophényl)thiazol-2-ylméthane et 0,55 g de chlorhydrate de (RS)-3-[(3,5-difluorophényl)(méthylsulfonyl)méthyl]azétidin-3-ol et après chromatographie sur colonne de gel de silice (granulométrie 0,06-0,200 mm, diamètre 4,4 cm, hauteur 38 cm), sous une pression de 0,5 bar d'argon en éluant avec un mélange d'acétate d'éthyle et cyclohexane (30/70 en volumes puis 40/60 dès la fraction 16) et en recueillant des fractions de 60 cm³, les fractions 21 à 35 sont réunies puis concentrées à sec sous pression réduite (2,7 kPa). On obtient 0,40 g du mélange des deux diastéréoisomères 1-[(4-chlorophényl)(thiazol-2-yl)méthyl-(RS)]-3-[(3,5-difluorophényl)(méthylsulfonyl)méthyl-(RS)]azétidin-3-ol, sous la forme d'un solide blanchâtre.

### Exemple 87

A une solution de 0,32 g de 1-{(R*)-[4-(chlorométhyl)phényl]-(4-chlorophényl)méthyl}-3-[(3,5-difluorophényl)(méthylsulfonyl)méthylène]azétidine, isomère forme A et 5 mg d'iodure de sodium dans 10 cm³ de dichlorométhane, on ajoute 50 mm³ de pyrrolidine. Après 20 heures d'agitation à 20°C, on ajoute au mélange 50 mm³ de pyrrolidine, agite pendant 8 heures puis ajoute à nouveau 50 mm³ de pyrrolidine et agite pendant 20 heures à 20°C. On lave le mélange réactionnel par de l'eau puis sèche la phase organique sur sulfate de magnésium, concentre à sec sous vide (2,7 kPa). Le résidu obtenu est chromatographié sur une colonne de gel de silice (granulométrie 0,06-0,200 mm, diamètre 1,2 cm, hauteur 30 cm), sous une pression de 0,1 bar d'argon en éluant avec du dichlorométhane puis avec un mélange dichlorométhane et méthanol (97,5/2,5 en volumes) et en recueillant des fractions de 3 cm³. Les fractions 12 à 40 sont réunies puis concentrées à sec sous pression réduite (2,7 kPa). On obtient 0,18 g de 1-{(R*)-(4-chlorophényl)[4-(pyrrolidinylméthyl)phényl]méthyl}-3-[(3,5-difluorophényl)(méthylsulfonyl)méthylène]azétidine, isomère forme A, sous la forme d'une meringue blanche [α]²⁰365nm = -22,5° +/- 0,7 (c = 0,5 %; dichlorométhane) [Spectre de R.M.N. ¹H (300 MHz, CDCl₃, δ en ppm) : 1,78 (mt : 4H); 2,51 (mt : 4H); 2,81 (s : 3H); 3,58 (s : 2H); 3,84 (mt : 2H); 4,33 (mt : 2H); 4,50 (s : 1H); 6,84 (tt, J = 9 et 2,5 Hz : 1H); 6,98 (mt : 2H); de 7,20 à 7,40 (mt : 8H)].

Le 1-{(R*)-[(4-chlorométhyl)phényl](4-chlorophényl)méthyl}-3-[(3,5-difluorophényl)(méthylsulfonyl)méthylène]azétidine, isomère forme A, peut être préparé en opérant de la façon suivante : A une solution de 28,0 g du mélange des 2 diastéréoisomères (formes A) 1-{(R*)-[(4-chlorométhyl)phényl]-(4-chlorophényl)méthyl}-3-[(R)-(3,5-difluorophényl)(méthylsulfonyl)méthyl)]azétidin-3-ol, et 1-{(R*)-[(4-chlorométhyl)phényl]-(4-chlorophényl)méthyl}-3-[(S)-(3,5-difluorophényl)(méthylsulfonyl)méthyl)]azétidin-3-ol, de 32 g de 4-diméthylaminopyridine, dans 500 cm³ de dichlorométhane, on ajoute 12,4 cm³ de chlorure de méthanesulfonyle. Après une heure d'agitation à 10°C, puis une heure à 20°C, le mélange réactionnel est lavé par 500 cm³ d'eau, la phase organique séchée sur sulfate de magnésium et concentrée à sec sous pression réduite (2,7 kPa). Le résidu est chromatographié sur une colonne de gel de silice (granulométrie 0,06-0,200 mm, diamètre 6 cm, hauteur 30 cm), sous une pression de 0,2 bar d'argon en éluant avec du dichlorométhane et en recueillant des fractions de 250 cm³. Les fractions 9 à 25 sont réunies puis concentrées à sec sous pression réduite (2,7 kPa). On obtient 6,3 g de 1-{(R*)-[4-(chlorométhyl)phényl]-(4-chlorophényl)méthyl}-3-[(3,5-difluorophényl)(méthylsulfonyl)méthylène]azétidine, isomère forme A, sous la forme d'une meringue blanche.

Le mélange des 2 diastéréoisomères (formes A) 1-{(R*)-[4-(chlorométhyl)phényl](4-chlorophényl)méthyl}-3-[(R)-(3,5-difluorophényl)(méthylsulfonyl)méthyl)]azétidin-3-ol, et 1-{(R*)-[4-(chlorométhyl)phényl](4-chlorophényl)méthyl}-3-[(S)-(3,5-difluorophényl)(méthylsulfonyl)méthyl)]azétidin-3-ol, peut être préparé en opérant de la façon suivante : A une solution de 0,20 g du mélange des 2 diastéréoisomères (formes A) 1-{(R*)-(4-chlorophényl)[4-(hydroxyméthyl)phényl]méthyl}-3-[(R)-(3,5-difluorophényl)(méthylsulfonyl)méthyl)]azétidin-3-ol, et 1-{(R*)-(4-chlorophényl)[4-(hydroxyméthyl)phényl]méthyl}-3-[(S)-(3,5-difluorophényl)(méthylsulfonyl)méthyl)]azétidin-3-ol, dans 10 cm³ de dichlorométhane, on ajoute 60 mm³ de chlorure de thionyle. Après 20 heures d'agitation à 20°C, on ajoute au mélange réactionnel 5 cm³ d'une solution aqueuse saturée d'hydrogénocarbonate de sodium, puis agite pendant 15 minutes. Le mélange est décanté, la phase organique est lavée avec de l'eau, séchée sur sulfate de magnésium, puis concentrée à sec sous pression réduite (2,7 kPa). Le résidu est chromatographié sur une colonne de gel de silice (granulométrie 0,04-0,06 mm, diamètre 1,0 cm, hauteur 20 cm), sous une pression de 0,2 bar d'argon en éluant avec un mélange cyclohexane et acétate d'éthyle (75/25 en volumes) et en recueillant des fractions de 20 cm³. Les fractions 4 à 7 sont réunies puis concentrées à sec sous pression réduite (2,7 kPa). On obtient 0,17 g du mélange des 2 diastéréoisomères (formes A) 1-{(R*)-[4-(chlorométhyl)phényl]-(4-chlorophényl)méthyl}-3-[(R)-(3,5-difluorophényl)(méthylsulfonyl)méthyl)]azétidin-3-ol, et 1-{(R*)-[4-(chlorométhyl)phényl]-(4-chlorophényl)méthyl}-3-[(S)-(3,5-difluorophényl)(méthylsulfonyl)méthyl)]azétidin-3-ol, sous la forme d'une meringue blanche.

Le mélange des 2 diastéréoisomères (formes A) 1-{(R*)-(4-chlorophényl)[4-(hydroxyméthyl)phényl]méthyl}-3-[(R)-(3,5-difluorophényl)(méthylsulfonyl)méthyl)]azétidin-3-ol et 1-{(R*)-(4-chlorophényl)[4-(hydroxyméthyl)phényl]méthyl}-3-[(S)-(3,5-difluorophényl)(méthylsulfonyl)méthyl)]azétidin-3-ol, peut être préparé en opérant de la façon suivante : A une solution maintenue sous argon et refroidie à -30°C de 0,58 g du mélange des 2 diastéréoisomères (formes A) 3-acétoxy-1-{(R*)-(4-chlorophényl)[4-(méthoxycarbonyl)phényl]méthyl}-3-[(R)-(3,5-difluorophényl)(méthylsulfonyl)méthyl)]azétidine, et 3-acétoxy-1-{(R*)-(4-chlorophényl)[4-(méthoxycarbonyl)phényl]méthyl}-3-[(S)-(3,5-difluorophényl)(méthylsulfonyl) méthyl)]azétidine, dans 10 cm³ de toluène anhydre, on ajoute 1,6 cm³ d'une solution 1,5M dans le toluène d'hydrure de diisobutylaluminium. Après 15 minutes d'agitation à -30°C, on ajoute à nouveau 1,0 cm³ de cette même solution d'hydrure, puis laisse le mélange revenir à 0°C. Après 30 minutes d'agitation, le mélange agité est additionné de 3 cm³ d'eau et 6 cm³ d'hydroxyde de sodium 1N puis extrait par 25 cm³ de dichlorométhane. La phase organique est lavée par 5 cm³ d'eau, 5 cm³ de saumure, puis séchée sur sulfate de magnésium et concentrée à sec sous pression réduite (2,7 kPa). Le résidu est chromatographié sur une colonne de gel de silice (granulométrie 0,06-0,200 mm, diamètre 1,2 cm, hauteur 30 cm), sous une pression de 0,1 bar d'argon en éluant avec un mélange cyclohexane et acétate d'éthyle (50/50 en volumes) et en recueillant des fractions de 30 cm³. Les fractions 4 à 12 sont réunies puis concentrées à sec sous pression réduite (2,7 kPa). On obtient 0,42 g du mélange des 2 diastéréoisomères (formes A) 1-{(R*)-(4-chlorophényl)[4-(hydroxyméthyl)phényl]méthyl}-3-[(R)-(3,5-difluorophényl)(méthylsulfonyl)méthyl)]azétidin-3-ol, et 1-{(R*)-(4-chlorophényl)[4-(hydroxyméthyl)phényl]méthyl}-3-[(S)-(3,5-difluorophényl)(méthylsulfonyl)méthyl)]azétidin-3-ol, sous la forme d'une laque blanche.

Le mélange des 2 diastéréoisomères (formes A) 3-acétoxy-1-{(R*)-(4-chlorophényl)[4-(méthoxycarbonyl)phényl]méthyl}-3-[(R)-(3,5-difluorophényl)(méthylsulfonyl)méthyl)]azétidine, et 3-acétoxy-1-{(R*)-(4-chlorophényl)[4-(méthoxycarbonyl)phényl]méthyl}-3-[(S)-(3,5-difluorophényl) (méthylsulfonyl)méthyl)]azétidine, peut être préparé en opérant comme il est décrit dans l'exemple 40, à partir de 1,0 g de (3,5-difluorobenzyl)méthylsulfone, 30 cm³ de tétrahydrofuranne, 3 cm³ d'une solution 1,6N de n butyllithium dans l'hexane, de 1,45 g de 1-{(R*)-(4-chlorophényl)[4-(méthoxycarbonyl)phényl]méthyl}azétidin-3-one, isomère forme A, et 0,43 cm³ de chlorure d'acétyle. On obtient 1,28 g du mélange des 2 diastéréoisomères (formes A) 3-acétoxy-1-{(R*)-(4-chlorophényl)[4-(méthoxycarbonyl)phényl]méthyl}-3-[(R)-(3,5-difluorophényl)(méthylsulfonyl) méthyl)]azétidine, et 3-acétoxy-1-{(R*)-(4-chlorophényl)[4-(méthoxycarbonyl) phényl]méthyl}-3-[(S)-(3,5-difluorophényl)(méthylsulfonyl)méthyl)]azétidine, sous la forme d'une meringue beige.

Le 1-{(R*)-(4-chlorophényl)[4-(méthoxycarbonyl)phényl]méthyl}azétidin-3-one, isomère forme A, peut être préparé en opérant comme il est décrit dans l'exemple 40, à partir de 0,55 cm³ de chlorure d'oxalyle, de 25 cm³ de dichlorométhane, de 0,90 cm³ de diméthylsulfoxyde, de 1,75 g de 1-{(4-chlorophényl)[4-(méthoxycarbonyl)phényl]méthyl} azétidin-3-ol, et 2,70 cm³ de triéthylamine. On obtient 1,45 g de 1-{(R*)-(4-chlorophényl)[4-(méthoxycarbonyl)phényl]méthyl}azétidin-3-one, isomère forme A, sous la forme d'une meringue jaune.

Le 1-{(R*)-(4-chlorophényl)[4-(méthoxycarbonyl)phényl]méthyl}azétidin-3-ol, isomère forme A, peut être préparé en opérant selon le mode opératoire décrit par KATRITZKY A.R. et coll., dans J. Heterocycl. Chem., (1994), 271 à partir de 2,0 g de (+)-4-[(R*)-amino-(4-chlorophényl)méthyl]benzoate de méthyle, de 30 cm³ d'éthanol, 0,60 g d'hydrogénocarbonate de sodium, et 0,60 cm³ d'épibromhydrine. On obtient 1,76 g de 1-{(R*)-(4-chlorophényl)[4-(méthoxycarbonyl) phényl]méthyl}azétidin-3-ol, isomère forme A, sous la forme d'un solide pâteux.

Le (+)-4-[(R*)-amino-(4-chlorophényl)méthyl]benzoate de méthyle peut être préparé en opérant de la façon suivante : A une solution de 9,2 g de 4-[(RS)-amino-(4-chlorophényl)méthyl]benzoate de méthyle dans 10 cm³ de méthanol, on ajoute 2,51 g d'acide D-(-)-tartrique. La solution est concentrée à sec sous pression réduite (2,7 kPa). La meringue crème obtenue est dissoute dans 50 cm³ d'éthanol contenant 5% d'eau et la solution résultante est laissée cristalliser pendant 20 heures à 20°C. Les cristaux sont filtrés, lavés avec l'éthanol à 5% d'eau, essorés, puis séchés sous pression réduite (2,7 kPa). On obtient 3,4 g de cristaux blancs que l'on nomme « cristaux A » [et que l'on conserve pour la préparation ultérieure du deuxième énantiomère (-)-4-[(R*)-amino-(4-chlorophényl)méthyl]benzoate de méthyle)]. Les liqueurs mères sont concentrées à sec, et on obtient une meringue blanche (8,1 g) qui est dissoute dans 100 cm³ d'acétate d'éthyle. La solution obtenue est additionnée de 50 cm³ d'hydroxyde de sodium 1N, agitée, décantée. La phase organique est lavée avec 50 cm³ d'eau, puis séchée sur sulfate de magnésium, et concentrée à sec sous pression réduite (2,7 kPa). On obtient un solide jaune, que l'on dissout dans 100 cm³ de méthanol. La solution obtenue est additionnée de 1,85 g d'acide L-(+)-tartrique et la solution résultante est concentrée à sec sous pression réduite (2,7 kPa). On obtient une meringue crème qui, une fois dissoute dans 27 cm3 d'éthanol à 4% d'eau, est laissée cristalliser pendant 20 heures à 20°C. Les cristaux sont filtrés, lavés avec de l'éthanol à 4% d'eau, essorés, puis séchés sous pression réduite (2,7 kPa). On obtient 3,4 g de cristaux de L-(+)-tartrate de (+)-4-[(R*)-amino-(4-chlorophényl)méthyl]benzoate de méthyle, que l'on recristallise dans 60 cm³ d'éthanol à 5% d'eau. Après essorage, puis séchage, on obtient 2,78 g de cristaux blancs que l'on dissout dans 50 cm³ d'acétate d'éthyle. La solution obtenue est additionnée de 100 cm³ d'hydroxyde de sodium 1N, agitée, décantée. La phase organique est lavée avec 50 cm³ d'eau, puis séchée sur sulfate de magnésium, et concentrée à sec sous pression réduite (2,7 kPa). On obtient 2,1 g de (+)-4-[(R*)-amino-(4-chlorophényl)méthyl]benzoate de méthyle, sous la forme d'un solide blanc.

Le 4-[(RS)-amino-(4-chlorophényl)méthyl]benzoate de méthyle peut être préparé en opérant de la façon suivante : A une suspension de 16,3 g de 4-[(RS)-phtalimido-(4-chlorophényl)méthyl]benzoate de méthyle dans 200 cm³ de méthanol, on ajoute 3,9 cm³ d'hydrate d'hydrazine. Après 5 heures d'agitation à la température du reflux puis 20 heures à 20°C, le mélange réactionnel est filtré, le filtrat est concentré à sec sous pression réduite (2,7 kPa). Le résidu obtenu est repris par un mélange de 200 cm³ d'eau et 200 cm³ d'acétate d'éthyle. Après 15 minutes d'agitation, la suspension résultante est filtrée, le filtrat décanté en ampoule à décanter, et la phase organique est lavée par 50 cm³ d'eau, séchée sur sulfate de magnésium, et concentré à sec sous pression réduite (2,7 kPa). On obtient 8,4 g de 4-[(RS)-amino-(4-chlorophényl)méthyl]benzoate de méthyle, sous la forme d'une huile jaune pâle.

Le 4-[(RS)-phtalimido-(4-chlorophényl)méthyl]benzoate de méthyle, peut être préparé en opérant de la façon suivante : A une solution de 11,6 g de 4-[(RS)-bromo-(4-chlorophényl)méthyl]benzoate de méthyle, dans 70 cm³ de diméthylformamide, on ajoute 12,6 g de phtalimide de potassium. Après 3 heures d'agitation à la température du reflux, le mélange réactionnel est refroidi à 20°C puis additionné de 300 cm³ d'acétate d'éthyle et 300 cm³ d'eau. Après agitation, le mélange est décanté, la phase aqueuse réextraite par 2 fois 100 cm³ d'acétate d'éthyle, les phases organiques réunies sont lavées par 2 fois 400 cm³ d'eau, puis séchées sur sulfate de magnésium, et concentrées à sec sous pression réduite (2,7 kPa). On obtient 16,3 g de 4-[(RS)-phtalimido-(4-chlorophényl)méthyl]benzoate de méthyle, sous la forme d'un solide jaune pâteux.

Le 4-[(RS)-bromo-(4-chlorophényl)méthyl]benzoate de méthyle, peut être préparé en opérant de la façon suivante : A une solution de 17,4 g de 4-[(RS)-(4-chlorophényl)(hydroxy)méthyl]benzoate de méthyle dans 200 cm³ d'acétonitrile, on ajoute 10,18 g de NN'-carbonyldiimidazole, et 54,3 cm³ de bromure d'allyle. Après 30 minutes d'agitation à 20°C, le mélange réactionnel est porté au reflux pendant 2 heures, agité pendant 20 heures à 20°C et concentré presqu'à sec sous pression réduite (2,7 kPa). Le mélange, repris par du dichlorométhane, est chromatographié sur une colonne de gel de silice (granulométrie 0,06-0,200 mm, diamètre 7 cm, hauteur 30 cm), sous une pression de 0,5 bar d'argon en éluant avec du dichlorométhane, et en recueillant des fractions de 500 cm³. Les fractions 3 à 6 sont réunies puis concentrées à sec sous pression réduite (2,7 kPa). On obtient 11,6 g de 4-[(RS)-bromo-(4-chlorophényl)méthyl]benzoate de méthyle, sous la forme d'une huile qui sera utilisée telle quelle à l'étape suivante.

Le 4-[(RS)-(4-chlorophényl)(hydroxy)méthyl]benzoate de méthyle peut être préparé en opérant de la façon suivante : A une suspension de 2,75 g de 4-(4-chlorobenzoyl)benzoate de méthyle, dans 200 cm³ de méthanol à 20°C, on ajoute lentement par petites fractions (il se produit un échauffement du milieu jusqu'à 50°C), 1,21 g de borohydrure de sodium. Après 20 heures d'agitation à 20°C, le mélange réactionnel est concentré à volume réduit puis additionné de 150 cm³ de dichlorométhane et en agitant de 100 cm³ d'acide chlorhydrique 0,5N. Après décantation, la phase organique est séchée sur sulfate de magnésium, concentrée à sec sous pression réduite (2,7 kPa). On obtient 2,5 g de 4-[(RS)-(4-chlorophényl)(hydroxy)méthyl]benzoate de méthyle, sous la forme d'une huile incolore qui cristallise lentement à 20°C, et qui sera utilisée telle quelle à l'étape suivante.

Le 4-(4-chlorobenzoyl)benzoate de méthyle, peut être préparé en opérant de la façon suivante : A une solution refroidie à -22°C de 19,3 g de chlorure de l'acide téréphtalique monométhylester dans 200 cm³ de tetrahydrofurane, on ajoute sous argon 27,4 cm³ de tri n-butylphosphine. Après 20 minutes d'agitation à -22°C, on coule en maintenant cette température, une solution de bromure de 4-chlorophénylmagnésien (préparée à partir de 19,15 g de bromo-4-chlorobenzène, de 2,43 g de magnésium et un cristal d'iode dans 100 cm³ d'oxyde de diéthyle au reflux). Après 30 minutes d'agitation à -22°C, on ajoute lentement 150 cm³ d'acide chlorhydrique 1N, laisse le mélange revenir à 20°C puis dilue le milieu avec 200 cm³ d'oxyde de diéthyle. La suspension blanche obtenue est filtrée, le solide est lavé par 2 fois 50 cm³ d'eau, puis par 2 fois 50 cm³ de d'oxyde de diéthyle. On obtient après essorage puis séchage sous pression réduite (2,7 kPa), 16,2 g de 4-(4-chlorobenzoyl)benzoate de méthyle, sous la forme d'un solide blanc fondant à 170°C

### Exemple 88

On opère comme il est décrit dans l'exemple 87, à partir de 0,05 g de 1-{(R*)-[4-(chlorométhyl)phényl]-(4-chlorophényl)méthyl}-3-[(3,5-dilluorophényl)(méthylsulfonyl)méthylène]azétidine, isomère forme A, 1,0 cm³ de dichlorométhane, et 0,025 g de 3,3-diméthylpipéridine. Le produit brut est chromatographié sur une colonne de gel de silice (granulométrie 0,06-0,200 mm, diamètre 8 mm, hauteur 8 cm), en éluant avec 80 cm³ de dichlorométhane puis en éluant avec un mélange dichlorométhane et méthanol (95/5 en volumes), en recueillant des fractions de 2,5 cm³ dès l'utilisation de ce mélange éluant. Les fractions 3 à 8 sont réunies puis concentrées à sec sous pression réduite (2,7 kPa). On obtient 0,040g de 1-{(R*)-(4-chlorophényl)[4-(3,3-diméthyl-pipéridin-1-yl-méthyl)phényl]méthyl}-3-[(3,5-difluorophényl)(méthylsulfonyl)méthylène]azétidine, isomère forme A, sous la forme d'une meringue blanche [Spectre de R.M.N. ¹H (300 MHz, CDCl₃, δ en ppm) : 0.94 (s : 6H); 1,21 (mt : 2H); de 1,50 à 1,65 (mt : 2H); 1,99 (s large : 2H); 2,27 (mf : 2H); 2,81 (s : 3H); 3,36 (s : 2H); 3,85 (mt : 2H); 4,33 (mt : 2H); 4,49 (s : 1H); 6,84 (tt, J = 8,5 et 2,5 Hz : 1H); 6,98 (mt : 2H); de 7,20 à 7,40 (mt : 8H)].

### Exemple 89

On opère comme il est décrit dans l'exemple 87, à partir de 0,05 g de 1-{(R*)-[4-(chlorométhyl)phényl]-(4-chlorophényl)méthyl}-3-[(3,5-difluorophényl)(méthylsulfonyl)méthylène]azétidine, isomère forme A) méthylsulfonyl méthyl, 1,0 cm³ de dichlorométhane, et 0,025 g de thiomorpholine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 3 à 8 sont réunies puis concentrées à sec sous pression réduite (2,7 kPa). On obtient 0,038g de 1-{(R*)-(4-chlorophényl)[4-(thiomorpholin-4-yl-méthyl)phényl]méthyl}-3-[(3,5-difluorophényl)(méthylsulfonyl)méthylène]azétidine, isomère forme A, sous la forme d'une meringue blanche [Spectre de R.M.N. ¹H (300 MHz, CDCl₃, δ en ppm) : de 2,60 à 2,75 (mt : 8H); 2,80 (s : 3H); 3,44 (s : 2H); 3,84 (mt : 2H); 4,33 (mt : 2H); 4,50 (s : 1H); 6,83 (tt, J = 8,5 et 2,5 Hz : 1H); 6,97 (mt : 2H); de 7,20 à 7,40 (mt : 8H)].

### Exemple 90

On opère comme il est décrit dans l'exemple 87, à partir de 0,05 g de1-{(R*)-[4-(chlorométhyl)phényl]-(4-chlorophényl)méthyl}-3-[(3,5-difluorophényl)(méthylsulfonyl)méthylène]azétidine, isomère forme A, 1,0 cm³ de dichlorométhane, et 0,025 g de N-cyclohexyl-N-éthyl-amine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 5 à 8 sont réunies puis concentrées à sec sous pression réduite (2,7 kPa). On obtient 0,022g de 1-{(R*)-(4-chlorophényl)[4-(N-éthyl-N-cyclohexyl-aminométhyl)phényl]méthyl}}-3-[(3,5-difluorophényl)(méthylsulfonyl)méthylène]azétidine, isomère forme A, sous la forme d'une meringue blanche [Spectre de R.M.N. ¹H (300 MHz, CDCl₃ avec ajout de quelques gouttes de CD₃COOD d4, δ en ppm) : de 1,15 à 1,25 (mt : 2H); 1,29 (t, J = 7,5 Hz : 3H); de 1,45 à 1,65 (mt : 4H); 1,88 (mt : 2H); 2,17 (mt : 2H); 2,81 (s : 3H); 3,05 (q, J = 7,5 Hz : 2H); 3,27 (mt : 1H); 3,95 (mt : 2H); 4,18 (s : 2H); 4,40 (mt : 2H); 4,66 (s : 1H); 6,82 (tt, J = 8,5 et 2,5 Hz : 1H); 7,00 (mt : 2H); de 7,20 à 7,40 (mt : 4H); 7,41 (d, J = 8 Hz : 2H); 7,53 (d, J = 8 Hz : 2H)].

### Exemple 91

On opère comme il est décrit dans l'exemple 87, à partir de 0,05 g de 1-{(R*)-[4-(chlorométhyl)phényl]-(4-chlorophényl)méthyl}-3-[(3,5-difluorophényl)(méthylsulfonyl)méthylène]azétidine, isomère forme A, 1,0 cm³ de dichlorométhane, et 0,032 g de 4-(éthoxycarbonyl)pipérazine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 2 à 8 sont réunies puis concentrées à sec sous pression réduite (2,7 kPa). On obtient 0,021 g de 1-{{(R*)-(4-chlorophényl) {4-[(4-éthoxycarbonylpipérazinyl)méthyl]phényl }méthyl}}-3-[(3,5-difluorophényl)(méthylsulfonyl)méthylène]azétidine, isomère forme A, sous la forme d'une meringue blanche [Spectre de R.M.N. ¹H (400 MHz, CDCl₃, δ en ppm) : 1,25 (t, J = 7 Hz : 3H); 2,36 (mt : 4H); 2,80 (s : 3H); 3,44 (s : 2H); 3,46 (mt : 4H); 3,85 (mt : 2H); 4,13 (q, J = 7 Hz : 2H); 4,34 (mt : 2H); 4,50 (s : 1H); 6,83 (tt, J = 9 et 2,5 Hz : 1H); 6 ,98 (mt : 2H); de 7,20 à 7,40 (mt : 8H)].

### Exemple 92

On opère comme il est décrit dans l'exemple 87, à partir de 0,05 g de 1-{(R*)-[4-(chlorométhyl)phényl]-(4-chlorophényl)méthyl }-3-[(3,5-difluorophényl)(méthylsulfonyl)méthylène]azétidine, isomère forme A, 1,0 cm³ de dichlorométhane, et 0,023 g de N-cyclopropyl-N-propyl-amine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 3 à 9 sont réunies puis concentrées à sec sous pression réduite (2,7 kPa). On obtient 0,026 g de 1-{(R*)-(4-chlorophényl)[4-N-cyclopropyl-N-propyl-aminométhyl)phényl]méthyl}-3-[(3,5-difluorophényl)(méthylsulfonyl)méthylène]azétidine, isomère forme A, sous la forme d'une meringue blanche [Spectre de R.M.N. ¹H (400 MHz, CDCl₃ avec ajout de quelques gouttes de CD₃COOD d4, δ en ppm) : 0,34 (mt : 2H); 0,70 (mt : 2H); 0,91 (t, J = 7 Hz : 3H); 1,08 (mt : 1H); 1,76 (mt : 2H); 2,82 (s : 3H); 2,92 (d, J = 7 Hz : 2H); 3,00 (mt : 2H); 3,90 (mt : 2H); 4,25 (s : 2H); 4,37 (mt : 2H); 4,59 (s : 1H); 6,83 (tt, J = 9 et 2,5 Hz : 1H); 7,00 (mt : 2H); de 7,20 à 7,45 (mt : 8H)].

### Exemple 93

On opère comme il est décrit dans l'exemple 87, mais en agitant le mélange réactionnel pendant 6 jours à 20°C, à partir de 0,05 g de 1-{(R*)-[4-(chlorométhyl)phényl]-(4-chlorophényl)méthyl}-3-[(3,5-difluorophényl)(méthylsulfonyl)méthylène]azétidine, isomère forme A, 1,0 cm³ de dichlorométhane, 5 mg d'iodure de sodium et 0,020 g de diisopropylamine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 2 à 8 sont réunies puis concentrées à sec sous pression réduite (2,7 kPa). On obtient 0,028g de 1-{(R*)-(4-chlorophényl)[4-(diisopropylaminométhyl)phényl]méthyl}-3-[(3,5-difluorophényl)(méthylsulfonyl)méthylène]azétidine, isomère forme A, sous la forme d'une meringue blanche [Spectre de R.M.N. ¹H (300 MHz, CDCl₃, δ en ppm) : 1,00 (mf : 12H); 2,80 (s : 3H); de 2,90 à 3,10 (mf : 2H); 3,58 (mt : 2H); 3,84 (mt : 2H); 4,33 (mt : 2H); 4,48 (s : 1H); 6,82 (tt, J = 8,5 et 2,5 Hz : 1H); 6,97 (mt : 2H); de 7,20 à 7,40 (mt : 8H)].

### Exemple 94

On opère comme il est décrit dans l'exemple 87, à partir de 0,05 g de 1-{(R*)-[4-(chlorométhyl)phényl]-(4-chlorophényl)méthyl}-3-[(3,5-difluorophényl)(méthylsulfonyl)méthylène]azétidine, isomère forme A, 1,0 cm³ de dichlorométhane, et 0,027 g de bis-(2-méthoxyéthyl)amine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 3 à 10 sont réunies puis concentrées à sec sous pression réduite (2,7 kPa). On obtient 0,014 g de 1-{{(R*)-(4-chlorophényl){4-[bis-(2-méthoxyéthyl)aminométhyl]phényl}méthyl}-3-[(3,5-difluorophényl)(méthylsulfonyl)méthylène]azétidine, isomère forme A, sous la forme d'une meringue blanche [Spectre de R.M.N. ¹H (300 MHz, CDCl₃, δ en ppm) : 2,70 (t large, J = 5,5 Hz : 4H); 2,81 (s : 3H); 3,29 (s : 6H); 3,46 (t large, J = 5,5 Hz : 4H); 3,65 (s large : 2H); 3,85 (mt : 2H); 4,33 (mt : 2H); 4,49 (s : 1H); 6,84 (tt, J = 9 et 2,5 Hz : 1H); 6,98 (mt : 2H); de 7,20 à 7,40 (mt : 8H)].

### Exemple 95

On opère comme il est décrit dans l'exemple 87, à partir de 0,05 g de 1-{(R*)-[4-(chlorométhyl)phényl]-(4-chlorophényl)méthyl}-3-[(3,5-difluorophényl)(méthylsulfonyl)méthylène]azétidine, isomère forme A, 1,0 cm³ de dichlorométhane, et 0,020 g de di-n-propylamine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 3 à 8 sont réunies puis concentrées à sec sous pression réduite (2,7 kPa). On obtient 0,025 g de 1-{(R*)-(4-chlorophényl)[4-(di-n-propylaminométhyl)phényl]méthyl}-3-[(3,5-difluorophényl)(méthylsul-fonyl)méthylène]azétidine, isomère forme A, sous la forme d'une meringue blanche [Spectre de R.M.N. ¹H (400 MHz, CDCl₃, δ en ppm) : 0,85 (t, J = 7,5 Hz : 6H); 1,45 (mt : 4H); 2,34 (t, J = 7,5 Hz : 4H); 2,80 (s : 3H); 3,48 (s : 2H); 3,84 (mt : 2H); 4,33 (mt : 2H); 4,50 (s : 1H); 6,83 (tt, J = 9 et 2,5 Hz : 1H); 6,98 (mt : 2H); de 7,20 à 7,40 (mt : 8H)].

### Exemple 96

On opère comme il est décrit dans l'exemple 87, à partir de 0,05 g de 1-{(R*)-[4-(chlorométhyl)phényl]-(4-chlorophényl)méthyl}-3-[(3,5-difluorophényl)(méthylsulfonyl)méthylène]azétidine, isomère forme A, 1,0 cm³ de dichlorométhane, et 0,017 g de pipéridine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 5 à 10 sont réunies puis concentrées à sec sous pression réduite (2,7 kPa). On obtient 0,035 g de 1-{(R*)-(4-chlorophényl)[4-(pipéridin-1-yl-méthyl)phényl]méthyl}-3-[(3,5-difluorophényl)(méthylsulfonyl)méthylène]azétidine, isomère forme A, sous la forme d'une meringue blanche [Spectre de R.M.N. ¹H (300 MHz, CDCl₃, δ en ppm) : de 1,35 à 1,65 (mt : 6H); 2,35 (mf : 4H); 2,80 (s : 3H); 3,41 (s large : 2H); 3,84 (mt : 2H); 4,33 (mt : 2H); 4,50 (s : 1H); 6,84 (tt, J = 8,5 et 2,5 Hz : 1H); 6,98 (mt : 2H); de 7,20 à 7,40 (mt : 8H)].

### Exemple 97

On opère comme il est décrit dans l'exemple 87, à partir de 0,05 g de 1-{(R*)-[4-(chlorométhyl)phényl]-(4-chlorophényl)méthyl}-3-[(3,5-difluorophényl)(méthylsulfonyl)méthylène]azétidine, isomère forme A, 1,0 cm³ de dichlorométhane, et 0,020 g de N-méthylpipérazine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 3 à 9 sont réunies puis concentrées à sec sous pression réduite (2,7 kPa). On obtient 0,025 g de 1-{(R*)-(4-chlorophényl)[4-(4-méthyl-pipérazin-1-yl-méthyl)phényl]méthyl}-3-[(3,5-difluorophényl)(méthylsulfonyl)méthylène]azétidine, isomère forme A, sous la forme d'une meringue blanche [Spectre de R.M.N. ¹H (300 MHz, CDCl₃, δ en ppm) : 2,28 (s : 3H); de 2,30 à 2,60 (mf : 8H); 2,80 (s : 3H); 3,45 (s : 2H); 3,84 (mt : 2H); 4,33 (mt : 2H); 4,50 (s : 1H); 6,84 (tt, J = 8,5 et 2,5 Hz : 1 H); 6,98 (mt : 2H); de 7,20 à 7,40 (mt : 8H)].

### Exemple 98

On opère comme il est décrit dans l'exemple 87, à partir de 0,05 g de 1-{(R*)-[4-(chlorométhyl)phényl]-(4-chlorophényl)méthyl }-3-[(3,5-difluorophényl)(méthylsulfonyl)méthylène]azétidine, isomère forme A, 1,0 cm³ de dichlorométhane, et 0,018 g de morpholine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 3 à 8 sont réunies puis concentrées à sec sous pression réduite (2,7 kPa). On obtient 0,022 g de 1-{(R*)-(4-chlorophényl)[4-(morpholin-4-yl-méthyl)phényl]méthyl}-3-[(3,5-difluorophényl)(méthylsulfonyl)méthylène] azétidine, isomère forme A, sous la forme d'une meringue blanche [Spectre de R.M.N ¹H (300 MHz, CDCl₃, δ en ppm) : 2,41 (t, J = 5 Hz, : 4H); 2,80 (s : 3H); 3,43 (s : 2H); 3,69 (t, J = 5 Hz : 4H); 3,85 (mt : 2H); 4,33 (mt : 2H); 4,50 (s : 1H); 6,84 (tt, J = 8,5 et 2,5 Hz : 1H); 6,98 (mt : 2H); de 7,20 à 7,40 (mt : 8H)].

### Exemple 99

On opère comme il est décrit dans l'exemple 87, à partir de 0,05 g de 1-{(R*)-[4-(chlorométhyl)phényl]-(4-chlorophényl)méthyl}-3-[(3,5-difluorophényl)(méthylsulfonyl)méthylène]azétidine, isomère forme A, 1,0 cm³ de dichlorométhane, et 0,020 cm³ de D-prolinol. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 3 à 8 sont réunies puis concentrées à sec sous pression réduite (2,7 kPa). On obtient 0,025 g de 1-{(R*)-(4-chlorophényl)[4-((2R)-hydroxyméthylpyrrolidin-1-yl-méthyl)phényl]méthyl}-3-[(3,5-difluorophényl)(méthylsulfonyl)méthylène]azétidine, isomère forme A, sous la forme d'une meringue blanche [Spectre de R.M.N. ¹H (300 MHz, (CD₃)₂SO d6 avec ajout de quelques gouttes de CD₃COOD d4, δ en ppm) : de 1,60 à 2,15 (mt : 4H); de 2,90 à 3,05 (mt : 1H); 2,98 (s : 3H); 3,13 (mt : 1H); 3,38 (mt : 1H); de 3,50 à 3,60 (mt : 1H); 3,56 (d, J = 5 Hz : 2H); 3,90 (mt : 2H); 4,04 (d, J = 13,5 Hz : 2H); 4,21 (mt : 2H); 4,40 (d, J = 13,5 Hz : 2H); 4,78 (s : 1H); 7,14 (mt : 2H); 7,27 (tt, J = 9 et 2,5 Hz : 1H); de 7,30 à 7,55 (mt : 8H)].

### Exemple 100

On opère comme il est décrit dans l'exemple 87, à partir de 0,05 g de 1-{(R*)-[4-(chlorométhyl)phényl]-(4-chlorophényl)méthyl}-3-[(3,5-difluorophényl)(méthylsulfonyl)méthylène]azétidine, isomère forme A, 1,0 cm³ de dichlorométhane, et 0,015 g de diéthylamine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 4 à 9 sont réunies puis concentrées à sec sous pression réduite (2,7 kPa). On obtient 0,025 g de 1-{(R*)-(4-chlorophényl)[4-(diéthylaminométhyl)phényl]méthyl}-3-[(3,5-difluorophényl)(méthylsulfonyl)méthylène]azétidine, isomère forme A, sous la forme d'une meringue blanche [Spectre de R.M.N. ¹H (400 MHz, CDCl₃, δ en ppm) : 1,03 (t, J = 7 Hz : 6H); 2,50 (q, J = 7 Hz : 4H); 2,81 (s : 3H); 3,50 (s : 2H); 3,85 (mt : 2H); 4,34 (mt : 2H); 4,49 (s : 1H); 6,84 (tt, J = 9 et 2,5 Hz : 1H); 6,99 (mt : 2H); de 7,20 à 7,40 (mt : 8H)].

### Exemple 101

On opère comme il est décrit dans l'exemple 87, à partir de 0,05 g de 1-{(R*)-[4-(chlorométhyl)phényl]-(4-chlorophényl)méthyl}-3-[(3,5-difluorophényl)(méthyl-sulfonyl)méthylène]azétidine, isomère forme A, 1,0 cm³ de dichlorométhane, et 0,026 g de N-(hydroxyéthyl)pipérazine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 3 à 10 sont réunies puis concentrées à sec sous pression réduite (2,7 kPa). On obtient 0,032 g de 1-{{(R*)-(4-chlorophényl){4-[4-(hydroxyéthyl)-pipérazin-1-yl-méthyl]phényl}méthyl}}-3-[(3,5-difluorophényl)(méthylsulfonyl)méthylène]azétidine, isomère forme A, sous la forme d'une meringue blanche [Spectre de R.M.N. ¹H (300 MHz, CDCl₃, δ en ppm) : de 2,40 à 2,60 (mt : 8H); 2,54 (t, J = 5,5 Hz : 2H); 2,80 (s : 3H); 3,44 (s : 2H); 3,60 (t, J = 5,5 Hz : 2H); 3,84 (mt : 2H); 4,33 (mt : 2H); 4,50 (s : 1H); 6,84 (tt, J = 9 et 2,5 Hz : 1H); 6,98 (mt : 2H); de 7,20 à 7,40 (mt : 8H)].

### Exemple 102

On opère comme il est décrit dans l'exemple 87, mais en agitant le mélange réactionnel pendant 4 jours à 20°C, à partir de 0,05 g de 1-{(R*)-[4-(chlorométhyl)phényl]-(4-chlorophényl)méthyl}-3-[(3,5-difluorophényl)(méthylsulfonyl)méthylène] azétidine, isomère forme A, 1,0 cm³ de dichlorométhane, et 0,023 g de 2(RS),6(RS)-diméthylpipéridine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 2 à 9 sont réunies puis concentrées à sec sous pression réduite (2,7 kPa). On obtient 0,024 g de 1-{(R*)-(4-chlorophényl)[4-(2(RS),6(RS)-diméthylpipéridin-1-yl-méthyl)phényl]méthyl}-3-[(3,5-difluorophényl)(méthylsulfonyl)méthylène]azétidine, mélange d'isomères, forme A, sous la forme d'une meringue blanche [Spectre de R.M.N. ¹H (400 MHz, CDCl₃ avec ajout de quelques gouttes de CD₃COOD d4, à une température de 353 K, δ en ppm) : de 1,20 à 1,45 (mt : 2H); 1,60 (d, J = 7 Hz : 6H); de 1,80 à 2,10 (mt : 4H); 2,80 (s : 3H); 3,17 (mt : 2H); 3,90 (mt : 2H); 4,34 (d large, J = 16 Hz : 1H); 4,40 (mt : 2H); 4,43 (d large, J = 16 Hz : 1H); 4,62 (s : 1H); 6,82 (tt, J = 9 et 2,5 Hz : 1H); 6,98 (mt : 2H); de 7,20 à 7,50 (mt : 8H)].

### Exemple 103

On opère comme il est décrit dans l'exemple 87, mais en agitant le mélange réactionnel pendant 4 jours à 20°C, à partir de 0,05 g de 1-{(R*)-[4-(chlorométhyl)phényl]-(4-chlorophényl)méthyl}-3-[(3,5-difluorophényl)(méthylsulfonyl)méthylène] azétidine, isomère forme A, 1,0 cm³ de dichlorométhane, et 0,024 g de pipérazin-2-one. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 3 à 8 sont réunies puis concentrées à sec sous pression réduite (2,7 kPa). On obtient 0,022 g de 1-{(R*)-(4-chlorophényl)[4-(pipérazin-2-one-4-ylméthyl)phényl]méthyl}-3-[(3,5-difluorophényl)(méthylsulfonyl)méthylène] azétidine, isomère forme A, sous la forme d'une meringue blanche [Spectre de R.M.N. ¹H (400 MHz, CDCl₃, δ en ppm) : 2,62 (t, J = 5,5 Hz : 2H); 2,80 (s : 3H); 3,11 (s : 2H); 3,34 (mt : 2H); 3,51 (s : 2H); 3,85 (mt : 2H); 4,34 (mt : 2H); 4,51 (s : 1H); 5,76 (mf : 1H); 6,84 (t large, J_{HF} = 9 Hz : 1H); 6,98 (mt : 2H); de 7,20 à 7,40 (mt : 8H)].

### Exemple 104

On opère comme il est décrit dans l'exemple 87, à partir de 0,05 g de 1-{(R*)-[4-(chlorométhyl)phényl]-(4-chlorophényl)méthyl}-3-[(3,5-difluorophényl)(méthylsul-fonyl)méthylène]azétidine, isomère forme A, 1,0 cm³ de dichlorométhane, et 0,020 g de L-prolinol. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 3 à 8 sont réunies puis concentrées à sec sous pression réduite (2,7 kPa). On obtient 0,028 g de 1-{{(R*)-(4-chlorophényl){4-[(2S)-(hydroxyméthyl)pyrrolidin-1-yl-méthyl]phényl}méthyl}-3-[(3,5-difluorophényl) (méthylsulfonyl)méthylène]azétidine, isomère forme A, sous la forme d'une meringue blanche [Spectre de R.M.N. ¹H (300 MHz, CDCl₃, δ en ppm) : de 1,50 à 2,00 (mt : 4H); 2,24 (mt : 1H); 2,71 (mt : 1H); 2,80 (s : 3H); 2,93 (mt : 1H); 3,28 (d, J = 13,5 Hz : 1H); 3,45 (mt : 1H); 3,65 (d, J = 11 et 4 Hz : 1H); 3,84 (mt : 2H); 3,91 (d, J = 13,5 Hz : 1H); 4,33 (mt : 2H); 4,50 (s : 1H); 6,83 (tt, J = 8,5 et 2,5 Hz : 1H); 6,98 (mt : 2H); de 7,20 à 7,40 (mt : 8H)].

### Exemple 105

On opère comme il est décrit dans l'exemple 87, à partir de 0,05 g de 1-{(R*)-[4-(chlorométhyl)phényl]-(4-chlorophényl)méthyl }-3-[(3,5-difluorophényl)(méthylsulfonyl)méthylène]azétidine, isomère forme A, 1,0 cm³ de dichlorométhane, et 0,023 g de (2S)-(méthoxyméthyl)pyrrolidine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 2 à 6 sont réunies puis concentrées à sec sous pression réduite (2,7 kPa). On obtient 0,037 g de 1-{{(R*)-(4-chlorophényl) {4-[(2S)-(méthoxyméthyl)pyrrolidin-1-yl-méthyl]phényl}méthyl}}-3-[(3,5-difluorophényl)(méthylsulfonyl)méthylène]azétidine, isomère forme A, sous la forme d'une meringue blanche [Spectre de R.M.N. ¹H (300 MHz, CDCl₃, δ en ppm) : 1,66 (mt : 2H); 1,90 (mt : 1H); 2,16 (mt : 1H); 2,68 (mt : 1H); 2,80 (s : 3H); 2,89 (mt : 1H); de 3,25 à 3,45 (mt : 4H); 3,31 (s : 3H); 3,84 (mt : 2H); 4,04 (d, J = 13,5 Hz : 1H); 4,33 (mt : 2H); 4,50 (s : 1H); 6,84 (tt, J = 8,5 et 2,5 Hz : 1H); 6,98 (mt : 2H); de 7,20 à 7,40 (mt : 8H)].

### Exemple 106

On opère comme il est décrit dans l'exemple 87, à partir de 0,05 g de 1-{(R*)-[4-(chlorométhyl)phényl]-(4-chlorophényl)méthyl }-3-[(3,5-difluorophényl)(méthylsulfonyl)méthylène]azétidine, isomère forme A, 1,0 cm³ de dichlorométhane, et 0,020 g de 2(RS),5(RS)-diméthylpyrrolidine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 3 à 6 sont réunies puis concentrées à sec sous pression réduite (2,7 kPa). On obtient 0,024 g de 1-{(R*)-(4-chlorophényl)[4-(2(RS),5(RS)-diméthylpyrrolidin-1-yl-méthyl)phényl]méthyl}-3-[(3,5-difluorophényl)(méthylsulfonyl)méthylène]azétidine, mélange d'isomères, forme A, sous la forme d'une meringue blanche [Spectre de R.M.N. ¹H (400 MHz, CDCl₃ avec ajout de quelques gouttes de CD₃COOD d4, δ en ppm) : 1,68 (d, J = 7 Hz : 6H); de 2,00 à 2,15 (mt : 4H); 2,82 (s : 3H); 3,22 (mt : 2H); 3,92 (mt : 2H); 4,30 (s : 2H); 4,33 (mt : 1H); 4,45 (d, J = 16,5 et 3 Hz : 1H); 4,63 (s : 1H); 6,84 (tt, J = 8,5 et 2,5 Hz : 1H); 7,00 (mt : 2H); de 7,20 à 7,55 (mt : 8H)].

### Exemple 107

On opère comme il est décrit dans l'exemple 87, à partir de 0,05 g de 1-{(R*)-[(4-chlorométhyl)phényl]-4-(chlorophényl)méthyl}-3-[(3,5-difluorophényl)(méthylsulfonyl)méthylène]azétidine, isomère forme A, 1,0 cm³ de dichlorométhane, et 0,023 g de L-prolinamide. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 2 à 5 sont réunies puis concentrées à sec sous pression réduite (2,7 kPa). On obtient 0,028 g de 1-{(R*)-(4-chlorophényl)[4-((2S)-carbamoylpyrrolidin-1-yl-méthyl)phényl]méthyl}-3-[(3,5-difluorophényl)(méthylsulfonyl)méthylène]azétidine, isomère forme A, sous la forme d'une meringue blanche [Spectre de R.M.N. ¹H (400 MHz, CDCl₃, δ en ppm) : de 1,65 à 1,85 (mt : 2H); 1,92 (mt : 1H); de 2,15 à 2,35 (mt : 2H); 2,80 (s : 3H); 3,00 (mt : 1H); 3,16 (dd, J = 10 et 5,5 Hz : 1H); 3,41 (d, J = 13,5 Hz : 1H); 3,86 (mt : 2H); 3,89 (d, J = 13,5 Hz : 1H); 4,33 (mt : 2H); 4,51 (s : 1H); 5,23 (mf : 1H); 6,84 (tt, J = 9 et 2,5 Hz : 1H); 6,98 (mt : 2H); 7,17 (mf : 1H); de 7,20 à 7,40 (mt : 8H)].

### Exemple 108

On opère comme il est décrit dans l'exemple 87, mais en agitant le mélange réactionnel pendant 4 jours à 20°C, à partir de 0,05 g de 1-{(R*)-[4-(chlorométhyl)phényl]-(4-chlorophényl)méthyl}-3-[(3,5-difluorophényl)(méthylsulfonyl)méthylène] azétidine, isomère forme A, 1,0 cm³ de dichlorométhane, et 0,021 g de diéthanolamine. Le produit brut est chromatographié sur une colonne de gel de silice (granulométrie 0,06-0,200 mm, diamètre 8 mm, hauteur 8 cm), en éluant avec 80 cm³ de dichlorométhane, puis avec un mélange dichlorométhane et méthanol (95/5 en volumes), en recueillant des fractions de 2,5 cm³ dès l'utilisation de ce mélange éluant. Les fractions 2 à 9 sont réunies puis concentrées à sec sous pression réduite (2,7 kPa). On obtient 0,004 g de 1-{(R*)-(4-chlorophényl)[4-(dihydroxyéthylaminométhyl)phényl]méthyl}-3-[(3,5-difluorophényl)(méthylsulfonyl)méthylène] azétidine, isomère forme A, sous la forme d'une meringue blanche [Spectre de R.M.N. ¹H (300 MHz, CDCl₃, δ en ppm) : 2,69 (t, J = 5,5 Hz : 4H); 2,80 (s : 3H); 3,61 (t, J = 5,5 Hz : 4H); 3,65 (s : 2H); 3,84 (mt : 2H); 4,33 (mt : 2H); 4,50 (s : 1H); 6,83 (tt, J = 9 et 2,5 Hz : 1H); 6,98 (mt : 2H); de 7,20 à 7,40 (mt : 8H)].

### Exemple 109

A une solution de 0,24 g de 1-{(R*)-[4-(chlorométhyl)phényl]-(4-chlorophényl)méthyl}-3-[(3,5-difluorophényl)(méthylsulfonyl)méthylène]azétidine, isomère forme A, dans 5 cm³ de dichlorométhane, on ajoute 0,055 g d'imidazole. Après 3 heures de reflux, le mélange est additionné de 5 mg d'iodure de sodium. Après 20 heures d'agitation au reflux, le mélange réactionnel est refroidi à 20°C, puis chromatographié sur une colonne de gel de silice (granulométrie 0,06-0,200 mm, diamètre 1,0 cm, hauteur 20 cm), en éluant avec 120 cm³ de dichlorométhane sans fractionner, puis avec un mélange dichlorométhane et méthanol (98/2 puis 96/4 en volumes), en recueillant des fractions de 4 cm³. Les fractions 12 à 14 sont réunies puis concentrées à sec sous pression réduite (2,7 kPa). On obtient 0,039 g de 1-{(R*)-(4-chlorophényl)[4-(imidazol-1-yl-méthyl)phényl]méthyl}-3-[(3,5-difluorophényl)(méthylsulfonyl)méthylène]azétidine, isomère forme A, sous la forme d'une meringue blanche.

### Exemple 110

On opère comme il est décrit dans l'exemple 87, à partir de 0,50 g de 1-{(R*)-[4-(chlorométhyl)phényl]-(4-chlorophényl)méthyl }-3-[(3,5-difluorophényl)(méthylsulfonyl)méthylène]azétidine, isomère forme B, 5 mg d'iodure de sodium, 15 cm³ de dichlorométhane, et 0,190 g de pyrrolidine. Le produit brut est chromatographié sur une colonne de gel de silice (granulométrie 0,06-0,200 mm, diamètre 1,5 cm, hauteur 20 cm), sous une pression de 0,1 bar d'argon en éluant avec du dichlorométhane puis avec un mélange dichlorométhane et méthanol (95/5 en volumes) et en recueillant des fractions de 25 cm³. Les fractions 20 à 40 sont réunies puis concentrées à sec sous pression réduite (2,7 kPa). On obtient 0,28 g de 1-{(R*)-(4-chlorophényl)[4-(pyrrolidin-1-yl-méthyl)phényl]méthyl}-3-[(3,5-difluorophényl)(méthylsulfonyl) méthylène]azétidine, isomère forme B, sous la forme d'une meringue blanche. [α]²⁰365nm = +26,8° +/- 0,8 (c = 0,5 %; dichlorométhane) [Spectre de R.M.N. ¹H (300 MHz, CDCl₃, δ en ppm) : 1,78 (mt : 4H); 2,50 (mt : 4H); 2,80 (s : 3H); 3,57 (s : 2H); 3,84 (mt : 2H); 4,34 (mt : 2H); 4,50 (s : 1H); 6,84 (tt, J = 9 et 2,5 Hz : 1H); 6,98 (mt : 2H); de 7,20 à 7,40 (mt : 8H)].

Le 1-{(R*)-[4-(chlorométhyl)phényl]-(4-chlorophényl)méthyl}-3-[(3,5-difluorophényl)(méthylsulfonyl)méthylène]azétidine, isomère forme B, peut être préparé en opérant comme il est décrit dans l'exemple 87, à partir de 7,3 g du mélange des 2 diastéréoisomères (formes B) 1-{(R*)-[4-(chlorométhyl)phényl]-(4-chlorophényl)méthyl}-3-[(R)-(3,5-difluorophényl)(méthylsulfonyl)méthyl)]azétidin-3-ol et 1-{(R*)-[4-(chlorométhyl)phényl]-(4-chlorophényl)méthyl}-3-[(S)-(3,5-difluorophényl)(méthylsulfonyl)méthyl)]azétidin-3-ol, de 8,2 g de 4-diméthylaminopyridine, de 150 cm³ de dichlorométhane, et 3,2 cm³ de chlorure de méthanesulfonyle. Le produit brut est chromatographié sur une colonne de gel de silice (granulométrie 0,04-0,06 mm, diamètre 3 cm, hauteur 30 cm), sous une pression de 0,2 bar d'argon en éluant avec du dichlorométhane et en recueillant des fractions de 100 cm³. Les fractions 15 à 30 sont réunies puis concentrées à sec sous pression réduite (2,7 kPa). On obtient 2,50 g de 1-{(R*)-[4-(chlorométhyl)phényl]-(4-chlorophényl)méthyl}-3-[(3,5-difluorophényl)(méthylsulfonyl)méthylène]azétidine, isomère forme B, sous la forme d'une meringue blanche.

Le mélange des 2 diastéréoisomères 1-{(R*)-[4-(chlorométhyl)phényl]-(4-chloro phényl)méthyl}-3-[(R)-(3,5-difluorophényl)(méthylsulfonyl)méthyl)]azétidin-3-ol, et 1- {(R*)-[4-(chlorométhyl)phényl]-(4-chlorophényl)méthyl}-3-[(S)-(3,5-difluorophényl)(méthylsulfonyl)méthyl)]azétidin-3-ol, peut être préparé en opérant comme il est décrit dans l'exemple 87, à partir de 11,0 g du mélange des 2 diastéréoisomères 1-{(R*)-(4-chlorophényl)[4-(hydroxyméthyl)phényl]méthyl}-3-[(R)-(3,5-difluorophényl)(méthylsulfonyl)méthyl)]azétidin-3-ol et 1-{(R*)-(4-chlorophényl)[4-(hydroxyméthyl)phényl]méthyl}-3-[(S)-(3,5-difluorophényl)(méthylsulfonyl) méthyl)]azétidin-3-ol, de 250 cm³ de dichlorométhane, et 3,1 cm³ de chlorure de thionyle. Le produit brut est chromatographié sur une colonne de gel de silice (granulométrie 0,04-0,06 mm, diamètre 3 cm, hauteur 30 cm), sous une pression de 0,2 bar d'argon en éluant avec un mélange cyclohexane et acétate d'éthyle (70/30 en volumes) et en recueillant des fractions de 50 cm³. Les fractions 9 à 25 sont réunies puis concentrées à sec sous pression réduite (2,7 kPa). On obtient 7,3 g du mélange des 2 diastéréoisomères (formes B) 1-{(R*)-[4-(chlorométhyl)phényl]-(4-chlorophényl)méthyl }-3-[(R)-(3,5-difluorophényl)(méthylsulfonyl)méthyl)]azétidin-3-ol, et 1-{(R*)-[4-(chlorométhyl)phényl]-(4-chlorophényl)méthyl}-3-[(S)-(3,5-difluorophényl)(méthylsulfonyl)méthyl)]azétidin-3-ol, sous la forme d'une meringue blanche.

Le mélange des 2 diastéréoisomères (formes B) 1-{(R*)-(4-chlorophényl)[4-(hydroxyméthyl)phényl]méthyl}-3-[(R)-(3,5-difluorophényl)(méthylsulfonyl)méthyl)]azétidin-3-ol et 1-{(R*)-(4-chlorophényl)[4-(hydroxyméthyl)phényl]méthyl}-3-[(R)-(3,5-difluorophényl)(méthylsulfonyl)méthyl)]azétidin-3-ol, peut être préparé en opérant comme il est décrit dans l'exemple 87, à partir de 18,0 g du mélange des 2 diastéréoisomères formes B) 3-acétoxy-1-{(R*)-(4-chlorophényl)[4-(méthoxycarbonyl)phényl]méthyl}-3-[(R)-(3,5-difluorophényl)(méthylsulfonyl)méthyl]azétidine, et 3-acétoxy-1-{(R*)-(4-chlorophényl)[4-(méthyloxycarbonyl)phényl]méthyl}-3-[(S)-(3,5-difluorophényl)(méthylsulfonyl)méthyl]azétidine, 150 cm³ de toluène anhydre, et 100 cm³ d'une solution à 20% dans le toluène d'hydrure de diisobutylaluminium. Le produit brut est chromatographié sur une colonne de gel de silice (granulométrie 0,06-0,200 mm, diamètre 3 cm, hauteur 30 cm), sous une pression de 0,1 bar d'argon en éluant avec un mélange cyclohexane et acétate d'éthyle (50/50 en volumes) et en recueillant des fractions de 50 cm³. Les fractions 15 à 30 sont réunies puis concentrées à sec sous pression réduite (2,7 kPa). On obtient 11,0 g du mélange des 2 diastéréoisomères (formes B) 1-{(R*)-(4-chlorophényl)[4-(hydroxyméthyl)phényl]méthyl}-3-[(R)-(3,5-difluorophényl)(méthylsulfonyl)méthyl] azétidin-3-ol, et 1-{(R*)-(4-chlorophényl)[4-(hydroxy-méthyl)phényl]méthyl}-3-[(S)-(3,5-difluorophényl)(méthylsulfonyl)méthyl]azétidin-3-ol, sous la forme d'une meringue blanche.

Le mélange des 2 diastéréoisomères (formes B) 3-acétoxy-1-{(R*)-(4-chlorophényl)[4-(méthoxycarbonyl)phényl]méthyl}-3-[(R)-(3,5-difluorophényl) (méthylsulfonyl)méthyl]azétidine, et 3-acétoxy-1-{(R*)-(4-chlorophényl)[4-(méthoxycarbonyl)phényl]méthyl}-3-[(R)-(3,5-difluorophényl)(méthylsulfonyl) méthyl]azétidine, peut être préparé en opérant comme il est décrit dans l'exemple 40, à partir de 11,2 g de (3,5-difluorobenzyl)méthylsulfone, 350 cm³ de tétrahydrofuranne, 34 cm³ d'une solution 1,6N de n butyllithium dans l'hexane, de 11,2 g de 1-{(R*)-(4-chlorophényl)[4-(méthoxycarbonyl)phényl]méthyl} azétidin-3-one, isomère forme B, et 5,5 cm³ de chlorure d'acétyle. Le produit brut est chromatographié sur une colonne de gel de silice (granulométrie 0,06-0,200 mm, diamètre 4 cm, hauteur 40 cm), en éluant avec un mélange cyclohexane et acétate d'éthyle (70/30 en volumes) et en recueillant des fractions de 100 cm³. Les fractions 10 à 30 sont réunies puis concentrées à sec sous pression réduite (2,7 kPa). On obtient 21 g d'une meringue crème encore impure que l'on chromatographie sur une colonne de gel de silice (granulométrie 0,06-0,200 mm, diamètre 4 cm, hauteur 40 cm), en éluant avec du dichlorométhane et en recueillant des fractions de 100 cm³. Les fractions 11 à 30 sont réunies puis concentrées à sec sous pression réduite (2,7 kPa). On obtient 20,0 g du mélange des 2 diastéréoisomères (formes B) 3-acétoxy-1-{(R*)-(4-chlorophényl)[4-(méthoxycarbonyl)phényl]méthyl}-3-[(R)-(3,5-difluorophényl)(méthylsulfonyl)méthyl)]azétidine, et 3-acétoxy- {(R*)-(4-chlorophényl)[4-(méthoxycarbonyl)phényl]méthyl}-3-[(S)-(3,5-difluorophényl) (méthylsulfonyl)méthyl]azétidine, sous la forme d'une meringue blanche.

Le 1-{(R*)-(4-chlorophényl)[4-(méthoxycarbonyl)phényl]méthyl}azétidin-3-one, isomère forme B, peut être préparé en opérant comme il est décrit dans l'exemple 40, à partir de 8,7 cm³ de chlorure d'oxalyle, de 350 cm³ de dichlorométhane, de 14,2 cm³ de diméthylsulfoxyde, de 29,0 g de 1-{(R*)-(4-chlorophényl)[4-(méthoxycarbonyl)phényl]méthyl}azétidin-3-ol, isomère forme B, et 43 cm³ de triéthylamine. Le produit brut est chromatographié sur une colonne de gel de silice (granulométrie 0,06-0,200 mm, diamètre 4 cm, hauteur 40 cm), en éluant avec du dichlorométhane et en recueillant des fractions de 250 cm³. Les fractions 7 à 25 sont réunies puis concentrées à sec sous pression réduite (2,7 kPa). On obtient 15,5 g de 1-{(R*)-(4-chlorophényl)[4-(méthoxycarbonyl)phényl]méthyl}azétidin-3-one, isomère forme B, sous la forme d'une huile orange.

Le 1-{(R*)-(4-chlorophényl)[4-(méthoxycarbonyl)phényl]méthyl}azétidin-3-ol, isomère forme B, peut être préparé selon le mode opératoire décrit par KATRITZKY A.R. et coll., dans J. Heterocycl. Chem., (1994), 271, à partir de 25,5g de (-)-4-[1-(R*)-amino-1-(4-chlorophényl)méthyl]benzoate de méthyle, de 250 cm³ d'éthanol, 7,9 g d'hydrogénocarbonate de sodium, et 7,7 cm³ d'épibromhydrine. On obtient 29 g de 1-{(R*)-(4-chlorophényl)[4-(méthoxycarbonyl)phényl]méthyl}azétidin-3-ol, isomère forme B, sous la forme d'une huile jaune.

Le (-)-4-[(R*)-amino-(4-chlorophényl)méthyl]benzoate de méthyle, peut être préparé en effectuant deux recristallisations successives des cristaux blancs (3,4 g) nommés « cristaux A » de l'exemple 87, dans 68 cm³ d'éthanol à 5% d'eau au reflux. Les cristaux obtenus sont filtrés, essorés puis séchés sous pression réduite (2,7 kPa). On obtient 2,2 g de D-(-)-tartrate de (-)-4-[(R*)-amino-(4-chlorophényl)méthyl]benzoate de méthyle, sous la forme de cristaux blancs que l'on dissout dans 50 cm³ d'acétate d'éthyle. La solution obtenue est additionnée de 50 cm³ d'hydroxyde de sodium 1N, agitée, puis décantée. La phase organique est lavée avec 50 cm³ d'eau, puis séchée sur sulfate de magnésium, et concentrée à sec sous pression réduite (2,7 kPa). On obtient 1,9 g de (-)-4-[(R*)-amino-(4-chlorophényl)méthyl]benzoate de méthyle, sous la forme d'un solide blanc. [α]20°C, 365 nm = -58,1° +/- 1 (c = 0,5%)

### Exemple 111

On opère comme il est décrit dans l'exemple 110, mais en agitant le mélange réactionnel pendant 48 heures à 20°C, à partir de 0,05 g de 1-{(R*)-[4-(chlorométhyl)phényl]-(4-chlorophényl)méthyl }-3-[(3,5-difluorophényl)(méthylsulfonyl)méthylène]azétidine, isomère forme B, 1,0 cm³ de dichlorométhane, et 0,030 cm³ de N-méthylpipérazine. Le produit brut est chromatographié sur une colonne de gel de silice (granulométrie 0,06-0,200 mm, diamètre 8 mm, hauteur 5 cm), en éluant avec 50 cm³ de dichlorométhane, puis avec un mélange dichlorométhane et méthanol (95/5 en volumes), en recueillant des fractions de 3 cm³ dès l'utilisation de ce mélange éluant. Les fractions 4 à 10 sont réunies puis concentrées à sec sous pression réduite (2,7 kPa). On obtient 0,025 g de 1-{(R*)-(4-chlorophényl)[4-(4-méthyl-pipérazin-1-yl-méthyl)phényl]méthyl}-3-[(3,5-difluorophényl)(méthylsulfonyl)méthylène]azétidine, isomère forme B, sous la forme d'une meringue crème [Spectre de R.M.N. ¹H (300 MHz, CDCl₃, δ en ppm) : 2,28 (s : 3H); 2,44 (mf : 8H); 2,80 (s : 3H); 3,45 (s : 2H); 3,85 (mt : 2H); 4,34 (mt : 2H); 4,50 (s : 1H); 6,84 (tt, J = 9 et 2,5 Hz : 1H); 6,99 (mt : 2H); de 7,20 à 7,40 (mt : 8H)].

### Exemple 112

On opère comme il est décrit dans l'exemple 110, mais en agitant le mélange réactionnel pendant 48 heures à 20°C, à partir de 0,05 g de 1-{(R*)-[4-(chlorométhyl)phényl]-(4-chlorophényl)méthyl}-3-[(3,5-difluorophényl)(méthylsulfonyl)méthylène]azétidine, isomère forme B, 1,0 cm³ de dichlorométhane, et 0,030 cm³ de L-Prolinol. Le produit brut est chromatographié sur une colonne de gel de silice (granulométrie 0,06-0,200 mm, diamètre 8 mm, hauteur 5 cm), en éluant avec 50 cm³ de dichlorométhane, puis avec un mélange dichlorométhane et méthanol (95/5 en volumes), en recueillant des fractions de 3 cm³ dès l'utilisation de ce mélange éluant. Les fractions 2 à 8 sont réunies puis concentrées à sec sous pression réduite (2,7 kPa). On obtient 0,025 g de 1-{{(R*)-(4-chlorophényl){4-[(2S)-(hydroxyméthyl)pyrrolidin-1-yl-méthyl]phényl}méthyl}}-3-[(3,5-difluorophényl)(méthylsulfonyl)méthylène]azétidine, isomère forme B, sous la forme d'une meringue crème [Spectre de R.M.N. ¹H (300 MHz, CDCl₃, δ en ppm) : de 1,80 à 2,00 (mt : 4H); 2,24 (mt : 1H); 2,72 (mt : 1H); 2,80 (s : 3H); 2,94 (mt : 1H); 3,28 (d, J = 13,5 Hz : 1H); 3,45 (mt : 1H); 3,65 (d, J = 10,5 et 3,5 Hz : 1H); 3,85 (mt : 2H); 3,92 (d, J = 13,5 Hz : 1H); 4,34 (mt : 2H); 4,50 (s : 1H); 6,84 (tt, J = 8,5 et 2,5 Hz : 1H); 6,98 (mt : 2H); de 7,15 à 7,40 (mt : 8H)].

### Exemple 113

On opère comme il est décrit dans l'exemple 110, mais en agitant le mélange réactionnel pendant 48 heures à 20°C, à partir de 0,05 g de 1-{(R*)-[4-(chlorométhyl)phényl]-(4-chlorophényl)méthyl}-3-[(3,5-difluorophényl)(méthylsulfonyl)méthylène]azétidine, isomère forme B, 1,0 cm³ de dichlorométhane, et 0,030 cm³ de D-Prolinol. Le produit brut est chromatographié sur une colonne de gel de silice (granulométrie 0,06-0,200 mm, diamètre 8 mm, hauteur 5 cm), en éluant avec 50 cm³ de dichlorométhane, puis avec un mélange dichlorométhane et méthanol (95/5 en volumes), en recueillant des fractions de 3 cm³ dès l'utilisation de ce mélange éluant. Les fractions 2 à 9 sont réunies puis concentrées à sec sous pression réduite (2,7 kPa). On obtient 0,029 g de 1-{{(R*)-(4-chlorophényl){4-[(2R)-(hydroxyméthyl)pyrrolidin-1-yl-méthyl]phényl}méthyl}}-3-[(3,5-difluorophényl)(méthylsulfonyl)méthylène]azétidine, isomère forme B, sous la forme d'une meringue crème [Spectre de R.M.N. ¹H (300 MHz, CDCl₃, δ en ppm) : de 1,50 à 2,00 (mt : 4H); 2,24 (mt : 1H); 2,71 (mt : 1H); 2,81 (s : 3H); 2,93 (mt : 1H); 3,28 (d, J = 13,5 Hz : 1H); 3,44 (t dédoublé, J = 10,5 et 2,5 Hz : 1H); 3,66 (dd, J = 10,5 et 3,5 Hz : 1H); 3,85 (mt : 2H); 3,92 (d, J = 13,5 Hz : 1H); 4,33 (mt : 2H); 4,50 (s : 1H); 6,84 (tt, J = 9 et 2,5 Hz : 1H); 6,98 (mt : 2H); de 7,15 à 7,40 (mt : 8H)].

### Exemple 114

On opère comme il est décrit dans l'exemple 110, mais en agitant le mélange réactionnel pendant 48 heures à 20°C, à partir de 0,05 g de 1-{(R*)-[4-(chlorométhyl)phényl]-(4-chlorophényl)méthyl}-3-[(3,5-difluorophényl)(méthylsulfonyl)méthylène]azétidine, isomère forme B, 1,0 cm³ de dichlorométhane, et 0,030 cm³ de morpholine. Le produit brut est chromatographié sur une colonne de gel de silice (granulométrie 0,06-0,200 mm, diamètre 8 mm, hauteur 5 cm), en éluant avec 50 cm³ de dichlorométhane, puis avec un mélange dichlorométhane et méthanol (95/5 en volumes), en recueillant des fractions de 3 cm³ dès l'utilisation de ce mélange éluant. Les fractions 2 à 9 sont réunies puis concentrées à sec sous pression réduite (2,7 kPa). On obtient 0,047 g de 1-{(R*)-(4-chlorophényl)[4-(morpholin-1-yl-méthyl)phényl]méthyl}-3-[(3,5-difluorophényl)(méthylsulfonyl)méthylène]azétidine, isomère forme B, sous la forme d'une meringue blanche [Spectre de R.M.N. ¹H (300 MHz, CDCl₃, δ en ppm) : 2,40 (mt : 4H); 2,81 (s : 3H); 3,43 (s : 2H); 3,69 (mt : 4H); 3,84 (mt : 2H); 4,34 (mt : 2H); 4,50 (s : 1H); 6,84 (tt, J = 8,5 et 2,5 Hz : 1H); 6,99 (mt : 2H); de 7,20 à 7,40 (mt : 8H)].

### Exemple 115

On opère comme il est décrit dans l'exemple 110, mais en agitant le mélange réactionnel pendant 48 heures à 20°C, à partir de 0,05 g de 1-{(R*)-[4-(chlorométhyl)phényl]-(4-chlorophényl)méthyl}-3-[(3,5-difluorophényl)(méthylsulfonyl)méthylène]azétidine, isomère forme B, 1,0 cm³ de dichlorométhane, et 0,030 cm³ de thiomorpholine. Le produit brut est chromatographié sur une colonne de gel de silice (granulométrie 0,06-0,200 mm, diamètre 8 mm, hauteur 5 cm), en éluant avec 50 cm³ de dichlorométhane, puis avec un mélange dichlorométhane et méthanol (95/5 en volumes), en recueillant des fractions de 3 cm³ dès l'utilisation de ce mélange éluant. Les fractions 2 à 9 sont réunies puis concentrées à sec sous pression réduite (2,7 kPa). On obtient 0,047 g de 1-{(R*)-(4-chlorophényl)[4-(thiomorpholin-4-yl-méthyl)phényl]méthyl}-3-[(3,5-difluorophényl)(méthylsulfonyl)méthylène] azétidine, isomère forme B, sous la forme d'une meringue blanche [Spectre de R.M.N. ¹H (300 MHz, CDCl₃, δ en ppm) : de 2,60 à 2,75 (mt : 8H); 2,81 (s : 3H); 3,44 (s : 2H); 3,85 (mt : 2H); 4,34 (mt : 2H); 4,50 (s : 1H); 6,84 (tt, J = 8,5 et 2,5 Hz : 1H); 6,98 (mt : 2H); de 7,15 à 7,40 (mt : 8H)].

### Exemple 116

On opère comme il est décrit dans l'exemple 110, mais en agitant le mélange réactionnel pendant 48 heures à 20°C, à partir de 0,200 g de 1-{(R*)-[4-(chlorométhyl)phényl]-(4-chlorophényl)méthyl}-3-[(3,5-difluorophényl)(méthylsulfonyl)méthylène]azétidine, isomère forme B, 5,0 cm³ de dichlorométhane, et 0,120 g de pipérazin-2-one. Le produit brut est chromatographié sur une colonne de gel de silice (granulométrie 0,06-0,200 mm, diamètre 1,0 cm, hauteur 10 cm), en éluant avec 50 cm³ de dichlorométhane, puis avec un mélange dichlorométhane et méthanol (95/5 en volumes), en recueillant des fractions de 5 cm³ dès l'utilisation de ce mélange éluant. Les fractions 3 à 13 sont réunies puis concentrées à sec sous pression réduite (2,7 kPa). On obtient 0,090 g de 1-{(R*)-(4-chlorophényl)[4-(pipérazin-2-on-4-yl-méthyl)phényl]méthyl }-3-[(3,5-difluorophényl)(méthylsulfonyl) méthylène]azétidine, isomère forme B, sous la forme d'une poudre blanche.

### Exemple 117

On opère comme il est décrit dans l'exemple 110, à partir de 0,200 g de 1-{(R*)-[4-(chlorométhyl)phényl]-(4-chlorophényl)méthyl}-3-[(3,5-difluorophényl)(méthylsulfonyl)méthylène]azétidine, isomère forme B, 5,0 cm³ de dichlorométhane, et 0,120g de 3,3-diméthylpipéridine. Le produit brut est chromatographié sur une colonne de gel de silice (granulométrie 0,06-0,200 mm, diamètre 1,0 cm, hauteur 10 cm), en éluant avec 50 cm³ de dichlorométhane, puis avec un mélange dichlorométhane et méthanol (95/5 en volumes), en recueillant des fractions de 5 cm³ dès l'utilisation de ce mélange éluant. Les fractions 4 à 11 sont réunies puis concentrées à sec sous pression réduite (2,7 kPa). On obtient 0,120 g de 1-{(R*)-(4-chlorophényl)[4-(3,3-diméthylpipéridinyl-méthyl)phényl]méthyl}-3-[(3,5-difluorophényl)(méthylsulfonyl)méthylène]azétidine, isomère forme B, sous la forme d'une poudre blanche.

### Exemple 118

On opère comme dans l'exemple 110, en agitant pendant 72 heures à 20°C, à partir de 0,200 g de 1-{(R*)-[4-(chlorométhyl)phényl]-(4-chlorophényl)méthyl}-3-[(3,5-difluorophényl)(méthylsulfonyl)méthylène]azétidine, isomère forme B, 5,0 cm³ de dichlorométhane, et 0,080 g d'imidazole. Le mélange réactionnel est directement chromatographié sur une colonne de gel de silice (granulométrie 0,06-0,200 mm, diamètre 1,0 cm, hauteur 10 cm), en éluant avec 100 cm³ de dichlorométhane sans fractionner, puis avec un mélange dichlorométhane et méthanol (98/2 puis 96/4 en volumes), en recueillant des fractions de 5 cm³. Les fractions 5 à 12 sont réunies puis concentrées à sec sous pression réduite (2,7 kPa).On obtient 0,035 g de 1-{(R*)-(4-chlorophényl)[4-(imidazol-1-yl-méthyl)phényl]méthyl}-3-[(3,5-difluorophényl)(méthylsulfonyl)méthyléne]}azétidine, isomère forme B,sous la forme d'une poudre blanche. [α]²⁰D = -6,7° (c = 0,5% dichlorométhane)

### Exemple 119

A une suspension de 6,12 g de 1-[bis(4-chlorophényl)méthyl]azétidin-3-one et 5,15 g de 5-(méthylsulfonylméthyl)thiophène-2-carboxylate de méthyle dans 200 cm³ de tétrahydrofuranne, sous atmosphère d'argon, refroidie à -70°C, on ajoute 2,47 g de *tert*-butylate de potassium. Le mélange est agité 1 heure 30 à une température voisine de -70°C, puis on ajoute 1,7 cm³ de chlorure de méthanesulfonyle en solution dans 8 cm³ d'éther éthylique. Après 1 heure d'agitation à une température voisine de -70°C le mélange est laissé revenir à température ambiante, puis on ajoute 80 cm³ d'eau distillée. Le mélange est concentré au rotavapor juqu'au tiers de son volume initial, puis est extrait par 500 cm³ de dichlorométhane. La phase organique est lavée par 3 fois 80 cm³ d'eau distillée, séchée sur sulfate de magnésium et concentrée à sec sous pression réduite (2,7 kPa). Le résidu obtenu est chromatographié sur colonne de gel de silice (granulométrie 0,02-0,04 mm, diamètre 7,5 cm, hauteur 35 cm), en éluant sous une pression de 0,5 bar d'azote par un mélange cyclohexane et acétate d'éthyle (70/30 en volumes) et en recueillant des fractions de 40 cm³. Les fractions 19 à 29 sont réunies puis concentrées à sec sous pression réduite (2,7 kPa). On obtient 1,6 g de 1-[bis(4-chlorophényl)méthyl]-3-[(méthylsulfonyl)(2-méthoxycar-bonylthién-5-yl)méthylène]azétidine sous forme d'une meringue crème [Spectre de R.M.N ¹H (400 MHz, CDCl₃, δ en ppm) : 2,91 (s : 3H); 3,88 (s : 3H); 4,08 (mt : 2H); 4,37 (mt : 2H); 4,53 (s : 1H); de 7,25 à 7,45 (mt : 9H); 7,71 (d, J = 3,5 Hz : 1H)].

Les fractions 34 à 48 sont réunies puis concentrées à sec sous pression réduite (2,7 kPa). On obtient 2,6 g de 1-[bis(4-chlorophényl)méthyl]-3-hydroxy-3-[(méthylsulfonyl)(2-méthoxycarbonylthién-5-yl)méthyl]azétidine-(RS) sous forme d'une poudre crème [Spectre de R.M.N. ¹H (400 MHz, (CD₃)₂SO d6, δ en ppm) : 2,87 (s : 3H); 2,89 (d, J = 8 Hz : 1H); 2,96 (d, J = 8 Hz : 1H); 3,21 (d, J = 8 Hz : 1H); 3,76 (d, J = 8 Hz : 1H); 3,82 (s : 3H); 4,55 (s : 1H); 4,86 (s : 1H); 6,86 (s : 1H); de 7,35 à 7,45 (mt : 9H); 7,73 (d, J = 4 Hz : 1H)].

Le 5-(méthylsulfonylméthyl)thiophène-2-carboxylate de méthyle peut être préparé de la façon suivante : A une solution de 16 g de 5-bromométhyl-thiophène-2-carboxylate de méthyle dans 150 cm³ de tétrahydrofuranne on ajoute, à température ambiante, 6,94 g de méthanesulfinate de sodium. La suspension est agitée 2 heures 30 au reflux, puis après addition de 50 cm³ d'éthanol est agitée à nouveau 3 heures au reflux. Le mélange est concentré à sec sous pression réduite (2,7 kPa) et le résidu obtenu est additionné de 150 cm³ d'eau distillée, puis est extrait par 2 fois 300 cm³ d'acétate d'éthyle. La phase organique est lavée successivement par 100 cm³ d'eau distillée et 2 fois 50 cm³ de solution aqueuse saturée de chlorure de sodium, puis séchée sur sulfate de magnésium et concentrée à sec sous pression réduite (2,7 kPa). On obtient ainsi 14 g de 5-(méthylsulfonylméthyl)thiophène-2-carboxylate de méthyle sous forme d'un solide jaune fondant vers 133°C [Spectre de R.M.N. ¹H (400 MHz, (CD₃)₂SO d6, à une température de 373K, δ en ppm) : 3,05 (s : 3H); 4,22 (mt : 2H); 4,40 (mt : 2H); 4,98 (s large : 1H); 7,30 (d, J = 3,5 Hz : 1H); 7,39 (d, J = 8 Hz : 4H); 7,50 (d, J = 8 Hz : 4H); 7,66 (d, J = 3,5 Hz : 1H)].

Le 5-bromométhyl-thiophène-2-carboxylate de méthyle peut être préparé selon Curtin M. L., Davidsen, S. K., Heyman H. R., Garland R. B., Sheppard G. S., J. Med. Chem.; 1998, 41 (1), 74-95.

### Exemple 120

A une solution de 163,5 mg du chlorhydrate de 1-[bis(4-chlorophényl)méthyl]-3-[(méthylsulfonyl)(2-hydroxycarbonylthién-5-yl)méthylène]azétidine dans 3 cm³ de dichlorométhane, à température ambiante, on ajoute 47 µl de *N,N'*-diisopropylcarbodiimide, 3,66 mg de 4-diméthylaminopyridine et 60 µl d'isobutylamine. Le mélange est agité pendant 18 heures à température ambiante, puis chromatographié sur colonne de gel de silice (granulométrie 0,04-0,06 mm), en éluant par un mélange dichlorométhane et acétate d'éthyle (90/10 en volumes). On obtient ainsi 60 mg de 1-[bis(4-chlorophényl)méthyl]-3-[(2-*iso*butylaminocarbonylthién-5-yl)(méthylsulfonyl) méthylène]azétidine sous forme d'une laque incolore [Spectre de R.M.N.¹H (400 MHz, CDCl₃, δ en ppm) : 0,97 (d, J = 7 Hz : 6H); 1,88 (mt : 1H); 2,90 (s : 3H); 3,25 (t, J = 7 Hz : 2H); 4,08 (mt : 2H); 4,36 (mt : 2H); 4,52 (s : 1H); 4,56 (t large, J = 7 Hz : 1H); de 7,20 à 7,40 (mt : 10H)].

Le chlorhydrate de 1-[bis(4-chlorophényl)méthyl]-3-[(méthylsulfonyl)(2-hydroxycarbonylthién-5-yl)méthylène]azétidine peut être préparé de la manière suivante : à une solution de 14 g de 1-[bis(4-chlorophényl)méthyl]-3-[(méthylsulfonyl)(2-méthoxycarbonylthién-5-yl)méthylène]azétidine dans 250 cm³ de d'acide acétique, à température ambiante, on ajoute 250 cm³ d'acide chlorhydrique concentré. Le mélange est agité pendant 38 heures à une température de 50°C, puis est concentré à sec sous pression réduite (2,7 kPa). Par trois fois le résidu est additionné de 250 cm³ de toluène et concentré à sec sous pression réduite (2,7 kPa). Après trituration du résidu dans 400 cm³ d'éther éthylique on obtient 14,2 g du chlorhydrate de 1-[bis(4-chlorophényl)méthyl]-3-[(méthylsulfonyl)(2-hydroxycarbonylthién-5-yl)méthylène] azétidine sous forme d'une poudre beige.

### Exemple 121

A une solution de 0,92 g de 1-[bis(4-chlorophényl)méthyl]-azétidin-3-one et 0,75 g de [(3-méthoxycarbonylphényl)méthyl]méthylsulfone dans 30 cm³ de tétrahydrofuranne, sous atmosphère d'argon, refroidie à -70°C, on ajoute 0,37 g de tert-butylate de potassium et agite 2 heures à -70°C. On ajoute ensuite 10 cm³ d'une solution d'acide chlorhydrique 0,1N et on laisse le mélange revenir à température ambiante. Après ajout de 50 cm³ d'acétate d'éthyle, le mélange réactionnel est décanté, séché sur sulfate de magnésium, filtré puis concentré à sec sous pression réduite (2,7 kPa). Le résidu est chromatographié sur colonne de gel de silice (granulométrie 0,20-0,06 mm, diamètre 3 cm, hauteur 50 cm), en éluant sous une pression de 0,8 bar d'azote par un mélange cyclohexane et acétate d'éthyle (70/30 en volumes) et en recueillant des fractions de 120 cm³. Les fractions 11 à 18 sont réunies puis concentrées à sec sous pression réduite (2,7 kPa). Le résidu est cristallisé dans 10 cm³ d'éther isopropylique et 30 cm³ de pentane. On obtient ainsi 0,30 g de 1-[bis(4-chlorophényl)méthyl]-3-[(3-méthoxycarbonylphényl)(méthylsulfonyl)méthyl-(RS)]azétidin-3-ol sous forme d'un solide blanc [Spectre de R.M.N ¹H (400 MHz, CDCl₃, δ en ppm) : 2,73 (s : 3H); 3,05 (AB, J = 9 Hz : 2H); 3,27 (d, J = 9 Hz : 1H); 3,63 (s : 1H); 3,79 (d, J = 9 Hz : 1H); 3,95 (s : 3H); 4,32 (s : 1H); 4,59 (s : 1H); de 7,15 à 7,35 (mt : 8H); 7,51 (t, J = 8 Hz : 1H); 7,94 (d large, J = 8 Hz : 1H); 8,10 (d large, J = 8 Hz : 1H); 8,32 (s large : 1H)].

### Exemple 122

En opérant selon le mode opératoire de l'exemple 1 à partir de 0,66 g de méthyl-(pyridin-4-yl-méthyl)sulfone et 1,18 g de 1-[bis(4-chlorophényl)méthyl]-azétidin-3-one, on obtient après purification sur colonne de gel de silice (granulométrie 0,20-0,06 mm, diamètre 3 cm, hauteur 50 cm) sous une pression de 0,5 bar d'azote avec un mélange cyclohexane et acétate d'éthyle (70/30 en volumes) comme éluant et en recueillant des fractions de 120 cm³, 0,20 g d'un solide blanc. Celui-ci est repris par 20 cm³ d'oxyde de diisopropyle. Après filtration, essorage et séchage, on obtient 0,16 g de 1-[bis(4-chlorophényl)méthyl]-3-[(méthylsulfonyl)(pyridin-4-yl)méthyl-(RS)]-azétidin-3-ol [Spectre de R.M.N ¹H (400 MHz, CDCl₃, δ en ppm) : 2,76 (s : 3H); 3,03 (AB, J = 9 Hz : 2H); 3,27 (d, J = 9 Hz : 1H); 3,53 (s : 1H); 3,83 (d, J = 9 Hz : 1H); 4,32 (s : 1H); 4,51 (s : 1H); de 7,20 à 7,30 (mt : 8H); 7,63 (d, J = 6 Hz : 2H); 8,68 (d, J = 6 Hz : 2H)].

La méthyl-(pyridin-4-yl-méthyl)sulfone peut être préparée selon la référence JP43002711

### Exemple 123

En opérant selon le mode opératoire de l'exemple 1 à partir de 0,47 g de méthyl-(pyridin-3-yl-méthyl)sulfone et 0,83 g de 1-[bis(4-chlorophényl)méthyl]-azétidin-3-one, on obtient après purification sur colonne de gel de silice (granulométrie 0,20-0,06 mm, diamètre 3 cm, hauteur 50 cm) sous une pression de 0,5 bar d'azote avec un mélange cyclohexane et acétate d'éthyle (70/30 en volumes) comme éluant et en recueillant des fractions de 120 cm³, 0,50 g d'un solide blanc. Celui-ci est repris par 30 cm³ d'oxyde de diisopropyle. Après filtration, essorage et séchage, on obtient 0,40 g de 1-[bis(4-chlorophényl)méthyl]-3-[(méthylsulfonyl)(pyridin-3-yl)méthyl-(RS)]-azétidin-3-ol [Spectre de R.M.N ¹H (300 MHz, CDCl₃, δ en ppm) : 2,77 (s : 3H); 3,03 (AB, J = 9 Hz : 2H); 3,28 (d, J = 9 Hz : 1H); 3,66 (s : 1H); 3,83 (d, J = 9 Hz : 1H); 4,33 (s : 1H); 4,55 (s : 1H); de 7,20 à 7,30 (mt : 8H); 7,37 (dd, J = 8 et 5 Hz : 1H); 8,16 (dt, J = 8 et 2 Hz : 1H); 8,68 (dd, J = 5 et 1,5 Hz : 1H); 8,83 (d, J = 2 Hz : 1H)].

La méthyl-(pyridin-3-yl-méthyl)sulfone peut être préparée selon la référence JP43002711.

### Exemple 124

A une suspension de 150 mg d'acide 3-({1-[bis-(4-chlorophényl)méthyl]-azétidin-3-ylidène}-méthanesulfonyl-méthyl)benzoïque activé sur résine TFP (165 µM) dans 3 cm³ de dichlorométhane, pré-agitée 90 minutes à une température voisine de 20°C, est ajouté à la même température 0,0388 cm³ de N-(3-aminopropyl)morpholine. La suspension est agitée à une température voisine de 20 °C pendant 22 heures, puis filtrée sur fritté. Le résidu solide est relavé avec 2 fois 1,5 cm³ de dichlorométhane. Les filtrats sont réunis et concentrés à sec sous pression réduite (2,7 kPa) à une température voisine de 40°C. On obtient ainsi 60 mg de 3-({1-[bis-(4-chlorophényl)méthyl]-azétidin-3-ylidène}-méthanesulfonyl-méthyl)N-(3-morpholin-4-yl-propyl)benzamide sous forme d'une meringue jaune pâle.

Le 3-({1-[bis-(4-chlorophényl)méthyl]-azétidin-3-ylidène-méthanesulfonyl-méthyl)N-(3-morpholin-4-yl-propyl)benzamide peut être également préparé de la manière suivante : A une solution de 300 mg d'acide 3-({1-[bis-(4-chlorophényl)méthyl]-azétidin-3-ylidène}-méthanesulfonyl-méthyl)benzoïque dans 10 cm³ de dichlorométhane anhydre (sur chlorure de calcium) et 5 cm³ de diméthylformamide, sous atmosphère inerte d'azote, à une température voisine de 20°C, sont ajoutés successivement 0,083 cm³ de N-(3-aminopropyl)morpholine, 110 mg de chlorhydrate de 1-(3-diméthylaminopropyl)3-éthylcarbodiimide et 5 mg de 4-diméthylaminopyridine. La solution obtenue est agitée à une température voisine de 20°C pendant environ 22 heures, puis concentré à sec sous pression réduite (0,27 kPa) à une température voisine de 40°C. Le résidu solide est repris avec 25 cm³ de dichlorométhane, lavé avec 2 fois 20 cm³ d'une solution saturée aqueuse de bicarbonate de sodium. Après décantation, la phase organique est séchée sur sulfate de magnésium, filtrée, et concentrée à sec sous pression réduite (2,7 kPa) à une température voisine de 40°C. On obtient ainsi 400 mg d'une huile jaune que l'on purifie par chromatographie sous pression d'azote (0,8 bar) sur 60 cm³ de silice (0,040-0,063 mm) contenus dans une colonne de 2,2 cm de diamètre en éluant avec un mélange méthanol-dichlorométhane (2-98 en volumes). Les fractions ne contenant que le produit cherché sont réunies et concentrées à sec sous pression réduite (0,27 kPa) à 40°C pendant 2 heures. On obtient ainsi 130 mg de 3-({1-[bis-(4-chlorophényl)méthyl]-azétidin-3-ylidène}-méthanesulfonylméthyl)N-(3-morpholin-4-yl-propyl)benzamide sous forme d'une poudre blanche cristalline [Spectre de R.M.N. ¹H (300 MHz, (CD₃)₂SO d6, d en ppm) : 1,68 (mt : 2H); de 2,25 à 2,40 (mt : 6H); 2,97 (s : 3H); de 3,20 à 3,35 (mt : 2H); 3,57 (t, J = 4,5 Hz : 4H); 3,81 (mt : 2H); 4,22 (mt : 2H); 4,79 (s : 1H); 7,36 (d, J = 8,5 Hz : 4H); 7,46 (d, J = 8,5 Hz : 4H); de 7,50 à 7,60 (mt : 2H); 7,83 (s large : 1H); 7,86 (d large, J = 8 Hz : 1H); 8,53 (t, J= 5,5 Hz : 1H)].

### Exemple 125

On opère dans les conditions décrites dans l'exemple 124 à partir de 150 mg d'acide 3-( {1-[bis-(4-chlorophényl)méthyl]-azétidin-3-ylidène}-méthanesulfonylméthyl)benzoïque activé sur résine TFP (165 µM) et 0,033 cm³ de N,N-diméthyl-1,3-propanediamine. On obtient ainsi 52 mg de 3-({1-[bis-(4-chlorophényl)méthyl]-azétidin-3-ylidène}-méthanesulfonyl-méthyl)N-(3-diméthylamino-propyl)benzamide sous forme d'une poudre blanche [Spectre de R.M.N. ¹H (400 MHz, (CD₃)₂SO d6, d en ppm) : 1,65 (mt : 2H); 2,18 (s : 6H); de 2,20 à 2,35 (mt : 2H); 2,98 (s : 3H); de 3,25 à 3,45 (mt : 2H); 3,82 (mt : 2H); 4,23 (mt : 2H); 4,80 (s : 1H); 7,36 (d, J = 8,5 Hz : 4H); 7,46 (d, J = 8,5 Hz : 4H); de 7,50 à 7,60 (mt : 2H); 7,83 (s large : 1H); 7,86 (d large, J = 8 Hz : 1H); 8,57 (t, J = 5,5 Hz : 1H)].

### Exemple 126

On opère dans les conditions décrites dans l'exemple 124 à partir de 150 mg d'acide 3-({1-[bis-(4-chlorophényl)méthyl]-azétidin-3-ylidène}-méthanesulfonylméthyl)benzoïque activé sur résine TFP (165 µM) et 0,0333 cm³ 1-(aminoéthyl)pyrrolidine. On obtient ainsi 39 mg de 3-({1-[bis-(4-chlorophényl)méthyl]-azétidin-3-ylidène}-méthanesulfonyl-méthyl)N-(2-pyrrolidin-1-yl-éthyl)benzamide sous forme d'une poudre jaune pâle [Spectre de R.M.N.¹H (300 MHz, (CD₃)₂SO d6 avec ajout de quelques gouttes de CD₃COOD d4, d en ppm) : de 1,80 à 2,00 (mt : 4H); 2,97 (s : 3H); 3,20 (mt : 6H); 3,57 (t, J = 6,5 Hz : 2H); 3,80 (mt : 2H); 4,23 (mt : 2H); 4,77 (s : 1H); 7,35 (d, J = 8,5 Hz : 4H); 7,45 (d, J = 8,5 Hz : 4H); de 7,50 à 7,65 (mt : 2H); 7,87 (s large : 1H); 7,90 (d large, J = 7,5 Hz : 1H)].

### Exemple 127

On opère dans les conditions décrites dans l'exemple 124 à partir de 150 mg d'acide 3-({1-[bis-(4-chlorophényl)méthyl]-azétidin-3-ylidène}-méthanesulfonylméthyl)benzoïque activé sur résine TFP (165 µM) et 0,033 cm³ 1-(diméthylamino)2-propylamine. On obtient ainsi 49 mg de 3-({1-[bis-(4-chlorophényl)méthyl]-azétidin-3-ylidène}-méthanesulfonyl-méthyl)N-(2-diméthylamino-1-méthyl-éthyl)benzamide sous forme d'une poudre blanche [Spectre de R.M.N. ¹H (400 MHz, (CD₃)₂SO d6, d en ppm) : 1,13 (d, J = 6,5 Hz : 3H); de 2,10 à 2,25 (mt : 1H); 2,15 (s : 6H); 2,38 (dd, J = 13 et 8 Hz : 1H); 2,98 (s : 3H); 3,80 (mt : 2H); 4,14 (mt : 1H); 4,23 (mt : 2H); 4,79 (s : 1H); 7,36 (d, J = 8 Hz : 4H); de 7,45 à 7,60 (mt : 2H); 7,46 (d, J = 8 Hz : 4H); 7,83 (s large : 1H); 7,87 (d large, J = 8 Hz : 1H); 8,16 (d large, J = 8 Hz : 1H)].

### Exemple 128

On opère dans les conditions décrites dans l'exemple 124 à partir de 150 mg d'acide 3-({1-[bis-(4-chlorophényl)méthyl]-azétidin-3-ylidéne}-méthanesulfonyl-méthyl) benzoïque activé sur résine TFP (165 µM) et 0,026 cm³ de pipéridine. On obtient ainsi 56 mg de 3-({1-[bis-(4-chlorophényl)méthyl]-azétidin-3-ylidène}-méthanesulfonyl-méthyl)N-pipéridin-1-yl-benzamide sous forme d'une poudre blanche [Spectre de R.M.N. ¹H (400 MHz, (CD₃)₂SO d6, d en ppm) : de 1,45 à 1,70 (mt : 6H); de 2,90 à 3,05 (mt : 2H); 2,98 (s : 3H); 3,19 (mf: 1H); 3,57 (mf: 1H); 3,85 (mt : 2H); 4,23 (mt : 2H); 4,80 (s : 1H); de 7,30 à 7,55 (mt : 12H)].

### Exemple 129

On opère dans les conditions décrites dans l'exemple 124 à partir de 150 mg d'acide 3-( {1-[bis-(4-chlorophényl)méthyl]-azétidin-3-ylidène}-méthanesulfonylméthyl)benzoïque activé sur résine TFP (165 µM) et 0,0265 cm³ d'isobutylamine. On obtient ainsi 46 mg de 3-({1-[bis-(4-chlorophényl)méthyl]-azétidin-3-ylidène}-méthanesulfonyl-méthyl)N-isobutyl-benzamide sous forme d'une poudre blanche [Spectre de R.M.N. ¹H (400 MHz, (CD₃)₂SO d6, d en ppm) : 0,89 (d, J = 7 Hz : 6H); 1,85 (mt : 1H); 2,98 (s : 3H); 3,09 (t, J = 6,5 Hz : 2H); 3,82 (mt : 2H); 4,23 (mt : 2H); 4,79 (s : 1H); 7,36 (d, J = 8,5 Hz : 4H); 7,46 (d, J = 8,5 Hz : 4H); de 7,50 à 7,60 (mt : 2H); 7,84 (s large : 1H); 7,88 (d large, J = 8 Hz : 1H); 8,51 (t, J = 6 Hz : 1H)].

### Exemple 130

On opère dans les conditions décrites dans l'exemple 124 à partir de 150 mg d'acide 3-( {1-[bis-(4-chlorophényl)méthyl]-azétidin-3-ylidène}-méthanesulfonylméthyl)benzoïque activé sur résine TFP (165 µM) et 0,0316 cm³ de N-(3-aminopropyl)imidazole. On obtient ainsi 54 mg de 3-({1-[bis-(4-chlorophényl)méthyl]-azétidin-3-ylidène}-méthanesulfonyl-méthyl)N-(3-imidazol-1-yl-propyl)benzamide sous forme d'une meringue jaune [Spectre de R.M.N. ¹H (400 MHz, (CD₃)₂SO d6, d en ppm) : 1,97 (mt : 2H); 2,98 (s : 3H); 3,25 (mt : 2H); 3,81 (mt : 2H); 4,02 (t, J = 7 Hz : 2H); 4,23 (mt : 2H); 4,79 (s : 1H); de 6,85 à 6,95 (mt : 2H); 7,36 (d, J = 8,5 Hz : 4H); 7,46 (d, J = 8,5 Hz : 4H); de 7,50 à 7,60 (mt : 2H); 7,84 (s large : 1H); 7,88 (d large, J = 8 Hz : 1H); 8,56 (t, J = 5,5 Hz : 1H)].

### Exemple 131

On opère dans les conditions décrites dans l'exemple 124 à partir de 150 mg d'acide 3-({1-[bis-(4-chlorophényl)méthyl]-azétidin-3-ylidène}-méthanesulfonyl-méthyl)benzoïque activé sur résine TFP (165 µM) et 0,030 cm³ de N,N-(diméthyl)éthylènediamine. On obtient ainsi 53 mg de 3-({1-[bis-(4-chlorophényl)méthyl]-azétidin-3-ylidène}-méthanesulfonyl-méthyl)N-(2-diméthylamino-éthyl)benzamide sous forme d'une meringue ocre [Spectre de R.M.N. ¹H (400 MHz, (CD₃)₂SO d6, d en ppm) : 2,18 (2s : 6H); de 2,35 à 2,45 (mt : 2H); 2,98 (s : 3H); de 3,25 à 3,50 (mt : 2H); 3,81 (mt : 2H); 4,23 (mt : 2H); 4,79 (s : 1H); 7,36 (d, J = 8 Hz : 4H); 7,46 (d, J = 8 Hz : 4H); de 7,45 à 7,60 (mt : 2H); 7,83 (s large : 1H); 7,86 (d large, J = 8 Hz : 1H); 8,43 (t, J = 6,5 Hz : 1H)].

### Exemple 132

On opère dans les conditions décrites dans l'exemple 124 à partir de 150 mg d'acide 3-({1-[bis-(4-chlorophényl)méthyl]-azétidin-3-ylidène}-méthanesulfonyl-méthyl) benzoïque activé sur résine TFP (165 µM) et 0,0141 cm³ de méthyl-hydrazine. On obtient ainsi 42 mg de N'-méthyl-hydrazide de l'acide 3-({1-[bis-(4-chlorophényl)méthyl]-azétidin-3-ylidène}-méthanesulfonyl-méthyl)benzoïque sous forme d'une meringue jaune pâle [Spectre de R.M.N. ¹H (400 MHz, (CD₃)₂SO d6, d en ppm) : 2,96 (s : 3H); 3,18 (s large : 3H); 3,83 (mt : 2H); 4,22 (mt : 2H); 4,80 (s large : 2H); de 7,35 à 7,65 (mt : 12H)].

### Exemple 133

On opère dans les conditions décrites dans l'exemple 124 à partir de 150 mg d'acide 3-({1-[bis-(4-chlorophènyl)mèthyl]-azétidin-3-ylidène}-méthanesulfonyl-méthyl) benzoïque activé sur résine TFP (165 µM) et 0,0345 cm³ de N-(2-aminoéthyl)morpholine. On obtient ainsi 62 mg de 3-({1-[bis-(4-chlorophényl)méthyl]-azétidin-3-ylidène}-méthanesulfonyl-méthyl)N-(2-morpholin-4-yl-éthyl)benzamide sous forme d'une meringue ocre [Spectre de R.M.N. ¹H (400 MHz, (CD₃)₂SO d6, d en ppm) : de 2,30 à 2,45 (mt : 4H); 2,46 (t, J = 7,5 Hz : 2H); 2,98 (s : 3H); 3,38 (mt : 2H); de 3,50 à 3,65 (mt : 4H); 3,82 (mt : 2H); 4,24 (mt : 2H); 4,79 (s : 1H); 7,36 (d, J = 8,5 Hz : 4H); 7,46 (d, J = 8,5 Hz : 4H); de 7,50 à 7,60 (mt : 2H); 7,83 (s large : 1H); 7,85 (dd, J = 8 et 2 Hz : 1H); 8,45 (t, J = 6,5 Hz : 1H)].

### Exemple 134

On opère dans les conditions décrites dans l'exemple 124 à partir de 150 mg d'acide 3-({1-[bis-(4-chlorophényl)méthyl]-azétidin-3-ylidène}-méthanesulfonyl-méthyl) benzoïque activé sur résine TFP (165 µM) et 0,0396 cm³ de 2-(aminométhyl)N-éthylpyrrolidine. On obtient ainsi 58 mg de 3-({1-[bis-(4-chlorophényl)méthyl]-azétidin-3-ylidène}-méthanesulfonyl-méthyl)N-(1-éthyl-pyrrolidin-2-ylméthyl)benzamide sous forme d'une meringue ocre.

Le 3-({1-[bis-(4-chlorophényl)méthyl]-azétidin-3-ylidène}-méthanesulfonyl-méthyl)N-(1-éthyl-pyrrolidin-2-ylméthyl)benzamide peut être également préparé dans les conditions décrites dans l'exemple 126 à partir de 700 mg d'acide 3-({1-[bis-(4-chlorophényl)méthyl]-azétidin-3-ylidène}-méthanesulfonyl-méthyl)benzoïque activé sur résine TFP (770 µM), 0,324 cm³ de triéthylamine et 0,25 cm³ de 2-(aminométhyl)N-éthyl-pyrrolidine. On obtient ainsi 370 mg d'un solide que l'on purifie par chromatographie sous pression d'azote (0,7 bar) sur 100 cm³ de silice (0,040-0,063 mm) contenus dans une colonne de 2,5 cm de diamètre en éluant avec un mélange méthanol-dichlorométhane (15-85 en volumes). Les fractions ne contenant que le produit cherché sont réunies et concentrées à sec sous pression réduite (0,27 kPa) à 40°C pendant 2 heures. On obtient ainsi 160 mg de 3-({1-[bis-(4-chlorophényl)méthyl]-azétidin-3-ylidène}-méthanesulfonyl-méthyl)N-(1-éthylpyrrolidin-2-ylméthyl)benzamide sous forme d'une poudre jaune pâle [Spectre de R.M.N. ¹H (400 MHz, (CD₃)₂SO d6, d en ppm) : 1,04 (t, J = 7 Hz : 3H); de 1,50 à 1,70 (mt : 3H); 1,78 (mt : 1H); 2,14 (mt : 1H); 2,28 (mt : 1H); 2,59 (mt : 1H); 2,83 (mt : 1H); 2,98 (s : 3H); de 3,00 à 3,15 (mt : 2H); de 3,30 à 3,45 (mt : 1H); 3,82 (mt : 2H); 4,23 (mt : 2H); 4,79 (s : 1H); 7,36 (d, J = 8,5 Hz : 4H); 7,46 (d, J = 8,5 Hz : 4H); de 7,50 à 7,60 (mt : 2H); 7,86 (s large : 1H); 7,85 (d large, J = 8 Hz : 1H); 8,41 (t, J = 6 Hz : 1H)].

### Exemple 135

On opère dans les conditions décrites dans l'exemple 124 à partir de 110 mg d'acide 3-({1-[bis-(4-chlorophényl)méthyl]-azétidin-3-ylidène}-méthanesulfonyl-méthyl) benzoïque activé sur résine TFP (121 µM) et 0,023 cm³ de néo-pentylamine. On obtient ainsi 69 mg de 3-({1-[bis-(4-chlorophényl)méthyl]-azétidin-3-ylidène}-méthanesulfonyl-méthyl)N-(2,2-diméthyl-propyl)benzamide sous forme d'une poudre jaune pâle [Spectre de R.M.N. ¹H (400 MHz, (CD₃)₂SO d6, d en ppm) : 0,90 (s : 9H); 2,98 (s : 3H); 3,11 (d, J = 6,5 Hz : 2H); 3,82 (mt : 2H); 4,23 (mt : 2H); 4,79 (s : 1H); 7,36 (d, J = 8 Hz : 4H); 7,46 (d, J = 8 Hz : 4H); de 7,45 à 7,60 (mt : 2H); 7,83 (s large : 1H); 7,86 (d large, J = 8 Hz : 1H); 8,37 (t, J = 6,5 Hz : 1H)].

### Exemple 136

On opère dans les conditions décrites dans l'exemple 124 à partir de 110 mg d'acide 3-({1-[bis-(4-chlorophényl)méthyl]-azétidin-3-ylidène}-méthanesulfonyl-méthyl) benzoïque activé sur résine TFP (121 µM) et 0,025 cm³ d'amino-méthyl-cylohexane. On obtient ainsi 44 mg de 3-({1-[bis-(4-chlorophényl)méthyl]-azétidin-3-ylidène}-méthanesulfonyl-méthyl)N-cyclohexylméthyl-benzamide sous forme d'une poudre jaune pâle dont les caractéristiques sont les suivantes [Spectre de R.M.N. ¹H (400 MHz, (CD₃)₂SO d6, d en ppm) : 0,92 (mt : 2H); 1,17 (mt : 4H); de 1,45 à 1,80 (mt : 5H); 2,97 (s : 3H); 3,10 (d, J = 6 Hz : 2H); 3,80 (mt : 2H); 4,23 (mt : 2H); 4,79 (s : 1H); 7,36 (d, J = 8 Hz : 4H); 7,46 (d, J = 8 Hz : 4H); de 7,45 à 7,60 (mt : 2H); 7,83 (s large : 1H); 7,86 (d large, J = 8 Hz : 1H); 8,47 (t, J = 6 Hz : 1H)].

### Exemple 137

On opère dans les conditions décrites dans l'exemple 124 à partir de 110 mg d'acide 3-({1-[bis-(4-chlorophényl)méthyl]-azétidin-3-ylidène}-méthanesulfonyl-méthyl) benzoïque activé sur résine TFP (121 µM), 0,026 cm³ de triéthylamine et 21 mg de chlorhydrate d'amino-méthyl-cylopropane. On obtient ainsi 68 mg de 3-({1-[bis-(4-chlorophényl)méthyl]-azétidin-3-ylidène} -méthanesulfonyl-méthyl)N-cyclopropylméthyl-benzamide sous forme d'une meringue jaune [Spectre de R.M.N. ¹H (400 MHz, (CD₃)₂SO d6, d en ppm) : 0,24 (mt : 2H); 0,44 (mt : 2H); 1,03 (mt : 1H); 2,98 (s : 3H); 3,15 (t, J = 6 Hz : 2H); 3,82 (mt : 2H); 4,23 (mt : 2H); 4,79 (s : 1H); 7,36 (d, J = 8,5 Hz : 4H); 7,46 (d, J = 8,5 Hz : 4H); de 7,50 à 7,60 (mt : 2H); 7,86 (s large : 1H); 7,89 (d large, J = 8 Hz : 1H); 8,64 (t, J = 6 Hz : 1H)].

### Exemple 138

On opère dans les conditions décrites dans l'exemple 124 à partir de 110 mg d'acide 3-({1-[bis-(4-chlorophényl)méthyl]-azétidin-3-ylidène}-méthanesulfonylméthyl)benzoïque activé sur résine TFP (121 µM) et 0,023 cm³ de 2-méthylbutylamine. On obtient ainsi 49 mg de 3-({1-[bis-(4-chlorophényl)méthyl]-azétidin-3-ylidène}-méthanesulfonyl-méthyl)N-(2-méthyl-butyl)benzamide [Spectre de R.M.N. ¹H (400 MHz, (CD₃)₂SO d6, d en ppm) : de 0,80 à 0,95 (mt : 6H); de 1,05 à 1,20 (mt : 1H); 1,41 (mt : 1H); 1,64 (mt : 1H); 2,98 (s : 3H); 3,06 (mt : 1H); 3,19 (mt : 1H); 3,81 (mt : 2H); 4,23 (mt : 2H); 4,79 (s : 1H); 7,36 (d, J = 8 Hz : 4H); 7,46 (d, J = 8 Hz : 4H); de 7,35 à 7,60 (mt : 2H); 7,84 (s large : 1H); 7,87 (d large, J = 8 Hz : 1H); 8,49 (t, J = 5,5 Hz : 1H)].

### Exemple 139

On opère dans les conditions décrites dans l'exemple 124 à partir de 110 mg d'acide 3-({1-[bis-(4-chlorophényl)méthyl]-azétidin-3-ylidène}-méthanesulfonyl-méthyl) benzoïque activé sur résine TFP (121 µM) et 0,028 cm³ de 2-méthyl-phénéthylamine. On obtient ainsi 42 mg de 3-({1-[bis-(4-chlorophényl)méthyl]-azétidin-3-ylidène}-méthanesulfonyl-méthyl)N-(2-phényl-propyl)benzamide sous forme d'une pâte jaune [Spectre de R.M.N. ¹H (400 MHz, (CD₃)₂SO d6, d en ppm) : 1,24 (d, J = 7 Hz : 3H); 2,97 (s : 3H); 3,07 (mt : 1H); de 3,20 à 3,50 (mt : 2H); 3,80 (mt : 2H); 4,23 (mt : 2H); 4,80 (s : 1H); de 7,10 à 7,40 (mt : 5H); 7,38 (d, J = 8 Hz : 4H); 7,47 (d, J = 8 Hz : 4H); de 7,50 à 7,60 (mt : 2H); 7,77 (s large : 1H); 7,79 (d large, J = 8 Hz : 1H); 8,55 (t, J = 6 Hz : 1H)].

### Exemple 140

On opère dans les conditions décrites dans l'exemple 124 à partir de 110 mg d'acide 3-({1-[bis-(4-chlorophényl)méthyl]-azétidin-3-ylidène-méthanesulfonyl-méthyl) benzoïque activé sur résine TFP (121 µM) et 0,020 cm³ de tétrahydrofurfurylméthylamine. On obtient ainsi 42 mg de 3-({1-[bis-(4-chlorophényl)méthyl]-azétidin-3-ylidène}-méthanesulfonyl-méthyl)N-(tetrahydrofuran-2-ylméthyl)benzamide sous forme d'une pâte jaune [Spectre de R.M.N. ¹H (400 MHz, (CD₃)₂SO d6, d en ppm) : 1,58 (mt : 1H); de 1,75 à 2,00 (mt : 3H); 2,98 (s : 3H); de 3,20 à 3,40 (mt : 2H); 3,63 (mt : 1H); 3,77 (mt : 1H); 3,82 (mt : 2H); 3,98 (mt : 1H); 4,23 (mt : 2H); 4,79 (s : 1H); 7,36 (d, J = 8 Hz : 4H); 7,46 (d, J = 8 Hz : 4H); de 7,50 à 7,60 (mt : 2H); 7,84 (s large : 1H); 7,88 (d large, J = 8 Hz : 1H); 8,60 (t, J = 6 Hz : 1H)].

### Exemple 141

On opère dans les conditions décrites dans l'exemple 124 à partir de 110 mg d'acide 3-({1-[bis-(4-chlorophényl)méthyl]-azétidin-3-ylidène}-méthanesulfonyl-méthyl) benzoïque activé sur résine TFP (121 µM) et 39 mg de 2,2-diphényléthylamine. On obtient ainsi 39 mg de 3-({1-[bis-(4-chlorophényl)méthyl]-azétidin-3-ylidène}-méthanesulfonyl-méthyl)N-(2,2-diphényl-éthyl)benzamide sous forme d'une pâte jaune [Spectre de R.M.N. ¹H (400 MHz, (CD₃)₂SO d6, d en ppm) : 2,95 (s : 3H); 3,77 (mt : 2H); 3,90 (dd, J = 8 et 6,5 Hz : 2H); 4,22 (mt : 2H); 4,42 (t, J = 8 Hz : 1H); 4,79 (s : 1H); de 7,10 à 7,40 (mt : 10H); 7,38 (d, J = 8,5 Hz : 4H);de 7,45 à 7,60 (mt : 2H); 7,48 (d, J = 8,5 Hz : 4H); 7,70 (mt : 2H); 8,56 (t, J = 6,5 Hz : 1H)].

### Exemple 142

On opère dans les conditions décrites dans l'exemple 124 à partir de 110 mg d'acide 3-( {1-[bis-(4-chlorophényl)méthyl]-azétidin-3-ylidène}-méthanesulfonyl-méthyl) benzoïque activé sur résine TFP (121 µM) et 19 mg de 2-éthyl-butylamine. On obtient ainsi 47 mg de 3-({1-[bis-(4-chlorophényl)méthyl]-azétidin-3-ylidène}-méthanesulfonyl-méthyl)N-(2-éthyl-butyl)benzamide sous forme d'une poudre jaune pâle [Spectre de R.M.N. ¹H (400 MHz, (CD₃)₂SO d6, d en ppm) : 0,86 (t, J = 7 Hz : 6H); de 1,20 à 1,40 (mt : 4H); 1,50 (mt : 1H); 2,98 (s : 3H); 3,19 (t, J = 6 Hz : 2H); 3,82 (mt : 2H); 4,24 (mt : 2H); 4,79 (s : 1H); 7,36 (d, J = 8,5 Hz : 4H); 7,46 (d, J = 8,5 Hz : 4H); de 7,45 à 7,60 (mt : 2H); 7,83 (s large : 1H); 7,86 (d large, J = 8 Hz : 1H); 8,42 (t, J = 6 Hz : 1H)].

### Exemple 143

On opère dans les conditions décrites dans l'exemple 124 à partir de 110 mg d'acide 3-({1-[bis-(4-chlorophényl)méthyl]-azétidin-3-ylidène}-méthanesulfonyl-méthyl) benzoïque activé sur résine TFP (121 µM), 0,026 cm³ de triéthylamine et 39 mg de chlrohydrate de l'ester méthylique de l'acide 4-aminométhyl-cyclohexanecarboxylique. On obtient ainsi 47 mg de l'ester méthylique de l'acide 4-{[3-({1-[bis-(4-chlorophényl)méthyl]-azétidin-3-ylidène}-méthanesulfonyl-méthyl)benzoylamino]-méthyl}-cyclohexanecarboxylique sous forme d'une pâte jaune pâle [Spectre de R.M.N. ¹H (400 MHz, (CD₃)₂SO d6, d en ppm) : de 0,90 à 1,05 (mt : 2H); de 1,20 à 1,40 (mt : 2H); 1,52 (mt : 1H); de 1,70 à 2,00 (mt : 4H); 2,27 (mt : 1H); 2,98 (s : 3H); 3,12 (t, J = 6,5 Hz : 2H); 3,60 (s : 3H); 3,80 (mt : 2H); 4,23 (mt : 2H); 4,79 (s : 1H); 7,36 (d, J = 8 Hz : 4H); 7,46 (d, J = 8 Hz : 4H); de 7,45 à 7,60 (mt : 2H); 7,83 (s large : 1H); 7,87 (d large, J = 8 Hz : 1H); 8,50 (t, J = 6 Hz : 1H)].

L'acide 3-({1-[bis-(4-chlorophényl)méthyl]-azétidin-3-ylidène}-méthanesulfonylméthyl)benzoïque activé sur résine TFP peut être préparé dans les conditions suivantes : A une suspension de 1,07 g de résine TFP (fonction phénol libre, 1,1 mmole/g, soit 1,17 mM) dans 15 cm³ de diméthylformamide anhydre, préagitée pendant 10 minutes à une température voisine de 20°C, est ajoputé, à la même température, 1,18 g d'acide 3-({1-[bis-(4-chlorophényl)méthyl]-azétidin-3-ylidène}-méthanesulfonyl-méthyl)benzoïque. Après 10 minutes d'agitation à une température voisine de 20°C, on ajoute 14 mg de 4-diméthylaminopyridine, puis après 10 minutes d'agitation à la mêmetempérature, 0,185 cm³ de 1,3-diisopropylcarbodiimide. Après 23 heures d'agitation à une température voisine de 20°C, la suspension est filtrée, la résine est lavée avec 45 cm³ de diméthylformamide, 45 cm³ de tétrahydrofuranne, 45 cm³ de dichlorométhane, puis séchée sous vide à poids constant. On obtient ainsi 1,5 g d'acide 3-({1-[bis-(4-chlorophényl)méthyl]-azétidin-3-ylidène}-méthanesulfonylméthyl)benzoïque activé sur résine TFP sous forme d'une résine jaune pâle. La résine TFP (structure ci-dessous) peut être préparée de la manière suivante :

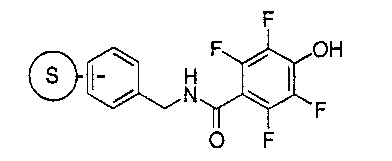

A une suspension de 2 g de résine aminométhyl polystyrène commerciale (0,39 mmol/g; 0,78 mmol) dans 15 cm³ de diméthylformamide, pré-agitée 5 minutes à une température voisine de 20°C, on ajoute successivement 492 mg de diisopropylcarbodiimide, 819,3 mg d'acide 2,3,5,6-tétrafluoro-4-hydroxy-benzoïque et 50 mg de 4-diméthylaminopyridine. Après environ 20 heures d'agitation, à une température voisine de 20°C, la suspension est filtrée, et la résine est rincée avec 3 fois 20 cm³ de diméthylformamide, 3 fois 20 cm³ de tétrahydrofuranne et 3 fois 20 cm³ de dichlorométhane. La résine obtenue est séchée sous pression réduite à une température voisine de 40°C. La résine obtenue est ensuite agitée, à une température voisine de 20°C, pendant environ 20 heures, en suspension dans un mélange pipéridine/diméthylformamide (10/90 en volumes). La suspension est filtrée, et la résine est rincée avec 3 fois 20 cm³ de diméthylformamide, 3 fois 20 cm³ de tétrahydrofuranne et 3 fois 20 cm³ de dichlorométhane. La résine obtenue est séchée sous pression réduite à une température voisine de 40°C et est utilisée en l'état.

### Exemple 144

Une solution de 76 mg de l'ester ter-butylique de l'acide (2-{4-[3-({1-[bis-(4-chlorophényl)méthyl]-azétidin-3-ylidène}-méthanesulfonyl-méthyl)phényl]-piperazin-1-yl}-2-oxo-éthyl)carbamique dans 2,5 cm³ d'acide formique est agitée 1 heure à une température voisine de 45°C. Le milieu réactionnel est concentré à sec sous pression réduite (5 kPa) à une température voisine de 30°C, repris avec 10 cm³ d'acétate d'éthyle et alcalinisé avec 10 cm³ d'une solution aqueuse saturée en bicarbonate de sodium. Après décantation, la phase organique est lavée avec 10 cm³ d'eau, séchée sur sulfate de magnésium, filtrée, et concentré à sec sous pression réduite (1 kPa) à une température voisine de 40°C. On obtient ainsi 51 mg de 2-amino-1-{4-[3-({1-[bis-(4-chlorophényl)méthyl]-azétidin-3-ylidène}-méthanesulfonyl-méthyl)phényl]-piperazin-1-yl}-éthanone sous forme d'une laque beige [Spectre de R.M.N. ¹H (300 MHz, CDCl₃, d en ppm) : de 1,95 à 2,25 (mf étalé : 2H); 2,77 (s : 3H); de 3,10 à 3,30 (mt : 4H); de 3,50 à 3,60 (mt : 2H); 3,56 (s large : 2H); de 3,75 à 3,90 (mt : 4H); 4,34 (mt : 2H); 4,50 (s : 1H); 6,84 (d large, J = 8 Hz : 1H); 6,91 (dd, J = 8 et 2 Hz : 1H); 7,01 (mt : 1H); de 7,20 à 7,40 (mt : 9H)].

### Exemple 145

A 108,5 mg de 1-[3-({1-[bis-(4-chlorophényl)méthyl]-azétidin-3-ylidène}-méthanesulfonyl-méthyl)phényl]-piperazine est ajouté successivement, à une température voisine de 20°C, 1,02 g d'EDCI supporté (5 mM), 44 mg de N-Boc-glycine puis 10 cm³ de dichlorométhane, Après 20 heures d'agitation, à une température voisine de 20°C, le mélange réactionnel est filtré sur verre fritté. La résine est rincée avec 3 fois 5 cm³ de dichlorométhane. Les filtrats réunis sont lavés avec 20 cm³ d'eau, séchés sur sulfate de magnésium, filtrés sur verre fritté, et concentrés à sec sous pression réduite (1 kPa) à une température voisine de 40°C. On obtient ainsi 143 mg de l'ester ter-butylique de l'acide (2-{4-[3-({1-[bis-(4-chlorophényl)méthyl]-azétidin-3-ylidène}-méthanesulfonyl-méthyl)phényl]-piperazin-1-yl}-2-oxo-éthyl)carbamique sous forme d'une laque crème [Spectre de R.M.N. ¹H (400 MHz, (CD₃)₂SO d6, d en ppm) : 1,40 (s : 9H); 2,93 (s : 3H); de 3,05 à 3,20 (mt : 4H); 3,57 (mt : 4H); 3,80 (mt : 2H); 3,84 (d, J = 6 Hz : 2H); 4,19 (mt : 2H); 4,78 (s : 1H); 6,79 (t, J = 6 Hz : 1H); 6,82 (d, J = 8 Hz : 1H); 6,93 (s large : 1H); 6,99 (dd, J = 8 et 2,5 Hz : 1H); 7,27 (t, J = 8 Hz : 1H); 7,36 (d, J = 8 Hz : 4H); 7,46 (d, J = 8 Hz : 4H)].

Le réactif EDCI supporté est commercial, et peut être également préparé selon la référence suivante : M. Desai, L. Stramiello, *Tetrahedron Letters, 34,* 48, 7685-7688 (1993).

### Exemple 146

Une solution de 81 mg de l'ester ter-butylique de l'acide (2-{4-[3-({1-[bis-(4-chlorophényl)méthyl]-azétidin-3-ylidène}-méthanesulfonyl-méthyl)phényl]-piperazin-1-yl}-2-oxo-éthyl)N-méthyl-carbamique dans 2,5 cm³ d'acide formique est agitée 1 heure à une température voisine de 45°C. Le milieu réactionnel est concentré à sec sous pression réduite (5 kPa) à une température voisine de 30°C, repris avec 10 cm³ d'acétate d'éthyle et alcalinisé avec 10 cm³ d'une solution aqueuse saturée en bicarbonate de sodium. Après décantation, la phase organique est lavée avec 10 cm³ d'eau, séchée sur sulfate de magnésium, filtrée, et concentré à sec sous pression réduite (1 kPa) à une température voisine de 40°C. On obtient ainsi 58 mg de 1-{4-[3-( {1-[bis-(4-chlorophényl)méthyl]-azétidin-3-ylidène -méthanesulfonylméthyl)phényl]-piperazin-1-yl}-2-méthylamino-éthanone sous forme d'une laque beige[Spectre de R.M.N. ¹H (300 MHz, CDCl₃, d en ppm) : de 1,95 à 2,15 (mf étalé : 1H); 2,51 (s large : 3H); 2,77 (s : 3H); de 3,10 à 3,30 (mt : 4H); 3,49 (s large : 2H); 3,58 (mt : 2H); de 3,75 à 3,90 (mt : 4H); 4,33 (mt : 2H); 4,49 (s : 1H); 6,83 (d large, J = 8 Hz : 1H); 6,90 (dd, J = 8 et 2 Hz : 1H); 7,00 (mt : 1H); de 7,20 à 7,40 (mt : 9H)].

### Exemple 147

A 108,5 mg de 1-[3-({1-[bis-(4-chlorophényl)méthyl]-azétidin-3-ylidène}-méthanesulfonyl-méthyl)phényl]-piperazine est ajouté successivement, à une température voisine de 20°C, 1,02 g d'EDCI supporté (5 mM), 47,3 mg de N-Boc-sarcosine puis 10 cm³ de dichlorométhane. Après 20 heures d'agitation, à une température voisine de 20°C, le mélange réactionnel est filtré sur verre fritté. La résine est rincée avec 3 fois 5 cm³ de dichlorométhane. Les filtrats réunis sont lavés avec 20 cm³ d'eau, séchés sur sulfate de magnésium, filtrés sur verre fritté, et concentrés à sec sous pression réduite (1 kPa) à une température voisine de 40°C. On obtient ainsi 143 mg de l'ester ter-butylique de l'acide (2-{4-[3-({1-[bis-(4-chlorophényl)méthyl]-azétidin-3-ylidène}-méthanesulfonyl-méthyl)phényl]-piperazin-1-yl}-2-oxo-éthyl)N-méthyl-carbamique sous forme d'une laque crème [Spectre de R.M.N ¹H (400 MHz, (CD₃)₂SO d6, d en ppm). Nous observons un mélange de rotamères à température ambiante; 1,31 et 1,41 (2s : 9H en totalité); 2,78 et 2,81 (2s : 3H en totalité); 2,93 (2s : 3H); de 3,10 à 3,25 (mf : 4H); de 3,45 à 3,65 (mt : 4H); 3,80 (mt : 2H); 4,06 et 4,09 (2s : 2H en totalité); 4,19 (mt : 2H); 4,78 (s : 1H); 6,83 (d large, J = 8 Hz : 1H); 6,93 (s large : 1H); 7,00 (dd, J = 8 et 2,5 Hz : 1H); 7,27 (t, J = 8 Hz : 1H); 7,36 (d, J = 8 Hz : 4H); 7,46 (d, J = 8 Hz : 4H)].

### Exemple 148

A 54,25 mg de 1-[3-({1-[bis-(4-chlorophényl)méthyl]-azétidin-3-ylidène}-méthanesulfonyl-méthyl)phényl]-piperazine est ajouté successivement, à une température voisine de 20°C, 2 cm³ de dichlorométhane puis 11 mg d'isothiocyanate de méthyle. Après 6 heures d'agitation, à une température voisine de 20°C, on ajoute au mélange réactionnel 0,05 cm³ d'eau. Après 15 minutes d'agitation à la même température, le milieu réactionnel est séché sur sulfate de magnésium, filtré, et concentré à sec sous pression réduite (1 kPa) à une température voisine de 40°C. On obtient ainsi 61 mg du N-méthylamide de l'acide 4-[3-({1-[bis-(4-chlorophényl)méthyl]-azétidin-3-ylidène}-méthanesulfonyl-méthyl)phényl]-piperazine-1-carbothioic sous forme d'une laque beige [Spectre de R.M.N. ¹H (400 MHz, CDCl₃, d en ppm) : 2,77 (s : 3H); 3,20 (d, J = 5 Hz : 3H); 3,32 (t, J = 5,5 Hz : 4H); 3,81 (mt : 2H); 4,00 (t, J = 5,5 Hz : 4H); 4,33 (mt : 2H); 4,49 (s : 1H); 5,63 (q large, J = 5 Hz : 1H); 6,80 (d, J = 8 Hz : 1H); 6,85 (dd, J = 8 et 2,5 Hz : 1H); 6,94 (s large : 1H); de 7,20 à 7,30 (mt : 5H); 7,32 (d, J = 8Hz:4H)].

### Exemple 149

A 54,25 mg de 1-[3-({1-[bis-(4-chlorophényl)méthyl]-azétidin-3-ylidène}-méthanesulfonyl-méthyl)phényl]-piperazine est ajouté successivement, à une température voisine de 20°C, 2 cm³ de dichlorométhane puis 11,5 mg d'isocyanate de méthyle. Après 4 heures d'agitation, à une température voisine de 20°C, on ajoute au mélange réactionnel 0,05 cm³ d'eau. Après 15 minutes d'agitation à la même température, le milieu réactionnel est séché sur sulfate de magnésium, filtré sur papier, et concentré à sec sous pression réduite (1 kPa) à une température voisine de 40°C. On obtient ainsi 66 mg du N-méthylamide de l'acide 4-[3-({1-[bis-(4-chlorophényl)méthyl]-azétidin-3-ylidène}-méthanesulfonyl-méthyl)phényl]-piperazine-1-carboxylique sous forme d'une laque beige [Spectre de R.M.N ¹H (400 MHz, CDCl₃, d en ppm) : 2,75 (s : 3H); 2,85 (d, J = 5 Hz : 3H); 3 ,19 (t large, J = 5,5 Hz : 4H); 3,52 (t large, J = 5,5 Hz : 4H); 3,80 (mt : 2H); 4,33 (mt : 2H); 4,45 (q large, J = 5 Hz : 1H); 4,49 (s : 1H); 6,81 (d, J = 8 Hz : 1H); 6,89 (dd, J = 8 et 2,5 Hz : 1H); 6,98 (s large : 1H); de 7,20 à 7,30 (mt : 5H); 7,32 (d, J = 8 Hz : 4H)].

### Exemple 150

A 54,25 mg de 1-[3-({1-[bis-(4-chlorophényl)méthyl]-azétidin-3-ylidène}-méthanesulfonyl-méthyl)phényl]-piperazine est ajouté successivement, à une température voisine de 20°C, 2 cm³ de pyridine puis 10,4 mg de chloroformate de méthyle. Après 6 heures d'agitation, à une température voisine de 20°C, le milieu réactionel est concentré à sec sous pression réduite (5 kPa) à une température voisine de 30°C. Le résidu obtenu est repris avec 5 cm³ d'acétate d'éthyle et 5 cm³ d'eau. Après décantation, la phase organique est lavée avec 2 cm³ d'eau, séchée sur sulfate de magnésium, filtrée, et concentrée à sec sous pression réduite (1 kPa) à une température voisine de 40°C. On obtient ainsi 62 mg de l'ester de méthyl de l'acide 4-[3-({1-[bis-(4-chlorophényl)méthyl]-azétidin-3-ylidène}-méthanesulfonyl-méthyl)phényl]-piperazine-1-carboxylique sous forme d'une laque beige [Spectre de R.M.N. ¹H (400 MHz, CDCl₃, d en ppm) : 2,75 (s : 3H); 3,15 (t large, J = 5,5 Hz : 4H); 3,62 (mt : 4H); 3,74 (s : 3H); 3,80 (mt : 2H); 4,32 (mt : 2H); 4,49 (s : 1H); 6,81 (d, J = 8 Hz : 1H); 6,90 (dd, J = 8 et 2,5 Hz : 1H); 6,99 (s large : 1H); de 7,20 à 7,40 (mt : 9H)].

### Exemple 151

A une solution de 54,25 mg de 1-[3-({1-[bis-(4-chlorophényl)méthyl]-azétidin-3-ylidène}-méthanesulfonyl-méthyl)phényl]-piperazine dans 2 cm³ de 1,2-dichloroéthane est ajouté successivement, à une température voisine de 20°C, 32 mg d'acétoxyborohydrure de sodium puis 22 mg d'isobutyraldéhyde. Après 4 heures d'agitation, à une température voisine de 20°C, on ajoute au milieu réactionnel 3 cm³ de dichlorométhane et 2 cm³ d'une solution aqueuse saturée en bicarbonate de sodium. Après décantation, la organique est séchée sur sulfate de magnésium, filtrée, et concentrée à sec sous pression réduite (1 kPa) à une température voisine de 40°C. On obtient ainsi 63 mg de la 1-[3-({1-[bis-(4-chlorophényl)méthyl]-azétidin-3-ylidène}-méthanesulfonyl-méthyl)phényl]-4-isobutyl-piperazine sous forme d'une laque beige [Spectre de R.M.N ¹H (400 MHz, CDCl₃, d en ppm) : 0,92 (d, J = 7 Hz : 6H); 1,82 (mt : 1H); 2,14 (d, J = 8 Hz : 2H); 2,54 (t, J = 5,5 Hz : 4H); 2,75 (s : 3H); 3,18 (t, J = 5,5 Hz : 4H); 3,81 (mt : 2H); 4,32 (mt : 2H); 4,49 (s : 1H); 6,78 (d, J = 8 Hz : 1H); 6,89 (dd, J = 8 et 2,5 Hz : 1H); 6,97 (s large : 1H); de 7,15 à 7,30 (mt : 5H); 7,32 (d, J = 8 Hz : 4H)].

### Exemple 152

A une solution de 54 mg de 1-[3-({1-[bis-(4-chlorophényl)méthyl]-azétidin-3-ylidène}-méthanesulfonyl-méthyl)phényl]-piperazine dans 2 cm³ de 1,2-dichloroéthane est ajouté successivement, à une température voisine de 20°C, 32 mg d'acétoxyborohydrure de sodium puis 13 mg d'acétaldéhyde. Après 21 heures d'agitation, à une température voisine de 20°C, on ajoute au milieu réactionnel 2 cm³ d'une solution aqueuse saturée en bicarbonate de sodium. Après décantation, la phase aqueuse est réextraite avec 2 cm³ de dichlorométhane. Les phases organiques rassemblées sont séchées sur sulfate de magnésium, filtrées, et concentrées à sec sous pression réduite (1 kPa) à une température voisine de 20°C. On obtient ainsi 60 mg d'un résidu solide qui est repris avec 2 cm³ de méthanol et 0,5 cm³ de dichlorométhane. La solution obtenue est déposée sur une cartouche de silice (500 mg de phase SCX). La cartouche est lavée avec 5 cm³ de méthanol, puis le produit attendu est élué avec 5 cm³ de méthanol ammoniacal (2N) puis 5 cm³ de méthanol supplémentaire. Le filtrat est concentré à sec sous pression réduite (1 kPa) à une température voisine de 30°C. On obtient ainsi 42 mg de la 1-[3-({1-[bis-(4-chlorophényl)méthyl]-azétidin-3-ylidène}-méthanesulfonyl-méthyl)phényl]-4-éthyl-piperazine sous forme d'une laque incolore [Spectre de R.M.N. ¹H (300 MHz, CDCl₃, d en ppm) : 1,14 (t, J = 7,5 Hz : 3H); 2,48 (q, J = 7,5 Hz : 2H); 2,60 (t large, J = 5 Hz : 4H); 2,77 (s : 3H); 3,22 (t large, J = 5 Hz : 4H); 3,82 (mt : 2H); 4,33 (mt : 2H); 4,49 (s : 1H); 6,79 (d large, J = 8 Hz : 1H); 6,91 (dd, J = 8 et 2 Hz : 1H); 6,98 (mt : 1H); de 7,20 à 7,40 (mt : 9H)].

### Exemple 153

A 54 mg de 1-[3-({1-[bis-(4-chlorophényl)méthyl]-azétidin-3-ylidène}-méthanesulfonyl-méthyl)phényl]-piperazine est ajouté successivement, à une température voisine de 20°C, 2 cm³ de pyridine puis 11,5 mg d'anhydride acétique. Après 23 heures d'agitation, à une température voisine de 20°C, le milieu réactionel est concentré à sec sous pression réduite (1 kPa) à une température voisine de 30°C. Le résidu obtenu est repris avec 5 cm³ d'acétate d'éthyle et 2 cm³ d'eau. Après décantation, la phase organique est lavée avec 2 cm³ d'eau, séchée sur sulfate de magnésium, filtrée, et concentrée à sec sous pression réduite (1 kPa) à une température voisine de 40°C. On obtient ainsi 52 mg de 4-acétyl 1-[3-({1-[bis-(4-chlorophényl)méthyl]-azétidin-3-ylidène}-méthanesulfonyl-méthyl)phényl]-piperazine sous forme d'une meringue beige [Spectre de R.M.N. ¹H (300 MHz, CDCl₃, d en ppm) : 2,16 (s : 3H); 2,77 (s : 3H); de 3,10 à 3,25 (mt : 4H); 3,63 (t large, J = 5,5 Hz : 2H); 3,78 (t large, J = 5,5 Hz : 2H); 3,82 (mt : 2H); 4,34 (mt : 2H); 4,50 (s : 1H); 6,84 (d large, J = 8 Hz : 1H); 6,92 (dd, J = 8 et 2 Hz : 1H); 7,02 (mt : 1H); de 7,20 à 7,40 (mt : 9H)].

### Exemple 154

A 54 mg de 1-[3-({1-[bis-(4-chlorophényl)méthyl]-azétidin-3-ylidène}-méthanesulfonyl-méthyl)phényl]-piperazine est ajouté successivement, à une température voisine de 20°C, 511 mg d'EDCI supporté (2,5 mM), 11,5 mg de N,N-diméthylglycine puis 5 cm³ de dichlorométhane. Après 24 heures d'agitation, à une température voisine de 20°C,on ajoute 35 mg de N,N-diméthylglycine. Après 96 heures d'agitation, à une température voisine de 20°C, le mélange réactionnel est filtré sur verre fritté. La résine est rincée avec 3 fois 2,5 cm³ de dichlorométhane. Les filtrats réunis sont lavés avec 10 cm³ d'eau, séchés sur sulfate de magnésium, filtrés, et concentrés à sec sous pression réduite (5 kPa) à une température voisine de 20°C. On obtient ainsi 53 mg de la 1-{4-[3-({1-[bis-(4-chlorophényl)méthyl]-azétidin-3-ylidène}-méthanesulfonylméthyl)phényl]-piperazin-1-yl}-2-diméthylamino-ethanone sous forme d'une meringue beige [Spectre de R.M.N. ¹H (400 MHz, (CD₃)₂SO d6, d en ppm) : 2,20 (s : 6H); 2,94 (s : 3H); 3,12 (s : 2H); 3,16 (mt : 4H); 3,58 (mt : 2H); 3,68 (mt : 2H); 3,80 (mt : 2H); 4,19 (mt : 2H); 4,78 (s : 1H); 6,81 (d large, J = 8 Hz : 1H); 6,93 (s large : 1H); 6,99 (dd, J = 8 et 2,5 Hz : 1H); 7,26 (t, J = 8 Hz : 1H); 7,36 (d, J = 8 Hz : 4H); 7,46 (d, J = 8 Hz : 4H)].

### Exemple 155

Une solution de 320 mg de l'ester tert-butylique de l'acide 4-[3-({1-[bis-(4-chlorophényl)méthyl]azétidin-3-ylidène}méthanesulfonylméthyl)phényl]pipérazine-1-carboxylique dans 5 cm³ d'acide formique est agitée 5 heures à une température voisine de 20°C, puis 1 heure à une température voisine de 45°C. Le milieu réactionnel est concentré à sec sous pression réduite (5 kPa) à une température voisine de 30°C, repris avec 20 cm³ d'acétate d'éthyle et alcalinisé avec 10 cm³ d'une solution aqueuse saturée en bicarbonate de sodium. Après décantation, la phase organique est lavée avec 3 fois 10 cm³ d'eau, séchée sur sulfate de magnésium, filtrée, et concentré à sec sous pression réduite (1 kPa) à une température voisine de 40°C. Le résidu obtenu est purifié par dépôt en solution dans un minimum de dichlorométhane sur gel de silice déposé sur plaque [(gel de 0,5 mm d'épaisseur, 5 plaques de 20 x 20 cm, éluant : dichlorométhane-méthanol (80-20 en volumes)]. La zone correspondant au produit cherché adsorbé, localisée aux rayons U.V., est grattée et la silice recueillie est lavée sur verre fritté par un mélange dichlorométhane-méthanol (75-25 en volumes). Les filtrats sont réunis et concentrés à sec sous pression réduite (1 kPa) à une température voisine de 30°C. On obtient ainsi 180 mg de 1-[3-({1-[bis-(4-chlorophényl)méthyl]azétidin-3-ylidène}méthanesulfonyl-méthyl)phényl]pipérazine sous forme d'une poudre blanche [Spectre de R.M.N. ¹H (300 MHz, CDCl₃, δ en ppm) : 2,77 (s : 3H); 3,05 (mt : 4H); 3,16 (mt : 4H); 3,81 (mt : 2H); 4,33 (mt : 2H); 4,49 (s : 1H); 6,79 (d large, J = 8 Hz : 1H); 6,90 (dd, J = 8 et 2,5 Hz : 1H); 6,98 (mt : 1H); de 7,20 à 7,40 (mt : 9H)].

L'ester tert-butylique de l'acide 4-[3-({1-[bis-(4-chlorophényl)méthyl]azétidin-3-ylidène}méthanesulfonylméthyl)phényl]pipérazine-1-carboxylique peut être préparé de la manière suivante : en opérant selon le mode opératoire de l'exemple 4 à partir de 1,32 g de l'ester tert-butylique de l'acide 4-[3-({1-[bis-(4-chlorophényl)méthyl]-3-hydroxy-azétidin-3-yl}-méthanesulfonyl-méthyl)phényl]pipérazine-1-carboxylique, de 0,232 cm³ de chlorure de méthanesulfonyle et 0,733 g de 4-diméthylaminopyridine, le résidu obtenu est purifié par chromatographie sur colonne de gel de silice (granulométrie 0,063-0,200 mm, diamètre 2 cm, hauteur 25 cm) à pression atmosphérique avec un mélange dichlorométhane-méthanol (99,5-0,5 en volumes) comme éluant et en recueillant des fractions de 15 cm³. Les fractions contenant le produit recherché sont réunies et concentrées à sec sous pression réduite (5 kPa) à une température voisine de 30°C. On obtient ainsi 0,86 g de l'ester tert-butylique de l'acide 4-[3-({1-[bis-(4-chlorophényl)méthyl]azétidin-3-ylidène}-méthanesulfonyl-méthyl)phényl]pipérazine-1-carboxylique sous forme d'une meringue blanche [Spectre de R.M.N ¹H (400 MHz, CDCl₃, δ en ppm) : 1,50 (s : 9H); 2,77 (s : 3H); 3,14 (t, J = 5 Hz : 4H); 3,57 (t, J = 5 Hz : 4H); 3,81 (mt : 2H); 4,34 (mt : 2H); 4,49 (s : 1H); 6,81 (d, J = 8 Hz : 1H); 6,90 (dd, J = 8 et 2,5 Hz : 1H); 6,99 (s large : 1H); de 7,20 à 7,30 (mt : 5H); 7,32 (d, J = 8 Hz : 4H)].

L'ester tert-butylique de l'acide 4-[3-({1-[bis-(4-chlorophényl)méthyl]-3-hydroxy-azétidin-3-yl}-méthanesulfonyl-méthyl)phényl]pipérazine-1-carboxylique peut être préparé selon la manière suivante : en opérant selon le mode opératoire de l'exemple 1 à partir de 0,886 g de l'ester tert-butylique de l'acide 4-(3-méthanesulfonylméthylphényl)pipérazine-1-carboxylique, de 0,765 g de 1-[bis(4-chlorophényl)méthyl]azétidin-3-one et 1,72 cm³ d'une solution 1,6 M de n.butyllithium dans l'hexane, on obtient 1,37 g de l'ester tert-butylique de l'acide 4-[3-({1-[bis-(4-chlorophényl)méthyl]-3-hydroxy-azétidin-3-yl}-méthanesulfonylméthyl)phényl]pipérazine-1-carboxylique sous forme d'une poudre beige.

L'ester tert-butylique de l'acide 4-(3-méthanesulfonylméthylphényl)pipérazine-1-carboxylique peut être préparé selon la manière suivante : en opérant selon le mode opératoire de l'exemple 10 à partir de 1,55 g de l'ester tert-butylique de l'acide 4-(3-chlorométhylphényl)pipérazine-1-carboxylique et 0,766 g de méthanesulfinate de sodium, on obtient 0,9 g de l'ester tert-butylique de l'acide 4-(3-méthanesulfonylméthylphényl)pipérazine-1-carboxylique sous forme d'une poudre beige.

L'ester tert-butylique de l'acide 4-(3-chlorométhylphényl)pipérazine-1-carboxylique peut être préparé selon la manière suivante : par réaction de 16,4 g de l'ester tert-butylique de l'acide 4-(3-hydroxyméthylphényl)pipérazine-1-carboxylique dans 150 cm³ de dichlorométhane, à une température voisine de 20°C, avec 29 cm³ de diisopropyléthylamine et 8,7 cm³ de chlorure de méthanesulfonyle, on obtient après purification sur colonne de chromatographie (silice 0,063-0,200 mm, diamètre 6 cm, hauteur 45 cm, fractions de 100 cm³) en éluant avec du dichlorométhane, 15 g de l'ester tert-butylique de l'acide 4-(3-chlorométhylphényl)pipérazine-1-carboxylique sous forme d'une poudre beige.

L'ester tert-butylique de l'acide 4-(3-hydroxyméthylphényl)pipérazine-1-carboxylique peut être préparé de la manière suivante : par réaction de 15,8 g d'un mélange d'esters tert-butyliques des acides 4-(3-éthoxycarbonylphényl)pipérazine-1-carboxylique et 4-(3-n.butyloxycarbonylphényl)pipérazine-1-carboxylique en solution dans 500 cm³ de THF anhydre, à une température voisine de -10°C, et 102 cm³ d'hydrure de diisobutylaluminium en solution dans le toluène (20% en poids), on obtient 12,8 g de l'ester tert-butylique de l'acide 4-(3-hydroxyméthylphényl)pipérazine-1-carboxylique sous forme d'une huile beige.

Le mélange d'esters tert-butyliques des acides 4-(3-éthoxycarbonylphényl)pipérazine-1-carboxylique et 4-(3-n.butyloxycarbonylphényl)pipérazine-1-carboxylique peut être préparé selon la méthode décrite dans le brevet WO 9726250.

### Exemple 156

En opérant selon l'exemple 38 (méthode 2), à partir de 0,3 g de 3-acétoxy-1-[bis(4-méthoxycarbonylphényl)méthyl]-3-[(3,5-difluorophényl)(méthylsulfonyl)méthyl-(RS)]azétidine et 105 mg d'hydroxyde de lithium monohydrate dans 10 cm³ d'acétonitrile, à une température voisine de 70°C, on obtient 0,24 g de 1-[bis(4-méthoxycarbonylphényl)méthyl]-3-[(3,5-difluorophényl)(méthylsulfonyl)méthylène]azétidine sous forme d'une meringue orange [Spectre de R.M.N. ¹H (300 MHz, CDCl₃, δ en ppm) : 2,81 (s : 3H); de 3,85 à 3,95 (mt : 2H); 3,89 (s : 6H); 4,37 (mt : 2H); 4,67 (s : 1H); 6,84 (tt, J = 9 et 2,5 Hz : 1H); 6,99 (mt : 2H); 7,50 (d, J = 8 Hz : 4H); 7,97 (d, J = 8 Hz : 4H)].

### Exemple 157

En opérant selon le mode opératoire de l'exemple 40 à partir de 4,45 g de (3,5-difluorobenzyl)méthylsulfone, 6,36 g de 1-[bis(4-méthoxycarbonylphényl)méthyl]azétidin-3-one, 2,18 cm³ de chlorure d'acétyle et 17 cm³ d'une solution 1,6 M de n.butyllithium dans l'hexane, on obtient 10,8 g de 3-acétoxy-1-[bis(4-méthoxycarbonylphényl)méthyl]-3-[(3,5-difluorophényl)(méthylsulfonyl)méthyl-(RS)]azétidine sous forme d'une meringue jaune pâle [Spectre de R.M.N. ¹H (400 MHz, (CD₃)₂SO d6, δ en ppm) : 2,03 (s : 3H); 2,96 (s : 3H); de 3,25 à 3,40 (mt : 2H); 3,52 (d large, J = 8 Hz : 1H); de 3,75 à 3,90 (mt : 1H); 3,82 (s : 3H); 3,83 (s : 3H); 4,72 (s : 1H); 5,36 (s : 1H); 7,27 (d, J = 8 Hz : 2H); de 7,35 à 7,45 (mt : 2H); 7,43 (d, J = 8 Hz : 2H); 7,54 (tt, J = 9,5 et 2,5 Hz : 1H); 7,81 (d, J = 8 Hz : 2H); 7,88 (d, J = 8 Hz : 2H)].

La 1-[bis(4-méthoxycarbonylphényl)méthyl]azétidin-3-one peut être préparée de la même manière que la 1-{(R*)-(4-chlorophényl)[4-(méthoxycarbonyl)phényl]méthyl} azétidin-3-one (exemple 110) à partir du 1-[bis(4-méthoxycarbonylphényl)méthyl]azétidin-3-ol.

Le 1-[bis(4-méthoxycarbonylphényl)méthyl]azétidin-3-ol peut être préparé de la même manière que la 1-{(R*)-(4-chlorophényl)[4-(méthoxycarbonyl)phényl]méthyl} azétidin-3-ol (exemple 110) à partir de la bis(4-méthoxycarbonylphényl)méthylamine.

La bis(4-méthoxycarbonylphényl)méthylamine peu être préparée de la même manière que le 4-[(RS)-amino-(4-chlorophényl)méthyl]benzoate de méthyle (exemple 87) à partir de la 4,4'-diméthoxycarbonyl-benzophénone.

### Exemple 158

En opérant selon le mode opératoire de l'exemple 110, à partir de 40 mg de (RS)-1-{[4-(chlorométhyl)phényl](4-chlorophényl)méthyl}-3-[(3,5-difluomphényl)(méthylsulfonyl)méthyl)méthylsulfonyl méthylène]azétidine, 0,25 cm³ de dichlorométhane et 0,0196 cm³ de morpholine, on obtient 35,8 mg de (RS)-4-[4-((4-chlorophényl) {3-[(3,5-difluorophényl)méthanesulfonyl-méthylene]azétidin-1-yl}-méthyl)benzyl]morpholine sous forme d'une meringue blanche [Spectre de R.M.N. ¹H (400 MHz, CDCl₃, δ en ppm) : 2,41 (mt : 4H); 2,80 (s : 3H); 3,42 (s : 2H); 3,69 (t, J = 4,5 Hz : 4H); 3,84 (mt : 2H); 4,33 (mt : 2H); 4,50 (s : 1H); 6,83 (tt, J = 9 et 2,5 Hz : 1H); 6,98 (mt : 2H); de 7,20 à 7,40 (mt : 8H)].

La (RS)-1-{[4-(chlorométhyl)phényl](4-chlorophényl)méthyl}-3-[(3,5-difluorophényl)(méthylsulfonyl)méthyl)méthylsulfonyl méthylène]azétidine peut être préparée de la manière suivante : en opérant selon l'exemple 87, à partir de 415 mg de (RS)-1-{(4-chlorophényl)[4-(hydroxyméthyl)phényl]méthyl }-3-[(3,5-difluorophényl)(méthylsulfonyl)méthylène]azétidine, 5 cm³ de dichlorométhane, 0,19 cm³ de chlorure de méthane sulfonyle, et 0,53 cm³ de diisopropyléthylamine, on obtient 421,2 mg de (RS)-1-{[4-(chlorométhyl)phényl](4-chlorophényl)méthyl}-3-[(3,5-difluorophényl)(méthylsulfonyl)méthyl)méthylsulfonyl méthylène]azétidine sous forme d'une meringue crème.

### Exemple 159

A une solution de 33 mg de 1-benzhydryl-3-{[3-(4-bromo-butoxy)phényl]méthanesulfonyl-méthylene}-azétidine dans 2 cm³ d'acétonitrile anhydre, sont ajoutés successivement, à une température voisine de 20°C, sous atmosphère inerte d'argon, 25 mg de carbonate de potassium puis 0,016 cm³ de morpholine. Après 17 heures d'agitation à une température voisine de 20°C, le milieu réactionnel est dilué avec 10 cm³ d'acétate d'éthyle et 4 cm³ d'eau. La phase organique, séparée, est lavée avec 4 cm³ d'une solution aqueuse saturée en chlorure de sodium, séchée sur sulfate de magnésium, filtrée sur verre fritté, puis concentrée à sec sous pression réduite (1 kPa) à une température voisine de 40°C. Le résidu obtenu est purifié par dépôt en solution dans un minimum de dichlorométhane sur chromatographie sur gel de silice déposé sur plaque [(gel de 0,5 mm d'épaisseur, 2 plaques de 20 x 20 cm, éluant : dichlorométhane-méthanol (92,5-7,5 en volumes)]. La zone correspondant au produit cherché adsorbé, localisée aux rayons U.V., est grattée et la silice recueillie est lavée sur verre fritté par un mélange dichlorométhane-méthanol (80-20 en volumes). Les filtrats sont réunis et concentrés à sec sous pression réduite (1 kPa) à une température voisine de 40°C. On obtient ainsi 25,2 mg de 4-(4-{3-[(1-benzhydryl-azétidin-3-ylidene)méthanesulfonylméthyl]phénoxy}-butyl)morpholine sous forme d'une meringue jaune pâle [Spectre de R.M.N. ¹H (400 MHz, CDCl₃, δ en ppm) : 1,66 (mt : 2H); 1,81 (mt : 2H); 2,40 (t, J = 7,5 Hz : 2H); 2,46 (mt : 4H); 2,77 (s : 3H); 3,72 (t, J = 5 Hz : 4H); 3,84 (mt : 2H); 3,96 (t, J = 6,5 Hz : 2H); 4,36 (mt : 2H); 4,53 (s : 1H); 6,87 (dd, J = 8 et 2 Hz : 1H); 6,93 (d, J = 8 Hz : 1H); 6,96 (d, J = 2 Hz : 1H); de 7,10 à 7,35 (mt : 7H); 7,42 (d, J = 8 Hz : 4H)].

La 1-benzhydryl-3-{[3-(4-bromo-butoxy)phényl]méthanesulfonyl-méthylene}-azétidine peut être préparée de la manière suivante : A une solution de 500 mg de 1-benzhydryl-3-[(3-hydroxyphényl)(méthylsulfonyl)méthylène]azétidine dans 10 cm³ de méthyl-éthylcétone, on ajoute successivement à une température voisine de 20°C, sous atmosphère inerte d'argon, 0,586 cm³ de 1,4-dibromobutane et 255 mg de carbonate de potassium. Le mélange réactionnel est porté au reflux du solvant, sous atmosphère inerte d'argon, pendant 7 heures, puis laissé à une température voisine de 20°C pendant environ 4 jours. Le mélange réactionnel est filtré sur verre fritté garni de célite. Le résidu solide est rincé avec de l'acétate d'éthyle, puis le filtrat est concentré à sec sous pression réduite (10 kPa) à une température voisine de 40°C. L'huile brune obtenue est purifiée par chromatographie à pression atmosphérique sur 40 g de silice (0,063-0,200 mm) contenus dans une colonne de 3 cm de diamètre en éluant avec un mélange méthanol-dichlorométhane (0,5-99,5 en volumes). Les fractions (10 cm³) ne contenant que le produit cherché sont réunies et concentrées à sec sous pression réduite (0,27 kPa) à 40°C pendant 2 heures. On obtient ainsi 408,4 mg de 1-benzhydryl-3-{[3-(4-bromo-butoxy)phényl]méthanesulfonylméthylene}-azétidine sous forme d'une meringue brune.

### Exemple 160

A une solution de 45 mg de 1-benzhydryl-3-{[3-(4-bromo-propyloxy)phényl]méthanesulfonyl-méthylene}-azétidine dans 3,5 cm³ d'acétonitrile anhydre, sont ajoutés successivement, à une température voisine de 20°C, sous atmosphère inerte d'argon, 0,110 cm³ de morpholine puis 35 mg de carbonate de potassium. Après 20 heures d'agitation à une température voisine de 20°C, le milieu réactionnel est dilué avec 40 cm³ d'acétate d'éthyle et 10 cm³ d'eau. La phase organique, séparée, est lavée avec 10 cm³ d'eau, puis 2 fois 10 cm³ d'une solution aqueuse saturée en chlorure de sodium, séchée sur sulfate de magnésium, filtrée sur verre fritté, puis concentrée à sec sous pression réduite (9 kPa) à une température voisine de 40°C. La laque jaune obtenue est purifiée par dépôt en solution dans un minimum de dichlorométhane sur chromatographie sur gel de silice déposé sur plaque [(gel de 0,5 mm d'épaisseur, 2 plaques de 20 x 20 cm, éluant : dichlorométhane-méthanol (97,5-2,5 en volumes)]. La zone correspondant au produit cherché adsorbé, localisée aux rayons U.V., est grattée et la silice recueillie est lavée sur verre fritté par un mélange dichlorométhane-méthanol (85-15 en volumes). Les filtrats sont réunis et concentrés à sec sous pression réduite (1 kPa) à une température voisine de 40°C. On obtient ainsi 33 mg de 4-(4-{3-[(1-benzhydryl-azétidin-3-ylidene)méthanesulfonyl-méthyl]phénoxy}-propyl)morpholine sous forme d'une meringue blanche [Spectre de R.M.N. ¹H (300 MHz, (CD₃)₂SO d6, δ en ppm) : 1,87 (mt : 2H); 2,37 (mt : 4H); 2,42 (t, J = 7,5 Hz : 2H); 2,94 (s : 3H); 3,58 (mt : 4H); 3,80 (mt : 2H); 4,02 (t, J = 7 Hz : 2H); 4,20 (mt : 2H); 4,74 (s : 1H); 6,97 (mt : 3H); 7,22 (t, J = 7,5 Hz : 2H); de 7,25 à 7,40 (mt : 1H); 7,32 (t, J = 7,5 Hz : 4H); 7,48 (d, J = 7,5 Hz : 4H)].

La 1-benzhydryl-3-{[3-(4-bromo-propyloxy)phényl]méthanesulfonyl-méthylene}-azétidine peut être préparée de la manière suivante : A une solution de 500 mg de 1-benzhydryl-3-[(3-hydroxyphényl)(méthylsulfonyl)méthylène]azétidine dans 10 cm³ de méthyl-éthylcétone, on ajoute successivement à une température voisine de 20°C, sous atmosphère inerte d'argon, 0,5 cm³ de 1,3-dibromopropane et 255 mg de carbonate de potassium. Le mélange réactionnel est porté au reflux du solvant, sous atmosphère inerte d'argon, pendant 7 heures, puis laissé à une température voisine de 20°C pendant environ 4 jours. Le mélange réactionnel est filtré sur verre fritté garni de célite. Le résidu solide est rincé avec de l'acétate d'éthyle, puis le filtrat est concentré à sec sous pression réduite (10 kPa) à une température voisine de 40°C. L'huile brune obtenue est purifiée par chromatographie à pression atmosphérique sur 40 g de silice (0,063-0,200 mm) contenus dans une colonne de 3 cm de diamètre en éluant avec un mélange méthanol-dichlorométhane (0,5-99,5 en volumes). Les fractions (10 cm³) ne contenant que le produit cherché sont réunies et concentrées à sec sous pression réduite (0,27 kPa) à 40°C pendant 2 heures. On obtient ainsi 511,1 mg de 1-benzhydryl-3-{[3-(4-bromo-propyloxy)phényl]méthanesulfonyl-méthylene}-azétidine sous forme d'une meringue brune.

Les médicaments selon l'invention sont constitués par un composé de formule (I) ou un isomère ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés.+ Dans ces compositions, le principe actif selon l'invention est mélangé à un ou plusieurs diluants inertes, tels que amidon, cellulose, saccharose, lactose ou silice , sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops ets élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention des psychoses y compris la schizophrénie, des troubles anxieux, de la dépression, de l'épilepsie, de la neurodégénération, des désordres cérébelleux et spinocérébelleux, des désordres cognitifs, du trauma crânien, des attaques de panique, des neuropathies périphériques, des glaucomes, de la migraine, de la maladie de Parkinson, de la maladie d'Alzheimer, de la chorée de Huntington, du syndrome de Raynaud, des tremblements, du désordre compulso-obsessionnel, de la démence sénile, des désordres thymiques, du syndrome de Tourette, de la dyskinésie tardive, des désordres bipolaires, des cancers, des désordres du mouvement induit par les médicaments, des dystonies, des chocs endotoxémiques, des chocs hémorragiques, de l'hypotension, de l'insomnie, des maladies immunologiques, de la sclérose en plaques, des vomissements, de l'asthme, des troubles de l'appétit (boulimie, anorexie), de l'obésité, des troubles de la mémoire, des troubles du transit intestinal, dans le sevrage aux traitements chroniques et abus d'alcool ou de médicaments (opioides, barbituriques, cannabis, cocaïne, amphétamine, phencyclide, hallucinogènes, benzodiazépines par exemple), comme analgésiques ou potentialisateurs de l'activité analgésique des médicaments narcotiques et non narcotiques, comme antibactériens, antiviraux et antiparasitaires.

Les doses dépendent de l'effet recherché, de la durée du traitement et de la voie d'administration utilisée; elles sont généralement comprises entre 5 mg et 1000 mg par jour par voie orale pour un adulte avec des doses unitaires allant de 1 mg à 250 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol1, 6 ml
- Eau q.s.p. 4 ml

## Revendications

1. Composés de formule : 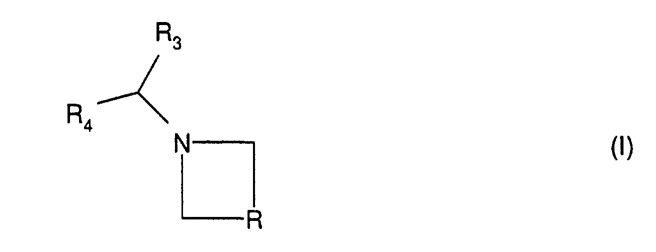 dans laquelle
R représente une chaîne 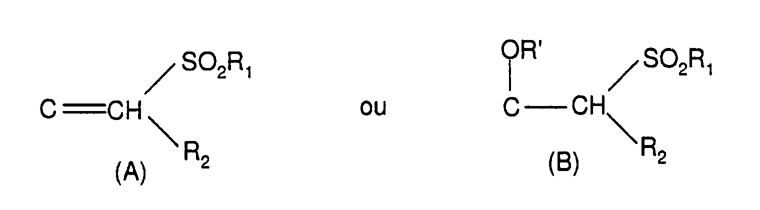 R₁ représente un radical méthyle ou éthyle,
R₂ représente soit un aromatique choisi parmi phényle, naphtyle ou indényle, ces aromatiques étant non substitués ou substitués par un ou plusieurs halogène, alkyle, alcoxy, -CO-alk, hydroxy, -COOR₅, formyle, trifluorométhyle, trifluorométhylsulfanyle, trifluorométhoxy, nitro, -NR₆R₇, -CO-NH-NR₆R₇, -N(alk)COOR₈, cyano, -CONHR₉, -CO-NR₁₆R₁₇, alkylsulfanyle, hydroxyalkyle, -O-alk-NR₁₂R₁₃ ou alkylthioalkyle soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, indolinyle, indolyle, isochromannyle, isoquinolyle, pyridyle, quinolyle, 1,2,3,4-tétrahydroisoquinolyle, 1,2,3,4-tétrahydroquinolyle, thiazolyle, thiényle, ces hétéroaromatiques pouvant être non substitués ou substitués par un halogène, alkyle, alcoxy, -COOR₅, trifluorométhyle, trifluorométhylsulfanyle, trifluorométhoxy, nitro, -NR₆R₇, -CO-NH-NR₆R₇, cyano, -CONHR₉, alkylsulfanyle, hydroxyalkyle ou alkylthioalkyle,
R₃ et R₄, identiques ou différents, représentent soit un aromatique choisi parmi phényle, naphtyle ou indényle, ces aromatiques étant non substitués ou substitués par un ou plusieurs halogène, alkyle, alcoxy, formyle, hydroxy, trifluorométhyle, trifluorométhoxy, -CO-alk, cyano, -COOR₅, -CONR₁₀R₁₁, -CO-NH-NR₆R₇, alkylsulfanyle, hydroxyalkyle, -alk-NR₆R₇ ou alkylthioalkyle; soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, furyle, isochromannyle, isoquinolyle, pyrrolyle, quinolyle, 1,2,3,4-tétrahydroisoquinolyle, thiazolyle, thiényle, ces hétéroaromatiques pouvant être non substitués ou substitués par un halogène, alkyle, alcoxy, hydroxy, trifluorométhyle, trifluorométhoxy, cyano, -COOR₅, -CO-NH-NR₆R₇, -CONR₁₀R₁₁, -alk-NR₆R₇, alkylsulfanyle, hydroxyalkyle ou alkylthioalkyle,
R₅ est un radical alkyle ou phényle éventuellement substitué par un ou plusieurs atomes d'halogène,
R₆ et R₇, identiques ou différents représentent un atome d'hydrogène ou un radical alkyle, -COOalk, cycloalkyle, alkylcycloalkyle, -alk-O-alk, hydroxyalkyle ou bien R₆ et R₇ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, -COalk, -COOalk, -CO-NHalk, -CS-NHalk, -CO-alk-NR₁₄R₁₅, oxo, hydroxyalkyle, -alk-O-alk, -CO-NH₂,
R₈ représente un radical alkyle,
R₉ représente un atome d'hydrogène ou un radical alkyle ou alkyle substitué par dialkylamino, phényle, cycloalkyle (éventuellement substitué par -COOalk) ou un hétérocycle mono ou bicyclique saturé ou insaturé ayant 3 à 10 chaînons, contenant éventuellement un ou plusieurs hétéroatomes choisis parmi oxygène, soufre et azote et étant éventuellement substitué par un ou plusieurs radicaux alkyle,
R₁₀ et R₁₁, identiques ou différents, représentent un atome d'hydrogène ou un radical alkyle ou bien R₁₀ et R₁₁ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un radical alkyle,
R₁₂ et R₁₃, identiques ou différents, représentent un atome d'hydrogène ou un radical alkyle, cycloalkyle ou bien R₁₂ et R₁₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un radical alkyle, -COalk, -COOalk, -CO-NHalk, -CS-NHalk, -CO-alk-NR₁₄R₁₅ ou un hétérocycle mono ou bicyclique saturé ayant 3 à 10 chaînons et contenant un hétéroatome choisi parmi oxygène, soufre et azote,
R₁₄ et R₁₅, identiques ou différents, représentent un atome d'hydrogène ou un radical alkyle ou -COOalk,
R₁₆ et R₁₇ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote,
R' représente un atome d'hydrogène ou un radical -CO-alk,
alk représente un radical alkyle ou alkylène,
étant entendu que les radicaux et portions alkyle et alkylène et les radicaux et portions alcoxy sont en chaîne droite ou ramifiée et contiennent 1 à 6 atomes de carbone, leurs isomères optiques et leurs sels avec un acide minéral ou organique.

2. Composés de formule (I) selon la revendication 1 pour lesquels lorsque R₆ et R₇ forment ensemble avec l'atome d'azote auquel ils sont rattachés un hétérocycle mono ou bicyclique, saturé ou insaturé ayant 3 à 10 chaînons celui-ci est un cycle azétidinyle, pyrrolidinyle, pipérazinyle, pipéridyle, morpholinyle, imidazolyle, thiomorpholinyle ou furyle, ces cycles étant éventuellement substitués par un radical alkyle, hydroxyalkyle, -alk-O-alk, -CONH₂, -COalk, -COOalk, oxo, -CSNHalk, -CONHalk ou -CO-alk-NR₁₄R₁₅ dans lequels alk, R₁₄ et R₁₅ ont les mêmes significations que dans la revendication 1,
leurs isomères optiques et leurs sels avec un acide minéral ou organique.

3. Composés de formule (I) selon l'une des revendications 1 ou 2 pour lesquels lorsque R₁₀ et R₁₁ forment ensemble avec l'atome d'azote auquel ils sont rattachés un hétérocycle mono ou bicyclique saturé et ayant 3 à 10 chaînons, celui-ci est un cycle azétidinyle, pyrrolidinyle, pipérazinyle, pipéridyle, morpholinyle ou thiomorpholinyle, ces cycles étant éventuellement substitués par un alkyle, leurs isomères optiques et leurs sels avec un acide minéral ou organique.

4. Composés de formule (I) selon l'une des revendications 1 à 3 pour lesquels lorsque R₁₂ et R₁₃ forment ensemble avec l'atome d'azote auquel ils sont rattachés un hétérocycle mono ou bicyclique saturé et ayant 3 à 10 chaînons, celui-ci est un cycle azétidinyle, pyrrolidinyle, pipérazinyle, pipéridyle, morpholinyle ou thiomorpholinyle, ces cycles étant éventuellement substitués par un radical alkyle, -COalk, -COOalk, -CO-NHalk, -CS-NHalk, -CO-alk-NR₁₄R₁₅ ou un hétérocycle mono ou bicyclique saturé ayant 3 à 10 chaînons et contenant un hétéroatome choisi parmi oxygène, soufre et azote, et, en particulier, par un radical thiomorpholinyle, alk, R₁₄ et R₁₅ ayant les mêmes significations que dans la revendication 1,
leurs isomères optiques et leurs sels avec un acide minéral ou organique.

5. Composés de formule (I) selon l'une des revendications 1 à 4 pour lesquels lorsque R₁₆ et R₁₇ forment ensemble avec l'atome d'azote auquel ils sont rattachés un hétérocycle mono ou bicyclique saturé et ayant 3 à 10 chaînons, celui-ci est un cycle pipéridyle,
leurs isomères optiques et leurs sels avec un acide minéral ou organique.

6. Composés de formule (I) selon l'une des revendications 1 à 5 pour lesquels R₉ représente un radical alkyle substitué par un hétérocycle mono ou bicyclique saturé ou insaturé ayant 3 à 10 chaînons, contenant éventuellement un ou plusieurs hétéroatomes choisis parmi oxygène, soufre et azote, ce dernier étant un cycle pyrrolidinyle, tétrahydrofuryle, morpholinyle, pyrrolyle et ces cycles étant éventuellement substitués par un ou plusieurs radicaux alkyle,
leurs isomères optiques et leurs sels avec un acide minéral ou organique.

7. Composés de formule (I) selon la revendication 1 pour lesquels
R représente une chaîne (A) ou (B),
R' représentant un atome d'hydrogène ou un radical -COalk,
R₁ représente un radical méthyle ou éthyle,
R₂ représente soit un aromatique choisi parmi phényle et naphtyle, ces aromatiques étant non substitués ou substitués par un ou plusieurs halogène, alkyle, alcoxy, hydroxy, -COOR₅, trifluorométhyle, trifluorométhylsulfanyle, trifluorométhoxy, -NR₆R₇, -CO-NH-NR₆R₇, cyano, -CONHR₉, alkylsulfanyle, hydroxyalkyle, nitro, -CO-NR₁₆R₁₇, -O-alkNR₁₂R₁₃ ou alkylthioalkyle ou un hétéroaromatique choisi parmi isoquinolyle, pyridyle, quinolyle, 1,2,3,4-tétrahydroisoquinolyle, 1,2,3,4-tétrahydroquinolyle, thiényle, ces hétéroaromatiques étant non substitués ou substitués par un halogène, alkyle, alcoxy, -COOR₅, trifluorométhyle, trifluorométhylsulfanyle, trifluorométhoxy, -NR₆R₇, -CO-NH-NR₆R₇, cyano, -CONHR₉, alkylsulfanyle, hydroxyalkyle, nitro ou alkylthioalkyle,
R₃ et R₄, identiques ou différents, représentent soit un aromatique choisi parmi phényle ou naphtyle , ces aromatiques étant non substitués ou substitués par un ou plusieurs halogène, alkyle, alcoxy, trifluorométhyle, trifluorométhoxy, -CONR₁₀R₁₁, -alk-NR₆R₇, hydroxyalkyle, formyle, -COOR₅, soit un hétéroaromatique choisi parmi les cycles thiazolyle ou thiényle, ces hétéroaromatiques étant non substitués ou substitués par un halogène, alkyle, alcoxy, -CONR₁₀R₁₁, -alk-NR₆R₇, hydroxyalkyle ou -COOR₅.
R₅ est alkyle ou phényle éventuellement substitué par un ou plusieurs halogène,
R₆ et R₇, identiques ou différents représentent un atome d'hydrogène ou un radical alkyle, -COOalk, cycloalkyle, alkylcycloalkyle, -alk-O-alk, hydroxyalkyle ou bien R₆ et R₇ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, -COalk, -COOalk, -CO-NHalk, -CS-NHalk, -CO-alk-NR₁₄R₁₅, oxo, hydroxyalkyle, -alk-O-alk, -CO-NH₂,
R₉ représente un atome d'hydrogène ou un radical alkyle ou alkyle substitué par dialkylamino, phényle, cycloalkyle (éventuellement substitué par -COOalk) ou un hétérocycle mono ou bicyclique saturé ou insaturé ayant 3 à 10 chaînons, contenant éventuellement un ou plusieurs hétéroatomes choisis parmi oxygène, soufre et azote et étant éventuellement substitué par un ou plusieurs radicaux alkyle,
R₁₀ et R₁₁, identiques ou différents, représentent un atome d'hydrogène ou un radical alkyle ou bien R₁₀ et R₁₁ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un radical alkyle,
R₁₂ et R₁₃, identiques ou différents, représentent un atome d'hydrogène ou un radical alkyle, cycloalkyle ou bien R₁₂ et R₁₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un radical alkyle, -COalk, -COOalk, -CO-NHalk, -CS-NHalk, -CO-alk-NR₁₄R₁₅ ou un hétérocycle mono ou bicyclique saturé ayant 3 à 10 chaînons et contenant un hétéroatome choisi parmi oxygène, soufre et azote,
R₁₄ et R₁₅, identiques ou différents, représentent un atome d'hydrogène ou un radical alkyle ou -COOalk,
R₁₆ et R₁₇ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote,
alk représente un radical alkyle ou alkylène,
étant entendu que les radicaux et portions alkyle et alkylène et les radicaux et portions alcoxy sont en chaîne droite ou ramifiée et contiennent 1 à 6 atomes de carbone,
leurs isomères optiques et leurs sels avec un acide minéral ou organique.

8. Composés de formule (I) selon la revendication 1 pour lesquels
R représente une chaîne (A) ou (B),
R' représentant un atome d'hydrogène ou un radical -COalk,
R₁ représente un radical méthyle ou éthyle,
R₂ représente soit un aromatique choisi parmi
naphtyle,
phényle,
phényle substitué par un ou plusieurs halogène, alkyle, alcoxy, hydroxy, -COOR₅ (dans lequel R₅ représente un radical alkyle ou phényle éventuellement substitué par plusieurs halogènes), trifluorométhyle, trifluorométhylsulfanyle, trifluorométhoxy, -NR₆R₇ (dans lequel R₆ et R₇, identiques ou différents représentent un atome d'hydrogène ou un radical alkyle ou -COOalk ou bien R₆ et R₇ forment ensemble avec l'atome d'azote auquel ils sont rattachés un hétérocycle choisi parmi pyrrolidinyle, pipéridyle, pipérazinyle ou pipérazinyle substitué par un ou plusieurs radicaux alkyle, -COalk, -COOalk, -CO-NHalk, -CS-NHalk, -CO-alk-NR₁₄R₁₅, dans lequel R₁₄ et R₁₅, identiques ou différents, représentent un atome d'hydrogène ou un radical alkyle), -CO-NH-NR₆R₇ (R₆ et R₇, identiques ou différents représentent un atome d'hydrogène ou un radical alkyle ou bien R₆ et R₇ forment ensemble avec l'atome d'azote auquel ils sont rattachés un hétérocycle choisi parmi pipéridyle, pyrrolyle, pipérazinyle ou pipérazyle substitué par un ou plusieurs radicaux alkyle), cyano, -CONHR₉ (dans lequel R₉ représente un atome d'hydrogène ou un radical alkyle ou alkyle substitué par dialkylamino, phényle, cycloalkyle (éventuellement substitué par -COOalk) ou un hétérocycle choisi parmi pyrrolidinyle (éventuellement substitué par alkyle), tétrahydrofuryle, morpholinyle), alkylsulfanyle, hydroxyalkyle, nitro, -CO-NR₁₆R₁₇, (dans lequel R₁₆ et R₁₇ forment ensemble avec l'atome d'azote auquel ils sont rattachés un cycle pipéridyle), -O-alkNR₁₂R₁₃ (dans lequel R₁₂ et R₁₃ forment ensemble avec l'atome d'azote auquel ils sont rattachés un cycle morpholino) ou alkylthioalkyle,
soit un hétéroaromatique choisi parmi
isoquinolyle,
pyridyle,
quinolyle,
1,2,3,4-tétrahydroisoquinolyle,
1,2,3,4-tétrahydroquinolyle,
thiényle, ou
thiényle substitué par un -COOR₅ (dans lequel R₅ représente un radical alkyle) ou -CONHR₉, (dans lequel R₉ représente un radical alkyle),
R₃ et R₄, identiques ou différents, représentent soit un aromatique choisi parmi phényle ou
phényle substitué par un ou plusieurs halogène, alkyle, alcoxy, trifluorométhyle, trifluorométhoxy, hydroxyalkyle, formyle, -COOR₅ (dans lequel R₅ est un radical alkyle), -CONR₁₀R₁₁ (dans lequel R₁₀ et R₁₁, identiques ou différents, représentent un atome d'hydrogène ou un radical alkyle), -alk-NR₆R₇, (dans lequel R₆ et R₇, identiques ou différents représentent un atome d'hydrogène ou un radical alkyle, cycloalkyle, -alk-O-alk, hydroxyalkyle ou bien R₆ et R₇ forment ensemble avec l'atome d'azote auquel ils sont rattachés un hétérocycle choisi parmi pipéridyle (éventuellement substitué par alkyle, oxo), pyrrolidinyle (éventuellement substitué par alkyle, hydroxyalkyle, -alk-O-alk, -CO-NH₂), thiomorpholinyle, morpholinyle, pyrrolyle, pipérazinyle éventuellement substitué par oxo, alkyle, hydroxyalkyle, -COOR₅ (dans lequel R₅ est un radical alkyle),
soit un hétéroaromatique choisi parmi
thiazolyle ou
thiényle,
alk représente un radical alkyle ou alkylène,
étant entendu que les radicaux et portions alkyle et alkylène et les radicaux et portions alcoxy sont en chaîne droite ou ramifiée et contiennent 1 à 6 atomes de carbone,
leurs isomères optiques et leurs sels avec un acide minéral ou organique.

9. Composés, selon la revendication 1, choisis parmi :
1-benzhydryl-3-[(méthylsulfonyl)(phényl)méthylène]azétidine,
1-benzhydryl-3-[(3-méthylphényl)(méthylsulfonyl)méthylène]azétidine,
1-benzhydryl-3-[(3-chlomphènyl)(méthylsulfonyl)méthylène]azétidine,
1 -benzhydryl-3-[(3,5-dichlorophényl)(méthylsulfonyl)méthylène]azétidine,
1-benzhydryl-3-[(2,5-dichlorophényl)(méthylsulfonyl)méthylène]azétidine,
1-benzhydryl-3-[(2,3-dichlorophényl)(méthylsulfonyl)méthylène]azétidine,
1 -benzhydryl-3-[(3-fluorophényl)(méthylsulfonyl)méthylène]azétidine,
1-benzhydryl-3-[(3,5-difluorophényl)(méthylsulfonyl)méthylène]azétidine,
I -benzhydryl-3-[(3-bromophényl)(méthylsulfonyl)méthylène]azétidine,
1-benzhydryl-3-[(3-iodophényl)(méthylsulfonyl)méthylène]azétidine,
1-benzhydryl-3-[(méthylsulfonyl)(3-trifluorométhoxyphényl)méthylène]azétidine,
1-benzhydryl-3-[(méthylsulfonyl)(3-trifluorométhylphényl)méthylène]azétidine,
1-benzhydryl-3-{[3,5-bis(trifluorométhyl)phényl](méthylsulfonyl)méthylène}azétidine,
1-benzhydryl-3-[(3,5-dibromophényl)(méthylsulfonyl)méthylène]azétidine,
1-benzhydryl-3-[(3-méthoxycarbonylphényl)(méthylsulfonyl)méthylène]azétidine,
1-benzhydryl-3-[(3-cyanophényl)(méthylsulfonyl)méthylène]azétidine,
1-benzhydryl-3-[(3-carbamoylphényl)(méthylsulfonyl)méthylène]azétidine,
I -benzhydryl-3-[(méthylsulfonyl)(napht-1-yl)(méthylsulfonyl)méthylène]azétidine,
1-[bis(4-chlorophényl)méthyl]-3-[(3,5-difluorophényl)(méthylsulfonyl)méthylène] azétidine,
1-[bis(4-méthoxyphényl)méthyl]-3-[(3,5-difluorophényl)(méthylsulfonyl)méthylène]azétidine,
1-[bis(4-méthylphényl)méthyl]-3-[(3,5-difluorophényl)(méthylsulfonyl)méthylène] azétidine,
(RS)-3-[(3,5-difluorophényl)(méthylsulfonyl)méthylène]-1-[(4-méthoxyphényl) (phényl)méthyl)]azétidine,
(R)-3-[(3,5-difluorophényl)(méthylsulfonyl)méthylène]-1-[(4-méthoxyphényl) (phényl)méthyl]azétidine,
(S)-3-[(3,5-difluorophényl)(méthylsulfonyl)méthylène]-1-[(4-méthoxyphényl) (phényl)méthyl]azétidine,
1 -[bis(4-trifluorométhoxyphényl)méthyl]-3-[(3,5-difluorophényl)(méthylsulfonyl) méthylène] azétidine,
1-[bis(4-trifluorométhylphényl)méthyl] -3-[(3,5-difluorophényl)(méthylsulfonyl)méthylène]azétidine,
1-[bis(4-chlorophényl) méthyl]3-{[3,5-bis(trifluorométhyl) phényl]méthylsulfonyl méthylène}azétidine,
(RS)-1-[(4-chlorophényl)(2,4-dichlorophényl)méthyl]-3-[(3,5-difluorophényl)(méthylsulfonyl)méthylène]azétidine,
(R)-1-[(4-chlorophényl)(2,4-dichlorophényl)méthyl]-3-[(3,5-difluorophényl)(méthylsulfonyl)méthylène]azétidine,
(S)-1-[(4-chlorophényl)(2,4-dichlorophényl)méthyl]-3-[(3,5-difluorophényl)(méthylsulfonyl)méthylène]azétidine,
(RS)-1-{(4-chlorophényl)[4-(hydroxyméthyl)phényl]méthyl} -3-[(3,5-difluorophényl)(méthylsulfonyl)méthylène]azétidine,
(R)-1-{(4-chlorophényl)[4-(hydroxyméthyl)phényl]méthyl}-3-[(3,5-difluorophényl)(méthylsulfonyl)méthylène]azétidine,
(S)-1-{(4-chlorophényl)[4-(hydroxyméthyl)phényl]méthyl}-3-[(3,5-difluorophényl)(méthylsulfonyl)méthylène]azétidine,
(RS)-1-{(4-chlorophényl)[4-(pyrrolidylméthyl)phényl]méthyl}-3-[(3,5-difluorophényl)(méthylsulfonyl)méthylène]azétidine,
(R)-1-{(4-chlorophényl)[4-(pyrrolidylméthyl)phényl]méthyl}-3-[(3,5 -difluorophényl)(méthylsulfonyl)méthylène]azétidine,
(S)-1-{(4-chlorophényl)[4-(pyrrolidylméthyl)phényl]méthyl}-3-[(3,5-difluorophényl)(méthylsulfonyl)méthylène]azétidine,
1-{(RS)-(4-chlorophényl) [4-(3,3-diméthyl-pipéridin-1-yl-méthyl) phényl]méthyl}-3-[(3,5-difluorophényl)(méthylsulfonyl)méthylène]azétidine,
1-{(R)-(4-chlorophényl) [4-(3,3-diméthyl-pipéridin-1-yl-méthyl) phényl]méthyl}-3-[(3,5-difluorophényl)(méthylsulfonyl)méthylène]azétidine,
1-{(S)-(4-chlorophényl) [4-(3,3-diméthyl-pipéridin-1-yl-méthyl) phényl]méthyl}-3-[(3,5-difluorophényl)(méthylsulfonyl)méthylène]azétidine,
1-{(RS)-(4-chlorophényl) [4-(thiomorpholin-4-yl-méthyl) phényl]méthyl}-3-[(3,5-difluorophényl)(méthylsulfonyl)méthylène]azétidine,
1-{(R)-(4-chlorophényl)[4-(thiomorpholin-4-yl-méthyl)phényl]méthyl}-3-[(3,5-difluorophényl)(méthylsulfonyl)méthylène]azétidine,
1-{(S)-(4-chlorophényl)[4-(thiomorpholin-4-yl-méthyl)phényl]méthyl}-3-[(3,5-difluorophényl)(méthylsulfonyl)méthylène]azétidine,
1-{(RS)-(4-chlorophényl)[4-(N-éthyl-N-cyclohexyl-aminométhyl)phényl]méthyl}-3-[(3,5-difluorophényl)(méthylsulfonyl)méthylène]azétidine,
1-{(R)-(4-chlorophényl)[ 4-(N-éthyl-N-cyclohexyl-aminométhyl)phényl]méthyl}-3-[(3,5-difluorophényl)(méthylsulfonyl)méthylène]azétidine,
1-{(S)-(4-chlorophényl)[ 4-(N-éthyl-N-cyclohexyl-aminométhyl)phényl]méthyl}-3-[(3,5-difluorophényl)(méthylsulfonyl)méthylène]azétidine,
1-{{(RS)-(4-chlorophényl){4-[(4-éthoxycarbonylpipérazinyl)méthyl]phényl}méthyl }}-3-[(3,5-difluorophényl)(méthylsulfonyl)méthylène]azétidine,
1 - { {(R)-(4-chlorophényl) {4-[(4-éthoxycarbonylpipérazinyl)méthyl]phényl}méthyl}}-3-[(3,5-difluorophényl)(méthylsulfonyl)méthylène]azétidine,
1-{{(S)-(4-chlorophényl) {4-[(4-éthoxycarbonylpipérazinyl)méthyl]phényl}méthyl}}-3-[(3,5-difluorophényl)(méthylsulfonyl)méthylène]azétidine,
1-{(RS)-(4-chlorophényl)[4-(N-cyclopropyl-N-propyl-aminométhyl)phényl]méthyl}-3-[(3,5-difluorophényl)(méthylsulfonyl)méthylène]azétidine,
1-{(R)-(4-chlorophényl)[4-(N-cyclopropyl-N-propyl-aminométhyl)phényl]méthyl}-3-[(3,5-difluorophényl)(méthylsulfonyl)méthylène]azétidine,
1-{ (S)-(4-chlorophényl) [4-(N-cyclopropyl-N-propyl-aminométhyl)phényl]méthyl}-3-[(3,5-difluorophényl)(méthylsulfonyl)méthylène]azétidine,
1- { (RS)-(4-chlorophényl)[4-(diisopropylaminométhyl)phényl]méthyl}-3-[(3,5-difluorophényl)(méthylsulfonyl)méthylène]azétidine,
1-{(R)-(4-chlorophényl)[4-(diisopropylaminométhyl)phényl]méthyl}-3-[(3,5-difluorophényl)(méthylsulfonyl)méthylène]azétidine,
1-{(S)-(4-chlorophényl)[4-(diisopropylaminométhyl)phényl]méthyl}-3-[(3,5-difluorophényl)(méthylsulfonyl)méthylène]azétidine,
1-{{(RS)-(4-chlorophényl){4-[bis-(2-méthoxyéthyl)aminométhyl]phényl}méthyl}}-3-[(3,5-difluorophényl)(méthylsulfonyl)méthylène]azétidine,
1-{((R)-(4-chlorophényl) { 4-[bis-(2-méthoxyéthyl)aminométhyl]phényl} méthyl}}-3-[(3,5-difluorophényl)(méthylsulfonyl)méthylène]azétidine,
1-{ {(S)-(4-chlorophényl){4-[bis-(2-méthoxyéthyl)aminométhyl]phényl}méthyl}}-3-[(3,5-difluorophényl)(méthylsulfonyl)méthylène]azétidine,
1-{(RS)-(4-chlorophényl)[4-(di-n-propylaminométhyl)phényl]méthyl}-3-[(3,5-difluorophényl)(méthylsulfonyl)méthylène]azétidine,
1-{(R)-(4-chlorophényl)[4-(di-n-propylaminométhyl)phényl]méthyl}-3-[(3,5-difluorophényl)(méthylsulfonyl)méthylène]azétidine,
1-{(S)-(4-chlorophényl)[4-(di-n-propylaminométhyl)phényl]méthyl}-3-[(3,5-difluorophényl)(méthylsulfonyl)méthylène]azétidine,
1-{(RS)-(4-chlorophényl)[4-(pipéridin-1-yl-méthyl)phényl]méthyl}-3-[(3,5-difluorophényl)(méthylsulfonyl)méthylène]azétidine,
1-{(R)-(4-chlorophényl)[4-(pipéridin-1-yl-méthyl)phényl]méthyl}-3-[(3,5-difluorophényl)(méthylsulfonyl)méthylène]azétidine,
1-{(S)-(4-chlorophényl)[4-(pipéridin-1-yl-méthyl)phényl]méthyl}-3-[(3,5-difluorophényl)(méthylsulfonyl)méthylène]azétidine,
1-{(RS)-(4-chlorophényl)[4-(4-méthyl-pipérazin-1-yl-méthyl)phényl]méthyl}-3-[(3,5-difluorophényl)(méthylsulfonyl)méthylène]azétidine,
1-{(R)-(4-chlorophényl) [4-(4-méthyl-pipérazin-1-yl-méthyl)phényl]méthyl}-3-[(3,5-difluorophényl)(méthylsulfonyl)méthylène]azétidine,
1-{(S)-(4-chlorophényl)[4-(4-méthyl-pipérazin-1-yl-méthyl)phényl]méthyl}-3-[(3,5-difluorophényl)(méthylsulfonyl)méthylène]azétidine,
1-{(RS)-(4-chlorophényl)[4-(morpholin-4-yl-méthyl)phényl]méthyl}-3-[(3,5-difluorophényl)(méthylsulfonyl)méthylène]azétidine,
1-{(R)-(4-chlorophényl)[4-(morpholin-4-yl-méthyl)phényl]méthyl}-3-[(3,5-difluorophényl)(méthylsulfonyl)méthylène]azétidine,
1-{(S)-(4-chlorophényl)[4-(morpholin-4-yl-méthyl)phényl]méthyl}-3-[(3,5-difluorophényl)(méthylsulfonyl)méthylène]azétidine,
1-{(RS)-(4-chlorophényl)[4-(diéthylaminométhyl)phényl]méthyl}-3-[(3,5-difluorophényl)(méthylsulfonyl)méthylène]azétidine,
1-{(R)-(4-chlorophényl)[4-(diéthylaminométhyl)phényl]méthyl}-3-[(3,5-difluorophényl)(méthylsulfonyl)méthylène]azétidine,
1-{(S)-(4-chlorophényl)[4-(diéthylaminométhyl)phényl]méthyl}-3-[(3,5-difluorophényl)(méthylsulfonyl)méthylène]azétidine,
1-{(RS)-(4-chlorophényl)[4-(pipérazin-2-one-4-yl-méthyl)phényl]méthyl}-3-[(3,5-difluorophényl)(méthylsulfonyl)méthylène]azétidine,
1-{(R)-(4-chlorophényl)[4-(pipérazin-2-one-4-yl-méthyl)phényl]méthyl}-3-[(3,5-difluorophényl)(méthylsulfonyl)méthylène]azétidine,
1-{(S)-(4-chlorophényl)[4-(pipérazin-2-one-4-yl-méthyl)phényl]méthyl}-3-[(3,5-difluorophényl)(méthylsulfonyl)méthylène]azétidine,
1-{(RS)-(4-chlorophényl)[4-(imidazol-1-yl-méthyl)phényl]méthyl}-3-[(3,5-difluorophényl)(méthylsulfonyl)méthylène]azétidine,
1-{(R)-(4-chlorophényl)[4-(imidazol-1-yl-méthyl)phényl]méthyl}-3-[(3,5-difluorophényl)(méthylsulfonyl)méthylène]azétidine,
1-{(S)-(4-chlorophényl)[4-(imidazol-1-yl-méthyl)phényl]méthyl}-3-[(3,5-difluorophényl)(méthylsulfonyl)méthylène] azétidine,
(RS)-1-{(4-chlorophényl)[4-(N,N-diméthylcarbamoyl)phényl]méthyl}-3-[(3,5-difluorophényl)(méthylsulfonyl)méthylène]azétidine,
(R)-1-{(4-chlorophényl)[4-(N,N-diméthylcarbamoyl)phényl]méthyl}-3-[(3,5-difluorophényl)(méthylsulfonyl)méthylène]azétidine,
(S)-1-{(4-chlorophényl)[4-(N,N-diméthylcarbamoyl)phényl]méthyl}-3-[(3,5-difluorophényl)(méthylsulfonyl)méthylène]azétidine,
(RS)-1-{(4-chlorophényl)[4-(N-éthylcarbamoyl)phényl]méthyl)}-3-[(3,5-difluorophényl)(méthylsulfonyl)méthylène]azétidine,
(R)-1-{(4-chlorophényl)[4-(N-éthylcarbamoyl)phényl]méthyl}-3-[(3,5-difluorophényl)(méthylsulfonyl)méthylène]azétidine,
(S)-1-{(4-chlorophényl)[4-(N-éthylcarbamoyl)phényl]méthyl}-3-[(3,5-difluorophényl)(méthylsulfonyl)méthylène]azétidine,
(RS)-1-[(4-carbamoylphényl)(4-chlorophényl)méthyl]-3-[(3,5-difluorophényl) (méthylsulfonyl)méthylène]azétidine,
(R)-1-[(4-carbamoylphényl)(4-chlorophényl)méthyl]-3-[(3,5-difluorophényl)(méthylsulfonyl)méthylène]azétidine,
(S)-1-[(4-carbamoylphényl)(4-chlorophényl)méthyl]-3-[(3,5-difluorophényl) (méthylsulfonyl)méthylène]azétidine,
1-[bis(4-chlorophényl)méthyl]-3-[(3,5-dichlorophényl)(méthylsulfonyl)méthylène] azétidine,
1-benzhydryl-3-[(3-méthylsulfanylphényl)(méthylsulfonyl)méthylène]azétidine,
1-benzhydryl-3-[(3-méthylsulfanylméthyl)phényl)](méthylsulfonyl)méthylène]azétidine,
1-[bis(4-chlorophényl)méthyl]-3-[(3-cyanophényl)(méthylsulfonyl)méthylène]azétidine,
1-[bis(4-chlorophényl)méthyl]-3-[(3-carbamoylphényl)(méthylsulfonyl)méthylène]azétidine,
1-[bis(4-chlorophényl)méthyl]-3-[(3-méthoxyphényl)(méthylsulfonyl)méthylène] azétidine,
1-[bis(4-chlorophényl)méthyl] -3-[(3-hydroxyphényl)(méthylsulfonyl)méthylène] azétidine,
1-[bis(4-chlorophényl)méthyl]-3-[(méthylsulfonyl)(3-pyrrolidinylphényl)méthylène] azétidine,
1-[bis(4-chlorophényl)méthyl]-3 - [(3-hydroxyméthylphényl)(méthylsulfonyl)méthylène]azétidine,
1-[bis(4-chlorophényl)méthyl]3-{(méthylsulfonyl)[3-(N-pipéridylcarbamoyl)phényl] méthylène} azétidine,
1-[bis(4-chlorophényl)méthyl]-3-[(méthylsulfonyl)(3-trifluorométhylsulfanylphényl)(méthylsulfonyl)méthylène]azétidine,
1-[bis(4-fluorophényl)méthyl]-3-[(3,5-difluorophényl)(méthylsulfonyl)méthylène] azétidine,
1-[bis(2-fluorophényl)méthyl]-3-[(3,5-difluorophényl)(méthylsulfonyl)méthylène] azétidine,
1-[bis(3-fluorophényl)méthyl]-3-[(3,5-difluorophényl)(méthylsulfonyl)méthylène] azétidine,
(RS)-1-[(4-chlorophényl)(thiazol-2-yl)méthyl]-3-[(méthylsulfonyl)(phényl)méthylène]azétidine,
(R)-1-[(4-chlorophényl)(thiazol-2-yl)méthyl]-3-[(méthylsulfonyl)(phényl)méthylène] azétidine,
(S)-1-[(4-chlorophényl)(thiazol-2-yl)méthyl]-3-[(méthylsulfonyl)(phényl)méthylène] azétidine,
(RS)-1-[(4-chlorophényl)(thién-2-yl)méthyl]-3-[(3,5-difluorophényl)(méthylsulfonyl)méthylène]azétidine,
(R)-1-[(4-chlorophényl)(thién-2-yl)méthyl]-3-[(3,5-difluorophényl)(méthylsulfonyl) méthylène]azétidine,
(S)-1-[(4-chlorophényl)(thién-2-yl)méthyl]-3-[(3,5-difluorophényl)(méthylsulfonyl) méthylène]azétidine,
1-benzhydryl-3-[(éthylsulfonyl)(phényl)méthylène]azétidine,
1-[bis(4-chlorophényl)méthyl]-3- {{3-[N-(4-méthylpipérazinyl)carbamoyl]phényl} (méthylsulfonyl)méthylène}azétidine,
1-[bis(4-chlorophényl)méthyl]-3-{[3-(2,2-diméthylcarbohydrazido)phényl](méthylsulfonyl)méthylène}azétidine,
1-[bis(thién-2-yl)méthyl]-3-[(3,5-difluorophényl)(méthylsulfonyl)méthylène]azétidine,
1-[bis(p-tolyl)méthyl]-3-[(méthylsulfonyl)(phényl)méthylène]azétidine,
1-[(4-chlorophényl)(4-hydroxyméthylphényl)méthyl]-3-[(3,5-difluorophényl) (méthylsulfonyl)méthylène]azétidine,
1-[bis(4-chlorophényl)méthyl] -3-[(3-méthylaminophényl)(méthylsulfonyl)méthylène]azétidine,
(RS)-1-[(4-chlorophényl)(thiazol-2-yl)méthyl]-3-[(3,5-difluorophényl)(méthylsulfonyl)méthylène]azétidine,
(R)-1-[(4-chlorophényl)(thiazol-2-yl)méthyl]-3-[(3,5-difluorophényl)(méthylsulfonyl)méthylène]azétidine,
(S)-1-[(4-chlorophényl)(thiazol-2-yl)méthyl]-3-[(3,5-difluorophényl)(méthylsulfonyl)méthylène]azétidine,
1-[bis(4-chlorophényl)méthyl]-3-[(méthylsulfonyl)(2-méthoxycarbonylthién-5-yl)méthylène]azétidine,
1-[bis(4-chlorophényl)méthyl]-3-hydroxy-3-[(méthylsulfonyl)(2-méthoxycarbonylthién-5-yl)méthyl]azétidine-(RS),
1-[bis(4-chlorophényl)méthyl]-3-[(2-*iso*butylaminocarbonylthién-5-yl)(méthylsulfonyl)méthylène]azétidine,
1-[bis(4-chlorophényl)méthyl]-3-[(3-méthoxycarbonylphényl)(méthylsulfonyl) méthyl-(RS)]azétidin-3-ol,
1-[bis(4-chlorophényl)méthyl]-3-[(méthylsulfonyl)(pyridin-4-yl)méthyl-(RS)]azétidin-3-ol,
1-[bis(4-chlorophényl)méthyl]-3-[(méthylsulfonyl)(pyridin-3-yl)méthyl-(RS)]azétidin-3-ol,
3-({1-[bis-(4-chlorophényl)méthyl]azétidin-3-ylidéne}-méthanesulfonyl-méthyl)N-(3-morpholin-4-yl-propyl)benzamide,
3-({1-[bis-(4-chlorophényl)méthyl]azétidin-3-ylidène}-méthanesulfonyl-méthyl)N-(3 -diméthylamino-propyl)benzamide,
3-({1-[bis-(4-chlorophényl)méthyl]azétidin-3-ylidène}-méthanesulfonyl-méthyl)N-(2-pyrrolidin-1-yl-éthyl)benzamide,
3-( {1-[bis-(4-chlorophényl)méthyl]azétidin-3-ylidène}-méthanesulfonyl-méthyl)N-(2-diméthylamino-1-méthyl-éthyl)benzamide,
3-({1-[bis-(4-chlorophényl)méthyl]azétidin-3-ylidène}-méthanesulfonyl-méthyl)N-pipéridin-1-yl-benzamide,
3-({1-[bis-(4-chlorophényl)méthyl]azétidin-3-ylidène}-méthanesulfonyl-méthyl)N-isobutyl-benzamide,
3-({1-[bis-(4-chlorophényl)méthyl]azétidin-3-ylidène}-méthanesulfonyl-méthyl)N-(3-imidazol-1-yl-propyl)benzamide,
3-({1-[bis-(4-chlorophényl)méthyl]azétidin-3-ylidène}-méthanesulfonyl-méthyl)N-(2-diméthylamino-éthyl)benzamide,
N'-méthyl-hydrazide de l'acide 3-({1-[bis-(4-chlorophényl)méthyl]azétidin-3-ylidène}-méthanesulfonyl-méthyl)benzoïque,
3-({1-[bis-(4-chlorophényl)méthyl]azétidin-3-ylidène}-méthanesulfonyl-méthyl)N-(2-morpholin-4-yl-éthyl)benzamide,
3-({1-[bis-(4-chlorophényl)méthyl]azétidin-3-ylidène}-méthanesulfonyl-méthyl)N-(1-éthyl-pyrrolidin-2-ylméthyl)benzamide,
3-({1-[bis-(4-chlorophényl)méthyl]azétidin-3-ylidène}-méthanesulfonyl-méthyl)N-(2,2-diméthyl-propyl)benzamide,
3-({1-[bis-(4-chlorophényl)méthyl]azétidin-3-ylidène}-méthanesulfonyl-méthyl)N-cyclohexylméthyl-benzamide,
3-({1-[bis-(4-chlorophényl)méthyl]azétidin-3-ylidène}-méthanesulfonyl-méthyl)N-cyclopropylméthyl-benzamide,
3-({1-[bis-(4-chlorophényl)méthyl]azétidin-3-ylidène}-méthanesulfonyl-méthyl)N-(2-méthyl-butyl)benzamide,
3-({1-[bis-(4-chlorophényl)méthyl]azétidin-3-ylidène}-méthanesulfonyl-méthyl)N-(2-phényl-propyl)benzamide,
3-({1-[bis-(4-chlorophényl)méthyl]azétidin-3-ylidène}-méthanesulfonyl-méthyl)N-(tetrahydro-furan-2-ylméthyl)benzamide,
3-({1-[bis-(4-chlorophényl)méthyl]azétidin-3-ylidène}-méthanesulfonyl-méthyl)N-(2,2-diphényl-éthyl)benzamide,
3-({1-[bis-(4-chlorophényl)méthyl]azétidin-3-ylidène}-méthanesulfonyl-méthyl)N-(2-éthyl-butyl)benzamide,
ester méthylique de l'acide 4-{[3-({1-[bis-(4-chlorophényl)méthyl]azétidin-3-ylidène}-méthanesulfonyl-méthyl)benzoylamino]méthyl}-cyclohexanecarboxylique,
2-amino-1-{4-[3-({1-[bis-(4-chlorophényl)méthyl]azétidin-3-ylidène}-méthanesulfonyl-méthyl)phényl]pipérazin-1-yl}-éthanone,
ester tert-butylique de l'acide (2-{4-[3-({1-[bis-(4-chlorophényl)méthyl]azétidin-3-ylidène }-méthanesulfonyl-méthyl)phényl]pipérazin-1-yl}-2-oxo-éthyl)carbamique,
1-{4-[3-( {1-[bis-(4-chlorophényl)méthyl]azétidin-3-ylidène}-méthanesulfonylméthyl)phényl]pipérazin-1-yl}-2-méthylamino-éthanone,
ester ter-butylique de l'acide (2-{4-[3-({1-[bis-(4-chlorophényl)méthyl]azétidin-3-ylidène}-méthanesulfonyl-méthyl)phényl]pipérazin-1-yl}-2-oxo-éthyl)N-méthylcarbamique,
N-méthylamide de l'acide 4-[3-({1-[bis-(4-chlorophényl)méthyl]azétidin-3-ylidène}-méthanesulfonyl-méthyl)phényl]pipérazine-1-carbothioic,
N-méthylamide de l'acide 4-[3-({1-[bis-(4-chlorophényl)méthyl]azétidin-3-ylidène}-méthanesulfonyl-méthyl)phényl]pipérazine-1-carboxylique,
ester de méthyl de l'acide 4-[3-({1-[bis-(4-chlorophényl)méthyl]azétidin-3-ylidène}-méthanesulfonyl-méthyl)phényl]pipérazine-1-carboxylique,
1-[3-({1-[bis-(4-chlorophényl)méthyl]azétidin-3-ylidène}-méthanesulfonyl-méthyl)phényl]-4-isobutyl-pipérazine,
1-[3-({1-[bis-(4-chlorophényl)méthyl]azétidin-3-ylidène}-méthanesulfonyl-méthyl)phényl]-4-éthyl-pipérazine,
4-acétyl 1-[3-({1-[bis-(4-chlorophényl)méthyl]azétidin-3-ylidène}-méthanesulfonylméthyl)phényl]pipérazine,
1-{4-[3-({1-[bis-(4-chlorophényl)méthyl]azétidin-3-ylidène}-méthanesulfonylméthyl)phényl]pipérazin-1-yl}-2-diméthylamino-éthanone,
1-[3-({1-[bis-(4-chlorophényl)méthyl]azétidin-3-ylidène}-méthanesulfonyl-méthyl)phényl]pipérazine,
ester tert-butylique de l'acide 4-[3-({1-[bis-(4-chlorophény])méthyl]azétidin-3-ylidène} -méthanesulfonyl-méthyl)phényl]pipérazine-1-carboxylique,
1-[bis(4-méthoxycarbonylphényl)méthyl]-3-[(3,5-difluorophényl)(méthylsulfonyl) méthylène]azétidine,
3-acétoxy-1-[bis(4-méthoxycarbonylphényl)méthyl]-3-[(3,5-difluorophényl) (méthylsulfonyl)méthyl-(RS)]azétidine,
(RS)-4-[4-((4-chlorophényl){3-[(3,5-difluorophényl)méthanesulfonyl-méthylene] azétidin-1-yl}-méthyl)benzyl]morpholine,
4-(4- {3-[(1-benzhydryl-azétidin-3-ylidene)méthanesulfonyl-méthyl]phénoxy}butyl)morpholine,
4-(4- {3-[(1-benzhydryl-azétidin-3-ylidene)méthanesulfonyl-méthyl]phénoxy}-propyl)morpholine,
leurs isomères optiques et leurs sels avec un acide minéral ou organique.

10. Les composés, selon la revendication 1, suivants :
1-[bis(4-chlorophényl)méthyl]-3-[(3,5-difluorophényl)(méthylsulfonyl)méthylène] azétidine,
1-[bis(4-chlorophényl)méthyl]-3-[(3,5-difluorophényl)(méthylsulfonyl)méthyl-(RS)]azétidin-3-ol,
3-acétoxy-1-[bis-(4-chlorophényl)méthyl]-3-[(3,5-difluorophényl)(méthyl-ulfonyl)méthyl)méthyl sulfonylméthyl-(RS)]azétidine
leurs isomères optiques et leurs sels avec un acide minéral ou organique.

11. Procédé de préparation des composés de formule (I) selon la revendication 1 pour lesquels R représente une chaîne de formule (A) **caractérisé en ce que** l'on déshydrate un composé de formule (Ia) correspondant : 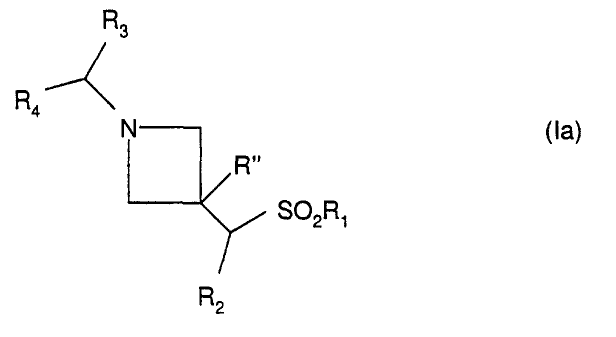 dans laquelle R₁, R₂, R₃ et R₄ ont les mêmes significations que dans la revendication 1 et R" représente un radical hydroxy, méthanesulfonyloxy ou acétyloxy, isole le produit et le transforme éventuellement en sel avec un acide minéral ou organique.

12. Procédé de préparation des composés de formule (I) selon la revendication 1 pour lesquels R représente une chaîne (B) dans laquelle R' est un atome d'hydrogène **caractérisé en ce que** l'on fait réagir un dérivé R₁SO₂CH₂R₂ (II) pour lequel R₁ et R₂ ont les mêmes significations que dans la revendication 1 sur une azétidinone de formule : 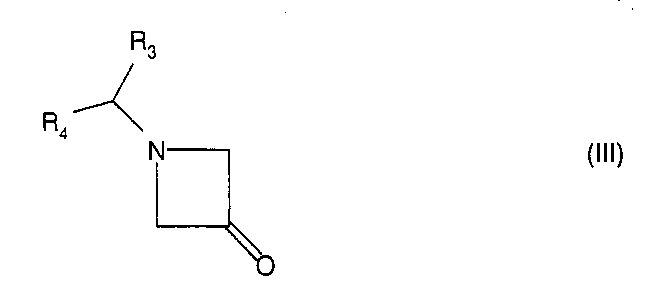 dans laquelle R₃ et R₄ ont les mêmes significations que dans la revendication 1, isole le produit et le transforme éventuellement en sel avec un acide minéral ou organique.

13. Procédé de préparation des composés de formule (I) selon la revendication 1 pour lesquels R représente une chaîne (B) dans laquelle R' est un atome d'hydrogène **caractérisé en ce que** l'on fait réagir un dérivé R₃CH(Br)R₄ pour lequel R₃ et R₄ ont les mêmes significations que dans revendication 1 sur un dérivé de formule : 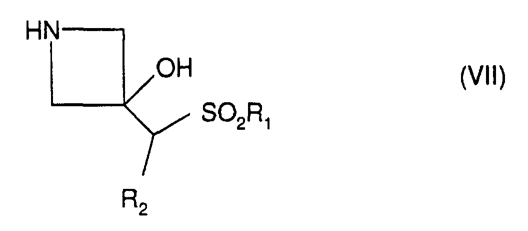 dans laquelle R₁ et R₂ ont les mêmes significations que dans la revendication 1, isole le produit et le transforme éventuellement en sel avec un acide minéral ou organique.

14. Procédé de préparation des composés de formule (I) selon la revendication 1 pour lesquels R est une chaîne (B) dans laquelle R' est un radical -CO-alk **caractérisé en ce que** l'on fait réagir un halogènure Hal-CO-alk dans lequel Hal représente un atome d'halogène et alk représente un radical alkyle en chaîne droite ou ramifiée et contenant 1 à 6 atomes de carbone sur un composé de formule (I) correspondant pour lequel R est une chaîne (B) dans laquelle R' est un atome d'hydrogène, isole le produit et le transforme éventuellement en sel avec un acide minéral ou organique.

15. Procédé de préparation des composés de formule (I) selon la revendication 1 pour lesquels R₂ représente un aromatique ou un hétéroaromatique substitué par -NR₆R₇ dans lequel R₆ et R₇ représentent chacun un atome d'hydrogène **caractérisé en ce que** l'on réduit un composé de formule (I) correpondant pour lequel R₂ représente un aromatique ou un hétéroaromatique substitué par nitro, isole le produit et le transforme éventuellement en sel avec un acide minéral ou organique.

16. Procédé de préparation des composés de formule (I) selon la revendication 1 pour lesquels R₂ représente un aromatique ou hétéroaromatique substitué par -CONHR₉ et/ou R₃ et/ou R₄ représentent un aromatique ou un hétaroaromatique substitué par -CONR₁₀R₁₁ **caractérisé en ce que** l'on fait réagir un composé de formule (I) correspondant pour lequel R₂ et/ou R₃ et/ou R₄ représentent un aromatique ou un hétéroaromatique substitué par -COOR₅ pour lequel R₅ est alkyle ou phényle éventuellement substitué par des halogènes, avec respectivement une amine H₂NR₉ ou HNR₁₀R₁₁ pour lesquelles R₉, R₁₀ et R₁₁ ont les mêmes significations que dans la revendication 1, isole le produit et le transforme éventuellement en sel avec un acide minéral ou organique.

17. Procédé de préparation des composés de formule (I) selon la revendication 1 pour lesquels R₂ représente un aromatique substitué par hydroxy et/ou R₃ et/ou R₄ représentent un aromatique ou un hétéroaromatique substitué par hydroxy **caractérisé en ce que** l'on hydrolyse un composé de formule (I) correspondant pour lequel R₂ représente un aromatique substitué par alcoxy et/ou R₃ et/ou R₄ représentent un aromatique ou un hétéroaromatique substitué par alcoxy, isole le produit et le transforme éventuellement en sel avec un acide minéral ou organique.

18. Procédé de préparation des composés de formule (I) selon la revendication 1 pour lesquels R₂ représente un aromatique substitué par -NR₆R₇ pour lequel R₆ représente un radical alkyle et R₇ représente un atome d'hydrogène **caractérisé en ce que** l'on déprotège un composé de formule (I) correspondant pour lequel R₂ représente un aromatique substitué par un -N(alk)COOR₈ dans lequel R₈ représente un radical tertbutyle, isole le produit et le transforme éventuellement en sel avec un acide minéral ou organique.

19. Procédé de préparation des composés de formule (I) selon la revendication 1 pour lesquels R₂ et/ou R₃ et/ou R₄ représentent un aromatique ou un hétéroaromatique substitué par -COOR₅ **caractérisé en ce que** l'on estérifie un dérivé de formule : 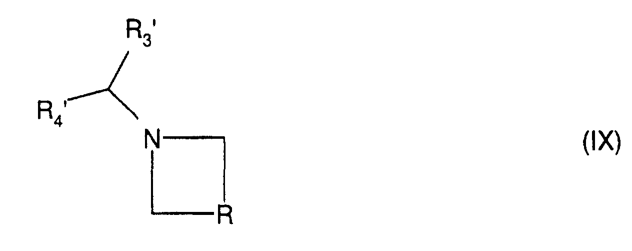 pour lequel R représente une chaîne C=C(SO₂R₁)R'₂ ou C(OR')CH(SO₂R₁)R'₂, R₁, R'₂, R'₃ et R'₄ ont les mêmes significations que les substituants R₁, R₂, R₃ et R₄ de la revendication 1 sous réserve qu'au moins un des substituants R'₂, R'₃, R'₄ représente un aromatique ou un hétéroaromatique substitué par carboxyle, au moyen d'un dérivé de formule R₅OH pour lequel R₅ est alkyle ou phényle éventuellement substitué par un ou plusieurs halogène, isole le produit et le transforme éventuellement en sel avec un acide minéral ou organique.

20. Procédé de préparation des composés de formule (I) selon la revendication 1 pour lesquels R₂ et/ou R₃ et/ou R₄ représentent un aromatique ou un hétéroaromatique substitué par alkylthioalkyle **caractérisé en ce que** l'on fait réagir un dérivé de formule 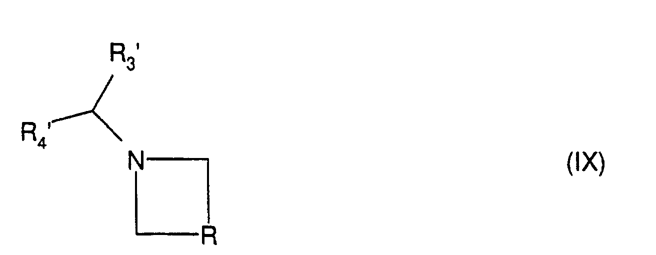 pour lequel R représente une chaîne C=C(SO₂R₁)R'₂ ou C(OR')CH(SO₂R₁)R'₂, R', R₁, R₂', R₃' et R₄' ont les mêmes significations que les substituants R', R₁, R₂, R₃ et R₄ de la revendication 1 sous réserve qu'au moins un des substituants R₂', R₃', R₄' représente un aromatique ou un hétéroaromatique substitué par halogénoalkyle sur un alkylthiolate de sodium pour lequel la partie alkyle est en chaîne droite ou ramifiée et contient 1 à 6 atomes de carbone, isole le produit et le transforme éventuellement en sel avec un acide minéral ou organique.

21. Procédé de préparation des composés de formule (I) selon la revendication 1 pour lesquels R₂ et/ou R₃ et/ou R₄ représentent un aromatique substitué par hydroxyalkyle dans lequel l'alkyle contient un atome de carbone **caractérisé en ce que** l'on réduit un composé de formule (I) pour lequel au moins un des substituants R₂, R₃, R₄ représente un aromatique substitué par formyle, isole le produit et le transforme éventuellement en sel avec un acide minéral ou organique.

22. Procédé de préparation des composés de formule (I) selon la revendication 1 pour lesquels R₃ et/ou R₄ représente un aromatique substitué par -alk-NR₆R₇ pour lequel alk est un alkyle contenant un atome de carbone **caractérisé en ce que** l'on fait réagir un composé de formule (I) pour lequel au moins un des substituants R₃, R₄ représente un aromatique substitué par formyle sur une amine HNR₆R₇ dans laquelle R₆ et R₇ ont les mêmes significations que dans la formule (I), isole le produit et le transforme éventuellement en sel avec un acide minéral ou organique.

23. Procédé de préparation des composés de formule (I) selon la revendication 1 pour lesquels R₂ représente un aromatique ou un hétéroaromatique substitué par -CONHR₉ et/ou R₃ et/ou R₄ représente un aromatique ou un hétéroaromatique substitué par -CO-NR₁₀R₁₁ **caractérisé en ce que** l'on fait réagir un dérivé de formule : 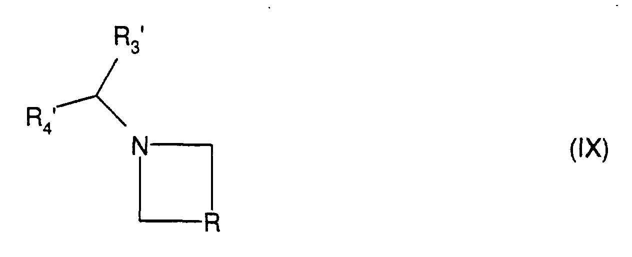 pour lequel R représente une chaîne C=C(SO₂R₁)R'₂ ou C(OR')CH(SO₂R₁)R'₂, R', R₁, R'₂, R'₃ et R'₄ ont les mêmes significations que les substituants R', R₁, R₂, R₃ et R₄ de la revendication 1 sous réserve qu'au moins un des substituants R'₂, R'₃, R'₄ représente un aromatique ou un hétéroaromatique substitué par carboxyle sur respectivement une amine H₂NR₉ ou HNR₁₀R₁₁ dans lesquelles R₉, R₁₀ et R₁₁ ont les mêmes significations que dans la formule (I), isole le produit et le transforme éventuellement en sel avec un acide minéral ou organique.

24. Procédé de préparation des composés de formule (I) selon la revendication 1 pour lesquels R₂ et/ou R₃ et/ou R₄ représentent un aromatique ou un hétéroaromatique substitué par -CO-NH-NR₆R₇ **caractérisé en ce que** l'on fait réagir un composé de formule (I) correspondant pour lequel R₂ et/ou R₃ et/ou R₄ représentent un aromatique ou un hétéroaromatique substitué par -COOR₅ et R₅ représente un radical alkyle ou phényle éventuellement substitué par des halogènes, sur une hydrazine H₂N-NR₆R₇ pour laquelle R₆ et R₇ ont les mêmes significations que dans la formule (I), isole le produit et le transforme éventuellement en sel avec un acide minéral ou organique.

25. Procédé de préparation des composés de formule (I) selon la revendication 1 pour lesquels R₂ représente un aromatique ou un hétéroaromatique substitué par -CO-NHR₉ dans lequel R₉ représente un atome d'hydrogène et/ou R₃ et/ou R₄ représentent un aromatique ou un hétéroaromatique substitué par -CO-NR₁₀R₁₁ dans lequel R₁₀ et R₁₁ sont des atomes d'hydrogène **caractérisé en ce que** l'on hydrolyse un composé de formule (I) correspondant pour lequel R₂ et/ou R₃ et/ou R₄ représentent un aromatique ou un hétéroaromatique substitué par cyano, isole le produit et le transforme éventuellement en sel avec un acide minéral ou organique.

26. Procédé de préparation des composés de formule (I) selon la revendication 1 pour lesquels R₂ représente un aromatique substitué par -O-alk-NR₁₂R₁₃ **caractérisé en ce que** l'on fait réagir un dérivé de formule : 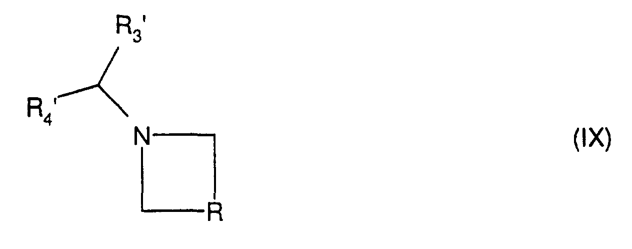 pour lequel R représente une chaîne C=C(SO₂R₁)R'₂ ou C(OR')CH(SO₂R₁)R'₂, R', R₁, R₂', R₃'et R₄' ont les mêmes significations que les substituants R', R₁, R₂, R₃ et R₄ de la revendication 1 sous réserve qu'au moins un des substituants R₂', R₃', R₄' représente un aromatique substitué par -O-alk-Hal dans lequel alk représente un radical alkyle en chaîne droite ou ramifiée et contenant 1 à 6 atomes de carbone et Hal représente un atome d'halogène sur une amine HNR₁₂R₁₃ dans laquelle R₁₂ R₁₃ ont les mêmes significations que dans la revendication 1, isole le produit et le transforme éventuellement en sel avec un acide minéral ou organique.

27. Procédé de préparation des composés de formule (I) selon la revendication 1 pour lesquels R₃ et/ou R₄ représente un aromatique substitué par -alk-NR₆R₇ **caractérisé en ce que** l'on fait réagir un dérivé de formule : 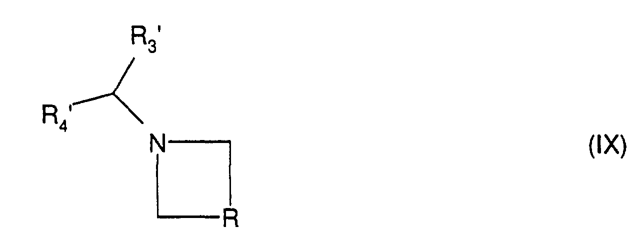 pour lequel R représente une chaîne C=C(SO₂R₁)R'₂ ou C(OR')CH(SO₂R₁)R'₂, R', R₁, R₂', R₃' et R₄' ont les mêmes significations que les substituants R', R₁, R₂, R₃ et R₄ de la revendication 1 sous réserve qu'au moins un des substituants R₃', R₄' représente un aromatique substitué par -alk-Cl dans lequel alk représente un radical alkyle contenant 1 à 6 atomes de carbone en chaîne droite ou ramifiée sur une amine HNR₆R₇ dans laquelle R₆ R₇ ont les mêmes significations que dans la revendication 1, isole le produit et le transforme éventuellement en sel avec un acide minéral ou organique.

28. Procédé de préparation des composés de formule (I) selon la revendication 1 pour lesquels R représente une chaîne B, R' représente un atome d'hydrogène et R₃ et/ou R₄ représente un aromatique substitué par hydroxyalkyle dans lequel le reste alkyle contient 1 atome de carbone **caractérisé en ce que** l'on fait réagir de l'hydrure de diisobutylaluminium sur un composé de formule (I) correspondant pour lequel R représente une chaîne B, R' représente un atome d'hydrogène et R₃ et/ou R₄ représente un aromatique substitué par un ou plusieurs radicaux -COOR₅ dans lequel R₅ est un radical alkyle, isole le produit et le transforme éventuellement en sel avec un acide minéral ou organique.

29. Procédé de préparation des composés de formule (I) selon la revendication 1 pour lesquels R₂ représente un radical phényle substitué par -NR₆R₇ représentant un cycle 1-pipérazinyl substitué en position -4 par un radical alkyle **caractérisé en ce que** l'on fait réagir un composé de formule (I) correspondant pour lequel R₂ représente un radical phényle substitué par un radical -NR₆R₇ représentant un cycle 1-pipérazinyl sur un dérivé alk-CHO dans lequel alk représente un radical alkyle en chaîne droite ou ramifiée contenant 1 à 5 atomes de carbone, isole le produit et le transforme éventuellement en sel avec un acide minéral ou organique.

30. Procédé de préparation des composés de formule (I) selon la revendication 1 pour lesquels R₂ représente un radical phényle substitué par -NR₆R₇ représentant un cycle 1-pipérazinyl substitué en position -4 par un radical -COOalk **caractérisé en ce que** l'on fait réagir un composé de formule (I) correspondant pour lequel R₂ représente un radical phényle substitué par un radical -NR₆R₇ représentant un cycle 1-pipérazinyl sur un dérivé de formule Hal-COOalk dans lequel alk représente un radical alkyle en chaîne droite ou ramifiée contenant 1 à 6 atomes de carbone et Hal représente un atome d'halogène, isole le produit et le transforme éventuellement en sel avec un acide minéral ou organique.

31. Procédé de préparation des composés de formule (I) selon la revendication 1 pour lesquels R₂ représente un radical phényle substitué par -NR₆R₇ représentant un cycle 1-pipérazinyl substitué en position -4 par un radical -CO-NHalk ou -CS-NHalk **caractérisé en ce que** l'on fait réagir un composé de formule (I) correspondant pour lequel R₂ représente un radical phényle substitué par -NR₆R₇ représentant un cycle 1-pipérazinyl sur un dérivé de formule Y=C=Nalk dans lequel alk représente un radical alkyle en chaîne droite ou ramifiée contenant 1 à 6 atomes de carbone et Y représente un atome de soufre ou d'oxygène, isole le produit et le transforme éventuellement en sel avec un acide minéral ou organique.

32. Procédé de préparation des composés de formule (I) selon la revendication 1 pour lesquels R₂ représente un radical phényle substitué par un radical -NR₆R₇ représentant un cycle 1-pipérazinyl substitué en position -4 par un radical -CO-alk-NR₁₄R₁₅ **caractérisé en ce que** l'on fait réagir un composé de formule (I) correspondant pour lequel R₂ représente un radical phényle substitué par un radical -NR₆R₇ représentant un cycle 1-pipérazinyl avec un acide de formule R₁₅R₁₄N-alk-COOH dans lequel alk représente un radical alkyle en chaîne droite ou ramifiée contenant 1 à 6 atomes de carbone et R₁₄ et R₁₅ ont les mêmes significations que dans la revendication 1 suivi éventuellement d'une déprotection du produit pour lequel R₁₄ est un radical tert-butoxycarbonyle pour obtenir les composés pour lesquels R₁₄ est un atome d'hydrogène, isole le produit et le transforme éventuellement en sel avec un acide minéral ou organique.

33. Procédé de préparation des composés de formule (I) selon la revendication 1 pour R₂ représente un radical phényle substitué par un radical -NR₆R₇ représentant un cycle 1-pipérazinyl substitué en position -4 par un radical -CO-alk dans lequel alk représente un radical méthyle **caractérisé en ce que** l'on fait réagir un composé de formule (I) correspondant pour lequel R₂ représente un radical phényle substitué par un radical -NR₆R₇ représentant un cycle 1-pipérazinyl avec l'anhydride acétique, isole le produit et le transforme éventuellement en sel avec un acide minéral ou organique.

34. Compositions pharmaceutiques contenant comme ingrédient actif au moins un composé de formule (I) selon l'une des revendications 1 à 10.

35. Composés de formule (I) selon l'une des revendication 1 à 10 à titre de médicament.

## Patentansprüche

1. Verbindungen der Formel 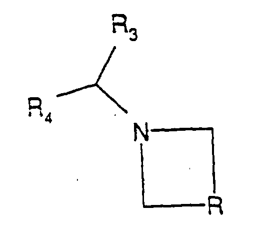 worin
R eine Kette 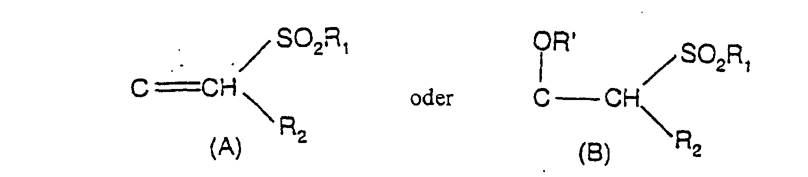 wiedergibt,
R₁ einen Methyl- oder Ethylrest bedeutet,
R₂ entweder einen aromatischen Rest, ausgewählt unter Phenyl, Naphthyl oder Indenyl, wobei diese aromatischen Reste unsubstituiert sind oder substituiert durch ein oder mehrere von Halogen, Alkyl, Alkoxy, -CO-alk, Hydroxy, -COOR₅, Formyl, Trifluormethyl, Trifluormethylsulfanyl, Trifluormethoxy, Nitro, -NR₆R₇, -CO-NH-NR₆R₇, -N(alk)COOR₈, Cyano, -CONHR₉, -CO-NR₁₆R₁₇, Alkylsulfanyl, Hydroxyalkyl, -O-alk-NR₁₂R₁₃ oder Alkylthioalkyl, oder einen heteroaromatischen Rest, ausgewählt unter den Ringen Benzofuryl, Benzothiazolyl, Benzothienyl, Benzoxazolyl, Chromanyl, 2,3-Dihydrobenzofuryl, 2,3-Dihydrobenzothienyl, Indolinyl, Indolyl, Isochromanyl, Isochinolyl, Pyridyl, Chinolyl, 1,2,3,4-Tetrahydroisochinolyl, 1,2,3,4-Tetrahydrochinolyl, Thiazolyl, Thienyl, wobei diese heteroaromatischen Reste unsubstituiert oder substituiert sein können durch ein Halogen, Alkyl, Alkoxy, -COOR₅, Trifluormethyl, Trifluormethylsulfanyl, Trifluormethoxy, Nitro, -NR₆R₇, -CO-NH-NR₆R₇, Cyano, -CONHR₉, Alkylsulfanyl, Hydroxyalkyl oder Alkylthioalkyl, bedeutet,
R₃und R₄ identisch oder voneinander verschieden entweder einen aromatischen Rest, ausgewählt unter Phenyl, Naphthyl oder Indenyl, wobei diese aromatischen Reste unsubstituiert oder substituiert sein können durch ein oder mehrere von Halogen, Alkyl, Alkoxy, Formyl, Hydroxy, Trifluormethyl, Trifluormethoxy, -CO-alk, Cyano, -COOR₅, -CONR₁₀R₁₁, -CO-NH-NR₆R₇, Alkylsulfanyl, Hydroxyalkyl, -alk-NR₆R₇, oder Alkylthioalkyl; oder einen heteroaromatischen Rest, ausgewählt unter den Ringen Benzofuryl, Benzothiazolyl, Benzothienyl, Benzoxazolyl, Chromanyl, 2,3-Dihydrobenzofuryl, 2,3-Dihydrobenzothienyl, Furyl, Isochromanyl, Isochinolyl, Pyrrolyl, Chinolyl, 1,2,3,4-Tetrahydroisochinolyl, Thiazolyl, Thienyl, wobei diese heteroaromatischen Reste unsubstituiert oder substituiert sein können durch ein Halogen, Alkyl, Alkoxy, Hydroxy, Trifluormethyl, Trifluormethoxy, Cyano, -COOR₅, -CO-NH-NR₆R₇, -CONR₁₀R₁₁, -alk-NR₆R₇, Alkylsulfanyl, Hydroxyalkyl oder Alkylthioalkyl, bedeuten,
R₅ ein Alkyl- oder ein Phenylrest ist, der gegebenenfalls durch ein oder mehrere Halogenatome substituiert ist,
R₆ und R₇ identisch oder voneinander verschieden ein Wasserstoffatom oder einen Rest Alkyl, -COOalk, Cycloalkyl, Alkylcycloalkyl, -alk-O-alk, Hydroxyalkyl bedeuten oder R₆ und R₇ gemeinsam mit dem Stickstoffatom, an das sie gebunden sind, einen gesättigten oder ungesättigten mono- oder bicyclischen Heterozyklus mit 3 bis 10 Gliedern bilden, der gegebenenfalls ein weiteres Heteroatom, ausgewählt unter Sauerstoff, Schwefel und Stickstoff, enthält, und gegebenenfalls substituiert ist durch einen oder mehrere Reste Alkyl, -COalk, -COOalk, -CO-NHalk, -CS-NHalk, -CO-alk-NR₁₄R₁₅, Oxo, Hydroxyalkyl, -alk-O-alk, -CO-NH₂,
R₈ einen Alkylrest bedeutet,
R₉ ein Wasserstoffatom oder einen Alkylrest oder Alkyl substituiert durch Dialkylamino, Phenyl, Cycloalkyl (gegebenenfalls durch -COOalk substituiert) oder einen gesättigten oder ungesättigten mono- oder bicyclischen Heterozyklus mit 3 bis 10 Gliedern, gegebenenfalls enthaltend ein oder mehrere Heteroatome, ausgewählt unter Sauerstoff, Schwefel und Stickstoff, und gegebenenfalls substituiert durch einen oder mehrere Alkylrest(e) bedeutet,
R₁₀ und R₁₁ identisch oder voneinander verschieden ein Wasserstoffatom oder einen Alkylrest bedeuten oder R₁₀ und R₁₁ gemeinsam mit dem Stickstoffatom, an das sie gebunden sind, einen gesättigten mono- oder bicyclischen Heterozyklus mit 3 bis 10 Gliedern bilden, der gegebenenfalls ein weiteres Heteroatom, ausgewählt unter Sauerstoff, Schwefel und Stickstoff, enthält und gegebenenfalls durch einen Alkylrest substituiert ist,
R₁₂ und R₁₃, identisch oder verschieden, ein Wasserstoffatom oder einen Rest Alkyl, Cycloalkyl bedeuten oder R₁₂ und R₁₃ gemeinsam mit dem Stickstoffatom, an das sie gebunden sind, einen gesättigten mono- oder bicyclischen Heterozyklus mit 3 bis 10 Gliedern, gegebenenfalls ein weiteres Heteroatom, ausgewählt unter Sauerstoff, Schwefel und Stickstoff, enthaltend und gegebenenfalls substituiert durch einen Rest Alkyl, -COalk, -COOalk, -CO-NHalk, -CS-NHalk, -CO-alk-NR₁₄R₁₅ oder einen gesättigten mono- oder bicyclischen Heterozyklus mit 3 bis 10 Gliedern und ein Heteroatom, ausgewählt unter Sauerstoff, Schwefel und Stickstoff enthaltend, bilden,
R₁₄ und R₁₅, identisch oder verschieden, ein Wasserstoffatom oder einen Rest Alkyl oder -COOalk bedeuten,
R₁₆ und R₁₇ gemeinsam mit dem Stickstoffatom, an das sie gebunden sind, einen gesättigten mono- oder bicyclischen Heterozyklus mit 3 bis 10 Gliedern, gegebenenfalls ein weiteres Heteroatom, ausgewählt unter Sauerstoff, Schwefel und Stickstoff, enthaltend, bilden,
R' ein Wasserstoffatom oder einen Rest -CO-alk bedeutet,
alk einen Alkyl- oder Alkylenrest bedeutet,
mit der Maßgabe, dass die Alkyl- oder Alkylenreste und -teile und die Alkoxyreste und -teile in gerader oder verzweigter Kette vorliegen und 1 bis 6 Kohlenstoffatome enthalten, deren optische Isomere und deren Salze mit einer Mineral- oder organischen Säure.

2. Verbindungen der Formel (I) gemäß Anspruch 1, worin R₆ und R₇ gemeinsam mit dem Stickstoffatom, an das sie gebunden sind, einen gesättigten oder ungesättigten mono- oder bicyclischen Heterozyklus mit 3 bis 10 Gliedern bilden, bei dem es sich um einen Azetidinyl-, Pyrrolidinyl-, Piperazinyl-, Piperidyl-, Morpholinyl-, Imidazolyl-, Thiomorpholinyl- oder Furylring handelt, wobei diese Ringe gegebenenfalls substituiert sind durch einen Rest Alkyl, Hydroxyalkyl, -alk-O-alk, -CONH₂, -COalk, -COOalk, Oxo, -CSNHalk, -CONHalk oder -CO-alk-NR₁₄R₁₅, worin alk, R₁₄ und R₁₅ die in Anspruch 1 angegebenen Bedeutungen besitzen, deren optische Isomere und deren Salze mit einer Mineral- oder organischen Säure.

3. Verbindungen der Formel (I) gemäß einem der Ansprüche 1 oder 2, worin R₁₀ und R₁₁ gemeinsam mit dem Stickstoffatom, an das sie gebunden sind, einen gesättigten mono- oder bicyclischen Heterozyklus mit 3 bis 10 Gliedern bilden, bei dem es sich um einen Azetidinyl-, Pyrrolidinyl-, Piperazinyl-, Piperidyl-, Morpholinyl- oder Thiomorpholinylring handelt, wobei diese Ringe gegebenenfalls durch ein Alkyl substituiert sind, deren optische Isomere und deren Salze mit einer Mineral- oder organischen Säure.

4. Verbindungen der Formel (I) gemäß einem der Ansprüche 1 bis 3, worin R₁₂ und R₁₃ gemeinsam mit dem Stickstoffatom, an das sie gebunden sind, einen gesättigten mono- oder bicyclischen Heterozyklus mit 3 bis 10 Gliedern bilden, bei dem es sich um einen Azetidinyl-, Pyrrolidinyl, Piperazinyl-, Piperidyl-, Morpholinyl- oder Thiomorpholinylring handelt, wobei diese Ringe gegebenenfalls substituiert sind durch einen Rest Alkyl, -COalk, -COOalk, -CO-NHalk, -CS-NHalk, -CO-alk-NR₁₄R₁₅ oder einen gesättigten mono- oder bicyclischen Heterozyklus mit 3 bis 10 Gliedern, der ein Heteroatom, ausgewählt unter Sauerstoff, Schwefel und Stickstoff, enthält, und insbesondere durch einen Thiomorpholinylrest, wobei alk, R₁₄ und R₁₅ die in Anspruch 1 angegebenen Bedeutungen besitzen, deren optische Isomere und deren Salze mit einer Mineral- oder organischen Säure.

5. Verbindungen der Formel (I) gemäß einem der Ansprüche 1 bis 4, worin R₁₆ und R₁₇ gemeinsam mit dem Stickstoffatom, an das sie gebunden sind, einen gesättigten mono- oder bicyclischen Heterozyklus mit 3 bis 10 Gliedern bilden, bei dem es sich um einen Piperidylring handelt, deren optische Isomere und deren Salze mit einer Mineraloder organischen Säure.

6. Verbindungen der Formel (I) gemäß einem der Ansprüche 1 bis 5, worin R₉ einen Alkylrest bedeutet, substituiert durch einen gesättigten oder ungesättigten monooder bicyclischen Heterozyklus mit 3 bis 10 Gliedern, der gegebenenfalls ein oder mehrere Heteroatom(e), ausgewählt unter Sauerstoff, Schwefel und Stickstoff, enthält, wobei es sich bei diesem letztgenannten um einen Pyrrolidinyl-, Tetrahydrofuryl-, Morpholinyl, Pyrrolylring handelt und diese Ringe gegebenenfalls durch einen oder mehrere Alkylrest(e) substituiert sind, deren optische Isomere und deren Salze mit einer Mineraloder organischen Säure.

7. Verbindungen der Formel (I) gemäß Anspruch 1, worin
R eine Kette (A) oder (B) bedeutet,
R' ein Wasserstoffatom oder einen Rest -COalk bedeutet,
R₁ einen Methyl- oder Ethylrest bedeutet,
R₂ entweder einen Aromaten, ausgewählt unter Phenyl und Naphthyl, wobei diese Aromaten unsubstituiert oder substituiert sind durch einen oder mehrere von Halogen, Alkyl, Alkoxy, Hydroxy, -COOR₅, Trifluormethyl, Trifluormethylsuflanyl, Trifluormethoxy, -NR₆R₇, -CO-NH-NR₆R₇, Cyano, -CONHR₉, Alkylsulfanyl, Hydroxyalkyl, Nitro, -CO-NR₁₆R₁₇, -O-alkNR₁₂R₁₃, oder Alkylthioalkyl oder einen Heteroaromaten, ausgewählt unter Isochinolyl, Pyridyl, Chinolyl, 1,2,3,4-Tetrahydroisochinolyl, 1,2,3,4-Tetrahydrochinolyl, Thienyl, wobei diese Heteroaromaten unsubstituiert oder substituiert sind durch einen von Halogen, Alkyl, Alkoxy, -COOR₅, Trifluormethyl, Trifluormethylsulfanyl, Trifluormethoxy, -NR₆R₇, -CO-NH-NR₆R₇, Cyano, -CONHR₉, Alkylsulfanyl, Hydroxyalkyl, Nitro oder Alkylthioalkyl bedeutet,
R₃ und R₄, gleich oder verschieden, entweder einen Aromaten, ausgewählt unter Phenyl oder Naphthyl, wobei diese Aromaten unsubstituiert oder substituiert sind durch einen oder mehrere von Halogen, Alkyl, Alkoxy, Trifluormethyl, Trifluormethoxy, -CONR₁₀R₁₁, -alk-NR₆R₇, Hydroxyalkyl, Formyl, -COOR₅, oder einen Heteroaromaten, ausgewählt unter den Thiazolyl- oder Thienylringen, wobei diese Heteroaromaten unsubstituiert oder substituiert sind durch ein Halogen, Alkyl, Alkoxy, -CONR₁₀R₁₁, -alk-NR₆R₇, Hydroxyalkyl oder -COOR₅ bedeuten,
R₅ Alkyl oder Phenyl, gegebenenfalls substituiert durch ein oder mehrere Halogen(e) bedeutet,
R₆ und R₇, gleich oder verschieden, ein Wasserstoffatom oder einen Rest Alkyl, -COOalk, Cycloalkyl, Alkylcycloalkyl, -alk-O-alk, Hydroxyalkyl bedeuten oder R₆ und R₇ gemeinsam mit dem Stickstoffatom, an das sie gebunden sind, einen gesättigten oder ungesättigten mono- oder bicyclischen Heterozyklus mit 3 bis 10 Gliedern bilden, der gegebenenfalls ein weiteres Heteroatom, ausgewählt unter Sauerstoff, Schwefel und Stickstoff, enthält und gegebenenfalls substituiert ist durch einen oder mehrere Reste Alkyl, -COalk, -COOalk, -CO-NHalk, -CS-NHalk, -CO-alk-NR₁₄R₁₅, Oxo, Hydroxyalkyl, -alk-O-alk, -CO-NH₂,
R₉ ein Wasserstoffatom oder einen Alkylrest oder Alkyl substituiert durch Dialkylamino, Phenyl, Cycloalkyl (gegebenenfalls durch -COOalk substituiert) oder einen gesättigten oder ungesättigten mono- oder bicyclischen Heterozyklus mit 3 bis 10 Gliedern, gegebenenfalls ein oder mehrere Heteroatome, ausgewählt unter Sauerstoff, Schwefel und Stickstoff, enthaltend und gegebenenfalls substituiert durch einen oder mehrere Alkylreste bedeutet,
R₁₀ und R₁₁, gleich oder verschieden, ein Wasserstoffatom oder einen Alkylrest bedeuten oder R₁₀ und R₁₁ gemeinsam mit dem Stickstoffatom, an das sie gebunden sind, einen gesättigten mono- oder bicyclischen Heterozyklus mit 3 bis 10 Gliedern bilden, der gegebenenfalls ein weiteres Heteroatom, ausgewählt unter Sauerstoff, Schwefel und Stickstoff, enthält und gegebenenfalls durch einen Alkylrest substituiert ist,
R₁₂ und R₁₃, gleich oder verschieden, ein Wasserstoffatom oder einen Rest Alkyl, Cycloalkyl bedeuten oder R₁₂ und R₁₃ gemeinsam mit dem Stickstoffatom, an das sie gebunden sind, einen gesättigten mono- oder bicyclischen Heterozyklus mit 3 bis 10 Gliedern bilden, der gegebenenfalls ein weiteres Heteroatom, ausgewählt unter Sauerstoff, Schwefel und Stickstoff, enthält und gegebenenfalls substituiert ist durch einen Rest Alkyl, -COalk, -COOalk, -CO-NHalk, -CS-NHalk, -CO-alk-NR₁₄R₁₅ oder einen gesättigten mono- oder bicyclischen Heterozyklus mit 3 bis 10 Gliedern, der ein Heteroatom, ausgewählt unter Sauerstoff, Schwefel und Stickstoff, enthält,
R₁₄ und R₁₅, gleich oder verschieden, ein Wasserstoffatom oder einen Rest Alkyl oder -COOalk bedeuten,
R₁₆ und R₁₇ gemeinsam mit dem Stickstoffatom, an das sie gebunden sind, einen gesättigten mono- oder bicyclischen Heterozyklus mit 3 bis 10 Gliedern bilden, der gegebenenfalls ein weiteres Heteroatom, ausgewählt unter Sauerstoff, Schwefel und Stickstoff, enthält,
alk einen Alkyl- oder Alkylenrest bedeutet,
mit der Maßgabe, dass die Alkyl- und Alkylenreste und -teile und die Alkoxyreste und -teile geradkettig oder verzweigt sind und 1 bis 6 Kohlenstoffatome enthalten,
deren optische Isomere und deren Salze mit einer Mineral- oder organischen Säure.

8. Verbindungen der Formel (I) gemäß Anspruch 1, worin
R eine Kette (A) oder (B) bedeutet,
R' ein Wasserstoffatom oder einen Rest -COalk bedeutet,
R₁ einen Methyl- oder Ethylrest bedeutet,
R₂ entweder einen Aromaten, ausgewählt unter
Naphthyl
Phenyl
Phenyl substituiert durch einen oder mehrere von Halogen, Alkyl, Alkoxy, Hydroxy, -COOR₅ (worin R₅ einen Rest Alkyl oder Phenyl gegebenenfalls durch mehrere Halogene substituiert bedeutet), Trifluormethyl, Trifluormethylsulfanyl, Trifluormethoxy, -NR₆R₇ (worin R₆ und R₇ gleich oder verschieden ein Wasserstoffatom oder einen Rest Alkyl oder -COOalk bedeuten oder R₆ und R₇ gemeinsam mit dem Stickstoffatom, an das sie gebunden sind, einen Heterozyklus, ausgewählt unter Pyrrolidinyl, Piperidyl, Piperazinyl oder Piperazinyl substituiert durch einen oder mehrere Reste, Alkyl, -COalk, -COOalk, -CO-NHalk, -CS-NHalk, -CO-alk-NR₁₄R₁₅ bilden, worin R₁₄ und R₁₅, gleich oder verschieden, ein Wasserstoffatom oder einen Alkylrest bedeuten), -CO-NH-NR₆R₇ (R₆ und R₇, gleich oder verschieden, ein Wasserstoffatom oder einen Alkylrest bedeuten oder R₆ und R₇ gemeinsam mit dem Stickstoffatom, an das sie gebunden sind, einen Heterozyklus, ausgewählt unter Piperidyl, Pyrrolyl, Piperazinyl oder Piperazyl substituiert durch einen oder mehrere Alkylrest(e) bilden), Cyano, -CONHR₉ (worin R₉ ein Wasserstoffatom oder einen Rest Alkyl oder Alkyl substituiert durch Dialkylamino, Phenyl, Cycloalkyl (gegebenenfalls durch -COOalk substituiert) oder einen Heterozyklus, ausgewählt unter Pyrrolidinyl (gegebenenfalls durch Alkyl substituiert), Tetrahydrofuryl, Morpholinyl bedeutet), Alkylsulfanyl, Hydroxyalkyl, Nitro, -CO-NR₁₆R₁₇ (worin R₁₆ und R₁₇ gemeinsam mit dem Stickstoffatom, an das sie gebunden sind, einen Piperidylring bilden), -O-alkNR₁₂R₁₃ (worin R₁₂ und R₁₃ gemeinsam mit dem Stickstoffatom, an das sie gebunden sind, einen Morpholinoring bilden) oder Alkylthioalkyl,
oder einen Heteroaromaten, ausgewählt unter
Isochinolyl,
Pyridyl,
Chinolyl,
1,2,3,4-Tetrahydroisochinolyl,
1,2,3,4-Tetrahydrochinolyl
Thienyl, oder
Thienyl substituiert durch ein -COOR₅ (worin R₅ einen Alkylrest bedeutet) oder -CONHR₉ (worin R₉ einen Alkylrest bedeutet) bedeutet,
R₃ und R₄, gleich oder verschieden, entweder einen Aromaten, ausgewählt unter
Phenyl, oder
Phenyl substituiert durch einen oder mehrere von Halogen, Alkyl, Alkoxy, Trifluormethyl, Trifluormethoxy, Hydroxyalkyl, Formyl, -COOR₅ (worin R₅ einen Alkylrest bedeutet), -C0NR₁₀R₁₁ (worin R₁₀ und R₁₁, gleich oder verschieden, ein Wasserstoffatom oder einen Alkylrest bedeuten), -alk-NR₆R₇ (worin R₆ und R₇, gleich oder verschieden, ein Wasserstoffatom oder einen Rest Alkyl, Cycloalkyl, -alk-O-alk, Hydroxyalkyl bedeuten oder R₆ und R₇ gemeinsam mit dem Stickstoffatom, an das sie gebunden sind, einen Heterozyklus bilden, ausgewählt unter Piperidyl (gegebenenfalls substituiert durch Alkyl, Oxo), Pyrrolidinyl (gegebenenfalls substituiert durch Alkyl, Hydroxyalkyl, -alk-O-alk, -CO-NH₂), Thiomorpholinyl, Morpholinyl, Pyrrolyl, Piperazinyl gegebenenfalls substituiert durch Oxo, Alkyl, Hydroxyalkyl, -COOR₅ (worin R₅ einen Alkylrest bedeutet),
oder einen Heteroaromaten, ausgewählt unter
Thiazolyl oder
Thienyl, bedeuten,
alk einen Alkyl- oder Alkylenrest bedeuten,
mit der Maßgabe, dass die Alkyl- und Alkylenreste und -teile und die Alkoxyreste und -teile geradekettig oder verzweigt sind und 1 bis 6 Kohlenstoffatome enthalten,
deren optische Isomere und deren Salze mit einer Mineral- oder organischen Säure.

9. Verbindungen gemäß Anspruch 1, ausgewählt unter
1-Benzhydryl-3-[(methylsulfonyl)-(phenyl)-methylen]-azetidin
1-Benzhydryl-3-[(methylphenyl)-(methylsulfonyl)-methylen] -azetidin
1-Benzhydryl-3-[(3-chlorphenyl)-(methylsulfonyl)-methylen]-azetidin
1-Benzhydryl-3-[(3,5-dichlorphenyl)-(methylsulfonyl)-methylen]-azetidin
1-Benzhydryl-3-[(2,5-dichlorphenyl)-(methylsulfonyl)-methylen]-azetidin
1-Benzhydryl-3-[(2,3-dichlorphenyl)-(methylsulfonyl)-methylen)-azetidin
1-Benzhydryl-3-[(3-fluorphenyl)-(methylsulfonyl)-methylen]-azetidin
1-Benzhydryl-3-[(3,5-difluorphenyl)-(methylsulfonyl)-methylen]-azetidin
1-Benzhydryl-3-[(3-bromphenyl)-(methylsulfonyl)-methylen]-azetidin
1-Benzhydryl-3-[(3-iodphenyl)-(methylsulfonyl)-methylen]-azetidin
1-Benzhydryl-3-[(methylsulfonyl)-(3-trifluormethoxyphenyl)-methylen]-azetidin
1-Benzhydryl-3-[(methylsulfonyl)-(3-trifluormethylphenyl)-methylen]-azetidin
1-Benzhydryl-3-[(3,5-bis(trifluormethyl)-phenyl]-(methylsulfonyl)-methylen]-azetidin
1-Benzhydryl-3-[(3,5-dibromphenyl)-(methylsulfonyl)-methylen]-azetidin
1-Benzhydryl-3-[(3-methoxycarbonylphenyl)-(methylsulfonyl)-methylen]-azetidin
1-Benzhydryl-3-[(3-cyanophenyl)-(methylsulfonyl)-methylen]-azetidin
1-Benzhydryl-3-[(3-carbamoylphenyl)-(methylsulfonyl)-methylen]-azetidin
1-Benzhydryl-3-[(methylsulfonyl)-(napth-1-yl)-(methylsulfonyl)-methylen]-azetidin
1-[Bis-(4-chlorphenyl)-methyl]-3-[(3,5-difluorphenyl)-(methylsulfonyl)-methylen]-azetidin
1-[Bis-(4-methoxyphenyl)-methyl]-3-[(3,5-difluorphenyl)-(methylsulfonyl)-methylen]-azetidin
1-[Bis-(4-methylphenyl)-methyl]-3-[(3,5-difluorphenyl)-(methylsulfonyl)-methylen]-azetidin
(RS)-3-[(3,5-Difluorphenyl)-(methylsulfonyl)-methylen]-1-[(4-methoxyphenyl)-(phenyl)-methyl)-]-azetidin
(R)-3-[(3,5-Difluorphenyl)-(methylsulfonyl)-methylen]-1-[(4-methoxyphenyl)-(phenyl)-methyl)-]-azetidin
(S)-3-[(3,5-Difluorphenyl)-(methylsulfonyl)-methylen]-1-[(4-methoxyphenyl)-(phenyl)-methyl)-]-azetidin
1-[Bis-(4-trifluormethoxyphenyl)-methyl]-3-[(3,5-difluorphenyl)-(methylsulfonyl)-methylen-azetidin
1-[Bis-(4-trifluormethylphenyl)-methyl]-3-[(3,5-difluorphenyl)-(methylsulfonyl)-methylen-azetidin
1-[Bis-(4-chlorphenyl)-methyl]-3-{[3,5-bis-(trifluormethyl)-phenyl)]-(methylsulfonyl)-methylen-azetidin
(RS)-1-[(4-Chlorphenyl)-(2,4-dichlorphenyl)-methyl]-3-[(3,5-difluorphenyl)-(methylsulfonyl)-methylen]-azetidin
(R)-1-[(4-Chlorphenyl)-(2,4-dichlorphenyl)-methyl]-3-[(3,5-difluorphenyl)-(methylsulfonyl)-methylen]-azetidin
(S)-1-[(4-Chlorphenyl)-(2,4-dichlorphenyl)-methyl]-3-[(3,5-difluorphenyl)-(methylsulfonyl)-methylen]-azetidin
(RS)-1-{(4-Chlorphenyl)[4-hydroxymethyl)-phenyl]-methyl}-3-[3,5-difluorphenyl)-(methylsulfonyl)-methylen]-azetidin
(R)-1-{(4-Chlorphenyl)[4-hydroxymethyl)-phenyl]-methyl}-3-[3,5-difluorphenyl)-(methylsulfonyl)-methylen]-azetidin
(S)-1-{(4-Chlorphenyl)[4-hydroxymethyl)-phenyl]-methyl}-3-[3,5-difluorphenyl)-(methylsulfonyl)-methylen]-azetidin
(RS)-1-{(4-Chlorphenyl)-[4-pyrrolidylmethyl)-phenyl]-methyl}-3-[(3,5-difluorphenyl)-(methylsulfonyl)-methylen]-azetidin
(R)-1-{(4-Chlorphenyl)-[4-pyrrolidylmethyl)-phenyl]-methyl}-3-[(3,5-difluorphenyl)-(methylsulfonyl)-methylen]-azetidin
(S)-1-{(4-Chlorphenyl)-[4-pyrrolidylmethyl)-phenyl]-methyl}-3-[(3,5-difluorphenyl)-(methylsulfonyl)-methylen]-azetidin
1-{(RS)-(4-Chlorphenyl)-[4-(3,3-dimethylpiperidin-1-yl-methyl)-phenyl]-methyl}-3-[(3,5-difluorphenyl)-(methylsulfonyl)-methylen]-azetidin
1-{(R)-(4-Chlorphenyl)-[4-(3,3-dimethylpiperidin-1-yl-methyl)-phenyl]-methyl} -3-[(3,5-difluorphenyl)-(methylsulfonyl)-methylen]-azetidin
1-{(S)-(4-Chlorphenyl)-[4-(3,3-dimethylpiperidin-1-yl-methyl)-phenyl]-methyl}-3-[(3,5-difluorphenyl)-(methylsulfonyl)-methylen]-azetidin
1-{(RS)-(4-Chlorphenyl)-[4-(thiomorpholin-4-yl-methyl) -phenyl]-methyl}-3-[(3,5-difluorphenyl)-(methylsulfonyl)-methylen]-azetidin
1-{(R)-(4-Chlorphenyl)-[4-(thiomorpholin-4-yl-methyl)-phenyl]-methyl}-3-[(3,5-difluorphenyl)-(methylsulfonyl)-methylen]-azetidin
1-{(S)-(4-Chlorphenyl)-[4-(thiomorpholin-4-yl-methyl) -phenyl]-methyl}-3-[(3,5-difluorphenyl)-(methylsulfonyl)-methylen]-azetidin 1-{(RS)(4-Chlorphenyl)-[4-(N-ethyl-N-cyclohexyl-aminomethyl)-phenyl)-methyl}-3-[(3,5 -difluorphenyl)-(methylsulfonyl)-methylen]-azetidin
1-{(R)(4-Chlorphenyl)-[4-(N-ethyl-N-cyclohexyl-aminomethyl)-phenyl)-methyl}-3-[(3,5-difluorphenyl)-(methylsulfonyl)-methylen]-azetidin
1-{(S)(4-Chlorphenyl)-[4-(N-ethyl-N-cyclohexyl-aminomethyl)-phenyl)-methyl}-3-[(3,5-difluorphenyl)-(methylsulfonyl)-methylen]-azetidin
1 {{(RS)-(4-Chlorphenyl)- {4-[(4-ethoxycarbonylpiperazinyl)-methyl]-phenyl}methyl} }-3-[(3,5-difluorphenyl)-(methylsulfonyl)-methylen]-azetidin
1 {{(R)-(4-Chlorphenyl)- {4-[(4-ethoxycarbonylpiperazinyl)-methyl]-phenyl}-methyl} }-3-[(3,5-difluorphenyl)-(methylsulfonyl)-methylen]-azetidin
1 {{(S)-(4-Chlorphenyl)-{4-[(4-ethoxycarbonylpiperazinyl)-methyl]-phenyl}-methyl}}-3-[(3,5-difluorphenyl)-(methylsulfonyl)-methylen]-azetidin
1-{(RS)-(4-Chlorphenyl)-[4-(N-cyclopropyl-N-propyl-aminomethyl)-phenyl]-methyl}-3-[(3,5-difluorphenyl)-(methylsulfonyl)-methylen]-azetidin
1-{(R)-(4-Chlorphenyl)-[4-(N-cyclopropyl-N-propyl-aminomethyl)-phenyl]-methyl}-3-[(3,5-difluorphenyl)-(methylsulfonyl)-methylen]-azetidin
1-{(S)-(4-Chlorphenyl)-[4-(N-cyclopropyl-N-propyl-aminomethyl)-phenyl]-methyl}-3-[(3,5-difluorphenyl)-(methylsulfonyl)-methylen]-azetidin
1-{(RS)-(4-Chlorphenyl)-[4-(diisopropylaminomethyl)-phenyl]-methyl}-3-[(3,5-difluorphenyl)-(methylsulfonyl)-methylen]-azetidin
1-{(R)-(4-Chlorphenyl)-[4-(diisopropylaminomethyl)-phenyl]-methyl}-3-[(3,5-difluorphenyl)-(methylsulfonyl)-methylen]-azetidin
1-{(S)-(4-Chlorphenyl)-[4-(diisopropylaminomethyl)-phenyl]-methyl}-3-[(3,5-difluorphenyl)-(methylsulfonyl)-methylen]-azetidin
1{{(RS)-(4-Chlorphenyl)-{4-[bis-(2-methoxyethyl)-aminomethyl]-phenyl}-methyl}}-3-[(3,5-difluorphenyl)-(methylsulfonyl)-methylen]-azetidin
1{{(R)-(4-Chlorphenyl)- {4-[bis-(2-methoxyethyl)-aminomethyl]-phenyl}-methyl}}-3-[(3,5-difluorphenyl)-(methylsulfonyl)-methylen]-azetidin
1{{(S)-(4-Chlorphenyl)-{4-[bis-(2-methoxyethyl)-aminomethyl]-phenyl}-methyl}}-3-[(3,5-difluorphenyl)-(methylsulfonyl)-methylen]-azetidin
1-{(RS)-(4-Chlorphenyl)-[4-(di-n-propylaminomethyl)-phenyl]-methyl}-3-[(3,5-difluorphenyl)-(methylsulfonyl)-methylen]-azetidin
1-{(R)-(4-Chlorphenyl)-[4-(di-n-propylaminomethyl)-phenyl]-methyl}-3-[(3,5-difluorphenyl)-(methylsulfonyl)-methylen]-azetidin
1-{(S)-(4-Chlorphenyl)-[4-(di-n-propylaminomethyl)-phenyl]-methyl}-3-[(3,5-difluorphenyl)-(methylsulfonyl)-methylen]-azetidin
1- {(RS)-(4-Chlorphenyl)-[4-(piperidin-1-yl-methyl)-phenyl]-methyl}-3-[(3,5-difluorphenyl)-(methylsulfonyl)-methylen]-azetidin
1-{(R)-(4-Chlorphenyl)-[4-(piperidin-1-yl-methyl)-phenyl]-methyl} -3-[(3,5-difluorphenyl)-(methylsulfonyl)-methylen]-azetidin
1-{(S)-(4-Chlorphenyl)-[4-(piperidin-1-yl-methyl)-phenyl]-methyl}-3-[(3,5-difluorphenyl)-(methylsulfonyl)-methylen]-azetidin
1-{(RS)-(4-Chlorphenyl)-[4-(4-methyl-piperazin-1-yl-methyl)-phenyl]-methyl}-3-[(3,5-difluorphenyl)-(methylsulfonyl)-methylen]-azetidin
1-{(R)-(4-Chlorphenyl)-[4-(4-methyl-piperazin-1-yl-methyl)-phenyl]-methyl}-3-[(3,5-difluorphenyl)-(methylsulfonyl)-methylen]-azetidin
1-{(S)-(4-Chlorphenyl)-[4-(4-methyl-piperazin-1-yl-methyl)-phenyl]-methyl}-3-[(3,5-difluorphenyl)-(methylsulfonyl)-methylen]-azetidin
1-{(RS)-(4-Chlorphenyl)-[4-(morpholin-4-yl-methyl)-phenyl]-methyl}-3-[(3,5-difluorphenyl)-(methylsulfonyl)-methylen]-azetidin
1-{(R)-(4-Chlorphenyl)-[4-(morpholin-4-yl-methyl)-phenyl]-methyl}-3-[(3,5-difluorphenyl)-(methylsulfonyl)-methylen]-azetidin
1-{(S)-(4-Chlorphenyl)-[4-(morpholin-4-yl-methyl)-phenyl]-methyl}-3-[(3,5-difluorphenyl)-(methylsulfonyl)-methylen]-azetidin
1-{(RS)-(4-Chlorphenyl)-[4-(diethylaminomethyl)-phenyl]-methyl}-3-[(3,5-difluorphenyl)-(methylsulfonyl)-methylen]-azetidin
1-{(R)-(4-Chlorphenyl)-[4-(diethylaminomethyl)-phenyl]-methyl}-3-[(3,5-difluorphenyl)-(methylsulfonyl)-methylen]-azetidin
1-{(S)-(4-Chlorphenyl)-[4-(diethylaminomethyl)-phenyl]-methyl}-3-[(3,5-difluorphenyl)-(methylsulfonyl)-methylen]-azetidin
1-{(RS)-(4-Chlorphenyl)-[4-(piperazin-2-on-4-yl-methyl)-phenyl]-methyl}-3-[3,5-difluorphenyl)-(methylsulfonyl)-methylen]-azetidin
1-{(R)-(4-Chlorphenyl)-[4-(piperazin-2-on-4-yl-methyl)-phenyl]-methyl}-3-[3,5-difluorphenyl)-(methylsulfonyl)-methylen]-azetidin
1-{(S)-(4-Chlorphenyl)-[4-(piperazin-2-on-4-yl-methyl)-phenyl]-methyl}-3-[3,5-difluorphenyl)-(methylsulfonyl)-methylen]-azetidin
1-{(RS)-(4-Chlorphenyl)-[4-(imidazol-1-yl-methyl)-phenyl]-methyl}-3-[(3,5-difluorphenyl)-(methylsulfonyl)-methylen]-azetidin
1-{(R)-(4-Chlorphenyl)-[4-(imidazol-1-yl-methyl)-phenyl]-methyl}-3-[(3,5-difluorphenyl)-(methylsulfonyl)-methylen]-azetidin
1-{(S)-(4-Chlorphenyl)-[4-(imidazol-1-yl-methyl)-phenyl]-methyl}-3-[(3,5-difluorphenyl)-(methylsulfonyl)-methylen]-azetidin
(RS)-1-{(4-Chlorphenyl)-[4-(N,N-dimethylcarbamoyl)-phenyl]-methyl}-3-[(3,5-difluorphenyl)-(methylsulfonyl)-methylen]-azetidin
(R)-1-{(4-Chlorphenyl)-[4-(N,N-dimethylcarbamoyl)-phenyl]-methyl}-3-[(3,5-difluorphenyl)-(methylsulfonyl)-methylen]-azetidin
(S)-1-{(4-Chlorphenyl)-[4-(N,N-dimethylcarbamoyl)-phenyl]-methyl}-3-[(3,5-difluorphenyl)-(methylsulfonyl)-methylen]-azetidin
(RS)-1-{(4-Chlorphenyl)-[4-(N-ethylcarbamoyl)-phenyl]-methyl}-3-[(3,5-difluorphenyl)-(methylsulfonyl)-methylen]-azetidin
(R)-1-{(4-Chlorphenyl)-[4-(N-ethylcarbamoyl)-phenyl]-methyl}-3-[(3,5-difluorphenyl)-(methylsulfonyl)-methylen]-azetidin
(S)-1-{(4-Chlorphenyl)-[4-(N-ethylcarbamoyl)-phenyl]-methyl}-3-[(3,5-difluorphenyl)-(methylsulfonyl)-methylen]-azetidin
(RS)-1-[(4-Carbamoylphenyl)-(4-chlorphenyl)-methyl]-3-[(3,5-difluorphenyl)-(methylsulfonyl)-methylen]-azetidin
(R)-1-[(4-Carbamoylphenyl)-(4-chlorphenyl)-methyl]-3-[(3,5-difluorphenyl)-(methylsulfonyl)-methylen]-azetidin
(S)-1-[(4-Carbamoylphenyl)-(4-chlorphenyl)-methyl]-3-[(3,5-difluorphenyl)-(methylsulfonyl)-methylen]-azetidin
1-Bis-(4-chlorphenyl)-methyl]-3-[(3,5-dichlorphenyl)-(methylsulfonyl)-methylen]-azetidin
1-Benzhydryl-3-[(3-methylsulfanylphenyl)-(methylsulfonyl)-methylen]-azetidin
1-Benzhydryl-3-[(3-methylsulfanylmethyl)-phenyl)]-(methylsulfonyl)-methylen]-azetidin
1-[Bis-(4-chlorphenyl)-methyl]-3-[(3-cyanophenyl)-(methylsulfonyl)-methylen]-azetidin
1-[Bis-(4-chlorphenyl)-methyl]-3-[(3-carbamoylphenyl)-(methylsulfonyl)-methylen]-azetidin
1-[Bis-(4-chlorphenyl)-methyl]-3-[(3-methoxyphenyl)-(methylsulfonyl)-methylen]-azetidin
1-[Bis-(4-chlorphenyl)-methyl]-3-[(3-hydroxyphenyl)-(methylsulfonyl)-methylen]-azetidin
1-[Bis-(4-chlorphenyl)-methyl]-3-[(methylsulfonyl)-(3-pyrrolidinylphenyl)-methylen]-azetidin
1-[Bis-(4-chlorphenyl)-methyl]-3-[(3-hydroxymethylphenyl)-(methylsulfonyl)-methylen]-azetidin
1-[Bis-(4-chlorphenyl)-methyl]-3-{(methylsulfonyl)-[3-(N-piperidylcarbamoyl)-phenyl]-methylen]-azetidin
1-[Bis-(4-chlorphenyl)-methyl]-3-[(methylsulfonyl)-(3-trifluormethylsulfanylphenyl)-(methylsulfonyl)-methylen]-azetidin
1-[Bis-(4-fluorphenyl)-methyl]-3-[(difluorphenyl)-(methylsulfonyl)-methylen]-azetidin
1-[Bis-(2-fluorphenyl)-methyl]-3-[(3,5-difluorphenyl)-(methylsulfonyl)-methylen]-azetidin
1-[Bis-(3-fluorphenyl)-methyl]-3-[(3,5-difluorphenyl)-(methylsulfonyl)-methylen]-azetidin
(RS)-1-[(4-Chlorphenyl)-(thiazol-2-yl)-methyl]-3-[(methylsulfonyl)-(phenyl)-methylen]-azetidin
(R)-1-[(4-Chlorphenyl)-(thiazol-2-yl)-methyl]-3-[(methylsulfonyl)-(phenyl)-methylen]-azetidin
(S)-1-[(4-Chlorphenyl)-(thiazol-2-yl)-methyl]-3-[(methylsulfonyl)-(phenyl)-methylen]-azetidin
(RS)-1-[(4-Chlorphenyl)-(thien-2-yl)-methyl]-3-[(3,5-difluorphenyl)-(methylsulfonyl)-methylen]-azetidin
(R)-1-[(4-Chlorphenyl)-(thien-2-yl)-methyl]-3-[(3,5-difluorphenyl)-(methylsulfonyl)-methylen]-azetidin
(S)-1-[(4-Chlorphenyl)-(thien-2-yl)-methyl]-3-[(3,5-difluorphenyl)-(methylsulfonyl)-methylen]-azetidin
1-Benzhydryl-3-[(ethylsulfonyl)-(phenyl)-methylen]-azetidin
1-Bis-(4-chlorphenyl)-methyl]-3-{{3-[N-(4-methylpiperazinyl)-carbamoyl]-phenyl}-(methylsulfonyl)-methylen} -azetidin
1-Bis-(4-chlorphenyl)-methyl]-3-{[3-(2,2-dimethylcarbohydrazido)-phenyl]-(methylsulfonyl)-methylen}-azetidin
1-[Bis-(thien-2-yl)-methyl]-3-[(3,5-difluorphenyl)-(methylsulfonyl)-methylen]-azetidin
1-[Bis-(p-tolyl)-methyl]-3-[(methylsulfonyl)-(phenyl)-methylen]-azetidin
1-[(4-Chlorphenyl)-(4-hydroxymethylphenyl)-methyl]-3-[(3,5-difluorphenyl)-(methylsulfonyl)-methylen]-azetidin
1-[Bis-(4-chlorphenyl)-methyl]-3-[(3-methylaminophenyl)-(methylsulfonyl)-methylen]-azetidin
(RS)-1-[(4-chlorphenyl)-(thiazol-2-yl)-methyl]-3-[(3,5-difluorphenyl)-(methylsulfonyl)-methylen]-azetidin
(R)-1-[(4-chlorphenyl)-(thiazol-2-yl)-methyl]-3-[(3,5-difluorphenyl)-(methylsulfonyl)-methylen]-azetidin
(S)-1-[(4-chlorphenyl)-(thiazol-2-yl)-methyl]-3-[(3,5-difluorphenyl)-(methylsulfonyl)-methylen]-azetidin
1-[Bis-(4-chlorphenyl)-methyl]-3-[(methylsulfonyl)-(2-methoxycarbonylthien-5-yl)-methylen]-azetidin
1-[Bis-(4-chlorphenyl)-methyl]-3-hydroxy-3-[(methylsulfonyl)-(2-methoxycarbonylthien-5-yl)-methyl]-azetidin-(RS)
1-[Bis-(4-chlorphenyl)-methyl]-3-[(2-isobutylaminocarbonylthien-5-yl)-(methylsulfonyl)-methylen]-azetidin
1-[Bis-(4-chlorphenyl)-methyl]-3-[(3-methoxycarbonylphenyl)-(methylsulfonyl)-methyl(RS)-azetidin-3-ol
1-[Bis-(4-chlorphenyl)-methyl]-3-[(methylsulfonyl)-(pyridin-4-yl)-methyl-(RS)]-azetidin-3-ol
1-[Bis-(4-chlorphenyl)-methyl]-3-[(methylsulfonyl)-(pyridin-3-yI)-methyl-(RS)]-azetidin-3-ol
3-({1-[Bis-(4-chlorphenyl)-methyl]-azetidin-3-yliden}-methansulfonyl-methyl)-N-(3-morpholin-4-yl-propyl)-benzamid,
3-({1-[Bis-(4-chlorphenyl)-methyl]-azetidin-3-yliden}-methansulfonyl-methyl)-N-(3-dimethylamino-propyl)-benzamid,
3-({1-[Bis-(4-chlorphenyl)-methyl]-azetidin-3-yliden}-methansulfonyl-methyl)-N-(2-pyrrolidin-1-yl-ethyl)-benzamid,
3-({1-[Bis-(4-chlorphenyl)-methyl]-azetidin-3-yliden}-methansulfonyl-methyl)-N-(2-dimethylamino-1-methyl-ethyl)-benzamid,
3-({1-[Bis-(4-chlorphenyl)-methyl]-azetidin-3-yliden}-methansulfonyl-methyl)-N-piperidin-1-yl-benzamid,
3-({1-[Bis-(4-chlorphenyl)-methyl]-azetidin-3-yliden}-methansulfonyl-methyl)-N-isobutyl-benzamid
3-({1-[Bis-(4-chlorphenyl)-methyl]-azetidin-3-yliden}-methansulfonyl-methyl)-N-(3-imidazol-1-yl-propyl)-benzamid,
3-({1-[Bis-(4-chlorphenyl)-methyl]-azetidin-3-yliden}-methansulfonyl-methyl)-N-(2-dimethylamino-ethyl)-benzamid,
N'-Methylhydrazid der 3-({1-[Bis-(4-chlorphenyl)-methyl]-azetidin-3-yliden}methansulfonyl-methyl]-benzoesäure
3-({1-[Bis-(4-chlorphenyl)-methyl]-azetidin-3-yliden}-methansulfonyl-methyl)-N-(2-morpholin-4-yl-ethyl)-benzamid,
3-({-[Bis-(4-chlorphenyl)-methyl]-azetidin-3-yliden}-methansulfonyl-methyl)-N-(1-ethylpyrrolidin-2-ylmethyl)-benzamid,
3-({1-[Bis-(4-chlorphenyl)-methyl]-azetidin-3-yliden}-methansulfonyl-methyl)-N-(2,2-dimethylpropyl)-benzamid
3-({1-[Bis-(4-chlorphenyl)-methyl]-azetidin-3-yliden}-methansulfonyl-methyl)-N-cyclohexylmethyl-benzamid
3-({1-[Bis-(4-chlorphenyl)-methyl]-azetidin-3-yliden}-methansulfonyl-methyl)-N-cyclopropylmethyl-benzamid
3-({1-[Bis-(4-chlorphenyl)-methyl]-azetidin-3-yliden}-methansulfonyl-methyl)-N-(2-methyl-butyl)-benzamid
3-({1-[Bis-(4-chlorphenyl)-methyl]-azetidin-3-yliden}-methansulfonyl-methyl)-N-(2-phenyl-propyl)-benzamid
3-({1-[Bis-(4-chlorphenyl)-methyl]-azetidin-3-yliden}-methansulfonyl-methyl)-N-(tetrahydrofuran-2-ylmethyl)-benzamid
3-({1-[Bis-(4-chlorphenyl)-methyl]-azetidin-3-yliden}-methansulfonyl-methyl)-N-(2,2-diphenyl-ethyl)-benzamid
3-({1-[Bis-(4-chlorphenyl)-methyl]-azetidin-3-yliden}-methansulfonyl-methyl)-N-(2-ethyl-butyl)-benzamid
Methylester der 4-{[3-([1-[Bis-(4-chlorphenyl)-methyl]-azetidin-3-yliden}methansulfonyl-methyl)-benzoylamino]-methyl}-cyclohexancarbonsäure
2-Amino-1-{4-[3-({1-[bis-(4-chlorphenyl)-methyl]-azetidin-3-yliden}-methansulfonylmethyl)-phenyl]-piperazin-1-yl}ethanon
tert-Butylester der (2-{4-[3-({1-[Bis-(4-chlorphenyl)-methyl]-azetidin-3-yliden}methansulfonyl-methyl)-phenyl]-piperazin-1-yl}-2-oxo-ethyl)-carbamidsäure
1-{4-[3-({1-[Bis-(4-Chlorphenyl)methyl]-azetidin-3-yliden}-methansulfonylmethyl)-phenyl]-piperazin-1-yl}-2-methylamino-ethanon
tert-Butylester der (2-{4-[3-({1-[Bis-(4-chlorphenyl)-methyl]-azetidin-3-yliden}methansulfonyl-methyl)-phenyl]-piperazin-1-yl}-2-oxo-ethyl)-N-methyl-carbamidsäure N-Methylamid der 4-[3-({1-{Bis-(4-chlorphenyl)-methyl]-azetidin-3-yliden}methansulfonyl-methyl)-phenyl]-piperazin-1-carbothiosäure
N-Methylamid der 4-[3-({1-[Bis-(4-chlorphenyl)-methyl]-azetidin-3-yliden}methansulfonyl-methyl)-phenyl]-piperazin-1-carbonsäure
Methylester der 4-[3-({1-[Bis-(4-chlorphenyl)-methyl]-azetidin-3-yliden}methansulfonyl-methyl)-phenyl]-piperazin-1-carbonsäure
1-[3-({1-[Bis-(4-chlorphenyl)-methyl]-azetidin-3-yliden}-methansulfonyl-methyl)-phenyl]-4-isobutyl-piperazin
1-[3-({1-[Bis-(4-chlorphenyl)-methyl]-azetidin-3-yliden}-methansulfonyl-methyl)-phenyl]4-ethyl-piperazin
4-Acetyl-1-[3-({1-[bis-(4-chlorphenyl)-methyl]-azetidin-3-yliden}methansulfonylmethyl)-phenyl]-piperazin
1-{4-[3-({1-[Bis-(4-chlorphenyl)-methyl]-azetidin-3-yliden}-methansulfonyl-methyl)-phenyl]-piperazin-1-yl}-2-dimethylamino-ethanon
1-[3-({1-[Bis-(4-Chlorphenyl)-methyl]-azetidin-3-yliden}-methansulfonyl-methyl)-phenyl]-piperazin
tert-Butylester der 4-[3-({1-[Bis-(4-chlorphenyl)-methyl]-azetidin-3-yliden}methansulfonyl-methyl)-phenyl]-piperazin-1-carbonsäure
1-[Bis-(4-methoxycarbonylphenyl)-methyl]-3-[(3,5-difluorphenyl)-(methylsulfonyl)-methylen]-azetidin
3-Acetoxy-1-[bis-(4-methoxycarbonylphenyl)-methyl]-3-[(3,5-difluorphenyl)-(methylsulfonyl)-methyl-(RS)-azetidin
(RS)-4-[4-((4-Chlorphenyl)-{3-[(3,5-difluorphenyl)-methansulfonyl-methylen]-azetidin-1 -yl}-methyl)-benzyl] -morpholin
4-(4- {3-[(1-Benzhydryl-azetidin-3-yliden)-methansulfonyl-methyl]-phenoxy}-butyl)-morpholin
4-(4- {3-[(1-Benzhydryl-azetidin-3-yliden}-methansulfonyl-methyl]-phenoxy}-propyl)-morpholin
deren optische Isomere und deren Salze mit einer Mineral- oder organischen Säure.

10. Die folgenden Verbindungen gemäß Anspruch 1,
1-[Bis-(4-chlorophenyl)-methyl]-3-[(3,5-difluorphenyl)-(methylsulfonyl)-methylen]-azetidin,
1-[Bis-(4-chlorophenyl)-methyl]-3-[(3,5-difluorphenyl)-(methylsulfonyl)-methyl-(RS)]-azetidin-3-ol,
3-Acetoxy-1-[bis-(4-chlorophenyl)-methyl]-3-[(3,5-difluorphenyl)-(methylsulfonyl)-methyl)-methyl-sulfonylmethyl-(RS)]-azetidin
deren optische Isomere und deren Salze mit einer Mineral- oder organischen Säure.

11. Verfahren zur Herstellung der Verbindungen der Formel (I) gemäß Anspruch 1, worin R eine Kette der Formel (A) bedeutet, **dadurch gekennzeichnet, dass** man eine entsprechende Verbindung der Formel (Ia) 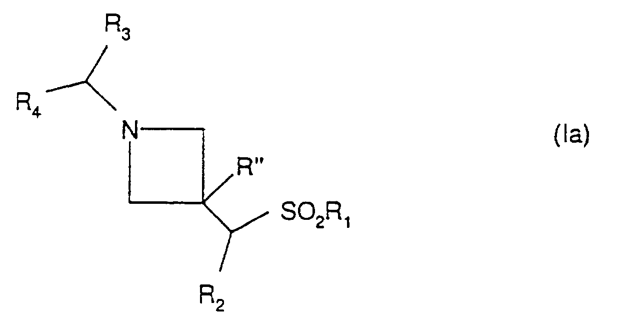 worin R₁, R₂, R₃ und R₄ die in Anspruch 1 angegebenen Bedeutungen besitzen und R" einen Hydroxy-, Methansulfonyloxy- oder Acetyloxyrest bedeutet, dehydratisiert, das Produkt isoliert und es gegebenenfalls mit einer Mineral- oder organischen Säure in ein Salz überführt.

12. Verfahren zur Herstellung der Verbindungen der Formel (I) gemäß Anspruch 1, worin R eine Kette (B) bedeutet, worin R' ein Wasserstoffatom ist, **dadurch gekennzeichnet, dass** man ein Derivat R₁SO₂CH₂R₂ (II), worin R₁ und R₂ die in Anspruch 1 angegebenen Bedeutungen besitzen, mit einem Azetidinon der Formel: 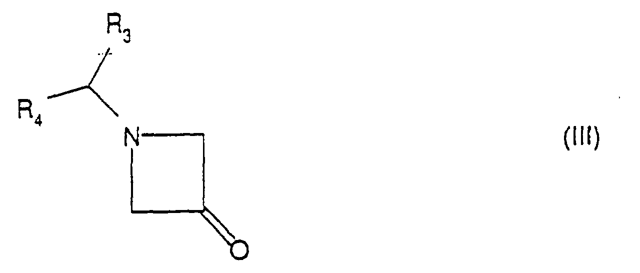 worin R₃ und R₄ die in Anspruch 1 angegebenen Bedeutungen besitzen, umsetzt, das Produkt isoliert und es gegebenenfalls mit einer Mineral- oder organischen Säure in ein Salz überführt.

13. Verfahren zur Herstellung der Verbindungen der Formel (I) gemäß Anspruch 1, worin R eine Kette (B) bedeutet, worin R' ein Wasserstoffatom ist, **dadurch gekennzeichnet, dass** man ein Derivat R₃CH(Br)R₄, worin R₃ und R₄ die in Anspruch 1 angegebenen Bedeutungen besitzen, mit einem Derivat der Formel: 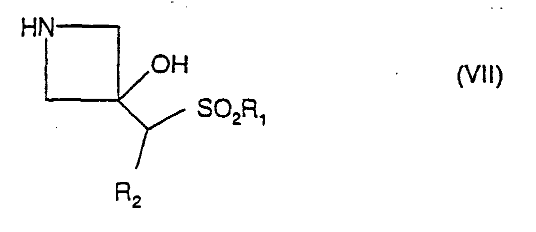 worin R₁ und R₂ die in Anspruch 1 angegebenen Bedeutungen besitzen, umsetzt, das Produkt isoliert und es gegebenenfalls mit einer Mineral- oder organischen Säure in ein Salz überführt.

14. Verfahren zur Herstellung der Verbindungen der Formel (I) gemäß Anspruch 1, worin R eine Kette (B) bedeutet, worin R' einen Rest -CO-alk wiedergibt, **dadurch gekennzeichnet, dass** man ein Halogenid Hal-CO-alk, worin Hal ein Halogenatom bedeutet und alk einen Alkylrest in gerader oder verzweigter Kette mit 1 bis 6 Kohlenstoffatomen wiedergibt, mit einer entsprechenden Verbindung der Formel (I), worin R eine Kette (B) bedeutet, worin R' ein Wasserstoffatom ist, umsetzt, das Produkt isoliert und es gegebenenfalls mit einer Mineral- oder organischen Säure in ein Salz überführt.

15. Verfahren zur Herstellung der Verbindungen der Formel (I) gemäß Anspruch 1, worin R₂ einen Aromaten oder einen Heteroaromaten substituiert durch -NR₆R₇ bedeutet, worin R₆ und R₇ jeweils ein Wasserstoffatom bedeuten, **dadurch gekennzeichnet, dass** man eine entsprechende Verbindung der Formel (I), worin R₂ einen Aromaten oder einen Heteroaromaten substituiert durch Nitro bedeutet, reduziert, das Produkt isoliert und es gegebenenfalls mit einer Mineral- oder organischen Säure in ein Salz überführt.

16. Verfahren zur Herstellung der Verbindungen der Formel (I) gemäß Anspruch 1, worin R₂ einen Aromaten oder Heteroaromaten substituiert durch -CONHR₉ bedeutet und/oder R₃ und/oder R₄ einen Aromaten oder einen Heteroaromaten substituiert durch -CONR₁₀R₁₁ bedeuten, **dadurch gekennzeichnet, dass** man eine entsprechende Verbindung der Formel (I), worin R₂ und/oder R₃ und/oder R₄ einen Aromaten oder einen Heteroaromaten substituiert durch -COOR₅ bedeuten, worin R₅ ein Alkyl oder Phenyl gegebenenfalls durch Halogene substituiert bedeutet, mit einem Amin H₂NR₉ bzw. HNR₁₀R₁₁, worin R₉, R₁₀ und R₁₁ die in Anspruch 1 angegebenen Bedeutungen besitzen, umsetzt, das Produkt isoliert und es gegebenenfalls mit einer Mineral- oder organischen Säure in ein Salz überführt.

17. Verfahren zur Herstellung der Verbindungen der Formel (I) gemäß Anspruch 1, worin R₂ einen durch Hydroxy substituierten Aromaten bedeutet und/oder R₃ und/oder R₄ einen Aromaten oder einen Heteroaromaten substituiert durch Hydroxy bedeuten, **dadurch gekennzeichnet, dass** man eine entsprechende Verbindung der Formel (I), worin R₂ einen Aromaten substituiert durch Alkoxy bedeutet, und/oder R₃ und/oder R₄ einen Aromaten oder einen Heteroaromaten substituiert durch Alkoxy bedeuten, hydrolysiert, das Produkt isoliert und es gegebenenfalls mit einer Mineral- oder organischen Säure in ein Salz überführt.

18. Verfahren zur Herstellung der Verbindungen der Formel (I) gemäß Anspruch 1, worin R₂ einen Aromaten substituiert durch -NR₆R₇ bedeutet, worin R₆ einen Alkylrest wiedergibt und R₇ ein Wasserstoffatom bedeutet, **dadurch gekennzeichnet, dass** man eine Schutzgruppenentfernung vornimmt an einer entsprechenden Verbindung der Formel (I), worin R₂ einen Aromaten substituiert durch ein -N(alk)COOR₈ bedeutet, worin R₈ einen tert-Butylrest bedeutet, das Produkt isoliert und es gegebenenfalls mit einer Mineral- oder organischen Säure in ein Salz überführt.

19. Verfahren zur Herstellung der Verbindungen der Formel (I) gemäß Anspruch 1, worin R₂ und/oder R₃ und/oder R₄ einen Aromaten oder einen Heteroaromaten substituiert durch -COOR₅ bedeuten, **dadurch gekennzeichnet, dass** man ein Derivat der Formel 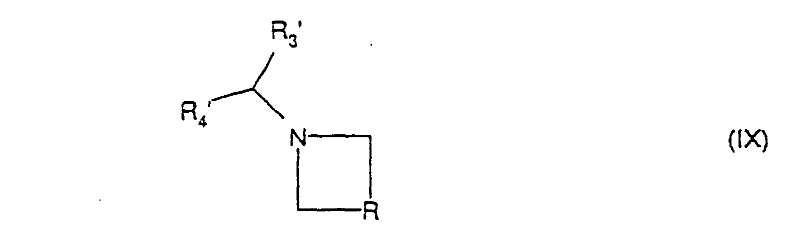 worin R eine Kette C=C(SO₂R₁)R'₂ oder C(OR')CH(SO₂R₁)R'₂ bedeutet, R₁, R'₂, R'₃ und R'₄ die gleichen Bedeutungen wie die Substituenten R₁, R₂, R₃ und R₄ von Anspruch 1 besitzen, vorausgesetzt, dass zumindest einer der Substituenten R'₂, R'₃, R'₄ einen Aromaten oder einen Heteroaromaten substituiert durch Carboxyl bedeutet, mittels eines Derivats der Formel R₅OH, worin R₅ Alkyl oder Phenyl gegebenenfalls durch ein oder mehrere Halogen(e) substituiert bedeutet, verestert, das Produkt isoliert und es gegebenenfalls mit einer Mineral- oder organischen Säure in ein Salz überführt.

20. Verfahren zur Herstellung der Verbindungen der Formel (I) gemäß Anspruch 1, worin R₂ und/oder R₃ und/oder R₄ einen Aromaten oder einen Heteroaromaten substituiert durch Alkylthioalkyl bedeuten, **dadurch gekennzeichnet, dass** man ein Derivat der Formel 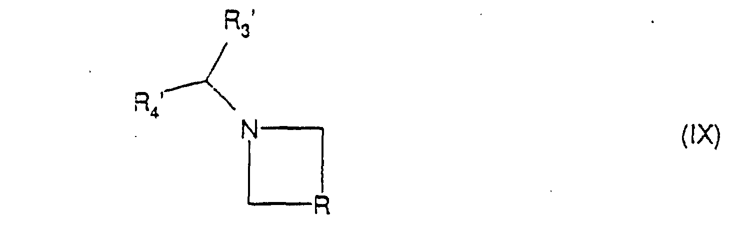 worin R eine Kette C=C(SO₂R₁)R'₂ oder C(OR')CH(SO₂R₁)R'₂ wiedergibt, R', R₁, R'₂, R'₃ und R'₄ die gleichen Bedeutungen besitzen wie die Substituenten R', R₁, R₂, R₃ und R₄ von Anspruch 1, wobei jedoch zumindest einer der Substituenten R'₂, R'₃, R'₄ einen Aromaten oder einen Heteroaromaten substituiert durch Halogenalkyl bedeutet, mit einem Natriumalkylthiolat, worin der Alkylteil geradkettig oder verzweigt ist und 1 bis 6 Kohlenstoffatom(e) aufweist, umsetzt, das Produkt isoliert und es gegebenenfalls mit einer Mineral- oder organischen Säure in ein Salz überführt.

21. Verfahren zur Herstellung der Verbindungen der Formel (I) gemäß Anspruch 1, worin R₂ und/oder R₃ und/oder R₄ einen Aromaten substituiert durch Hydroxyalkyl bedeuten, worin Alkyl ein Kohlenstoffatom enthält, **dadurch gekennzeichnet, dass** man eine Verbindung der Formel (I), worin zumindest einer der Substituenten R₂, R₃, R₄ einen durch Formyl substituierten Aromaten wiedergibt, reduziert, das Produkt isoliert und es gegebenenfalls mit einer Mineral- oder organischen Säure in ein Salz überführt.

22. Verfahren zur Herstellung der Verbindungen der Formel (I) gemäß Anspruch 1, worin R₃ und/oder R₄ einen Aromaten substituiert durch -alk-NR₆R₇, worin alk ein Alkyl mit einem Kohlenstoffatom ist, bedeutet, **dadurch gekennzeichnet, dass** man eine Verbindung der Formel (I), worin zumindest einer der Substituenten R₃, R₄ einen durch Formyl substituierten Aromaten bedeutet, mit einem Amin HNR₆R₇, worin R₆ und R₇ die fiir Formel (I) angegebenen Bedeutungen besitzen, umsetzt, das Produkt isoliert und es gegebenenfalls mit einer Mineral- oder organischen Säure in ein Salz überführt.

23. Verfahren zur Herstellung der Verbindungen der Formel (I) gemäß Anspruch 1, worin R₂ einen Aromaten oder einen Heteroaromaten substituiert durch -CONHR₉ bedeutet, und/oder R₃ und/oder R₄ einen Aromaten oder einen Heteroaromaten substituiert durch -CO-NR₁₀R₁₁ bedeutet, **dadurch gekennzeichnet, dass** man ein Derivat der Formel 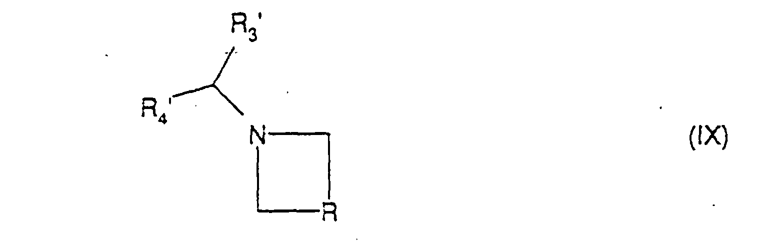 worin R eine Kette C=C(SO₂R₁)R'₂ oder C(OR')CH(SO₂R₁)R'₂ wiedergibt, R', R₁, R'₂, R'₃ und R'₄ die gleichen Bedeutungen besitzen wie die Substituenten R', R₁, R₂, R₃ und R₄ von Anspruch 1, wobei jedoch zumindest einer der Substituenten R'₂, R'₃, R'₄ einen Aromaten oder einen Heteroaromaten substituiert durch Carboxyl bedeutet, mit einem Amin H₂NR₉ bzw. HNR₁₀R₁₁, worin R₉, R₁₀ und R₁₁ die für Formel (I) angegebenen Bedeutungen besitzen, umsetzt, das Produkt isoliert und es gegebenenfalls mit einer Mineral- oder organischen Säure in ein Salz überführt.

24. Verfahren zur Herstellung der Verbindungen der Formel (I) gemäß Anspruch 1, worin R₂ und/oder R₃ und/oder R₄ einen Aromaten oder einen Heteroaromaten substituiert durch -CO-NH-NR₆R₇ bedeuten, **dadurch gekennzeichnet, dass** man eine entsprechende Verbindung der Formel (I), worin R₂ und/oder R₃ und/oder R₄ einen Aromaten oder einen Heteroaromaten substituiert durch -COOR₅ bedeuten, und R₅ ein Rest Alkyl oder Phenyl gegebenenfalls substituiert durch Halogene bedeutet, mit einem Hydrazin H₂N-NR₆R₇, worin R₆ und R₇ die für Formel (I) angegebenen Bedeutungen besitzen, umsetzt, das Produkt isoliert und es gegebenenfalls mit einer Mineral- oder organischen Säure in ein Salz überführt.

25. Verfahren zur Herstellung der Verbindungen der Formel (I) gemäß Anspruch 1, worin R₂ einen Aromaten oder einen Heteroaromaten substituiert durch -CO-NHR₉, worin R₉ ein Wasserstoffatom bedeutet, wiedergibt und/oder R₃ und/oder R₄ einen Aromaten oder einen Heteroaromaten substituiert durch -CO-NR₁₀R₁₁, worin R₁₀ und R₁₁ Wasserstoffatome sind, bedeuten, **dadurch gekennzeichnet, dass** man eine entsprechende Verbindung der Formel (I), worin R₂ und/oder R₃ und/oder R₄ einen Aromaten oder einen Heteroaromaten substituiert durch Cyano bedeuten, hydrolysiert, das Produkt isoliert und es gegebenenfalls mit einer Mineral- oder organischen Säure in ein Salz überführt.

26. Verfahren zur Herstellung der Verbindungen der Formel (I) gemäß Anspruch 1, worin R₂ einen Aromaten substituiert durch -O-alk-NR₁₂R₁₃ bedeutet, **dadurch gekennzeichnet, dass** man ein Derivat der Formel 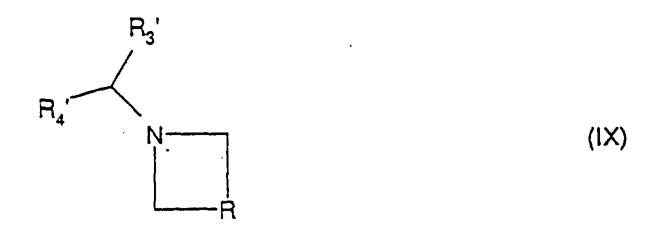 worin R eine Kette C=C(SO₂R₁)R'₂ oder C(OR')CH(SO₂R₁)R'₂ wiedergibt, R', R₁, R₂', R₃' und R₄' die gleichen Bedeutungen wie die Substituenten R', R₁, R₂, R₃ und R₄ von Anspruch 1 besitzen, wobei jedoch zumindest einer der Substituenten R₂', R₃', R₄' einen Aromaten substituiert durch -O-alk-Hal bedeutet, worin alk einen geradkettigen oder verzweigten Alkylrest mit 1 bis 6 Kohlenstoffatomen wiedergibt und Hal ein Halogenatom bedeutet, mit einem Amin HNR₁₂R₁₃, worin R₁₂, R₁₃ die gleichen Bedeutungen wie in Anspruch 1 besitzen, umsetzt, das Produkt isoliert und es gegebenenfalls mit einer Mineral- oder organischen Säure in ein Salz überführt.

27. Verfahren zur Herstellung der Verbindungen der Formel (I) gemäß Anspruch 1, worin R₃ und/oder R₄ einen Aromaten substituiert durch -alk-NR₆R₇ bedeutet, **dadurch gekennzeichnet, dass** man ein Derivat der Formel 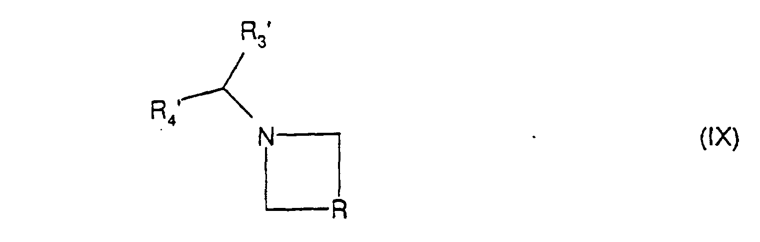 worin R eine Kette C=C(SO₂R₁)R'₂ oder C(OR')CH(SO₂R₁)R'₂ wiedergibt, R', R₁, R₂', R₃' und R₄' die gleichen Bedeutungen besitzen wie die Substituenten R', R₁, R₂, R₃ und R₄ von Anspruch 1, wobei jedoch zumindest einer der Substituenten R₃', R₄' einen Aromaten substituiert durch -alk-Cl bedeutet, worin alk einen Alkylrest mit 1 bis 6 Kohlenstoffatom(en) in gerader oder verzweigter Kette wiedergibt, mit einem Amin NHR₆R₇, worin R₆, R₇ die in Anspruch 1 angegebenen Bedeutungen besitzen, umsetzt, das Produkt isoliert und es gegebenenfalls mit einer Mineral- oder organischen Säure in ein Salz überführt.

28. Verfahren zur Herstellung der Verbindungen der Formel (I) gemäß Anspruch 1, worin R eine Kette (B) bedeutet, R' ein Wasserstoffatom wiedergibt und R₃ und/oder R₄ einen Aromaten substituiert durch Hydroxyalkyl bedeutet, worin der Rest Alkyl ein Kohlenstoffatom enthält, **dadurch gekennzeichnet, dass** man Diisobutylaluminiumhydrid mit einer entsprechenden Verbindung der Formel (I) umsetzt, worin R eine Kette (B) bedeutet, R' ein Wasserstoffatom bedeutet und R₃ und/oder R₄ einen Aromaten substituiert durch einen oder mehrere Reste -COOR₅ bedeutet, worin R₅ ein Alkylrest ist, das Produkt isoliert und es gegebenenfalls mit einer Mineral- oder organischen Säure in ein Salz überführt.

29. Verfahren zur Herstellung der Verbindungen der Formel (I) gemäß Anspruch 1, worin R₂ einen Phenylrest bedeutet, der substituiert ist durch -NR₆R₇, welches einen durch einen Alkylrest in 4-Stellung substituierten 1-Piperazinylring bedeutet, **dadurch gekennzeichnet, dass** man eine entsprechende Verbindung der Formel (1), worin R₂ einen Phenylrest bedeutet, der substituiert ist durch einen Rest -NR₆R₇, welcher einen 1-Piperazinylring wiedergibt, mit einem Derivat alk-CHO umsetzt, worin alk einen Alkylrest in gerader oder verzweigter Kette mit 1 bis 5 Kohlenstoffatomen bedeutet, das Produkt isoliert und es gegebenenfalls mit einer Mineral- oder organischen Säure in ein Salz überführt.

30. Verfahren zur Herstellung der Verbindungen der Formel (I) gemäß Anspruch 1, worin R₂ einen Phenylrest bedeutet, der substituiert ist durch -NR₆R₇, welches einen in 4-Stellung durch einen Rest -COOalk substituierten 1-Piperazinylring wiedergibt, **dadurch gekennzeichnet, dass** man eine entsprechende Verbindung der Formel (I), worin R₂ einen Phenylrest bedeutet, substituiert durch einen Rest -NR₆R₇, der einen 1-Piperazinylring wiedergibt, mit einem Derivat der Formel Hal-COOalk umsetzt, worin alk einen Alkylrest in gerader oder verzweigter Kette mit 1 bis 6 Kohlenstoffatom(en) bedeutet und Hal für ein Halogenatom steht, das Produkt isoliert und es gegebenenfalls mit einer Mineral- oder organischen Säure in ein Salz überführt.

31. Verfahren zur Herstellung der Verbindungen der Formel (I) gemäß Anspruch 1, worin R₂ einen Phenylrest bedeutet, substituiert durch -NR₆R₇, welches einen 1-Piperazinylring wiedergibt, der substituiert ist in 4-Stellung durch einen Rest -CO-NHalk oder -CS-NHalk, **dadurch gekennzeichnet, dass** man eine entsprechende Verbindung der Formel (I), worin R₂ einen Phenylrest bedeutet, substituiert durch -NR₆R₇, welches einen 1-Piperazinylring wiedergibt, mit einem Derivat der Formel Y=C=Nalk umsetzt, worin alk einen Alkylrest in gerader oder verzweigter Kette mit 1 bis 6 Kohlenstoffatom(en) bedeutet und Y ein Schwefel- oder Sauerstoffatom ist, das Produkt isoliert und es gegebenenfalls mit einer Mineral- oder organischen Säure in ein Salz überführt.

32. Verfahren zur Herstellung der Verbindungen der Formel (I) gemäß Anspruch 1, worin R₂ einen Phenylrest bedeutet, substituiert durch einen Rest -NR₆R₇, der einen 1-Piperazinylring bedeutet, welcher in 4-Stellung durch einen Rest -CO-alk-NR₁₄R₁₅ substituiert ist, **dadurch gekennzeichnet, dass** man eine entsprechende Verbindung der Formel (I), worin R₂ einen Phenylrest bedeutet, substituiert durch einen Rest -NR₆R₇, der einen 1-Piperazinylring wiedergibt, mit einer Säure der Formel R₁₅R₁₄N-alk-COOH umsetzt, worin alk einen Alkylrest in gerader oder verzweigter Kette mit 1 bis 6 Kohlenstoffatom(en) bedeutet und R₁₄ und R₁₅ die gleichen Bedeutungen wie in Anspruch 1 besitzen, gegebenenfalls gefolgt von einer Schutzgruppenabspaltung an dem Produkt, worin R₁₄ einen tert-Butoxycarbonylrest wiedergibt, um zu den Verbindungen zu gelangen, worin R₁₄ für ein Wasserstoffatom steht, das Produkt isoliert und es gegebenenfalls mit einer Mineral- oder organischen Säure in ein Salz überführt.

33. Verfahren zur Herstellung der Verbindungen der Formel (I) gemäß Anspruch 1, worin R₂ einen Phenylrest bedeutet, substituiert durch einen Rest -NR₆R₇, der einen 1-Piperazinylrest darstellt, welcher in 4-Stellung durch einen Rest -CO-alk substituiert ist, worin alk einen Methylrest bedeutet, **dadurch gekennzeichnet, dass** man eine entsprechende Verbindung der Formel (I), worin R₂ einen Phenylrest bedeutet, der substituiert ist durch einen Rest -NR₆R₇, welcher einen 1-Piperazinylring wiedergibt, mit Essigsäureanhydrid umsetzt, das Produkt isoliert und es gegebenenfalls mit einer Mineral- oder organischen Säure in ein Salz überführt.

34. Pharmazeutische Zusammensetzungen, enthaltend als Wirkstoff zumindest eine Verbindung der Formel (I) gemäß einem der Ansprüche 1 bis 10.

35. Verbindungen der Formel (I) gemäß einem der Ansprüche 1 bis 10 als Arzneimittel.

## Claims

1. Compounds of formula: 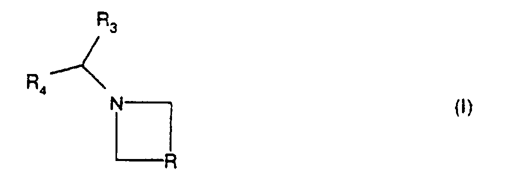 in which
R represents a chain 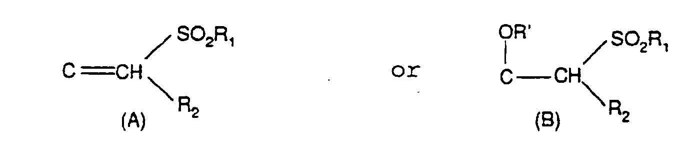 R₁ represents a methyl or ethyl radical,
R₂ represents either an aromatic chosen from phenyl, naphthyl or indenyl, these aromatics being nonsubstituted or substituted with one or more halogen, alkyl, alkoxy, -CO-alk, hydroxyl, -COOR₅, formyl, trifluoromethyl, trifluoromethylsulphanyl, trifluoromethoxy, nitro, -NR₆R₇, -CO-NH-NR₆R₇, -N(alk)COOR₈, cyano, -CONHR₉, -CO-NR₁₆R₁₇, alkylsulphanyl, hydroxyalkyl, -O-alk-NR₁₂R₁₃ or alkylthioalkyl or a heteroaromatic chosen from the benzofuryl, benzothiazolyl, benzothienyl, benzoxazolyl, chromanyl, 2,3-dihydrobenzofuryl, 2,3-dihydrobenzothienyl, indolinyl, indolyl, isochromanyl, isoquinolyl, pyridyl, quinolyl, 1,2,3,4-tetrahydroisoquinolyl, 1,2,3,4-tetrahydroquinolyl, thiazolyl, or thienyl rings, it being possible for these heteroaromatics to be nonsubstituted or substituted with a halogen, alkyl, alkoxy, -COOR₅, trifluoromethyl, trifluoromethylsulphanyl, trifluoromethoxy, nitro, -NR₆R₇, -CO-NH-NR₆R₇, cyano, -CONHR₉, alkylsulphanyl, hydroxyalkyl or alkylthioalkyl,
R₃ and R₄, which are identical or different, represent either an aromatic chosen from phenyl, naphthyl or indenyl, these aromatics being nonsubstituted or substituted with one or more halogen, alkyl, alkoxy, formyl, hydroxyl, trifluoromethyl, trifluoromethoxy, -CO-alk, cyano, -COOR₅, -CONR₁₀R₁₁, -CO-NH-NR₆R₇, alkylsulphanyl, hydroxyalkyl, -alk-NR₆R₇ or alkylthioalkyl; or a heteroaromatic chosen from benzofuryl, benzothiazolyl, benzothienyl, benzoxazolyl, chromanyl, 2,3-dihydrobenzofuryl, 2,3-dihydrobenzothienyl, furyl, isochromanyl, isoquinolyl, pyrrolyl, quinolyl, 1,2,3,4-tetrahydroisoquinolyl, thiazolyl or thienyl rings, it being possible for these heteroaromatics to be nonsubstituted or substituted with a halogen, alkyl, alkoxy, hydroxyl, trifluoromethyl, trifluoromethoxy, cyano, -COOR₅, -CO-NH-NR₆R₇, -CONR₁₀R₁₁, -alk-NR₆R₇, alkylsulphanyl, hydroxyalkyl or alkylthioalkyl,
R₅ is an alkyl or phenyl radical which is optionally substituted with one or more halogen atoms,
R₆ and R₇, which are identical or different, represent a hydrogen atom or an alkyl, -COOalk, cycloalkyl, alkylcycloalkyl, -alk-O-alk or hydroxyalkyl radical or alternatively R₆ and R₇ together form with the nitrogen atom to which they are attached a 3- to 10-membered saturated or unsaturated mono- or bicyclic heterocycle optionally containing another heteroatom chosen from oxygen, sulphur and nitrogen and being optionally substituted with one or more alkyl, -COalk, -COOalk, -CO-NHalk, -CS-NHalk, -CO-alk-NR₁₄R₁₅, oxo, hydroxyalkyl, -alk-O-alk or -CO-NH₂ radicals,
R₈ represents an alkyl radical,
R₉ represents a hydrogen atom or an alkyl radical or an alkyl radical substituted with dialkylamino, phenyl, cycloalkyl (optionally substituted with -COOalk) or a 3- to 10-membered saturated or unsaturated mono- or bicyclic heterocycle optionally containing one or more heteroatoms chosen from oxygen, sulphur and nitrogen and being optionally substituted with one or more alkyl radicals,
R₁₀ and R₁₁, which are identical or different, represent a hydrogen atom or an alkyl radical or alternatively R₁₀ and R₁₁ together form with the nitrogen atom to which they are attached a 3- to 10-membered saturated mono- or bicyclic heterocycle optionally containing another heteroatom chosen from oxygen, sulphur and nitrogen and being optionally substituted with an alkyl radical,
R₁₂ and R₁₃, which are identical or different, represent a hydrogen atom or an alkyl or cycloalkyl radical or alternatively R₁₂ and R₁₃ together form with the nitrogen atom to which they are attached a 3- to 10-membered saturated mono- or bicyclic heterocycle optionally containing another heteroatom chosen from oxygen, sulphur and nitrogen and being optionally substituted with an alkyl, -COalk, -COOalk, -CO-NHalk, -CS-NHalk or -CO-alk-NR₁₄R₁₅ radical or a 3- to 10-membered saturated mono- or bicyclic heterocycle containing a heteroatom chosen from oxygen, sulphur and nitrogen,
R₁₄ and R₁₅, which are identical or different, represent a hydrogen atom or an alkyl or -COOalk radical,
R₁₆ and R₁₇ together form with the nitrogen atom to which they are attached a 3- to 10-membered saturated mono- or bicyclic heterocycle optionally containing another heteroatom chosen from oxygen, sulphur and nitrogen,
R' represents a hydrogen atom or a -CO-alk radical,
alk represents an alkyl or alkylene radical, it being understood that the alkyl and alkylene radicals and portions and the alkoxy radicals and portions are in the form of a straight or branched chain and contain 1 to 6 carbon atoms,
their optical isomers and their salts with an inorganic or organic acid.

2. Compounds of formula (I) according to Claim 1, for which when R₆ and R₇ together form with the nitrogen atom to which they are attached a 3- to 10-membered saturated or unsaturated mono- or bicyclic heterocycle, the latter is an azetidinyl, pyrrolidinyl, piperazinyl, piperidyl, morpholinyl, imidazolyl, thiomorpholinyl or furyl ring, these rings being optionally substituted with an alkyl, hydroxyalkyl, -alk-O-alk, -CONH₂, -COalk, -COOalk, oxo, -CSNHalk, -CONHalk or -CO-alk-NR₁₄R₁₅ in which alk, R₁₄ and R₁₅ have the same meanings as in Claim 1,
their optical isomers and their salts with an inorganic or organic acid.

3. Compounds of formula (I) according to either of Claims 1 and 2, for which when R₁₀ and R₁₁ together form with the nitrogen atom to which they are attached a 3-to 10-membered saturated mono- or bicyclic heterocycle, the latter is an azetidinyl, pyrrolidinyl, piperazinyl, piperidyl, morpholinyl or thiomorpholinyl ring, these rings being optionally substituted with an alkyl, their optical isomers and their salts with an inorganic or organic acid.

4. Compounds of formula (I) according to one of Claims 1 to 3, for which when R₁₂ and R₁₃ together form with the nitrogen atom to which they are attached a 3-to 10-membered saturated mono- or bicyclic heterocycle, the latter is an azetidinyl, pyrrolidinyl, piperazinyl, piperidyl, morpholinyl or thiomorpholinyl ring, these rings being optionally substituted with an alkyl, -COalk, -COOalk, -CO-NHalk, -CS-NHalk or -CO-alk-NR₁₄R₁₅ radical or a 3- to 10-membered saturated mono- or bicyclic heterocycle containing a heteroatom chosen from oxygen, sulphur and nitrogen, and, in particular, with a thiomorpholinyl radical, alk, R₁₄ and R₁₅ having the same meanings as in Claim 1,
their optical isomers and their salts with an inorganic or organic acid.

5. Compounds of formula (I) according to one of Claims 1 to 4, for which when R₁₆ and R₁₇ together form with the nitrogen atom to which they are attached a 3-to 10-membered saturated mono- or bicyclic heterocycle, the latter is a piperidyl ring,
their optical isomers and their salts with an inorganic or organic acid.

6. Compounds of formula (I) according to one of Claims 1 to 5 for which R₉ represents an alkyl radical substituted with a 3- to 10-membered saturated or unsaturated mono- or bicyclic heterocycle optionally containing one or more heteroatoms chosen from oxygen, sulphur and nitrogen, the latter being a pyrrolidinyl, tetrahydrofuryl, morpholinyl or pyrrolyl ring, these rings being optionally substituted with one or more alkyl radicals,
their optical isomers and their salts with an inorganic or organic acid.

7. Compounds of formula (I) according to Claim 1 for which
R represents a chain (A) or (B),
R' representing a hydrogen atom or a -COalk radical,
R₁ represents a methyl or ethyl radical,
R₂ represents either an aromatic chosen from phenyl and naphthyl, these aromatics being nonsubstituted or substituted with one or more halogen, alkyl, alkoxy, hydroxyl, -COOR₅, trifluoromethyl, trifluoromethylsulphanyl, trifluoromethoxy, -NR₆R₇, -CO-NH-NR₆R₇, cyano, -CONHR₉, alkylsulphanyl, hydroxyalkyl, nitro, -CO-NR₁₆R₁₇, -O-alkNR₁₂R₁₃ or alkylthioalkyl or a heteroaromatic chosen from isoquinolyl, pyridyl, quinolyl, 1,2,3,4-tetrahydroisoquinolyl, 1,2,3,4-tetrahydroquinolyl and thienyl, these heteroaromatics being unsubstituted or substituted with a halogen, alkyl, alkoxy, -COOR₅, trifluoromethyl, trifluoromethylsulphanyl, trifluoromethoxy, -NR₆R₇, -CO-NH-NR₆R₇, cyano, -CONHR₉, alkylsulphanyl, hydroxyalkyl, nitro or alkylthioalkyl,
R₃ and R₄, which are identical or different, represent either an aromatic chosen from phenyl or naphthyl, these aromatics being nonsubstituted or substituted with one or more halogen, alkyl, alkoxy, trifluoromethyl, trifluoromethoxy, -CONR₁₀R₁₁, -alk-NR₆R₇, hydroxyalkyl, formyl or -COOR₅, or a heteroaromatic chosen from thiazolyl or thienyl rings, these heteroaromatics being nonsubstituted or substituted by a halogen, alkyl, alkoxy, -CONR₁₀R₁₁, -alk-NR₆R₇, hydroxyalkyl or -COOR₅,
R₅ is alkyl or phenyl which is optionally substituted with one or more halogens,
R₆ and R₇, which are identical or different, represent a hydrogen atom or an alkyl, -COOalk, cycloalkyl, alkylcycloalkyl, alk-O-alk or hydroxyalkyl radical or alternatively R₆ and R₇ together form with the nitrogen atom to which they are attached a 3- to 10-membered saturated or unsaturated mono- or bicyclic heterocycle optionally containing another heteroatom chosen from oxygen, sulphur and nitrogen and being optionally substituted with one or more alkyl, -COalk, -COOalk, -CO-NHalk, -CS-NHalk, -CO-alk-NR₁₄R₁₅, oxo, hydroxyalkyl, alk-O-alk or -CO-NH₂ radicals,
R₉ represents a hydrogen atom or an alkyl radical or an alkyl radical substituted with dialkylamino, phenyl, cycloalkyl (optionally substituted with -COOalk) or a 3- to 10-membered saturated or unsaturated mono- or bicyclic heterocycle optionally containing one or more heteroatoms chosen from oxygen, sulphur and nitrogen and being optionally substituted with one or more alkyl radicals,
R₁₀ and R₁₁, which are identical or different, represent a hydrogen atom or an alkyl radical or alternatively R₁₀ and R₁₁ together form with the nitrogen atom to which they are attached a 3- to 10-membered saturated mono- or bicyclic heterocycle optionally containing another heteroatom chosen from oxygen, sulphur and nitrogen and being optionally substituted with an alkyl radical,
R₁₂ and R₁₃, which are identical or different, represent a hydrogen atom or an alkyl or cycloalkyl radical or alternatively R₁₂ and R₁₃ together form with the nitrogen atom to which they are attached a 3- to 10-membered saturated mono- or bicyclic heterocycle optionally containing another heteroatom chosen from oxygen, sulphur and nitrogen and being optionally substituted with an alkyl, -COalk, -COOalk, -CO-NHalk, -CS-NHalk or -CO-alk-NR₁₄R₁₅ radical or a 3- to 10-membered saturated mono- or bicyclic heterocycle containing a heteroatom chosen from oxygen, sulphur and nitrogen,
R₁₄ and R₁₅, which are identical or different, represent a hydrogen atom or an alkyl or -COOalk radical,
R₁₆ and R₁₇ together form with the nitrogen atom to which they are attached a 3- to 10-membered saturated mono- or bicyclic heterocycle optionally containing another heteroatom chosen from oxygen, sulphur and nitrogen,
alk represents an alkyl or alkylene radical,
it being understood that the alkyl and alkylene radicals and portions and the alkoxy radicals and portions are in the form of a straight or branched chain and contain 1 to 6 carbon atoms,
their optical isomers and their salts with an inorganic or organic acid.

8. Compounds of formula (I) according to Claim 1, for which
R represents a chain (A) or (B),
R' representing a hydrogen atom or a -COalk radical,
R₁ represents a methyl or ethyl radical,
R₂ represents either an aromatic chosen from
naphthyl,
phenyl,
phenyl substituted with one or more halogen, alkyl, alkoxy, hydroxyl, -COOR₅ (in which R₅ represents an alkyl or phenyl radical optionally substituted with several halogens) trifluoromethyl, trifluoromethylsulphanyl, trifluoromethoxy, -NR₆R₇ (in which R₆ and R₇, which are identical or different, represent a hydrogen atom or an alkyl or -COOalk radical or alternatively R₆ and R₇ together form with the nitrogen atom to which they are attached a heterocycle chosen from pyrrolidinyl, piperidyl, piperazinyl or piperazinyl substituted with one or more alkyl, -COalk, -COOalk, -CO-NHalk, -CS-NHalk or -CO-alk-NR₁₄R₁₅ radicals, in which R₁₄ and R₁₅, which are identical or different, represent a hydrogen atom or an alkyl radical), -CO-NH-NR₆R₇ (R₆ and R₇, which are identical or different, represent a hydrogen atom or an alkyl radical or alternatively R₆ and R₇ together form with the nitrogen atom to which they are attached a heterocycle chosen from piperidyl, pyrrolyl, piperazinyl or piperazyl substituted with one or more alkyl radicals), cyano, -CONHR₉ (in which R₉ represents a hydrogen atom or an alkyl radical or an alkyl radical substituted with dialkylamino, phenyl, cycloalkyl (optionally substituted with -COOalk) or a heterocycle chosen from pyrrolidinyl (optionally substituted with alkyl), tetrahydrofuryl or morpholinyl), alkylsulphanyl, hydroxyalkyl, nitro, -CO-NR₁₆R₁₇, (in which R₁₆ and R₁₇ together form with the nitrogen atom to which they are attached a piperidyl ring), -O-alkNR₁₂R₁₃ (in which R₁₂ and R₁₃ together form with the nitrogen atom to which they are attached a morpholino ring) or alkylthioalkyl,
or a heteroaromatic chosen from
isoquinolyl,
pyridyl,
quinolyl,
1,2,3,4-tetrahydroisoquinolyl,
1,2,3,4-tetrahydroquinolyl,
thienyl, or
thienyl substituted with a -COOR₅ (in which R₅ represents an alkyl radical) or -CONHR₉, (in which R₉ represents an alkyl radical),
R₃ and R₄, which are identical or different, represent either an aromatic chosen from
phenyl or
phenyl substituted with one or more halogen, alkyl, alkoxy, trifluoromethyl, trifluoromethoxy, hydroxyalkyl, formyl, -COOR₅ (in which R₅ is an alkyl radical), -CONR₁₀R₁₁ (in which R₁₀ and R₁₁, which are identical or different, represent a hydrogen atom or an alkyl radical), -alk-NR₆R₇ (in which R₆ and R₇, which are identical or different, represent a hydrogen atom or an alkyl, cycloalkyl, -alk-O-alk or hydroxyalkyl radical or alternatively R₆ and R₇ together form with the nitrogen atom to which they are attached a heterocycle chosen from piperidyl (optionally substituted with alkyl or oxo), pyrrolidinyl (optionally substituted with alkyl, hydroxyalkyl, -alk-O-alk or -CO-NH₂), thiomorpholinyl, morpholinyl, pyrrolyl, piperazinyl optionally substituted with oxo, alkyl, hydroxyalkyl, -COOR₅ (in which R₅ is an alkyl radical),
or a heteroaromatic chosen from
thiazolyl or
thienyl,
alk represents an alkyl or alkylene radical,
it being understood that the alkyl and alkylene radicals and portions and the alkoxy radicals and portions are in the form of a straight or branched chain and contain 1 to 6 carbon atoms,
their optical isomers and their salts with an inorganic or organic acid.

9. Compounds according to Claim 1, chosen from:
1-benzhydryl-3-[(methylsulphonyl)(phenyl)methylene]-azetidine,
1-benzhydryl-3-[(3-methylphenyl)(methylsulphonyl)-methylene]azetidine,
1-benzhydryl-3-[(3-chlorophenyl)(methylsulphonyl)-methylene]azetidine,
1-benzhydryl-3-[(3,5-dichlorophenyl)(methylsulphonyl)-methylene]azetidine,
1-benzhydryl-3-[(2,5-dichlorophenyl)(methylsulphonyl)-methylene]azetidine,
1-benzhydryl-3-[(2,3-dichlorophenyl)(methylsulphonyl)-methylene]azetidine,
1-benzhydryl-3-[(3-fluorophenyl)(methylsulphonyl)-methylene]azetidine,
1-benzhydryl-3-[(3,5-difluorophenyl)(methylsulphonyl)-methylene]azetidine,
1-benzhydryl-3-[(3-bromophenyl)(methylsulphonyl)-methylene]azetidine,
1-benzhydryl-3-[(3-iodophenyl)(methylsulphonyl)-methylene]azetidine,
1-benzhydryl-3-[(methylsulphonyl)(3-trifluoromethoxyphenyl)methylene]azetidine,
1-benzhydryl-3-[(3-methylsulphonyl)(3-trifluoromethylphenyl)methylene]azetidine,
1-benzhydryl-3-{[3,5-bis(trifluoromethyl)phenyl]-(methylsulphonyl)methylene}azetidine,
1-benzhydryl-3-[(3,5-dibromophenyl)(methylsulphonyl)-methylene]azetidine,
1-benzhydryl-3-[(3-methoxycarbonylphenyl)-(methylsulphonyl)methylene]azetidine,
1-benzhydryl-3-[(3-cyanophenyl)(methylsulphonyl)-methylene]azetidine,
1-benzhydryl-3-[(3-carbamoylphenyl)(methylsulphonyl)-methylene]azetidine,
1-benzhydryl-3-[(methylsulphonyl)(naphth-1-yl)-(methylsulphonyl)methylene]azetidine,
1-[bis(4-chlorophenyl)methyl]-3-[(3,5-difluorophenyl)-(methylsulphonyl)methylene]azetidine,
1-[bis(4-methoxyphenyl)methyl]-3-[(3,5-difluorophenyl)-(methylsulphonyl)methylene]azetidine,
1-[bis(4-methylphenyl)methyl]-3-[(3,5-difluorophenyl)-(methylsulphonyl)methylene]azetidine,
(RS)-3-[(3,5-difluorophenyl)(methylsulphonyl)methylene]-1-[(4-methoxyphenyl)(phenyl)methyl]azetidine,
(R)-3-[(3,5-difluorophenyl)(methylsulphonyl)methylene]-1-[(4-methoxyphenyl)(phenyl)methyl]azetidine,
(S)-3-[(3,5-difluorophenyl)(methylsulphonyl)methylene]-1-[(4-methoxyphenyl)(phenyl)methyl]azetidine,
1-[bis(4-trifluoromethoxyphenyl)methyl]-3-[(3,5-difluorophenyl)(methylsulphonyl)methylene]azetidine,
1-[bis(4-trifluoromethylphenyl)methyl]-3-[(3,5-difluorophenyl)(methylsulphonyl)methylene]azetidine,
1-[bis(4-chlorophenyl)methyl]-3-{[3,5-bis-(trifluoromethyl)phenyl]methylsulphonylmethylene}-azetidine,
(RS)-1-[(4-chlorophenyl)(2,4-dichlorophenyl)methyl]-3-[(3,5-difluorophenyl)(methylsulphonyl)methylene]-azetidine,
(R)-1-[(4-chlorophenyl)(2,4-dichlorophenyl)methyl]-3-[(3,5-difluorophenyl)(methylsulphonyl)methylene]-azetidine,
(S)-1-[(4-chlorophenyl)(2,4-dichlorophenyl)methyl]-3-[(3,5-difluorophenyl)(methylsulphonyl)methylene]-azetidine,
(RS)-1-{(4-chlorophenyl)[4-(hydroxymethyl)phenyl]-methyl}-3-[(3,5-difluorophenyl)(methylsulphonyl)-methylene]azetidine,
(R)-1-{(4-chlorophenyl)[4-(hydroxymethyl)phenyl]-methyl}-3-[(3,5-difluorophenyl)(methylsulphonyl)-methylene]azetidine,
(S)-1-{(4-chlorophenyl)[4-(hydroxymethyl)phenyl]-methyl}-3-[(3,5-difluorophenyl)(methylsulphonyl)-methylene]azetidine,
(RS)-1-{(4-chlorophenyl)[4-(pyrrolidylmethyl)-phenyl]methyl}-3-[(3,5-difluorophenyl)(methylsulphonyl)methylene]azetidine,
(R)-1-{(4-chlorophenyl)[4-(pyrrolidylmethyl)phenyl]-methyl}-3-[(3,5-difluorophenyl)(methylsulphonyl)-methylene]azetidine,
(S)-1-{(4-chlorophenyl)[4-(pyrrolidylmethyl)phenyl]-methyl}-3-[(3,5-difluorophenyl)(methylsulphonyl)-methylene] azetidine,
1-{(RS)-(4-chlorophenyl)[4-(3,3-dimethylpiperidin-1-yl-methyl)phenyl]methyl}-3-[(3,5-difluorophenyl)-(methylsulphonyl)methylene]azetidine,
1-{(R)-(4-chlorophenyl)[4-(3,3-dimethylpiperidin-1-yl-methyl)phenyl]methyl}-3-[(3,5-difluorophenyl)-(methylsulphonyl)methylene]azetidine,
1-{(S)-(4-chlorophenyl)[4-(3,3-dimethylpiperidin-1-yl-methyl)phenyl]methyl}-3-[(3,5-difluorophenyl)-(methylsulphonyl)methylene]azetidine,
1-{(RS)-(4-chlorophenyl)[4-(thiomorpholin-4-ylmethyl)phenyl]methyl}-3-[(3,5-difluorophenyl)-(methylsulphonyl)methylene]azetidine,
1-{(R)-(4-chlorophenyl)[4-(thiomorpholin-4-ylmethyl)phenyl]methyl}-3-[(3,5-difluorophenyl)-(methylsulphonyl)methylene]azetidine,
1-{(S)-(4-chlorophenyl)[4-(thiomorpholin-4-ylmethyl)phenyl]methyl}-3-[(3,5-difluorophenyl)-(methylsulphonyl)methylene]azetidine,
1-{(RS)-(4-chlorophenyl)[4-(N-ethyl-N-cyclohexylaminomethyl)phenyl]methyl}-3-[(3,5-difluorophenyl)(methylsulphonyl)methylene]azetidine,
1-{(R)-(4-chlorophenyl)[4-(N-ethyl-N-cyclohexylaminomethyl)phenyl]methyl}-3-[(3,5-difluorophenyl)(methylsulphonyl)methylene]azetidine,
1-{(S)-(4-chlorophenyl)[4-(N-ethyl-N-cyclohexylaminomethyl)phenyl]methyl}-3-[(3,5-difluorophenyl)(methylsulphonyl)methylene]azetidine,
1-{{(RS)-(4-chlorophenyl){4-[(4-ethoxycarbonylpiperazinyl)methyl]phenyl}methyl}}-3-[(3,5-difluorophenyl)(methylsulphonyl)methylene]azetidine,
1-{{(R)-(4-chlorophenyl){4-[(4-ethoxycarbonylpiperazinyl)methyl]phenyl}methyl}}-3-[(3,5-difluorophenyl)(methylsulphonyl)methylene]azetidine,
1-{{(S)-(4-chlorophenyl){4-[(4-ethoxycarbonylpiperazinyl)methyl]phenyl}methyl}}-3-[(3,5-difluorophenyl)(methylsulphonyl)methylene]azetidine,
1-{(RS)-(4-chlorophenyl)[4-(N-cyclopropyl-N-propylaminomethyl)phenyl]methyl}-3-[(3,5-difluorophenyl)-(methylsulphonyl)methylene]azetidine,
1-{(R)-(4-chlorophenyl)[4-(N-cyclopropyl-N-propylaminomethyl)phenyl]methyl}-3-[(3,5-difluorophenyl)-(methylsulphonyl) methylene]azetidine,
1-{(S)-(4-chlorophenyl)[4-(N-cyclopropyl-N-propylaminomethyl)phenyl]methyl}-3-[(3,5-difluorophenyl)-(methylsulphonyl)methylene]azetidine,
1-{(RS)-(4-chlorophenyl)[4-(diisopropylaminomethyl)-phenyl]methyl}-3-[(3,5-difluorophenyl)(methylsulphonyl)methylene]azetidine,
1-{(R)-(4-chlorophenyl)[4-(diisopropylaminomethyl)-phenyl]methyl}-3-[(3,5-difluorophenyl)(methylsulphonyl)methylene]azetidine,
1-{(S)-(4-chlorophenyl)[4-(diisopropylaminomethyl)-phenyl]methyl}-3-[(3,5-difluorophenyl)(methylsulphonyl)methylene]azetidine,
1-{{(RS)-(4-chlorophenyl){4-[bis-(2-methoxyethyl)aminomethyl]phenyl}methyl}}-3-[(3,5-difluorophenyl)-(methylsulphonyl)methylene]azetidine,
1-{{(R)-(4-chlorophenyl){4-[bis-(2-methoxyethyl)aminomethyl]phenyl}methyl}}-3-[(3,5-difluorophenyl)-(methylsulphonyl)methylene]azetidine,
1-{{(S)-(4-chlorophenyl){4-[bis-(2-methoxyethyl)aminomethyl]phenyl}methyl}}-3-[(3,5-difluorophenyl)-(methylsulphonyl)methylene]azetidine,
1-{(RS)-(4-chlorophenyl)[4-(di-n-propylaminomethyl)phenyl]methyl}-3-[(3,5-difluorophenyl)-(methylsulphonyl)methylene]azetidine,
1-{(R)-(4-chlorophenyl)[4-(di-n-propylaminomethyl)phenyl]methyl}-3-[(3,5-difluorophenyl)-(methylsulphonyl)methylene]azetidine,
1-{(S)-(4-chlorophenyl)[4-(di-n-propylaminomethyl)phenyl]methyl}-3-[(3,5-difluorophenyl)-(methylsulphonyl)methylene]azetidine,
1-{(RS)-(4-chlorophenyl)[4-(piperidin-1-ylmethyl)-phenyl]methyl}-3-[(3,5-difluorophenyl)(methylsulphonyl)methylene]azetidine,
1-{(R)-(4-chlorophenyl)[4-(piperidin-1-ylmethyl)-phenyl]methyl}-3-[(3,5-difluorophenyl)(methylsulphonyl)methylene]azetidine,
1-{(S)-(4-chlorophenyl)[4-(piperidin-1-ylmethyl)-phenyl]methyl}-3-[(3,5-difluorophenyl)(methylsulphonyl)methylene]azetidine,
1-{(RS)-(4-chlorophenyl)[4-(4-methylpiperazin-1-yl-methyl)phenyl]methyl}-3-[(3,5-difluorophenyl)(methylsulphonyl)methylene]azetidine,
1-{(R)-(4-chlorophenyl)[4-(4-methylpiperazin-1-yl-methyl)phenyl]methyl}-3-[(3,5-difluorophenyl)(methylsulphonyl)methylene]azetidine,
1-{(S)-(4-chlorophenyl)[4-(4-methylpiperazin-1-yl-methyl)phenyl]methyl}-3-[(3,5-difluorophenyl)(methylsulphonyl)methylene]azetidine,
1-{(RS)-(4-chlorophenyl)[4-(morpholin-4-yl-methyl)phenyl]methyl}-3-[(3,5-difluorophenyl)-(methylsulphonyl)methylene]azetidine,
1-{(R)-(4-chlorophenyl)[4-(morpholin-4-yl-methyl)phenyl]methyl}-3-[(3,5-difluorophenyl)-(methylsulphonyl)methylene]azetidine,
1-{(S)-(4-chlorophenyl)[4-(morpholin-4-yl-methyl)phenyl]methyl}-3-[(3,5-difluorophenyl)-(methylsulphonyl)methylene]azetidine,
1-{(RS)-(4-chlorophenyl)[4-(diethylaminomethyl)-phenyl]methyl}-3-[(3,5-difluorophenyl)-(methylsulphonyl)methylene]azetidine,
1-{(R)-(4-chlorophenyl)[4-(diethylaminomethyl)-phenyl]methyl}-3-[(3,5-difluorophenyl)-(methylsulphonyl)methylene]azetidine,
1-{(S)-(4-chlorophenyl)[4-(diethylaminomethyl)-phenyl]methyl}-3-[(3,5-difluorophenyl)-(methylsulphonyl)methylene]azetidine,
1-{(RS)-(4-chlorophenyl)[4-(piperazin-2-on-4-yl-methyl)phenyl]methyl}-3-[(3,5-difluorophenyl)(methylsulphonyl)methylene]azetidine,
1-{(R)-(4-chlorophenyl)[4-(piperazin-2-on-4-yl-methyl)phenyl]methyl}-3-[3,5-difluorophenyl)(methylsulphonyl)methylene]azetidine,
1-{(S)-(4-chlorophenyl)[4-(piperazin-2-on-4-yl-methyl}phenyl]methyl}-3-[(3,5-difluorophenyl)(methylsulphonyl)methylene]azetidine,
1-{(RS)-(4-chlorophenyl)[4-(imidazol-1-yl-methyl)phenyl]methyl}-3-[(3,5-difluorophenyl)(methylsulphonyl)methylene]azetidine,
1-{(R)-(4-chlorophenyl)[4-(imidazol-1-yl-methyl)phenyl]methyl}-3-[(3,5-difluorophenyl)(methylsulphonyl)methylene]azetidine,
1-{(S)-(4-chlorophenyl)[4-(imidazol-1-yl-methyl)phenyl]methyl}-3-[(3,5-difluorophenyl)(methylsulphonyl)methylene]azetidine,
(RS)-1-{(4-chlorophenyl)[4-(N,N-dimethylcarbamoyl)-phenyl]methyl}-3-[(3,5-difluorophenyl)(methylsulphonyl)methylene]azetidine,
(R)-1-{(4-chlorophenyl)[4-(N,N-dimethylcarbamoyl)-phenyl]methyl}-3-[(3,5-difluorophenyl)(methylsulphonyl)methylene]azetidine,
(S)-1-{(4-chlorophenyl)[4-(N,N-dimethylcarbamoyl)-phenyl]methyl}-3-[(3,5-difluorophenyl)(methylsulphonyl)methylene]azetidine,
(RS)-1-{(4-chlorophenyl)[4-(N-ethylcarbamoyl)phenyl]-methyl}-3-[(3,5-difluorophenyl)(methylsulphonyl)-methylene]azetidine,
(R)-1-{(4-chlorophenyl)[4-(N-ethylcarbamoyl)phenyl]-methyl}-3-[(3,5-difluorophenyl)(methylsulphonyl)-methylene]azetidine,
(S)-1-{(4-chlorophenyl)[4-(N-ethylcarbamoyl)phenyl]-methyl}-3-[(3,5-difluorophenyl)(methylsulphonyl)-methylene]azetidine,
(RS)-1-[(4-carbamoylphenyl)(4-chlorophenyl)methyl]-3-[(3,5-difluorophenyl)(methylsulphonyl)methylene]-azetidine,
(R)-1-[(4-carbamoylphenyl)(4-chlorophenyl)methyl]-3-[(3,5-difluorophenyl)(methylsulphonyl)methylene]-azetidine,
(S)-1-[(4-carbamoylphenyl)(4-chlorophenyl)methyl]-3-[(3,5-difluorophenyl)(methylsulphonyl)methylene]-azetidine,
1-[bis(4-chlorophenyl)methyl]-3-[(3,5-dichlorophenyl)-(methylsulphonyl)methylene]azetidine,
1-benzhydryl-3-[(3-methylsulphanylphenyl)=
(methylsulphonyl)methylene]azetidine,
1-benzhydryl-3-[(3-methylsulphanylmethyl)phenyl)]-(methylsulphonyl)methylene]azetidine,
1-[bis(4-chlorophenyl)methyl]-3-[(3-cyanophenyl)-(methylsulphonyl)methylene]azetidine,
1-[bis(4-chlorophenyl)methyl]-3-[(3-carbamoylphenyl)-(methylsulphonyl)methylene]azetidine,
1-[bis(4-chlorophenyl)methyl]-3-[(3-methoxyphenyl)-(methylsulphonyl)methylene]azetidine,
1-[bis(4-chlorophenyl)methyl]-3-[(3-hydroxyphenyl)-(methylsulphonyl)methylene]azetidine,
1-[bis(4-chlorophenyl)methyl]-3-[(methylsulphonyl)-(3-pyrrolidinylphenyl)methylene]azetidine,
1-[bis(4-chlorophenyl)methyl]-3-[(3-hydroxymethylphenyl)(methylsulphonyl)methylene]azetidine,
1-[bis(4-chlorophenyl)methyl]3-{(methylsulphonyl)[3-(N-piperidylcarbamoyl)phenyl]methylene}azetidine,
1-[bis(4-chlorophenyl)methyl]-3-[(methylsulphonyl)-(3-trifluoromethylsulphanylphenyl)(methylsulphonyl)-methylene]azetidine,
1-[bis(4-fluorophenyl)methyl]-3-[(3,5-difluorophenyl)-(methylsulphonyl)methylene]azetidine,
1-[bis(2-fluorophenyl)methyl]-3-[3,5-difluorophenyl)-(methylsulphonyl)methylene]azetidine,
1-[bis(3-fluorophenyl)methyl]-3-[(3,5-difluorophenyl)-(methylsulphonyl)methylene]azetidine,
(RS)-1-[(4-chlorophenyl)(thiazol-2-yl)methyl]-3-[(methylsulphonyl)(phenyl)methylene]azetidine,
(R)-1-[(4-chlorophenyl)(thiazol-2-yl)methyl]-3-[(methylsulphonyl)(phenyl)methylene]azetidine,
(S)-1-[(4-chlorophenyl)(thiazol-2-yl)methyl]-3-[(methylsulphonyl)(phenyl)methylene]azetidine,
(RS)-1-[(4-chlorophenyl)(thien-2-yl)methyl]-3-[(3,5-difluorophenyl)(methylsulphonyl)methylene]azetidine,
(R)-1-[(4-chlorophenyl)(thien-2-yl)methyl]-3-[(3,5-difluorophenyl)(methylsulphonyl)methylene]azetidine,
(S)-1-[(4-chlorophenyl)(thien-2-yl)methyl]-3-[(3,5-difluorophenyl) (methylsulphonyl)methylene]azetidine,
1-benzhydryl-3-[(ethylsulphsnyl)(phenyl)methylene]-azetidine,
1-[bis(4-chlorophenyl)methyl]-3-{{3-[N-(4-methylpiperazinyl)carbamoyl]phenyl}(methylsulphonyl)-methylene}azetidine,
1-[bis(4-chlorophenyl)methyl]-3-{[3-(2,2-dimethylcarbohydrazido)phenyl](methylsulphonyl)-methylene}azetidine,
1-[bis(thien-2-yl)methyl]-3-[3,5-difluorophenyl)-(methylsulphonyl)methylene]azetidine,
1-[bis(p-tolyl)methyl]-3-[(methylsulphonyl)(phenyl)-methylene]azetidine,
1-[(4-chlorophenyl)(4-hydroxymethylphenyl)methyl]-3-[(3,5-difluorophenyl)(methylsulphonyl)methylene]-azetidine,
1-[bis(4-chlorophenyl)methyl]-3-[(3-methylaminophenyl)-(methylsulphonyl)methylene]azetidine,
(RS)-1-[(4-chlorophenyl)(thiazol-2-yl)methyl]-3-[(3,5-difluorophenyl)(methylsulphonyl)methylene]azetidine,
(R)-1-[(4-chlorophenyl)(thiazol-2-yl)methyl]-3-[(3,5-difluorophenyl)(methylsulphonyl)methylene]azetidine,
(S)-1-[(4-chlorophenyl)(thiazol-2-yl)methyl]-3-[(3,5-difluorophenyl)(methylsulphonyl)methylene]azetidine,
1-[bis(4-chlorophenyl)methyl]-3-[(methylsulphonyl)(2-methoxycarbonylthien-5-yl)methylene]azetidine,
1-[bis(4-chlorophenyl)methyl]-3-hydroxy-3-[(methylsulphonyl)(2-methoxycarbonylthien-5-yl)methyl]azetidine,
1-[bis(4-chlorophenyl)methyl]-3-[(2-isobutylaminocarbonylthien-5-yl)(methylsulphonyl)-methylene]azetidine,
1-[bis(4-chlorophenyl)methyl]-3-[(3-methoxycarbonylphenyl)(methylsulphonyl)methyl-(RS)]azetidin-3-ol,
1-[bis(4-chlorophenyl)methyl]-3-[(methylsulphonyl)-(pyridin-4-yl)methyl-(RS)]azetidin-3-ol,
1-[bis(4-chlorophenyl)methyl]-3-[(methylsulphonyl)-(pyridin-3-yl)methyl-(RS)]azetidin-3-ol,
3-({1-[bis(4-chlorophenyl)methyl]azetidin-3-ylidene}-methanesulphonylmethyl)-N-(3-morpholin-4-yl-propyl)benzamide,
3-({1-[bis(4-chlorophenyl)methyl]azetidin-3-ylidene}-methanesulphonylmethyl)-N-(3-dimethylaminopropyl)-benzamide,
3-({1-[bis(4-chlorophenyl)methyl]azetidin-3-ylidene}-methanesulphonylmethyl)-N-(2-pyrrolidin-1-ylethyl)-benzamide,
3-({1-[bis(4-chlorophenyl)methyl]azetidin-3-ylidene}-methanesulphonylmethyl)-N-(2-dimethylamino-1-methylethyl)benzamide,
3-({1-[bis(4-chlorophenyl)methyl]azetidin-3-ylidene}-methanesulphonylmethyl)-N-piperidin-1-ylbenzamide,
3-({1-[bis(4-chlorophenyl)methyl]azetidin-3-ylidene}-methanesulphonylmethyl)-N-isobutylbenzamide,
3-({1-[bis(4-chlorophenyl)methyl]azetidin-3-ylidene}-methanesulphonylmethyl)-N-(3-imidazol-1-ylpropyl)-benzamide,
3-({1-[bis(4-chlorophenyl)methyl]azetidin-3-ylidene}-methanesulphonylmethyl)-N-(2-dimethylaminoethyl)-benzamide,
3-({1-[bis-(4-chlorophenyl)methyl]azetidin-3-ylidene}methanesulphonylmethyl)benzoic acid N'-methylhydrazide,
3-({1-[bis-(4-chlorophenyl)methyl]azetidin-3-ylidene}-methanesulphonylmethyl)-N- (2-morpholin-4-ylethyl)-benzamide,
3-({1-[bis-(4-chlorophenyl)methyl]azetidin-3-ylidene}-methanesulphonylmethyl)-N-(1-ethylpyrrolidin-2-ylmethyl)benzamide,
3-({1-[bis-(4-chlorophenyl)methyl]azetidin-3-ylidene}-methanesulphonylmethyl)-N-(2,2-dimethylpropyl)benzamide,
3-({1-[bis-(4-chlorophenyl)methyl]azetidin-3-ylidene}-methanesulphonylmethyl)-N-cyclohexylmethylbenzamide,
3-({1-[bis-(4-chlorophenyl)methyl]azetidin-3-ylidene}-methanesulphonylmethyl) -N-cyclopropylmethylbenzamide,
3-({1-[bis-(4-chlorophenyl)methyl]azetidin-3-ylidene}-methanesulphonylmethyl)-N-(2-methylbutyl)benzamide,
3-({1-[bis-(4-chlorophenyl)methyl]azetidin-3-ylidene}-methanesulphonylmethyl)-N-(2-phenylpropyl)benzamide,
3-({1-[bis-(4-chlorophenyl)methyl]azetidin-3-ylidene}-methanesulphonylmethyl) -N- (tetrahydrofuran-2-ylmethyl)benzamide,
3-({1-[bis-(4-chlorophenyl)methyl]azetidin-3-ylidene}-methanesulphonylmethyl)-N-(2,2-diphenylethyl)benzamide,
3-({1-[bis-(4-chlorophenyl)methyl]azetidin-3-ylidene}-methanesulphonylmethyl)-N- (2-ethylbutyl)benzamide,
4-{[3-({1-[bis(4-chlorophenyl)methyl]azetidin-3-ylidene}methanesulphonylmethyl)benzoylamino]methyl}cyclohexanecarboxylic acid methyl ester,
2-amino-1-{4-[3-({1-[bis-(4-chlorophenyl)methyl]-azetidin-3-ylidene}methanesulphonylmethyl)phenyl]piperazin-1-yl}ethanone,
(2-{4-[3-({1-[bis(4-chlorophenyl)methyl]azetidin-3-ylidene}methanesulphonylmethyl)phenyl]piperazin-2-yl}-2-oxoethyl)carbamic acid tert-butyl ester,
1-{4-[3-({1-[bis(4-chlorophenyl)methyl]azetidin-3-ylidene}methanesulphonylmethyl)phenyl]piperazin-1-yl}-2-methylaminoethanone,
(2-{4-[3-({1-[bis(4-chlorophenyl)methyl]azetidin-3-ylidene}methanesulphonylmethyl)phenyl]piperazin-1-yl}-2-oxoethyl)-N-methylcarbamic acid tert-butyl ester,
4-[3-({1-[bis(4-chlorophenyl)methyl]azetidin-3-ylidene}methanesulphonylmethyl)phenyl]piperazine-1-carbothioic acid N-methylamide,
4-[3-({1-[bis(4-chlorophenyl)methyl]azetidin-3-ylidene}methanesulphonylmethyl)phenyl]piperazine-1-carboxylic acid N-methylamide,
4-[3-({1-[bis(4-chlorophenyl)methyl]azetidin-3-ylidene}methanesulphonylmethyl)phenyl]piperazine-1-carboxylic acid methyl ester,
1-[3-({1-[bis(4-chlorophenyl)methyl]azetidin-3-ylidene}methanesulphonylmethyl)phenyl]-4-isobutylpiperazine,
1-[3-({1-[bis(4-chlorophenyl)methyl]azetidin-3-ylidene}methanesulphonylmethyl)phenyl]-4-ethylpiperazine,
4-acetyl 1-[3-({1-[bis(4-chlorophenyl)methyl]azetidin-3-ylidene}methanesulphonylmethyl)phenyl]piperazine,
1-{4-[3-({1-[bis(4-chlorophenyl)methyl]azetidin-3-ylidene}methanesulphonylmethyl)phenyl]piperazin-1-yl}-2-dimethylaminoethanone,
1-[3-({1-[bis(4-chlorophenyl)methyl]azetidin-3-ylidene}methanesulphonylmethyl)phenyl]piperazine,
4-[3-({1-[bis(4-chlorophenyl)methyl]azetidin-3-ylidene}methanesulphonylmethyl)phenyl]piperazine-1-carboxylic acid tert-butyl ester,
1-[bis(4-methoxycarbonylphenyl)methyl]-3-[(3,5-difluorophenyl)(methylsulphonyl)methylene]azetidine,
3-acetoxy-1-[bis(4-methoxycarbonylphenyl)methyl]-3-[(3,5-difluorophenyl)(methylsulphonyl)methyl-(RS))azetidine,
(RS)-4-[4-((4-chlorophenyl){3-[(3,5-difluorophenyl)-methanesulphonylmethylene]azetidin-1-yl}methyl)-benzyl]morpholine,
4-(4-{3-[(1-benzhydrylazetidin-3-ylidene)methanesulphonylmethyl]phenoxy}butyl)morpholine,
4-(4-{3-[(1-benzhydrylazetidin-3-ylidene)methanesulphonylmethyl]phenoxy}propyl)morpholine,
their optical isomers and their salts with an inorganic or organic acid.

10. The following compounds according to Claim 1:
1-[bis(4-chlorophenyl)methyl]-3-[(3,5-difluorophenyl)(methylsulphonyl)methylene]azetidine,
1-[bis(4-chlorophenyl)methyl]-3-[(3,5-difluorophenyl)(methylsulphonyl)methyl-(RS)]azetidin-3-ol,
3-acetoxy-1-[bis-(4-chlorophenyl)methyl]-3-[(3,5-difluorophenyl)(methylsulphonyl)methyl)methylsulphonylmethyl-(RS)]azetidine
their optical isomers and their salts with an inorganic or organic acid.

11. Process for the preparation of the compounds of formula (I) according to Claim 1 for which R represents a chain of formula (A), **characterized in that** a corresponding compound of formula (Ia): 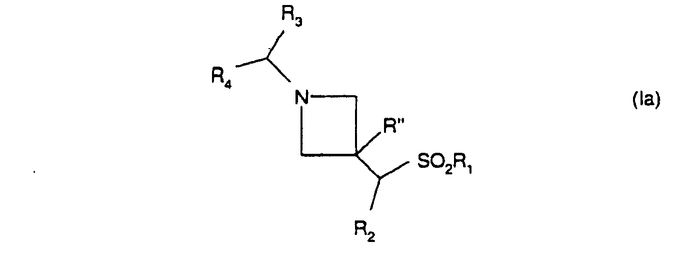 in which R₁, R₂, R₃ and R₄ have the same meanings as in Claim 1 and R" represents a hydroxyl, methanesulphonyloxy or acetyloxy radical, is dehydrated, the product isolated and optionally converted to a salt with an inorganic or organic acid.

12. Process for the preparation of the compounds of formula (I) according to Claim 1 for which R represents a chain (B) in which R' is a hydrogen atom, **characterized in that** a derivative R₁SO₂CH₂R₂ (II) for which R₁ and R₂ have the same meanings as in Claim 1 is reacted with an azetidinone of formula: 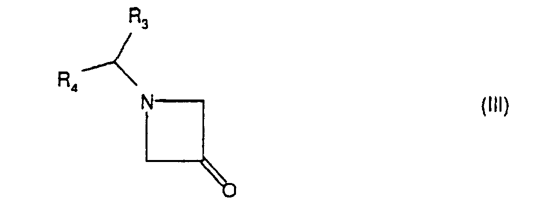 in which R₃ and R₄ have the same meanings as in Claim 1, the product isolated and optionally converted to a salt with an inorganic or organic acid.

13. Process for the preparation of the compounds of formula (I) according to Claim 1 for which R represents a chain (B) in which R' is a hydrogen atom, **characterized in that** a derivative R₃CH(Br)R₄ for which R₃ and R₄ have the same meanings as in Claim 1 is reacted with a derivative of formula: 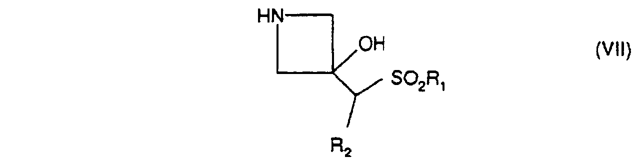 in which R₁ and R₂ have the same meanings as in Claim 1, the product isolated and optionally converted to a salt with an inorganic or organic acid.

14. Process for the preparation of the compounds of formula (I) according to Claim 1 for which R is a chain (B) in which R' is a -CO-alk radical, **characterized in that** a halide Hal-CO-alk in which Hal represents a halogen atom and alk represents a straight- or branched-chain alkyl radical containing 1 to 6 carbon atoms is reacted with a corresponding compound of formula (I) for which R is a chain (B) in which R' is a hydrogen atom, the product isolated and optionally converted to a salt with an inorganic or organic acid.

15. Process for the preparation of the compounds of formula (I) according to Claim 1 for which R₂ represents an aromatic or a heteroaromatic substituted with -NR₆R₇, in which R₆ and R₇ each represent a hydrogen atom, **characterized in that** a corresponding compound of formula (I) for which R₂ represents an aromatic or a heteroaromatic substituted with nitro is reduced, the product isolated and optionally converted to a salt with an inorganic or organic acid.

16. Process for the preparation of the compounds of formula (I) according to Claim 1 for which R₂ represents an aromatic or heteroaromatic substituted with -CONHR₉ and/or R₃ and/or R₄ represent an aromatic or a heteroaromatic. substituted with -CONR₁₀R₁₁, **characterized in that** a corresponding compound of formula (I) for which R₂ and/or R₃ and/or R₄ represent an aromatic or a heteroaromatic substituted with -COOR₅ for which R₅ is alkyl or phenyl optionally substituted with halogens is reacted respectively with an amine H₂NR₉ or HNR₁₀R₁₁ for which R₉, R₁₀ and R₁₁ have the same meanings as in Claim 1, the product isolated and optionally converted to a salt with an inorganic or organic acid.

17. Process for the preparation of the compounds of formula (I) according to Claim 1 for which R₂ represents an aromatic substituted by hydroxyl and/or R₃ and/or R₄ represent an aromatic or a heteroaromatic substituted with hydroxyl, **characterized in that** a corresponding compound of formula (I) for which R₂ represents an aromatic substituted by alkoxy and/or R₃ and/or R₄ represent an aromatic or a heteroaromatic substituted with alkoxy is hydrolysed, the product isolated and optionally converted to a salt with an inorganic or organic acid.

18. Process for the preparation of the compounds of formula (I) according to Claim 1 for which R₂ represents an aromatic substituted with -NR₆R₇ for which R₆ represents an alkyl radical and R₇ represents a hydrogen atom, **characterized in that** a corresponding compound of formula (I) for which R₂ represents an aromatic substituted with an -N(alk)COOR₈ in which R₈ represents a tert-butyl radical is deprotected, the product isolated and optionally converted to a salt with an inorganic or organic acid.

19. Process for the preparation of the compounds of formula (I) according to Claim 1 for which R₂ and/or R₃ and/or R₄ represent an aromatic or a heteroaromatic substituted with -COOR₅, **characterized in that** a derivative of formula: 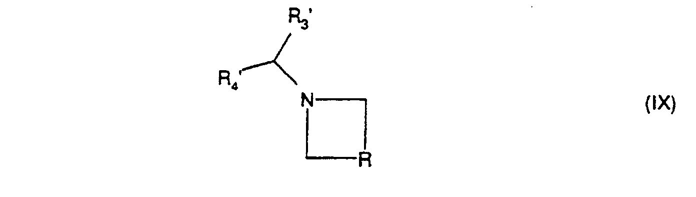 for which R represents a chain C=C(SO₂R₁)R'₂ or C(OR')CH(SO₂R₁)R'₂, R₁, R'₂, R'₃ and R'₄ have the same meanings as the substituents R₁, R₂, R₃ and R₄ of Claim 1 with the proviso that at least one of the substituents R'₂, R'₃ and R'₄ represents an aromatic or a heteroaromatic substituted with carboxyl, is esterified using a derivative of formula R₅OH for which R₅ is alkyl or phenyl optionally substituted with one or more halogen, the product isolated and optionally converted to a salt with an inorganic or organic acid.

20. Process for the preparation of the compounds of formula (I) according to Claim 1 for which R₂ and/or R₃ and/or R₄ represent an aromatic or a heteroaromatic substituted with alkylthioalkyl, **characterized in that** a derivative of formula: 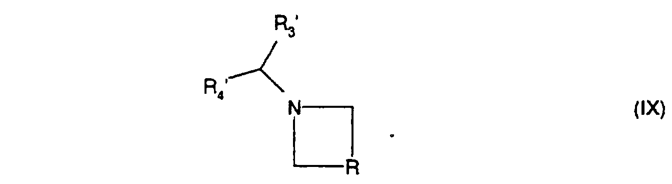 for which R represents a chain C=C(SO₂R₁)R'₂ or C(OR')CH(SO₂R₁)R'₂, R', R₁, R'₂, R'₃ and R'₄ have the same meanings as the substituents R', R₁, R₂, R₃ and R₄ of Claim 1 with the proviso that at least one of the substituents R₂', R₃' and R₄' represents an aromatic or a heteroaromatic substituted with haloalkyl, is reacted with a sodium alkylthiolate for which the alkyl portion is a straight or branched chain and contains 1 to 6 carbon atoms, the product isolated and optionally converted to a salt with an inorganic or organic acid.

21. Process for the preparation of the compounds of formula (I) according to Claim 1 for which R₂ and/or R₃ and/or R₄ represent an aromatic substituted with hydroxyalkyl in which the alkyl contains a carbon atom, **characterized in that** a compound of formula (I) for which at least one of the substituents R₂, R₃ and R₄ represents an aromatic substituted with formyl, is reduced, the product isolated and optionally converted to a salt with an inorganic or organic acid.

22. Process for the preparation of the compounds of formula (I) according to Claim 1 for which R₃ and/or R₄ represents an aromatic substituted with -alk-NR₆R₇ for which alk is an alkyl containing a carbon atom, **characterized in that** a compound of formula (I) for which at least one of the substituents R₃ and R₄ represents an aromatic substituted with formyl is reacted with an amine HNR₆R₇ in which R₆ and R₇ have the same meanings as in formula (I), the product isolated and optionally converted to a salt with an inorganic or organic acid.

23. Process for the preparation of the compounds of formula (I) according to Claim 1 for which R₂ represents an aromatic or a heteroaromatic substituted with -CONHR₉ and/or R₃ and/or R₄ represents an aromatic or a heteroaromatic substituted with -CO-NR₁₀R₁₁, **characterized in that** a derivative of formula: 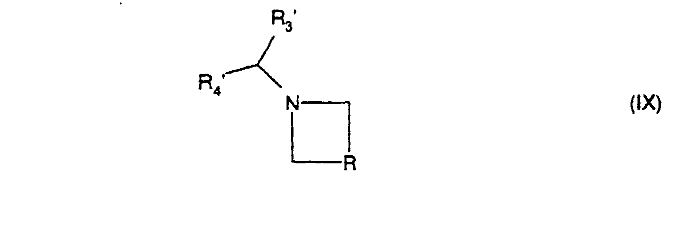 for which R represents a chain C=C(SO₂R₁)R'₂ or C(OR')CH(SO₂R₁)R'₂, R', R₁, R'₂, R'₃ and R'₄ have the same meanings as the substituents R', R₁, R₂, R₃ and R₄ of Claim 1 with the proviso that at least one of the substituents R'₂, R'₃ and R'₄ represents an aromatic or a heteroaromatic substituted with carboxyl, is reacted respectively with an amine H₂NR₉ or HNR₁₀R₁₁ in which R₉, R₁₀ and R₁₁ have the same meanings as in formula (I), the product isolated and optionally converted to a salt with an inorganic or organic acid.

24. Process for the preparation of the compounds of formula (I) according to Claim 1 for which R₂ and/or R₃ and/or R₄ represent an aromatic or. a heteroaromatic substituted with -CO-NH-NR₆R₇, **characterized in that** a corresponding compound of formula (I) for which R₂ and/or R₃ and/or R₄ represent an aromatic or a heteroaromatic substituted with -COOR₅ and R₅ represents an alkyl or phenyl radical which is optionally substituted by halogens, is reacted with a hydrazine H₂N-NR₆R₇ for which R₆ and R₇ have the same meanings as in formula (I), the product isolated and optionally converted to a salt with an inorganic or organic acid.

25. Process for the preparation of the compounds of formula (I) according to Claim 1 for which R₂ represents an aromatic or a heteroaromatic substituted with -CO-NHR₉ in which R₉ represents a hydrogen atom and/or R₃ and/or R₄ represent an aromatic or a heteroaromatic substituted with -CO-NR₁₀R₁₁ in which R₁₀ and R₁₁ are hydrogen atoms, **characterized in that** a corresponding compound of formula (I) for which R₂ and/or R₃ and/or R₄ represent an aromatic or a heteroaromatic substituted with cyano is hydrolysed, the product isolated and optionally converted to a salt with an inorganic or organic acid.

26. Process for the preparation of the compounds of formula (I) according to Claim 1 for which R₂ represents an aromatic substituted with -O-alk-NR₁₂R₁₃, **characterized in that** a derivative of formula: 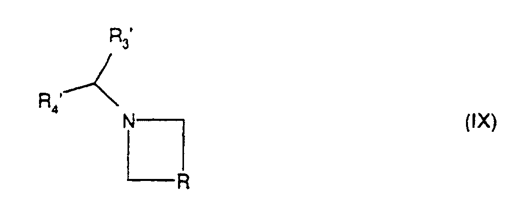 for which R represents a chain C=C(SO₂R₁)R'₂ or C(OR')CH(SO₂R₁)R'₂, R', R₁, R₂', R₃' and R₄' have the same meanings as the substituents R', R₁, R₂, R₃ and R₄ of Claim 1 with the proviso that at least one of the substituents R₂', R₃', R₄' represents an aromatic substituted with -O-alk-Hal in which alk represents a straight- or branched-chain alkyl radical containing 1 to 6 carbon atoms and Hal represents a halogen atom, is reacted with an amine HNR₁₂R₁₃ in which R₁₂, R₁₃ have the same meanings as in Claim 1, the product isolated and optionally converted to a salt with an inorganic or organic acid.

27. Process for the preparation of the compounds of the formula (I) according to Claim 1, for which R₃ and/or R₄ represents an aromatic substituted with -alk-NR₆R₇, **characterized in that** a derivative of formula: 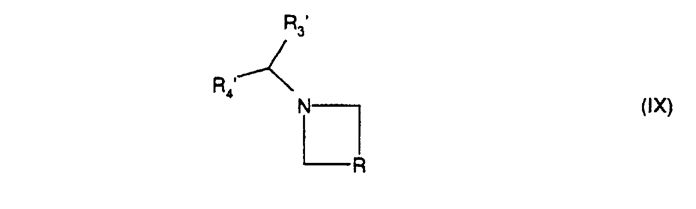 for which R represents a chain C=C(SO₂R₁)R'₂ or C(OR')CH(SO₂R₁)R'₂, R', R₁, R₂', R₃' and R₄' have the same meanings as the substituents R', R₁, R₂, R₃ and R₄ of Claim 1 with the proviso that at least one of the substituents R₃', R₄' represents an aromatic substituted with -alk-Cl in which alk represents a straight- or branched-chain alkyl radical containing 1 to 6 carbon atoms, is reacted with an amine HNR₆R₇ in which R₆, R₇ have the same meanings as in Claim 1, the product isolated and optionally converted to a salt with an inorganic or organic acid.

28. Process for the preparation of the compounds of formula (I) according to Claim 1 for which R represents a chain B, R' represents a hydrogen atom and R₃ and/or R₄ represents an aromatic substituted with hydroxyalkyl in which the alkyl residue contains one carbon atom, **characterized in that** diisobutylaluminium hydride is reacted with a corresponding compound of formula (I) for which R represents a chain B, R' represents a hydrogen atom and R₃ and/or R₄ represents an aromatic substituted with one or more -COOR₅ radicals, in which R₅ is an alkyl radical, the product isolated and optionally converted to a salt with an inorganic or organic acid.

29. Process for the preparation of the compounds of formula (I) according to Claim 1 for which R₂ represents a phenyl radical substituted with -NR₆R₇ representing a 1-piperazinyl ring substituted at the 4 position with an alkyl radical, **characterized in that** a corresponding compound of formula (I) for which R₂ represents a phenyl radical substituted with a radical -NR₆R₇ representing a 1-piperazinyl ring is reacted with an alk-CHO derivative in which alk represents a straight- or branched-chain alkyl radical containing 1 to 5 carbon atoms, the product isolated and optionally converted to a salt with an inorganic or organic acid.

30. Process for the preparation of the compounds of formula (I) according to Claim 1 for which R₂ represents a phenyl radical substituted with -NR₆R₇ representing a 1-piperazinyl ring substituted at the 4 position with a -COOalk radical, **characterized in that** a corresponding compound of formula (I) for which R₂ represents a phenyl radical substituted with a radical -NR₆R₇ representing a 1-piperazinyl ring is reacted with a derivative of formula Hal-COOalk in which alk represents a straight- or branched-chain alkyl radical containing 1 to 6 carbon atoms and Hal represents a halogen atom, the product isolated and optionally converted to a salt with an inorganic or organic acid.

31. Process for the preparation of the compounds of formula (I) according to Claim 1 for which R₂ represents a phenyl radical substituted with -NR₆R₇ representing a 1-piperazinyl ring substituted at the 4 position with a -CO-NHalk or -CS-NHalk radical, **characterized in that** a corresponding compound of formula (I) for which R₂ represents a phenyl radical substituted with -NR₆R₇ representing a 1-piperazinyl ring is reacted with a derivative of formula Y=C=Nalk in which alk represents a straight- or branched-chain alkyl radical containing 1 to 6 carbon atoms and Y represents a sulphur or oxygen atom, the product isolated and optionally converted to a salt with an inorganic or organic acid.

32. Process for the preparation of the compounds of formula (I) according to Claim 1 for which R₂ represents a phenyl radical substituted with a radical -NR₆R₇ representing a 1-piperazinyl ring substituted at the 4 position with a -CO-alk-NR₁₄R₁₅ radical, **characterized in that** a corresponding compound of formula (I) for which R₂ represents a phenyl radical substituted with a radical -NR₆R₇ representing a 1-piperazinyl ring is reacted with an acid of formula R₁₅R₁₄N-alk-COOH in which alk represents a straight- or branched-chain alkyl radical containing 1 to 6 carbon atoms and R₁₄ and R₁₅ have the same meanings as in Claim 1 optionally followed by deprotection of the product for which R₁₄ is a tert-butoxycarbonyl radical in order to obtain the compounds for which R₁₄ is a hydrogen atom, the product isolated and optionally converted to a salt with an inorganic or organic acid.

33. Process for the preparation of the compounds of formula (I) according to Claim 1 for which R₂ represents a phenyl radical substituted with a radical -NR₆R₇ representing a 1-piperazinyl ring substituted at the 4 position with a -CO-alk radical in which alk represents a methyl radical, **characterized in that** a corresponding compound of formula (I) for which R₂ represents a phenyl radical substituted with a radical -NR₆R₇ representing a 1-piperazinyl ring is reacted with acetic anhydride, the product isolated and optionally converted to a salt with an inorganic or organic acid.

34. Pharmaceutical compositions containing, as active ingredient, at least one compound of formula (I) according to one of Claims 1 to 10.

35. Compounds of formula (I) according to one of Claims 1 to 10, as medicaments.
